# EUROPEAN PATENT APPLICATION

(11) **EP 4 847 047 A1**
(43) Date of publication of application: **02.12.2026**
(21) Application number: 25744726.8
(22) Date of filing: 24.01.2025
(51) Int. Cl.: A61K 47/68, A61P 31/16

(54) **CONJUGATE, PREPARATION METHOD THEREFOR, AND USE THEREOF**

(30) Priority: 26.01.2024 CN 202410111150
(71) Applicant: ZHEJIANG YANGSHENGTANG INSTITUTE OF NATURAL MEDICATION CO., LTD., Hangzhou, Zhejiang 310024 (CN)
(72) Inventor: ZHOU, Xinbo, Beijing 100850 (CN); XIAO, Dian, Beijing 100850 (CN); FENG, Xun, Beijing 100850 (CN); LI, Song, Beijing 100850 (CN); ZHONG, Wu, Beijing 100850 (CN); XIE, Fei, Beijing 100850 (CN); LIU, Lianqi, Beijing 100850 (CN); LI, Xingzhou, Beijing 100850 (CN); CAO, Ruiyuan, Beijing 100850 (CN); XIAO, Junhai, Beijing 100850 (CN); ZHENG, Zhibing, Beijing 100850 (CN); FAN, Shiyong, Beijing 100850 (CN); LI, Wei, Beijing 100850 (CN); LI, Pengyun, Beijing 100850 (CN); WANG, Zihao, Beijing 100850 (CN); YAN, Linjie, Beijing 100850 (CN); HU, Liu, Hangzhou, Zhejiang 310024 (CN); JIANG, Jingqian, Hangzhou, Zhejiang 310024 (CN); ZHAO, Jinghua, Hangzhou, Zhejiang 310024 (CN); XU, Nuo, Hangzhou, Zhejiang 310024 (CN); CHEN, Guanfeng, Hangzhou, Zhejiang 310024 (CN); LIANG, Xiao, Hangzhou, Zhejiang 310024 (CN)
(74) Representative: J A Kemp LLP
(86) International application number: PCT/CN2025/074660
(87) International publication number: WO 2025/157263

(57) **Abstract**

The present invention relates to a conjugate for inhibiting viral replication, a preparation method therefor, and use thereof. The conjugate comprises a dimer or trimer of a portion that inhibits influenza virus neuraminidase (e.g., zanamivir, peramivir, oseltamivir, or an analog thereof), the dimer or trimer being conjugated to an Fc domain monomer, an Fc domain, an Fc-binding peptide, an albumin, or an albumin-binding peptide. The conjugate can be used to treat viral infections (e.g., influenza virus infections).

## Description

### Cross-Reference

The present application is based on and claims the benefit of priority from Chinese Application No. 202410111150.3, filed on January 26, 2024, the disclosures of which are incorporated herein by reference in its entirety.

### Technical Field

The application relates to a conjugate for inhibiting viral replication, a method for preparing the same, and use thereof.

### Background Art

In the medical field, new and highly effective anti-influenza virus therapeutic drugs and strategies are constantly being sought. Influenza viruses (pathogens of influenza) cause three to five million cases of serious illness per year, and approximately 500,000 deaths worldwide. Although the majority of individuals may will fully recover on their own within one to two weeks, some people will develop life-threatening complications such as pneumonia. Thus, influenza can be fatal for certain vulnerable individuals, particularly children, the elderly, patients suffering from chronic diseases, and people with weak or compromised immune systems. For example, patients with advanced HIV infection or organ transplant recipients (whose immune systems are medically suppressed to prevent rejection of transplant organs) are at a great risk of complications associated with influenza. Pregnant women and young children are also at a high risk of developing such complications.

The development of therapeutic drugs for anti-influenza virus has become an ongoing challenge. Several antiviral agents against influenza virus have been approved for clinical use. However, long-term administration has led to the emergence of drug-resistant strains to the most commonly used influenza inhibitors.

Therapeutic drugs for anti-influenza virus primarily target proteins on the surface of influenza virions. The envelope of the influenza virus contains two key glycoproteins, namely hemagglutinin (HA) and neuraminidase (NA), which play key roles in viral infection and spread. The neuraminidase, as an exoglycosidase, cleaves sialic acid from a glycan structure on the surface of infected host cells, thereby enabling the release of progeny virions and their dissemination to adjacent uninfected cells. Accordingly, the neuraminidase has become a pharmacological target of antiviral drugs. Multiple small-molecule neuraminidase inhibitors for inhibiting viral dissemination have been commercially available, including oseltamivir (tamiflu^{™}), zanamivir (Relenza^{™}), and zeramivir (Rapivab^{™}).

Targeted delivery conjugate drugs are an emerging therapeutic approach, whose basic principle lie in conjugating therapeutic agents to targeting molecules, enabling the drugs to selectively bind to disease-associated cells or tissues. In cancer therapy, encouraging outcomes have been achieved with the targeted delivery conjugate drugs. For example, several antibody-drug conjugates have been successfully used to treat diseases such as breast cancer, colon cancer, and lymphoma. Currently, macromolecule conjugation technology has also been applied to develop novel antiviral drugs. As an example, Cidara Therapeutics, Inc. developed a human IgG Fc protein-conjugated dimer dimeric neuraminidase (NA) inhibitor (CN 113194983 A, WO 2021/046549 A1), which has exhibited preliminary therapeutic potential against influenza.

However, since influenza viruses undergo frequent antigenic drift and antigenic shift, new subtypes are continuously generated, thereby increasing the likelihood of drug-resistant strains emergence. and there is a demand to develop a new and more efficient therapy for treating influenza.

### Summary of the Invention

The application relates to a conjugate for inhibiting viral replication, a method for preparing the same, and use thereof. In particular, the conjugates conjugate neuraminidase inhibitors against influenza viruses (such as zanamivir, peramivir, oseltamivir, or analogues thereof) to biomacromolecules such as antibodies, antibody fragments, or albumin. The neuraminidase inhibitor in the conjugates targets the neuraminidase on the surface of virion. The Fc region or Fc protein of the antibody in the conjugates binds to FcγRs (e.g., FcRn, FcγRI, FcγRIIa, FcγRIIc, FcγRIIIa, and FcγRIIIb) on immune cells (e.g., neutrophils), activating the phagocytic function of immune cells, such as antibody-dependent cell-mediated cytotoxicity (ADCC), thereby prompting the immune cells to phagocytose and destroy virions, and further enhancing the antiviral activity of the conjugates. The albumin may prolong the half-life of the conjugates, and for example, the albumin binds to FcRn to slow metabolic clearance of the conjugates.

Specifically, the conjugate comprises a dimer or trimer of a moieties that inhibit influenza virus neuraminidase (for example, zanamivir, peramivir, oseltamivir, or an analog thereof), which are conjugated to an Fc domain monomer, an Fc domain, an Fc-binding peptide, an albumin, or an albumin-binding peptide. The neuraminidase inhibitor in the conjugate targets the neuraminidase on the surface of virions. The Fc monomer or Fc domain in the conjugate binds to FcγR (e.g., FcRn, FcγRI, FcγRIIa, FcγRIIc, FcγRIIIa, and FcγRII1b) on immune cells (e.g., neutrophils) to activate phagocytosis and effector functions, such as antibody-dependent cell-mediated cytotoxicity (ADCC), thereby prompting the immune cells to phagocytose and destroy virions, and further enhancing the antiviral activity of the conjugate. The albumin or albumin-binding peptide can prolong the half-life of the conjugate via binding to the recycling neonatal Fc receptor (FcRn). The conjugate can be used to inhibit viral growth and treat viral infections (such as those infections caused by influenza A viruses, influenza B viruses, and influenza C viruses).

In one aspect, the present application provides a conjugate represented by Formula (I) or a pharmaceutically acceptable salt, a geometric or optical isomer, a hydrate, a solvate or a polymorph thereof, 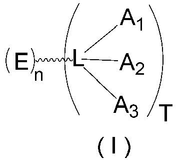
wherein A₁, A₂ and A₃ each independently are selected from the group consisting of Formula (A-I) to Formula (A-XIV), 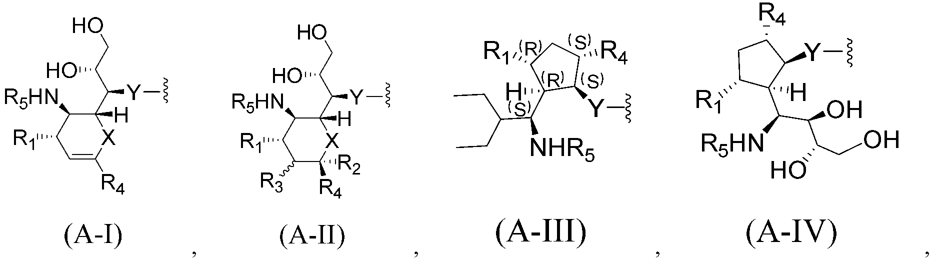 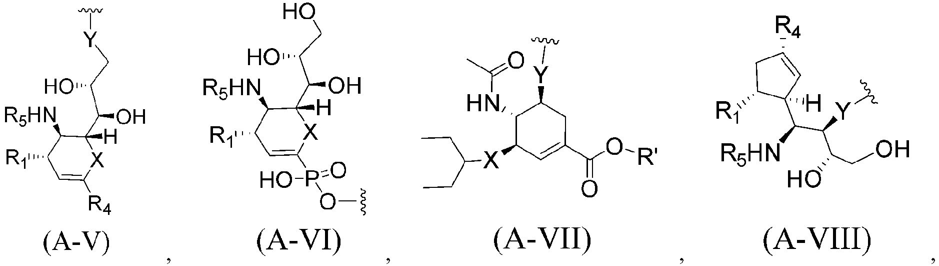 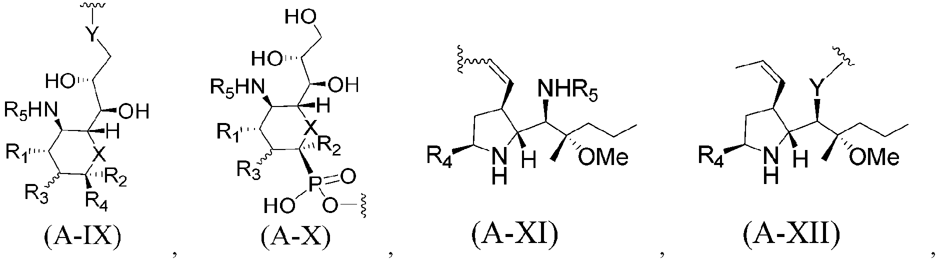 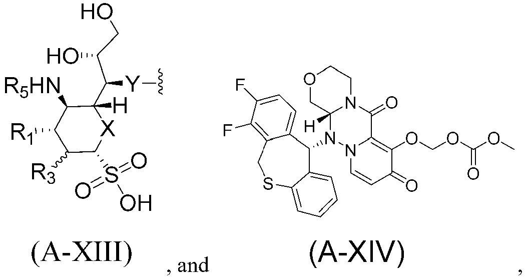
wherein, L may be linked to any possible sites of the compound of Formula (A-XIV);
wherein R₁ is selected from the group consisting of -OH, -NH₂, -NHC(=NH)NH₂ and -NHC(=NH)NHR₆;
R₂ and R₃ each independently are selected from the group consisting of -H, -OH, -F, -Cl and -Br;
R₄ is selected from the group consisting of -C(=O)OH, -P(=O)(OH)₂, -SO₃H, -C(=O)OCH₃ and -C(=O)OCF₃;
R₅ is selected from the group consisting of -C(=O)CH₃, -C(=O)CF₃, and -SO₂CH₃;
X is selected from the group consisting of -O- and -S-;
Y is selected from the group consisting of -O-, -S-, -NR₇-, -OC(=O)NR₇-, -OC(=S)NR₇-, -OC(=O)O-, -OC(=O)-, -NHC(=O)O-, -NHC(=O)-, -NHC(=NH)-, -NHC(=O)NR₇-, -NHC(=NH)NR₇-, -NHC(=S)NR₇-, -NHC(=S)-, -OCH₂C(=O)NR₇-, -NH(SO₂)- and -NH(SO₂)NR₇-;
R' is H or C₁-C₆ alkyl;
R₆ is selected from the group consisting of: 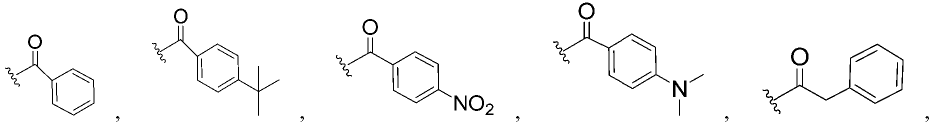 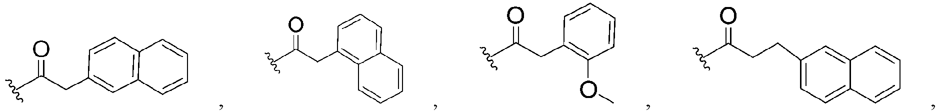 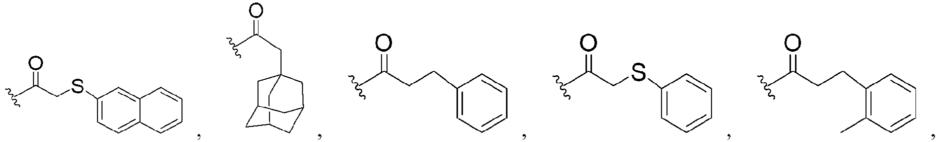 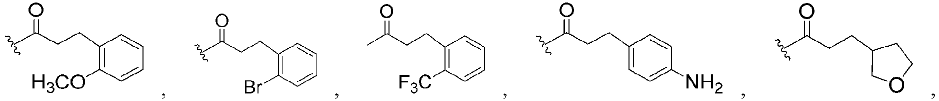 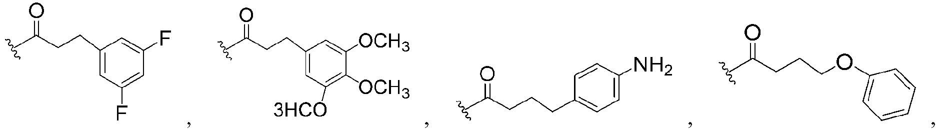 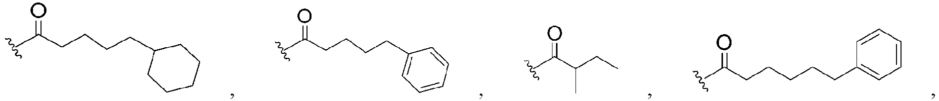 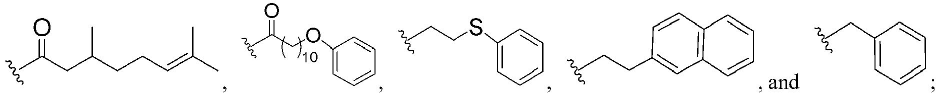
R₇ is selected from the group consisting of H, C₁-C₂₀ alkyl, C₃-C₂₀ cycloalkyl, C₃-C₂₀ heterocycloalkyl, C₅-C₁₅ aryl and C₅-C₁₅ heteroaryl; preferably, R₇ is H; preferably, R₇ is C₁-C₆ alkyl;
each E is a biomacromolecule; n is 1 or 2; T is a number between 1 and 20;
the wavy line linked to each E represents a covalent linking (e.g., via a covalent bond or a linker) of each 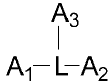 to an N atom in the each E (e.g., an N atom on the side chain amino group of a lysine, arginine, asparagine, or glutamine residue), and L is a linker covalently linked to each of groups E, A1, A2, and A3.

In some embodiments, in the conjugate represented by Formula (I), A₁, A₂ and A₃ each independently are selected from the group consisting of Formula (A-I) to Formula (A-XIII), 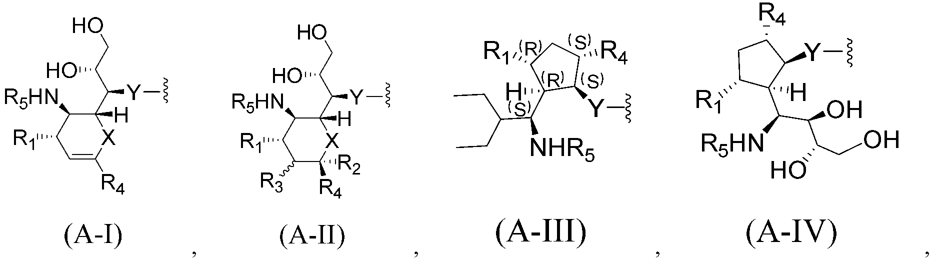 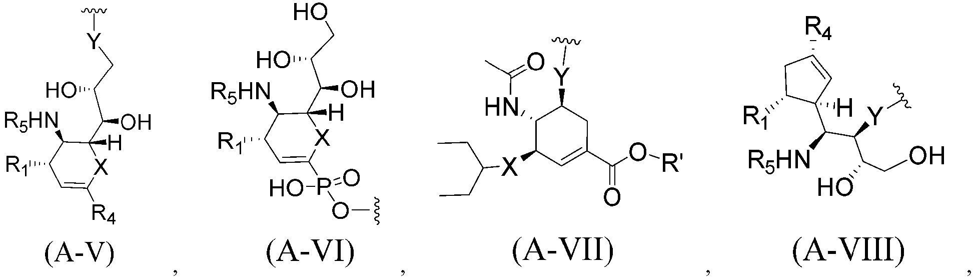 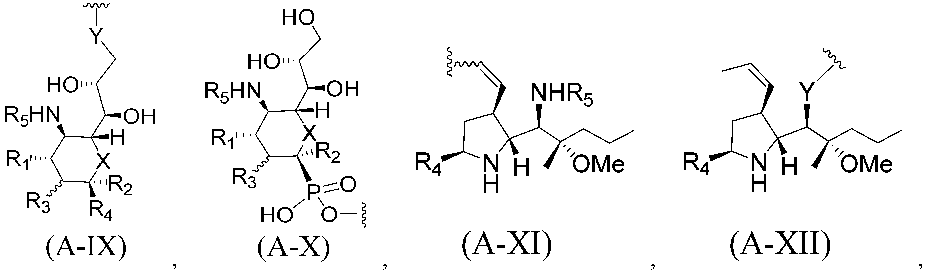 and 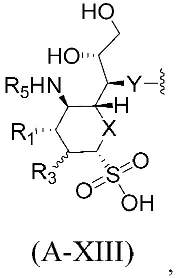 wherein R₁, R₂, R₃, R₄, R₅, R', X, and Y are as defined in any embodiment of the present application.

In some embodiments, in the conjugate represented by Formula (I), A₁, A₂ and A₃ each independently are selected from the group consisting of Formula (A-I) to Formula (A-IV) and Formula (A-VII), 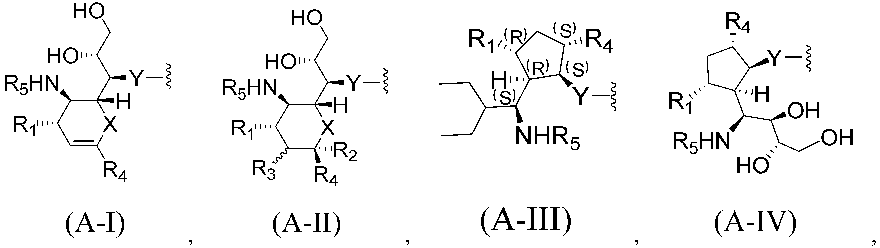 and 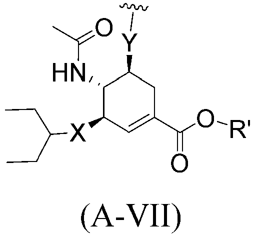 wherein R₁, R₂, R₃, R₄, R₅, R', X, and Y are as defined in any embodiment of the present application.

In some embodiments, in the conjugate represented by Formula (I), each E comprises an Fc domain monomer (e.g., an Fc domain monomer having a sequence as set forth in any one of SEQ ID NO: 1-68), albumin (e.g., albumin having a sequence as set forth in any one of SEQ ID NO:69-71), and an albumin-binding peptide, or an Fc-binding peptide; n is 1 or 2, wherein when n is 2 and E is an Fc domain monomer, the two E dimerize to form an Fc domain.

In some embodiments, in the conjugate represented by Formula (I), A₁, A₂ and A₃ each independently are selected from the group consisting of Formula (A-I) to Formula (A-III) and Formula (A-VII), 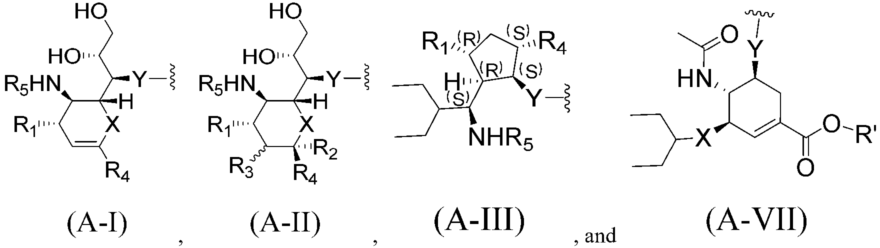 wherein R₁, R₂, R₃, R₄, R₅, R', X, and Y are as defined in any embodiment of the present application.

In some embodiments, in the conjugate represented by Formula (I), A₁, A₂ and A₃ each independently are selected from the group consisting of Formula (A-I), Formula (A-III) and Formula (A-VII), 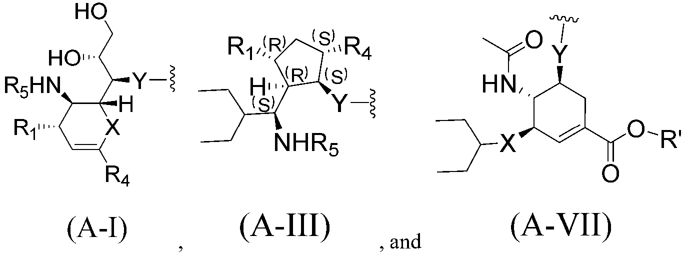 wherein R₁, R₄, R₅, R', X, and Y are as defined in any embodiment of the present application.

In some embodiments, in the conjugate represented by Formula (I), E comprises an Fc domain monomer (e.g., an Fc domain monomer having a sequence as set forth in any one of SEQ ID NO:1-68); n is 1 or 2, wherein when n is 2, the two E dimerize to form an Fc domain.

In some embodiments, in the conjugate represented by Formula (I), E is an Fc domain monomer, the Fc domain monomer having an amino acid sequence selected from the group consisting of:
i) any one of the sequences as set forth in SEQ ID NO:1-68;
ii) a sequence with substitution, deletion or addition of one or several amino acids (for example, substitution, deletion or addition of one, two, three, four or five amino acids) as compared with any one of the sequences as set forth in SEQ ID NO: 1-68; and
iii) a sequence with a sequence identity of at least 70% (such as 70%, 75%, 80%, 85%, 90%, 95%, 96%, 97%, 98%, 99% or 100%) as compared with any one of the sequences as set forth in SEQ ID NO:1-68.

In some embodiments, the substitution as described in (ii) is a conservative substitution.

In some embodiments, in the conjugate represented by Formula (I), E is an Fc domain monomer having a sequence with a sequence identity of at least 70% (such as 70%, 75%, 80%, 85%, 90%, 95%, 96%, 97%, 98%, 99% or 100%) as compared with the sequences as set forth in SEQ ID NO:64.

In some embodiments, in the conjugate represented by Formula (I), E is an Fc domain monomer having a sequence as set forth in SEQ ID NO:64.

In some embodiments, in the conjugate represented by Formula (I), E is an Fc domain monomer having a sequence as set forth in SEQ ID NO:64, n is 2, and the two E dimerize to form an Fc domain.

In some embodiments, in the conjugate represented by Formula (I), R₁ is selected from the group consisting of -OH, -NH₂, and -NHC(=NH)NH₂. In some embodiments, in the conjugate represented by Formula (I), R₁ is -NHC(=NH)NH₂.

In some embodiments, in the conjugate represented by Formula (I), R₄ is selected from the group consisting of -C(=O)OH, -C(=O)OCH₃ and -C(=O)OCF₃. In some embodiments, in the conjugate represented by Formula (I), R₄ is selected from the group consisting of -C(=O)OH and -C(=O)OCH₃.

In some embodiments, in the conjugate represented by Formula (I), R₅ is selected from the group consisting of -C(=O)CH₃ and -C(=O)CF₃. In some embodiments, in the conjugate represented by Formula (I), R₅ is -C(=O)CH₃.

In some embodiments, in the conjugate represented by Formula (I), R' is H or C₁-C₄ alkyl. In some embodiments, in the conjugate represented by Formula (I), R' is H, methyl or ethyl. In some embodiments, in the conjugate represented by Formula (I), R' is H or ethyl.

In some embodiments, in the conjugate represented by Formula (I), X is -O-.

In some embodiments, in the conjugate represented by Formula (I), Y is selected from the group consisting of -O-, -S-, and -NH-. In some embodiments, in the conjugate represented by Formula (I), Y is selected from the group consisting of -O- and -NH-.

In some embodiments, the conjugate represented by Formula (I) has a structure represented by Formula (I-II-A), Formula (I-II-B) or Formula (I-II-C), 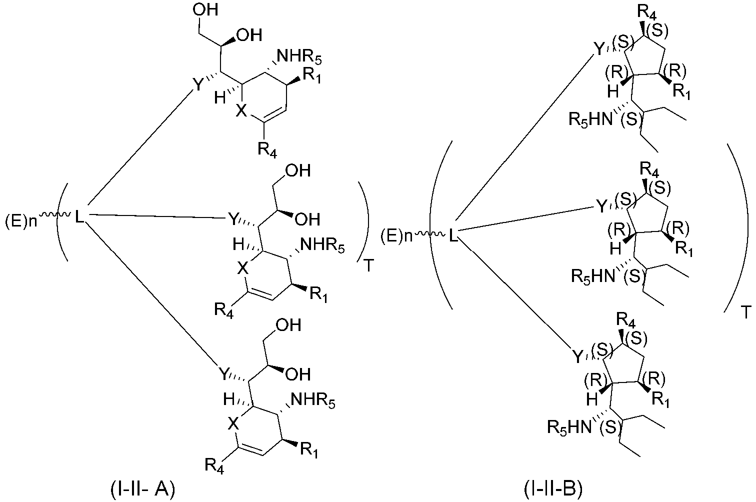 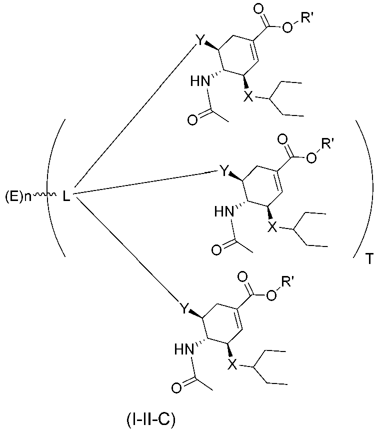 wherein R₁, R₄, R₅, R', X, Y, E, n, L, and T are as defined in any embodiment of the present application.

In some embodiments, the conjugate represented by Formula (I) has a structure represented by Formula (I-II-A'), Formula (I-II-B') or Formula (I-II-C'), 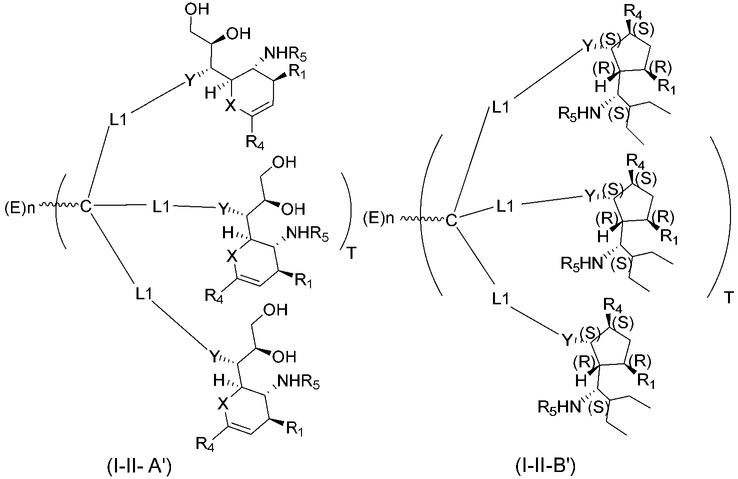 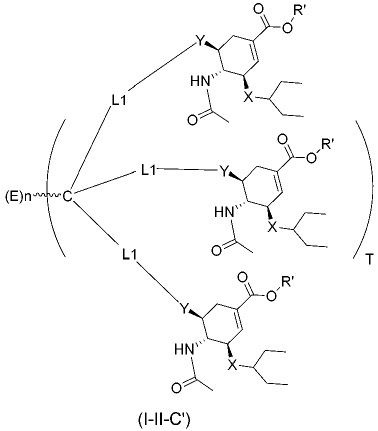
wherein R₁, R₄, R₅, R', X, Y, E, n, and T are as defined in any embodiment of the present application,
Li is selected from the group consisting of: 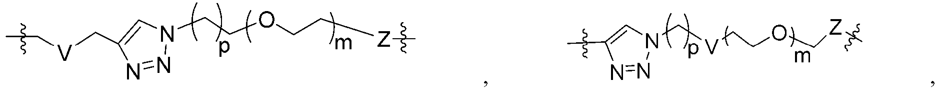 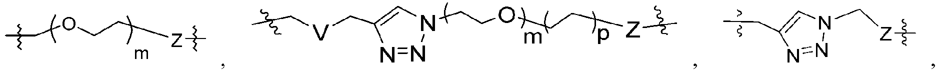 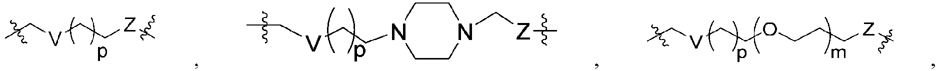 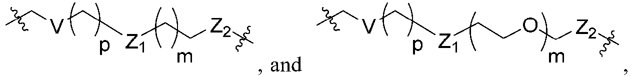 wherein V is -CH₂-, -O- or -S-; Z is - C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-; Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, - C(=O)- or -CH₂-; m and p each independently are an integer between 1 and 20.

In some embodiments, in the conjugate represented by Formula (I), L₁ is selected from the group consisting of: 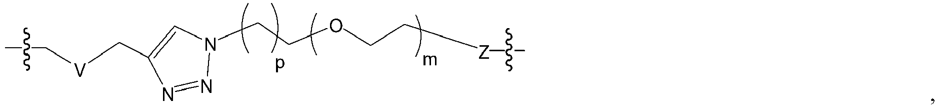 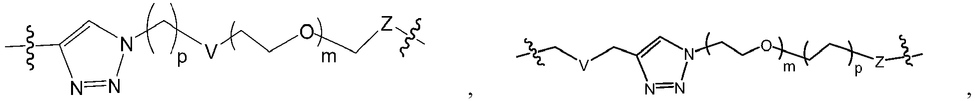 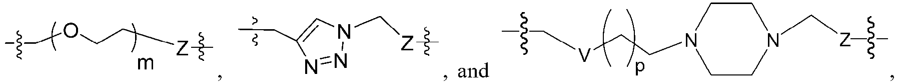 wherein V, p, m, and Z are as defined in any embodiment of the present application.

In some embodiments, in the conjugate represented by Formula (I), L₁ is 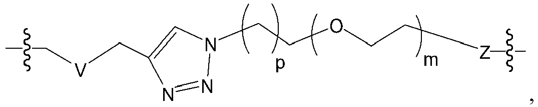 wherein V, p, m, and Z are as defined in any embodiment of the present application.

In some embodiments, in the conjugate represented by Formula (I), V is -CH₂- or -O-. In some embodiments, in the conjugate represented by Formula (I), V is -O-.

In some embodiments, in the conjugate represented by Formula (I), Z is -C(=O)-NH-, -CH₂-C(=O)-NH-, - NH-C(=O)-, -CH₂CH₂-C(=O)-, -N(CH₃)-C(=O)-, -N(CH₂CH₃)-C(=O)- or -C(=O)-. In some embodiments, in the conjugate represented by Formula (I), Z is -NH-C(=O)- or -C(=O)-.

In some embodiments, in the conjugate represented by Formula (I), p is an integer between 1 and 10. In some embodiments, in the conjugate represented by Formula (I), p is an integer between 1 and 6 (such as 1, 2, 3, 4, 5, 6).

In some embodiments, in the conjugate represented by Formula (I), m is an integer between 1 and 12. In some embodiments, in the conjugate represented by Formula (I), m is an integer between 1 and 10 (such as 1, 2, 3, 4, 5, 6, 7, 8, 9).

In some embodiments, the conjugate represented by Formula (I) has a structure represented by Formula (I-II-A'-1), Formula (I-II-B'-1), Formula (I-II-B'-2) or Formula (I-II-C'-1): 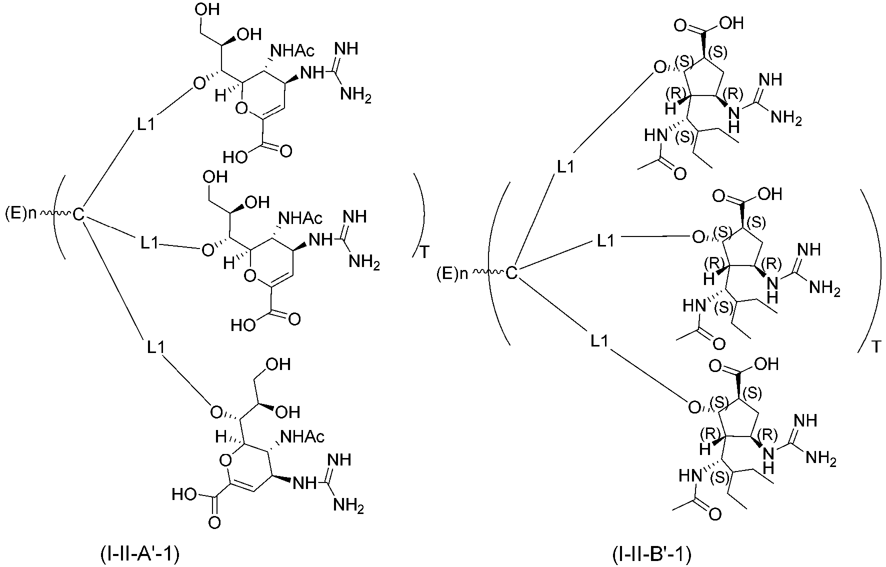 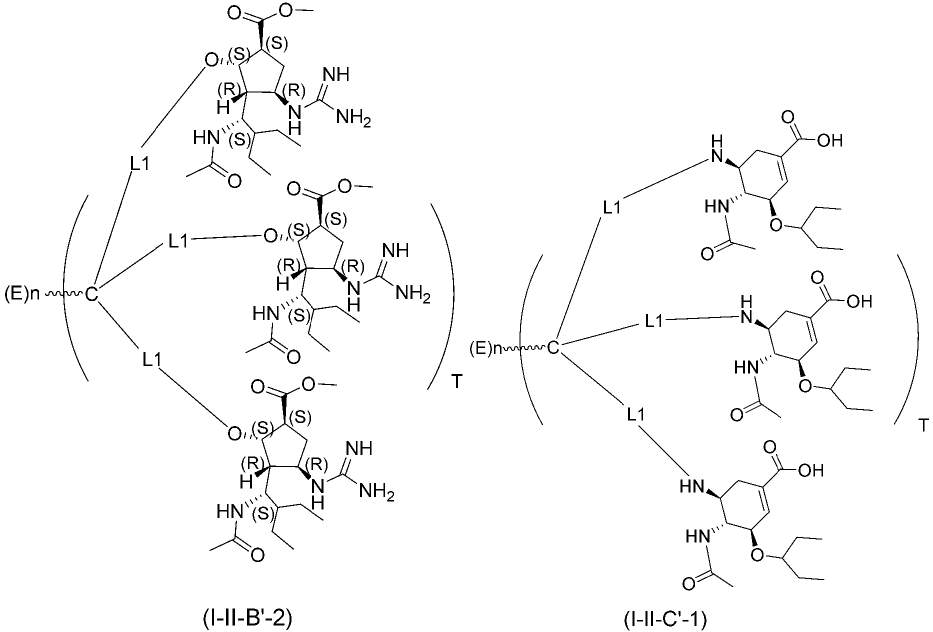 wherein E, n, T, and L₁ are as defined in any embodiment of the present application.

In some embodiments, the conjugate represented by Formula (I) has a structure represented by Formula (I-II-A-2), Formula (I-II-B-3), Formula (I-II-B-4) or Formula (I-II-C-2), 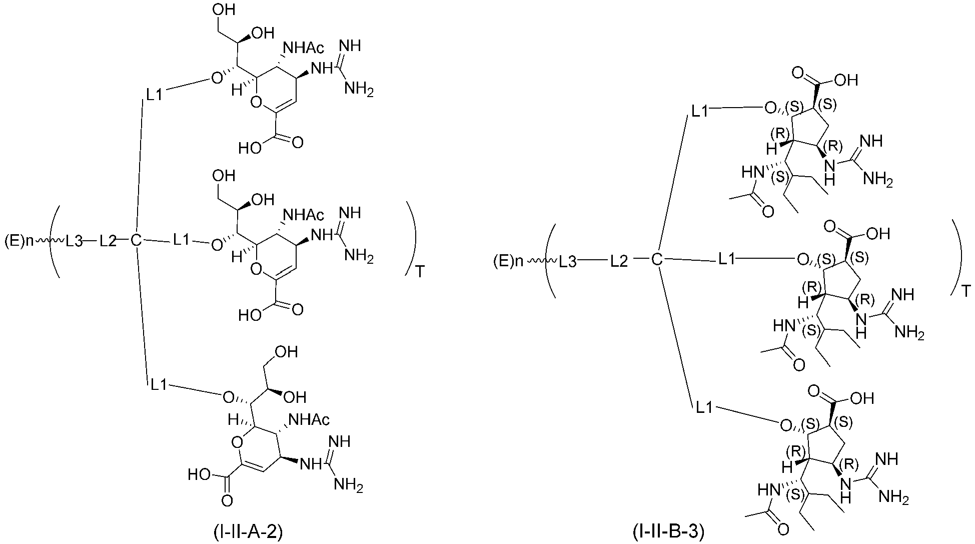 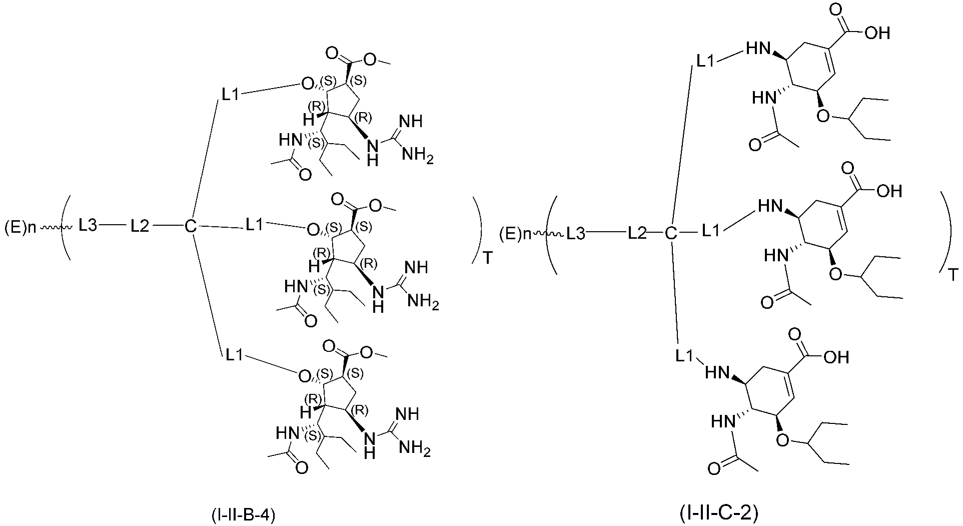
wherein E, n, T, and L₁ are as defined in any embodiment of the present application;
L₂ is selected from the group consisting of: 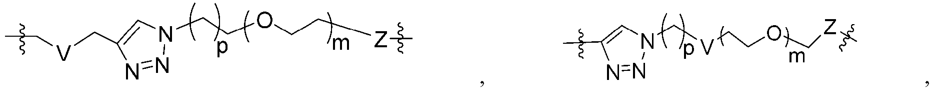 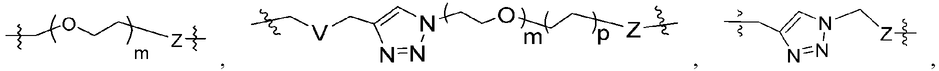 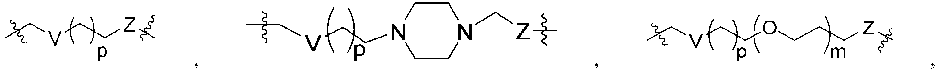 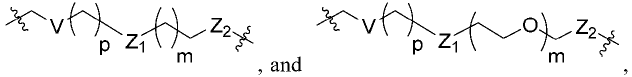 wherein V is -CH₂-, -O- or -S-; Z is - C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-; Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, - C(=O)- or -CH₂-; m and p each independently are an integer between 1 and 20;
L₃ is 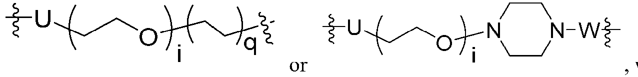 wherein U is -C(=O)-NH-, - NH-C(=O)-, -N(CH₃)-C(=O)-, -N(CH₂CH₃)-C(=O)-, -O-C(=O)-, -C(=O)-, or -CH₂-; W is -CH₂-, -O- or -S-; i and q each independently are an integer between 1 and 20, one end of L₃ is covalently linked to E, and the other end is covalently linked to L₂.

In some embodiments, in the conjugate represented by Formula (I), L₃ is covalently linked to E via a N atom, wherein the N atom comes from the N atom in the E (e.g., an N atom on the side chain amino group of a lysine, arginine, asparagine, or glutamine residue).

In some embodiments, in the conjugate represented by Formula (I), L₂ is selected from the group consisting of: 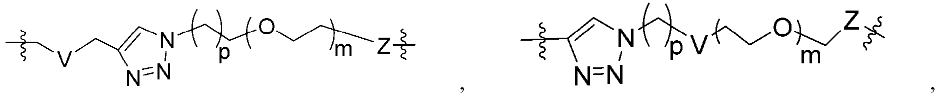 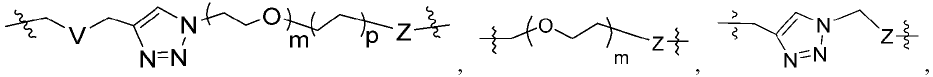 and 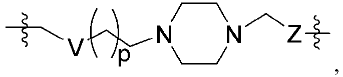 wherein V is -CH₂-, -O- or -S-; Z is -C(=O)-NH-, -CH₂-C(=O)-NH-, - NH-C(=O)-, -CH₂CH₂-C(=O)-, -N(CH₃)-C(=O)-, -N(CH₂CH₃)-C(=O)-, -O-C(=O)-, -C(=O)- or -CH₂-; m and p each independently are an integer between 1 and 20.

In some embodiments, in the conjugate represented by Formula (I), L₁ is 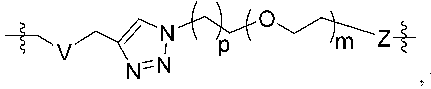 wherein V, p, m, and Z are as defined in any embodiment of the present application. In some embodiments, V is -CH₂- or -O-. In some embodiments, V is -O-. In some embodiments, Z is -C(=O)-NH-, -CH₂-C(=O)-NH-, -NH-C(=O)-, -CH₂CH₂-C(=O)-, -N(CH₃)-C(=O)-, - N(CH₂CH₃)-C(=O)- or -C(=O)-. In some embodiments, Z is -NH-C(=O)- or -C(=O)-. In some embodiments, p is an integer between 1 and 10. In some embodiments, p is an integer between 1 and 6 (such as 1, 2, 3, 4, 5, 6). In some embodiments, m is an integer between 1 and 12. In some embodiments, m is an integer between 1 and 10 (such as 1, 2, 3, 4, 5, 6, 7, 8, 9).

In some embodiments, in the conjugate represented by Formula (I), L₂ is 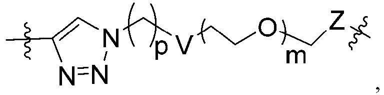 wherein V, p, m, and Z are as defined in any embodiment of the present application. In some embodiments, V is -CH₂- or -O-. In some embodiments, V is -O-. In some embodiments, Z is -C(=O)-NH-, -CH₂-C(=O)-NH-, -NH-C(=O)-, -CH₂CH₂-C(=O)-, -N(CH₃)-C(=O)-, or - N(CH₂CH₃)-C(=O)-. In some embodiments, Z is -C(=O)-NH-. In some embodiments, p is an integer between 1 and 10. In some embodiments, p is an integer between 1 and 6 (such as 1, 2, 3, 4, 5, 6). In some embodiments, m is an integer between 1 and 12. In some embodiments, m is an integer between 1 and 10 (such as 1, 2, 3, 4, 5, 6, 7, 8, 9).

In some embodiments, in the conjugate represented by Formula (I), L₃ is 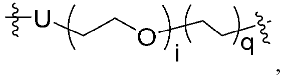 wherein U, i, and q are as defined in any embodiment of the present application. In some embodiments, U is - NH-C(=O)-, -N(CH₃)-C(=O)- or -N(CH₂CH₃)-C(=O)-. In some embodiments, U is -NH-C(=O)-. In some embodiments, the N atom in the U comes from the N atom in the E (e.g., an N atom on the side chain amino group of a lysine, arginine, asparagine, or glutamine residue). In some embodiments, i is an integer between 1 and 12. In some embodiments, i is an integer between 1 and 10 (such as 1, 2, 3, 4, 5, 6, 7, 8, 9). In some embodiments, q is an integer between 1 and 10. In some embodiments, q is an integer between 1 and 6 (such as 1, 2, 3, 4, 5, 6).

In some embodiments, in the conjugate represented by Formula (I), T is a number between 1 and 10. In some embodiments, in the conjugate represented by Formula (I), T is a number between 1 and 9. In some embodiments, in the conjugate represented by Formula (I), T is a number between 1 and 7. In some embodiments, in the conjugate represented by Formula (I), T is a number between 1 and 5. In some embodiments, in the conjugate represented by Formula (I), T is a number between 3 and 7. In some embodiments, in the conjugate represented by Formula (I), T is a number between 3 and 5.5.

In some embodiments, the conjugate represented by Formula (I) has a structure represented by Formula (I-II-A-3), Formula (I-II-B-5), Formula (I-II-B-6) or Formula (I-II-C-3), 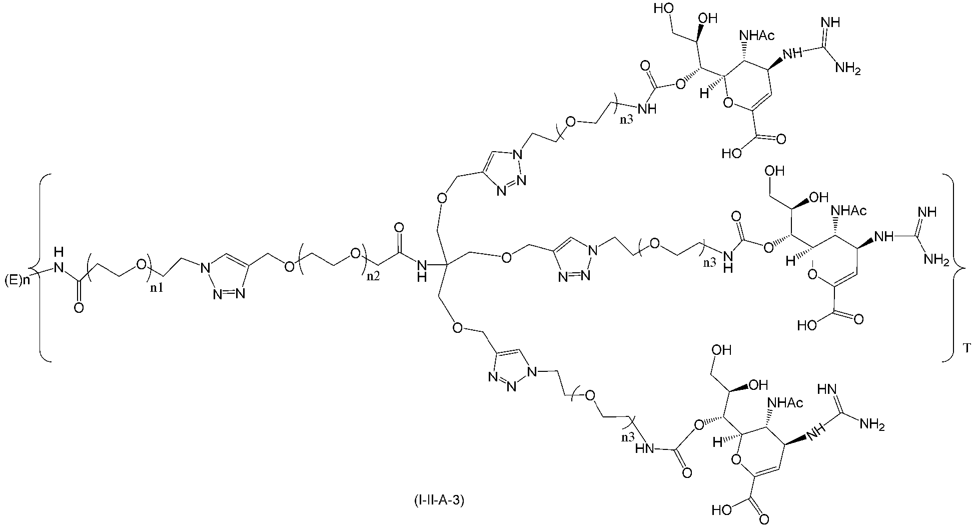 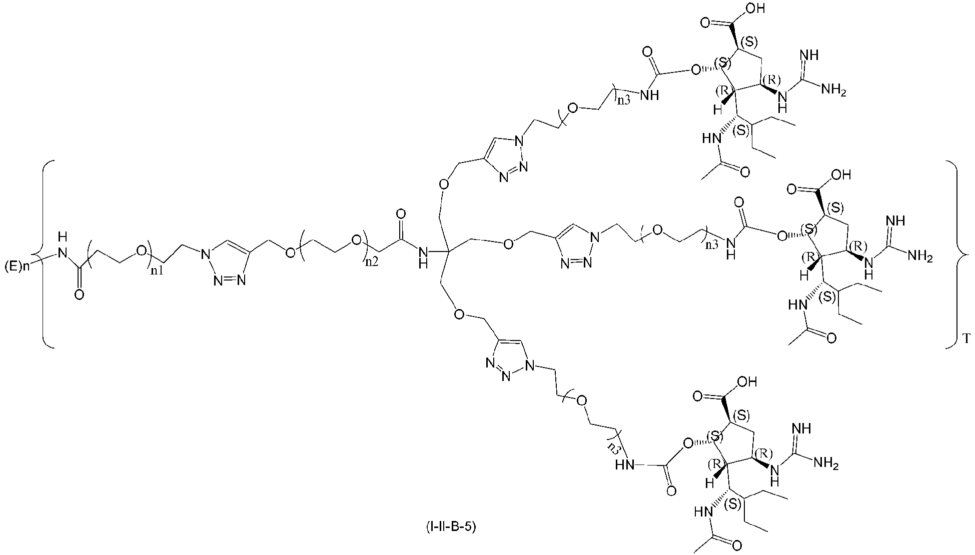 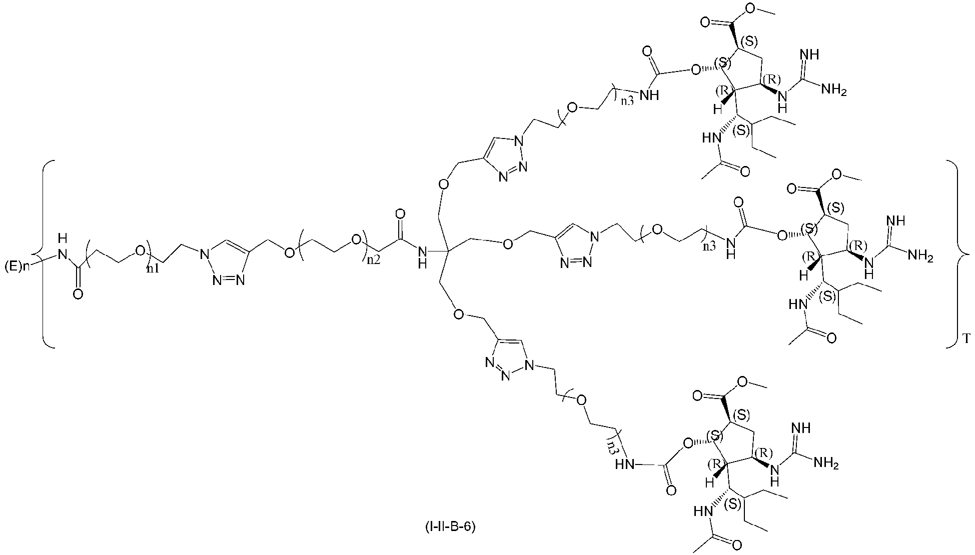 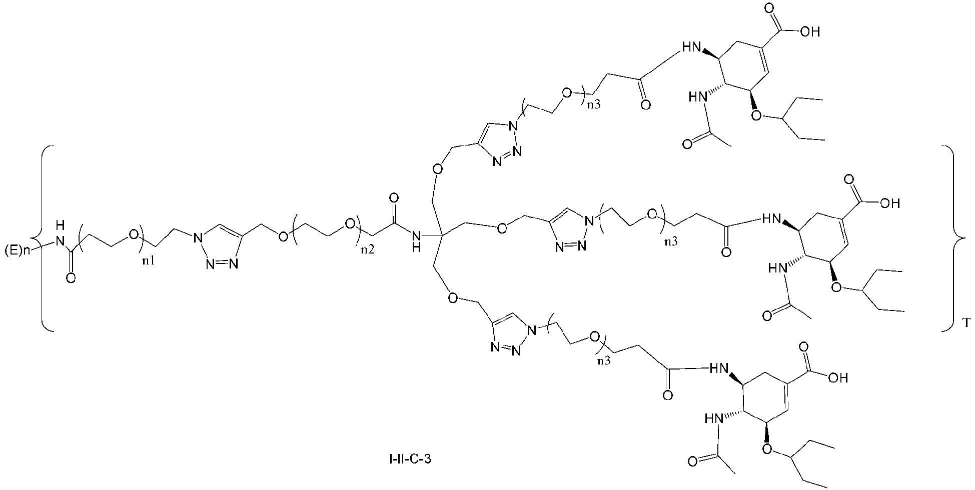 wherein E, n, and T are as defined in any embodiment of the present application; n1, n2, and n3 each independently are an integer between 1 and 20. In some embodiments, n1, n2, and n3 each independently are an integer between 1 and 12. In some embodiments, n1, n2, and n3 each independently are an integer between 1 and 10 (such as 1, 2, 3, 4, 5, 6, 7, 8, 9).

In some embodiments, the conjugate represented by Formula (I) is selected from the group consisting of: 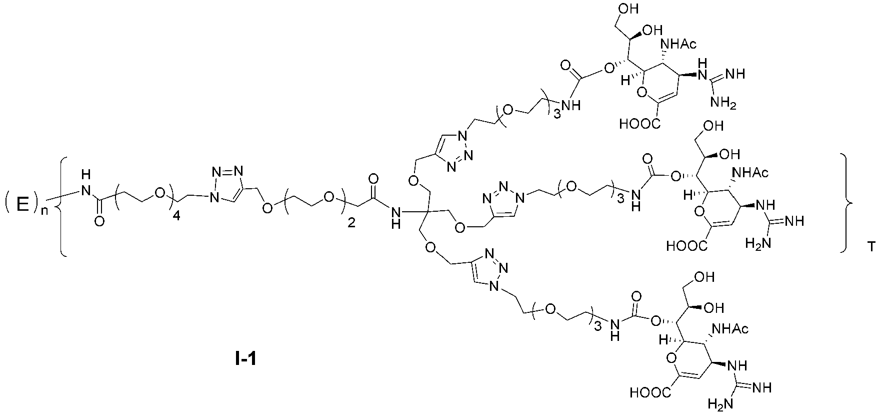 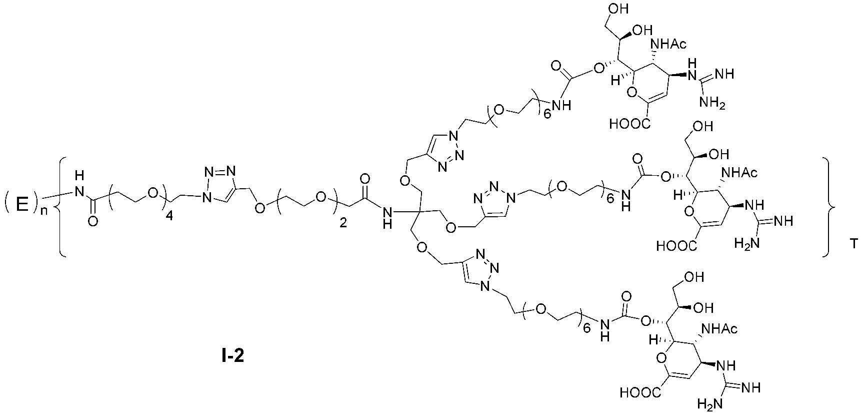 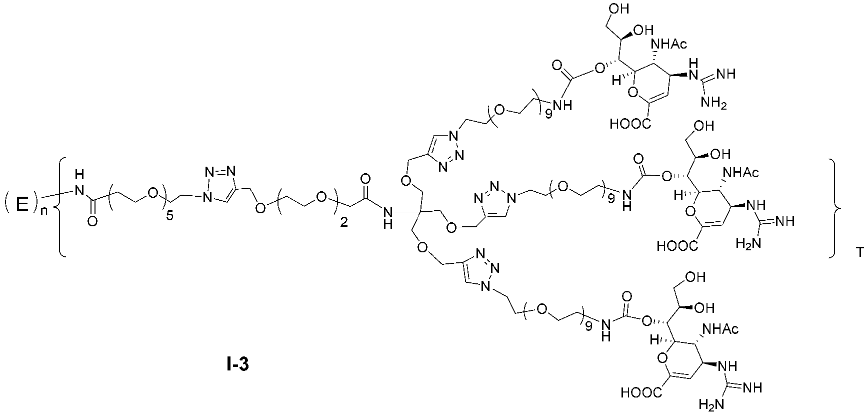 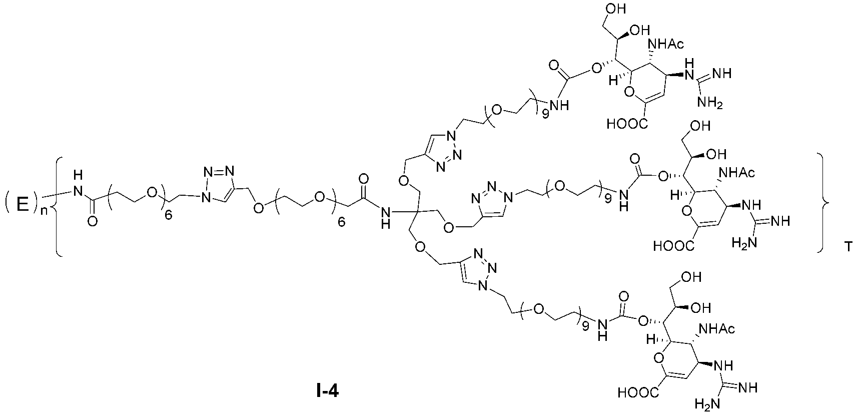 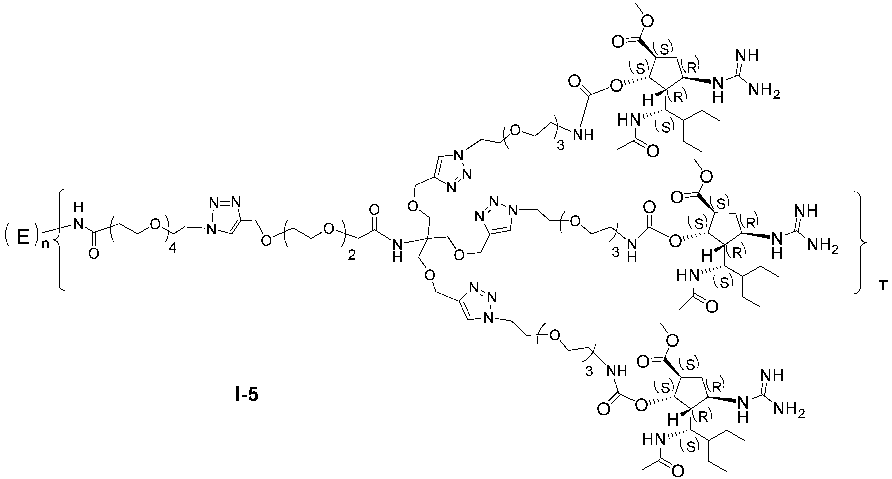 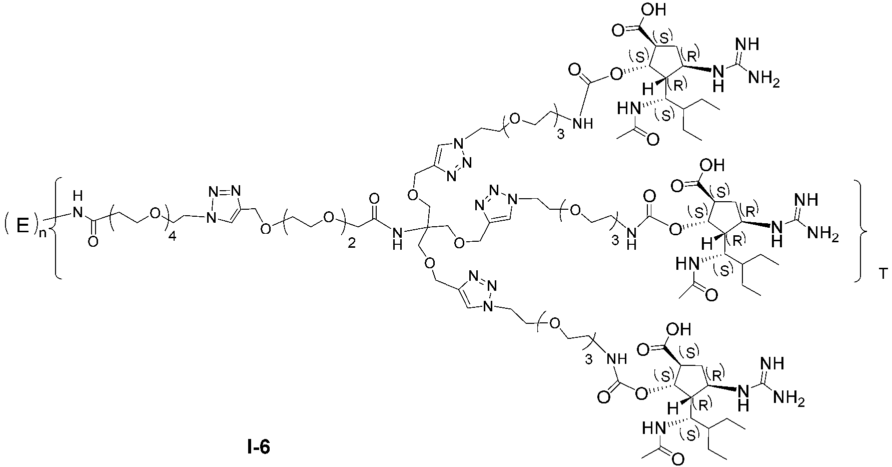 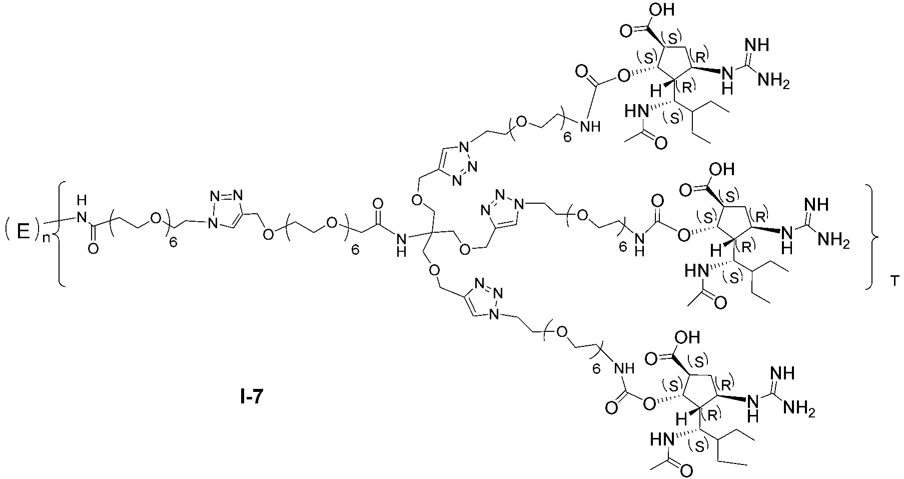 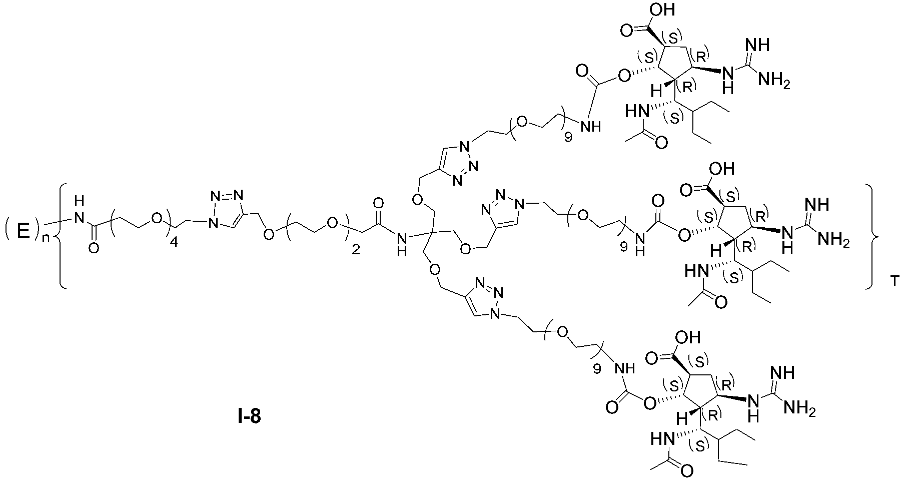 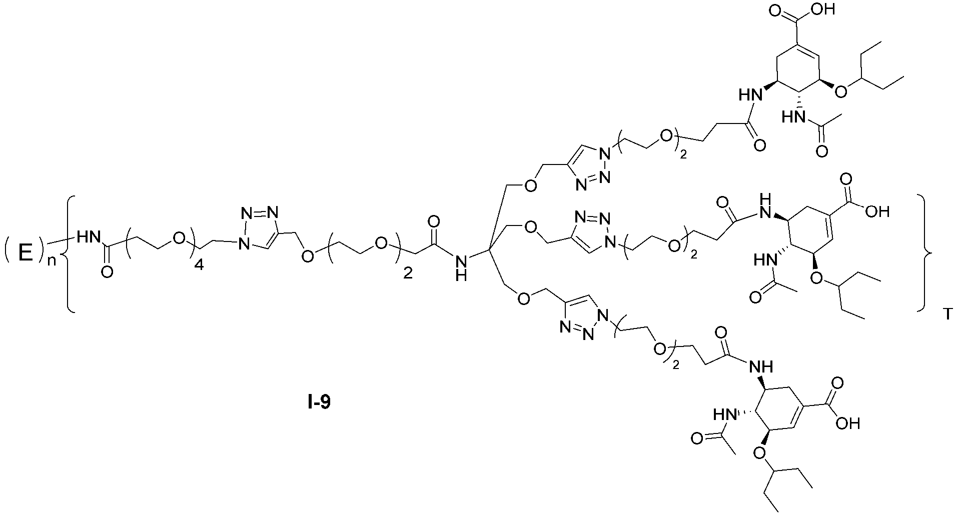 and 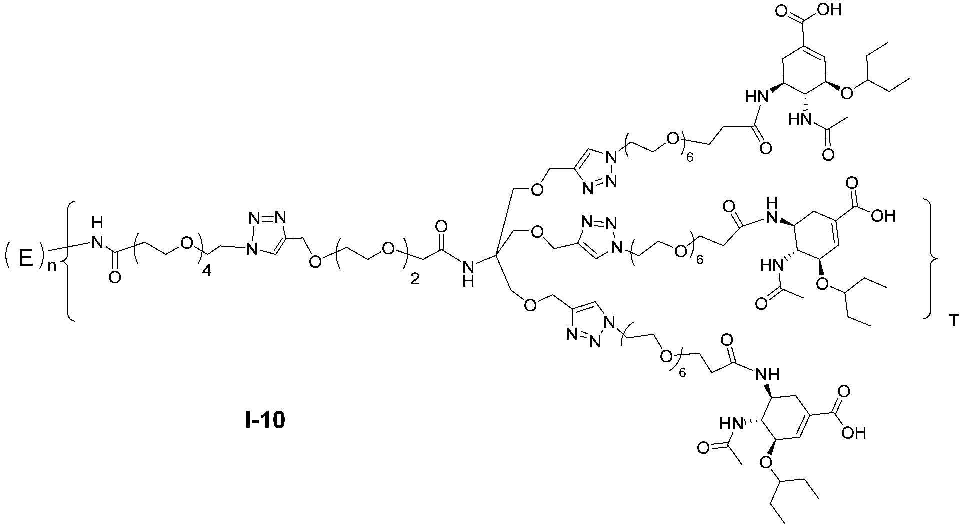 wherein E, n, and T are as defined in any embodiment of the present application.

In another aspect, the present application also provides a conjugate represented by Formula (II) or a pharmaceutically acceptable salt, a geometric or optical isomer, a hydrate, a solvate or a polymorph thereof. 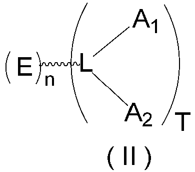
wherein A₁ and A₂ each independently are selected from the group consisting of Formula (A-I) to Formula (A-XIV), 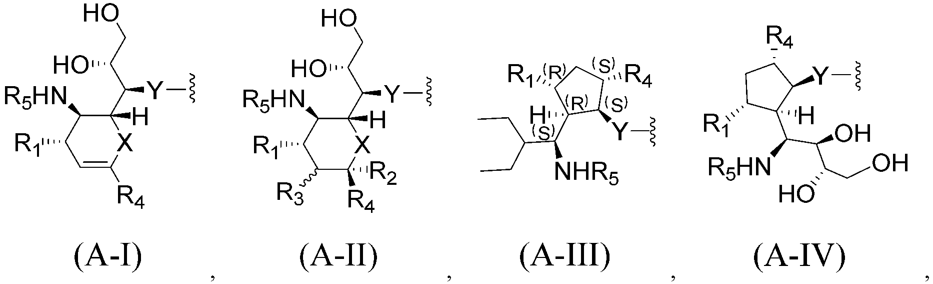 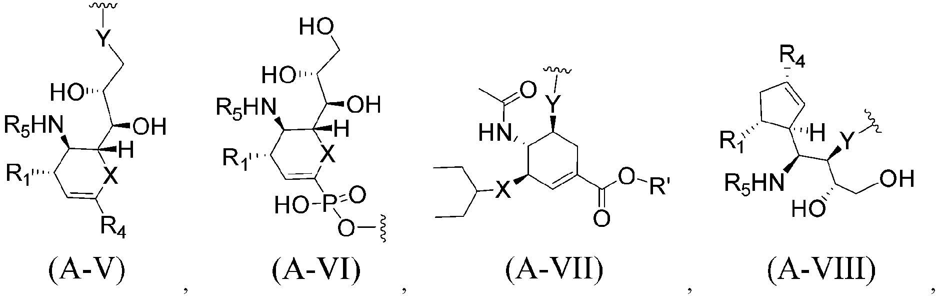 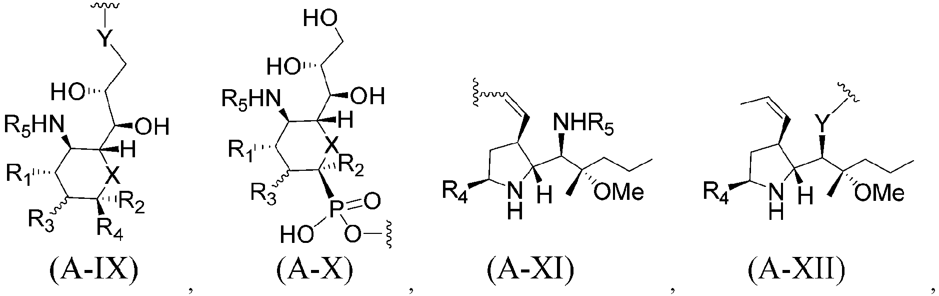 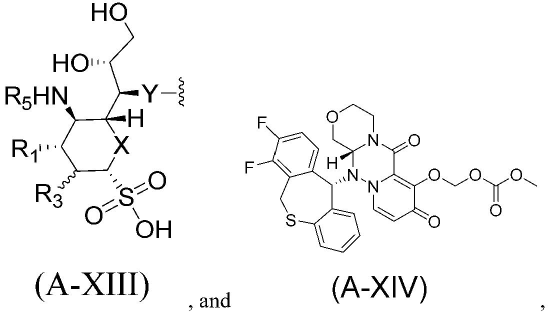
wherein, L may be linked to any possible sites of the compound of Formula (A-XIV);
wherein R₁ is selected from the group consisting of -OH, -NH₂, -NHC(=NH)NH₂ and -NHC(=NH)NHR₆;
R₂ and R₃ each independently are selected from the group consisting of -H, -OH, -F, -Cl and -Br;
R₄ is selected from the group consisting of -C(=O)OH, -P(=O)(OH)₂, -SO₃H, -C(=O)OCH₃ and -C(=O)OCF₃;
R₅ is selected from the group consisting of -C(=O)CH₃, -C(=O)CF₃, and -SO₂CH₃;
X is selected from the group consisting of -O- and -S-;
Y is selected from the group consisting of -O-, -S-, -NR₇-, -OC(=O)NR₇-, -OC(=S)NR₇-, -OC(=O)O-, -OC(=O)-, -NHC(=O)O-, -NHC(=O)-, -NHC(=NH)-, -NHC(=O)NR₇-, -NHC(=NH)NR₇-, -NHC(=S)NR₇-, -NHC(=S)-, -OCH₂C(=O)NR₇-, -NH(SO₂)- and -NH(SO₂)NR₇-;
R' is H or C₁-C₆ alkyl;
R₆ is selected from the group consisting of: 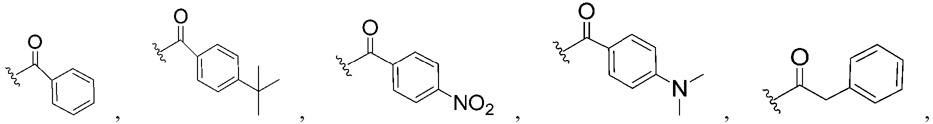 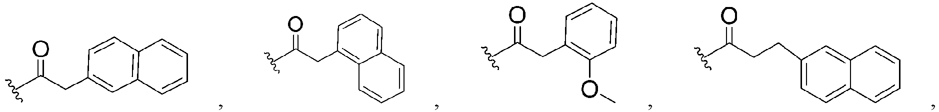 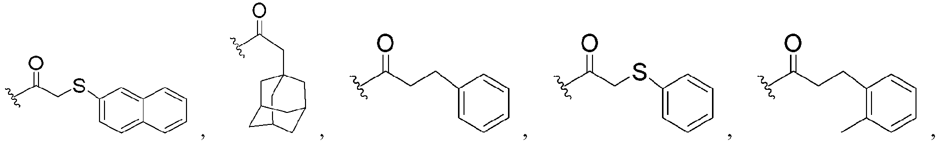 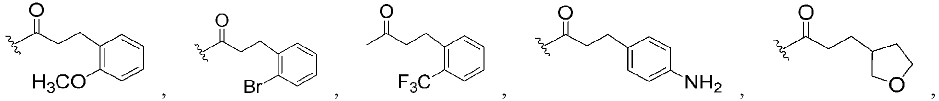 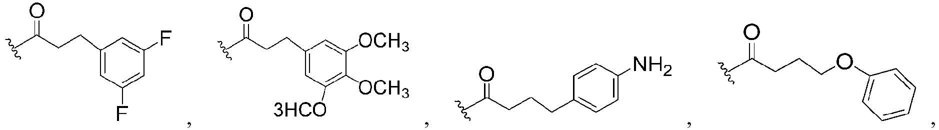 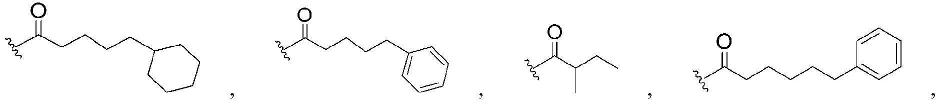 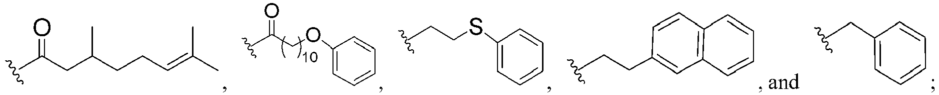
R₇ is selected from the group consisting of H, C₁-C₂₀ alkyl, C₃-C₂₀ cycloalkyl, C₃-C₂₀ heterocycloalkyl, C₅-C₁₅ aryl and C₅-C₁₅ heteroaryl; preferably, R₇ is H; preferably, R₇ is C₁-C₆ alkyl;
each E is a biomacromolecule; n is 1 or 2; T is a number between 1 and 20;
the wavy line linked to each E represents a covalent linking (e.g., via a covalent bond or a linker) of each A₁-L-A₂ to an N atom in the each E (e.g., an N atom on the side chain amino group of a lysine, arginine, asparagine, or glutamine residue), and L is a linker covalently linked to each of groups E, A₁, and A₂.

In some embodiments, in the conjugate represented by Formula (II), A₁ and A₂ each independently are selected from the group consisting of Formula (A-I) to Formula (A-XIII), 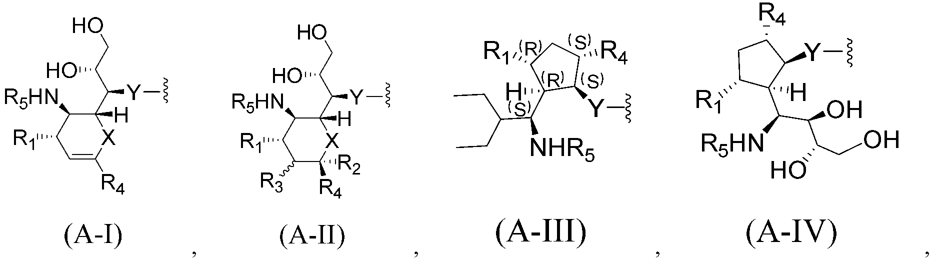 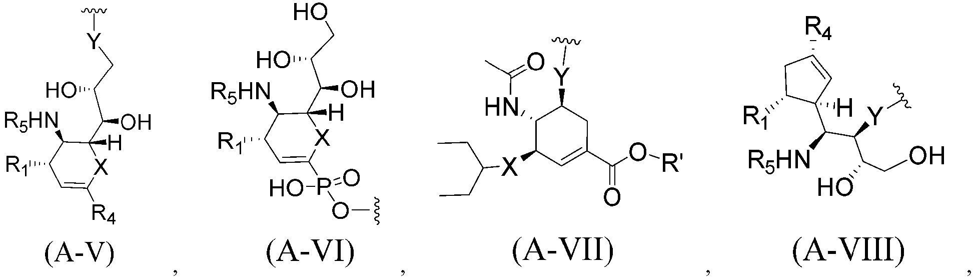 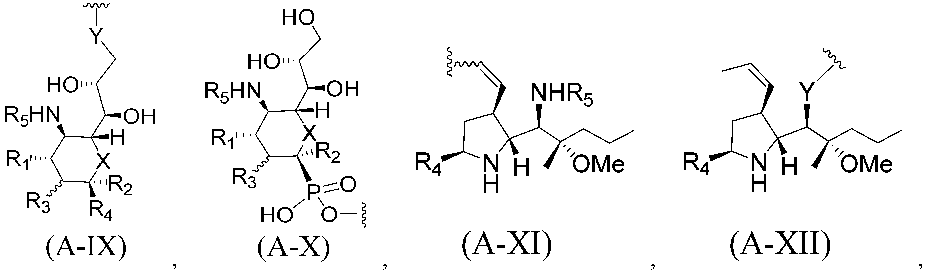 and 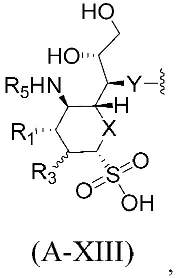 wherein R₁, R₂, R₃, R₄, R₅, R', X, and Y are as defined in any embodiment of the present application.

In some embodiments, in the conjugate represented by Formula (II), A₁ and A₂ each independently are selected from the group consisting of Formula (A-I) to Formula (A-IV) and Formula (A-VII), 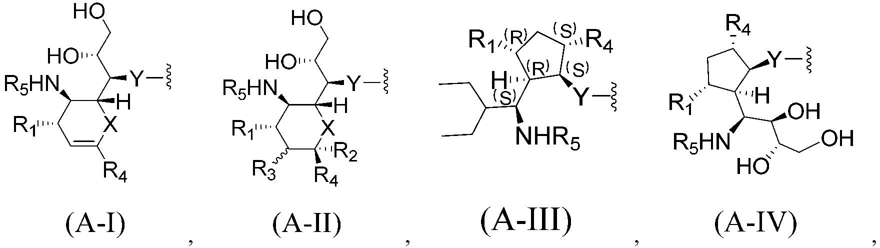 and 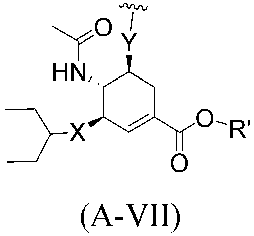 wherein R₁, R₂, R₃, R₄, R₅, R', X, and Y are as defined in any embodiment of the present application.

In some embodiments, in the conjugate represented by Formula (II), each E comprises an Fc monomer domain (e.g., an Fc domain monomer having a sequence as set forth in any one of SEQ ID NO: 1-68), albumin (e.g., albumin having a sequence as set forth in any one of SEQ ID NO:69-71), and an albumin-binding peptide, or an Fc-binding peptide; n is 1 or 2, wherein when n is 2 and E is the Fc domain monomer, the two E dimerize to form an Fc domain.

In some embodiments, in the conjugate represented by Formula (II), A₁ and A₂ each independently are selected from the group consisting of Formula (A-I) to Formula (A-III) and Formula (A-VII), 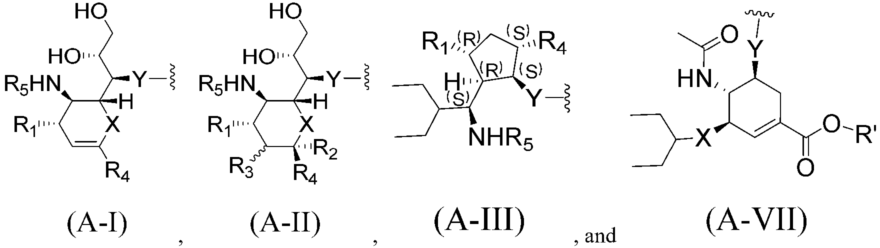 wherein R₁, R₂, R₃, R₄, R₅, R', X, and Y are as defined in any embodiment of the present application.

In some embodiments, in the conjugate represented by Formula (II), A₁ and A₂ each independently are selected from the group consisting of Formula (A-I), Formula (A-III) and Formula (A-VII), 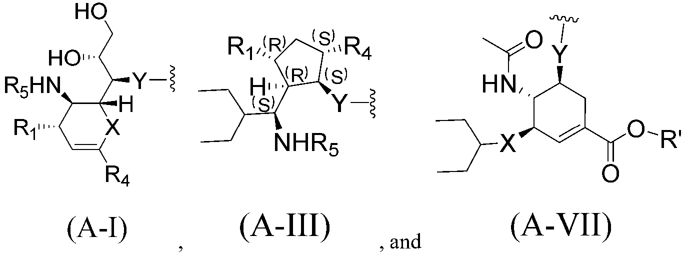 wherein R₁, R₄, R₅, R', X, and Y are as defined in any embodiment of the present application.

In some embodiments, in the conjugate represented by Formula (II), E comprises an Fc domain monomer (e.g., an Fc domain monomer having a sequence as set forth in any one of SEQ ID NO:1-68); n is 1 or 2, wherein when n is 2, the two E dimerize to form an Fc domain.

In some embodiments, in the conjugate represented by Formula (II,) E is an Fc domain monomer, the Fc domain monomer having an amino acid sequence selected from the group consisting of:
i) any one of the sequences as set forth in SEQ ID NO:1-68;
ii) a sequence with substitution, deletion or addition of one or several amino acids (for example, substitution, deletion or addition of one, two, three, four or five amino acids) as compared with any one of the sequences as set forth in SEQ ID NO: 1-68; and
iii) a sequence with a sequence identity of at least 70% (such as 70%, 75%, 80%, 85%, 90%, 95%, 96%, 97%, 98%, 99% or 100%) as compared with any one of the sequences as set forth in SEQ ID NO:1-68.

In some embodiments, the substitution as described in (ii) is a conservative substitution.

In some embodiments, in the conjugate represented by Formula (II), E is an Fc domain monomer of a sequence having a sequence identity of at least 70% (such as 70%, 75%, 80%, 85%, 90%, 95%, 96%, 97%, 98%, 99% or 100%) as compared with the sequences as set forth in SEQ ID NO:64.

In some embodiments, in the conjugate represented by Formula (II), E is an Fc domain monomer having a sequence as set forth in SEQ ID NO:64.

In some embodiments, in the conjugate represented by Formula (II) E is an Fc domain monomer having a sequence as set forth in SEQ ID NO:64, n is 2, and the two E dimerize to form an Fc domain.

In some embodiments, in the conjugate represented by Formula (II), R₁ is selected from the group consisting of -OH, -NH₂, and -NHC(=NH)NH₂. In some embodiments, in the conjugate represented by Formula (II), R₁ is -NHC(=NH)NH₂.

In some embodiments, in the conjugate represented by Formula (II), R₄ is selected from the group consisting of -C(=O)OH, -C(=O)OCH₃ and -C(=O)OCF₃. In some embodiments, in the conjugate represented by Formula (II), R₄ is selected from the group consisting of -C(=O)OH and -C(=O)OCH₃.

In some embodiments, in the conjugate represented by Formula (II), R₅ is selected from the group consisting of -C(=O)CH₃ and -C(=O)CF₃. In some embodiments, in the conjugate represented by Formula (II), R₅ is -C(=O)CH₃.

In some embodiments, in the conjugate represented by Formula (II), R' is H or C₁-C₄ alkyl. In some embodiments, in the conjugate represented by Formula (II), R' is H, methyl or ethyl. In some embodiments, in the conjugate represented by Formula (II), R' is H or ethyl.

In some embodiments, in the conjugate represented by Formula (II), X is -O-.

In some embodiments, in the conjugate represented by Formula (II), Y is selected from the group consisting of -O-, -S-, and -NH-. In some embodiments, in the conjugate represented by Formula (II), Y is selected from the group consisting of -O- and -NH-.

In some embodiments, the conjugate represented by Formula (II) has a structure represented by Formula (II-II-A), Formula (II-II-B) or Formula (II-II-C), 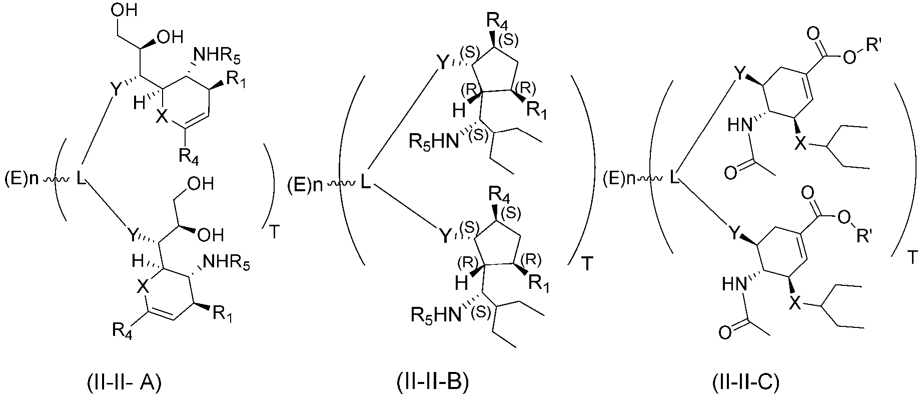 wherein R₁, R₄, R₅, R', X, Y, E, n, L, and T are as defined in any embodiment of the present application.

In some embodiments, the conjugate represented by Formula (II) has a structure represented by Formula (II-II-A'), Formula (II-II-B') or Formula (II-II-C'), 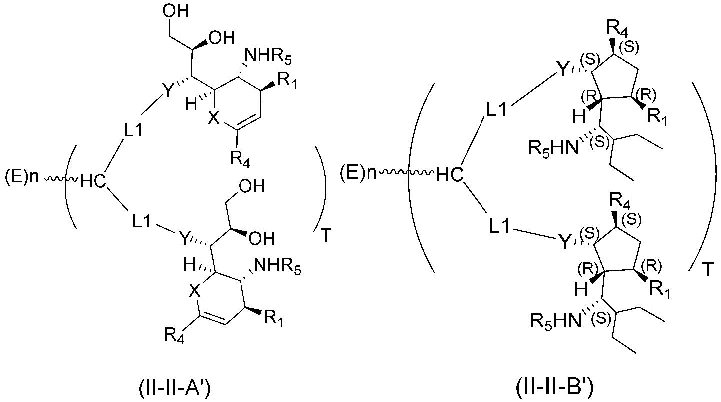 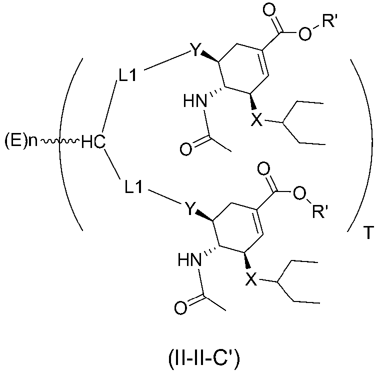
wherein R₁, R₄, R₅, R', X, Y, E, n, and T are as defined in any embodiment of the present application;
L₁ is selected from the group consisting of: 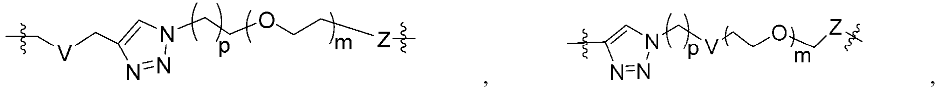 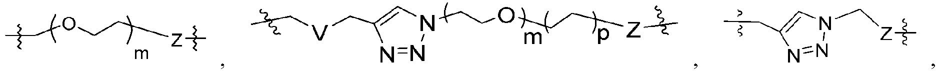 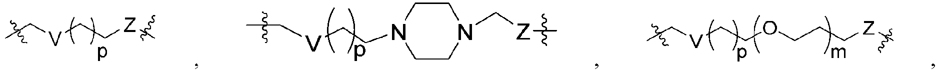 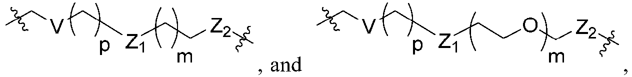 wherein V is -CH₂-, -O- or -S-; Z is - C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-; Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, - C(=O)- or -CH₂-; m and p each independently are an integer between 1 and 20.

In some embodiments, in the conjugate represented by Formula (II), L₁ is selected from the group consisting of: 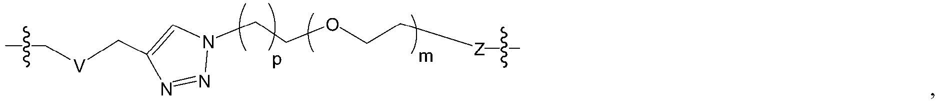 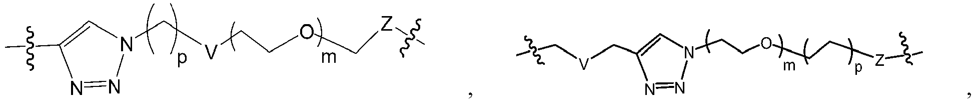 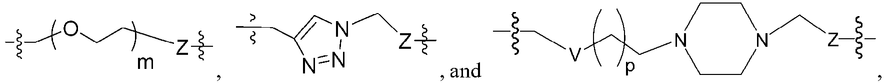 wherein V, p, m, and Z are as defined in any embodiment of the present application.

In some embodiments, in the conjugate represented by Formula (II), L₁ is 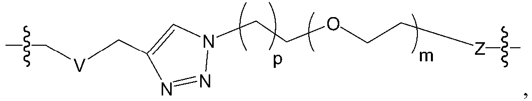 wherein V, p, m, and Z are as defined in any embodiment of the present application.

In some embodiments, in the conjugate represented by Formula (II), V is -CH₂- or -O-. In some embodiments, in the conjugate represented by Formula (II), V is -O-.

In some embodiments, in the conjugate represented by Formula (II), Z is -C(=O)-NH-, -CH₂-C(=O)-NH-, -NH-C(=O)-, -CH₂CH₂-C(=O)-, -N(CH₃)-C(=O)-, -N(CH₂CH₃)-C(=O)- or -C(=O)-. In some embodiments, in the conjugate represented by Formula (II), Z is -NH-C(=O)- or -C(=O)-.

In some embodiments, in the conjugate represented by Formula (II), p is an integer between 1 and 10. In some embodiments, in the conjugate represented by Formula (II), p is an integer between 1 and 6 (such as 1, 2, 3, 4, 5, 6).

In some embodiments, in the conjugate represented by Formula (II), m is an integer between 1 and 12. In some embodiments, in the conjugate represented by Formula (II), m is an integer between 1 and 10 (such as 1, 2, 3, 4, 5, 6, 7, 8, 9).

In some embodiments, the conjugate represented by Formula (II) has a structure represented by Formula (II-II-A-1), Formula (II-II-B-1), Formula (II-II-B-2) or Formula (II-II-C-1), 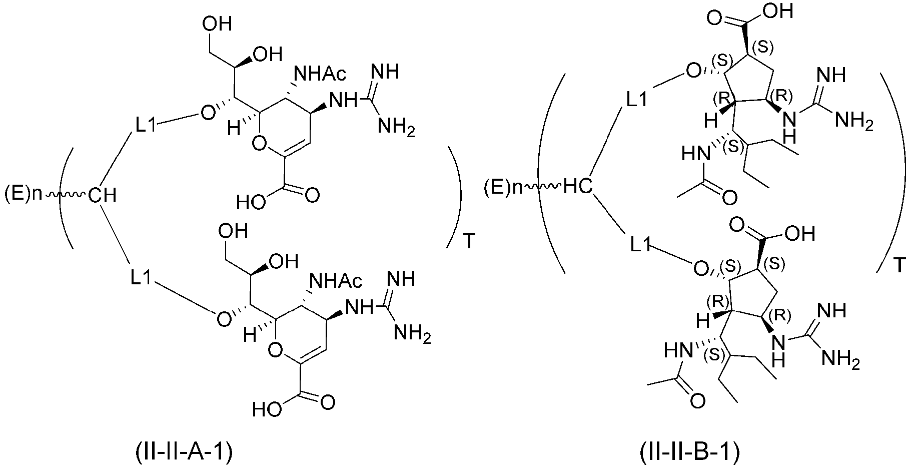 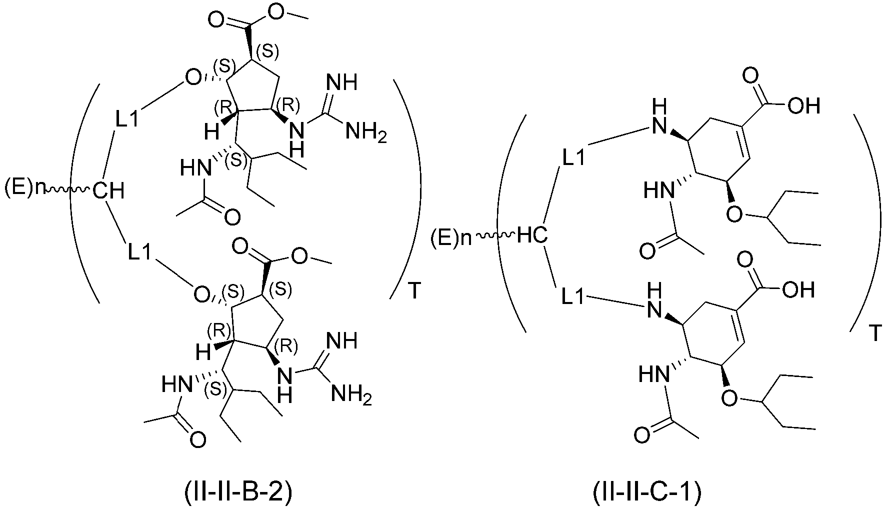 wherein E, n, T, and L₁ are as defined in any embodiment of the present application.

In some embodiments, the conjugate represented by Formula (II) has a structure represented by Formula (II-II-A-2), Formula (II-II-B-3), Formula (II-II-B-4) or Formula (II-II-C-2), 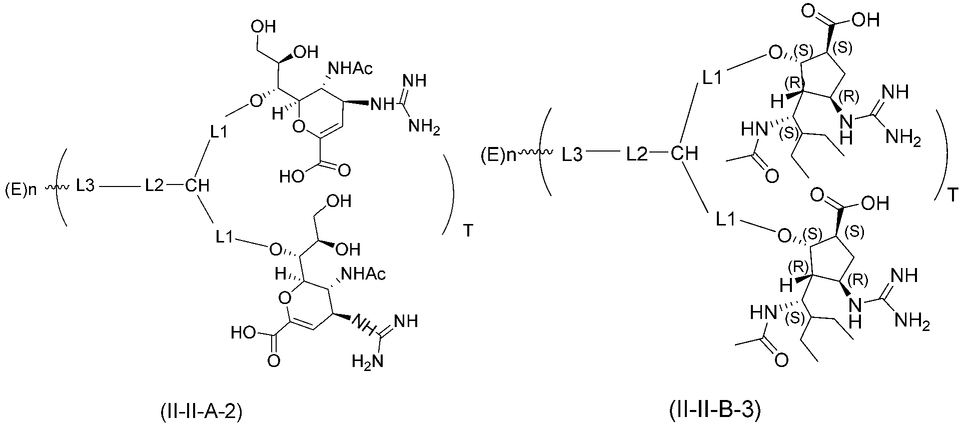 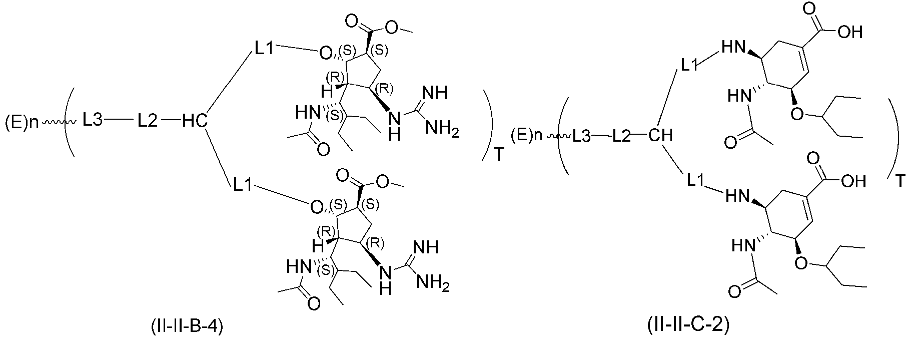
wherein E, n, T, and L₁ are as defined in any embodiment of the present application;
L₂ is selected from the group consisting of: 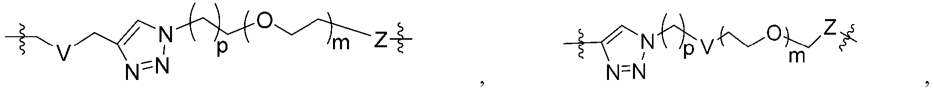 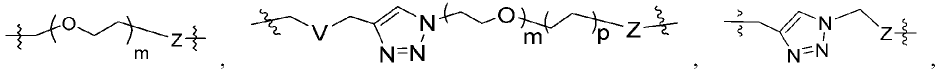 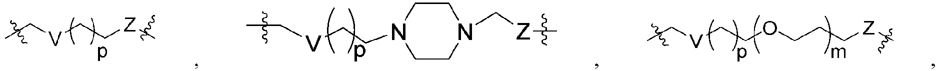 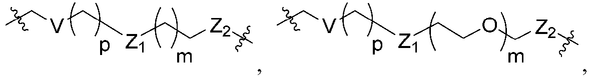 wherein V is -CH₂-, -O- or -S-; Z is -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, - C(=O)- or -CH₂-; Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or - CH₂-; m and p each independently are an integer between 1 and 20;
L₃ is 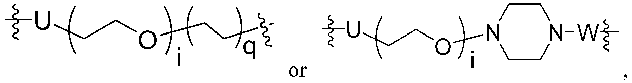 wherein U is -C(=O)-NH-, - NH-C(=O)-, -N(CH₃)-C(=O)-, -N(CH₂CH₃)-C(=O)-, -O-C(=O)-, -C(=O)-, or -CH₂-; W is -CH₂-, -O- or -S-; i and q each independently are an integer between 1 and 20, one end of L₃ is covalently linked to E, and the other end is covalently linked to L₂.

In some embodiments, in the conjugate represented by Formula (II), L₃ is covalently linked to E via a N atom, wherein the N atom comes from the N atom in the E (e.g., an N atom on the side chain amino group of a lysine, arginine, asparagine, or glutamine residue).

In some embodiments, in the conjugate represented by Formula (II), L₂ is selected from the group consisting of: 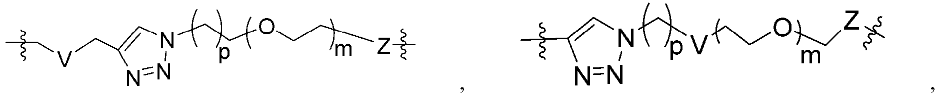 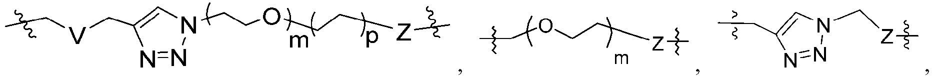 and 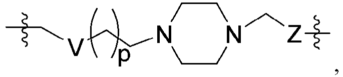 wherein V is -CH₂-, -O- or -S-; Z is -C(=O)-NH-, -CH₂-C(=O)-NH-, - NH-C(=O)-, -CH₂CH₂-C(=O)-, -N(CH₃)-C(=O)-, -N(CH₂CH₃)-C(=O)-, -O-C(=O)-, -C(=O)- or -CH₂-; m and p each independently are an integer between 1 and 20.

In some embodiments, in the conjugate represented by Formula (II), L₁ is 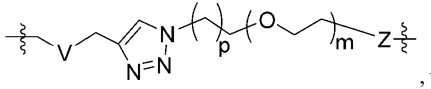 wherein V, p, m, and Z are as defined in any embodiment of the present application. In some embodiments, V is -CH₂- or -O-. In some embodiments, V is -O-. In some embodiments, Z is -C(=O)-NH-, -CH₂-C(=O)-NH-, -NH-C(=O)-, -CH₂CH₂-C(=O)-, -N(CH₃)-C(=O)-, - N(CH₂CH₃)-C(=O)- or -C(=O)-. In some embodiments, Z is -NH-C(=O)- or -C(=O)-. In some embodiments, p is an integer between 1 and 10. In some embodiments, p is an integer between 1 and 6 (such as 1, 2, 3, 4, 5, 6). In some embodiments, m is an integer between 1 and 12. In some embodiments, m is an integer between 1 and 10 (such as 1, 2, 3, 4, 5, 6, 7, 8, 9).

In some embodiments, in the conjugate represented by Formula (II), L₂ is 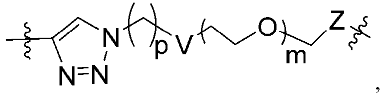 wherein V, p, m, and Z are as defined in any embodiment of the present application. In some embodiments, V is -CH₂- or -O. In some embodiments, V is -O-. In some embodiments, Z is -C(=O)-NH-, -CH₂-C(=O)-NH-, -NH-C(=O)-, -CH₂CH₂-C(=O)-, -N(CH₃)-C(=O)- or - N(CH₂CH₃)-C(=O)-. In some embodiments, Z is -C(=O)-NH-. In some embodiments, p is an integer between 1 and 10. In some embodiments, p is an integer between 1 and 6 (such as 1, 2, 3, 4, 5, 6). In some embodiments, m is an integer between 1 and 12. In some embodiments, m is an integer between 1 and 10 (such as 1, 2, 3, 4, 5, 6, 7, 8, 9).

In some embodiments, in the conjugate represented by Formula (II), L₃ is 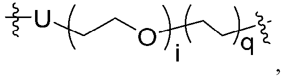 wherein U, i, and q are as defined in any embodiment of the present application. In some embodiments, U is - NH-C(=O)-, -N(CH₃)-C(=O)- or -N(CH₂CH₃)-C(=O)-. In some embodiments, U is -NH-C(=O)-. In some embodiments, the N atom in the U comes from the N atom in the E (e.g., an N atom on the side chain amino group of a lysine, arginine, asparagine, or glutamine residue). In some embodiments, i is an integer between 1 and 12. In some embodiments, i is an integer between 1 and 10 (such as 1, 2, 3, 4, 5, 6, 7, 8, 9). In some embodiments, q is an integer between 1 and 10. In some embodiments, q is an integer between 1 and 6 (such as 1, 2, 3, 4, 5, 6).

In some embodiments, in the conjugate represented by Formula (II), T is a number between 1 and 10. In some embodiments, in the conjugate represented by Formula (II), T is a number between 1 and 9. In some embodiments, in the conjugate represented by Formula (II), T is a number between 1 and 7. In some embodiments, in the conjugate represented by Formula (II), T is a number between 1 and 5. In some embodiments, in the conjugate represented by Formula (II), T is a number between 3 and 7. In some embodiments, in the conjugate represented by Formula (II), T is a number between 3 and 5.5.

In some embodiments, the conjugate represented by Formula (II) has a structure represented by Formula (II-II-A-3), Formula (II-II-B-5), Formula (II-II-B-6) or Formula (II-II-C-3), 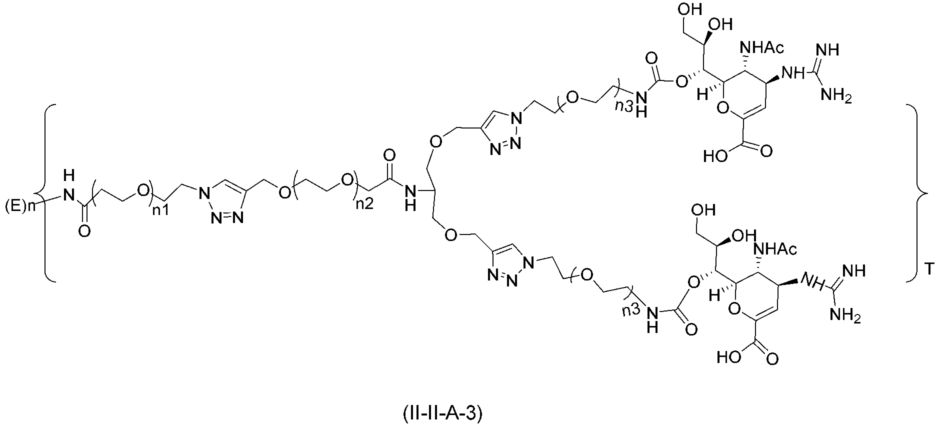 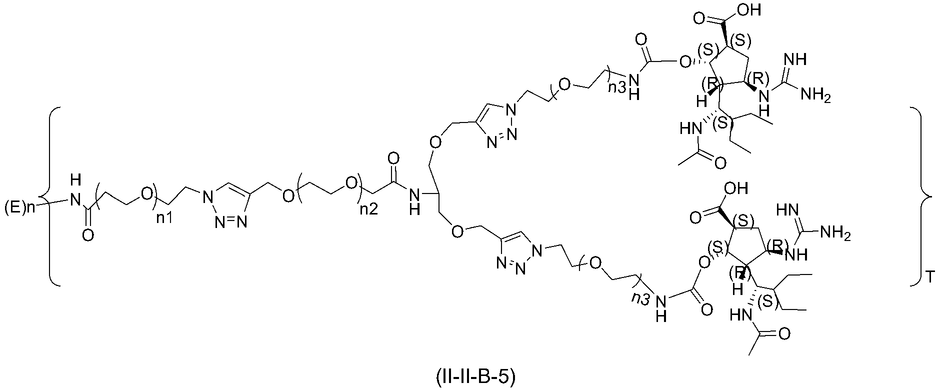 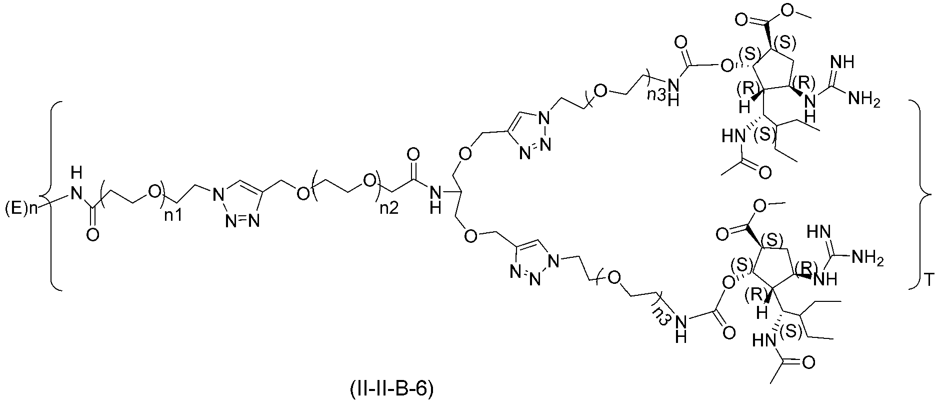 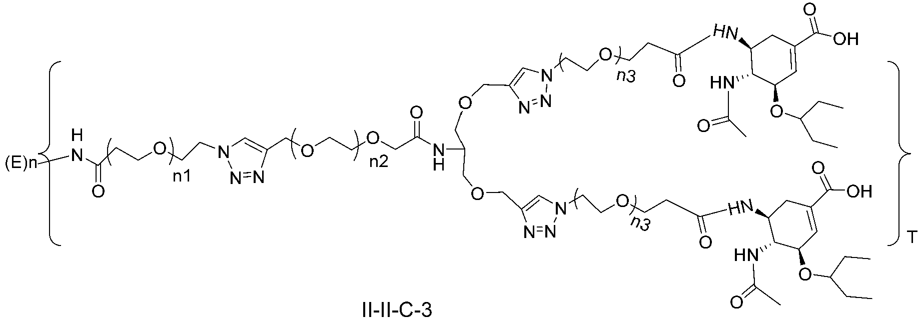 wherein E, n, and T are as defined in any embodiment of the present application; n1, n2, and n3 each independently are an integer between 1 and 20. In some embodiments, n1, n2, and n3 each independently are an integer between 1 and 12. In some embodiments, n1, n2, and n3 each independently are an integer between 1 and 10 (such as 1, 2, 3, 4, 5, 6, 7, 8, 9).

In some embodiments, the conjugate represented by Formula (II) is selected from the group consisting of: 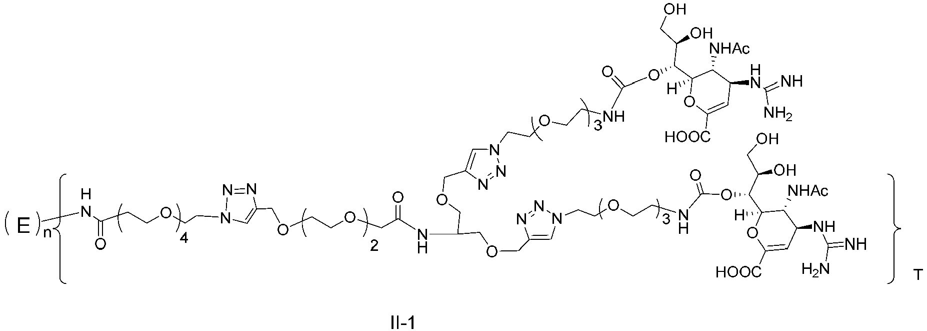 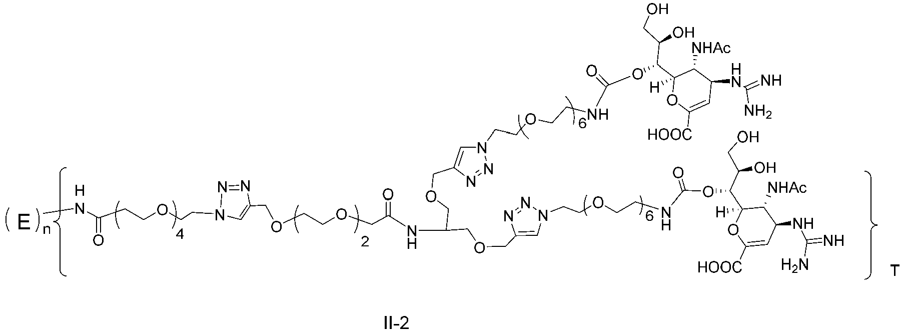 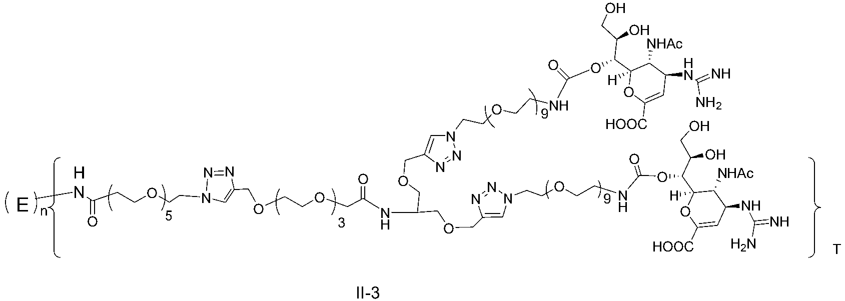 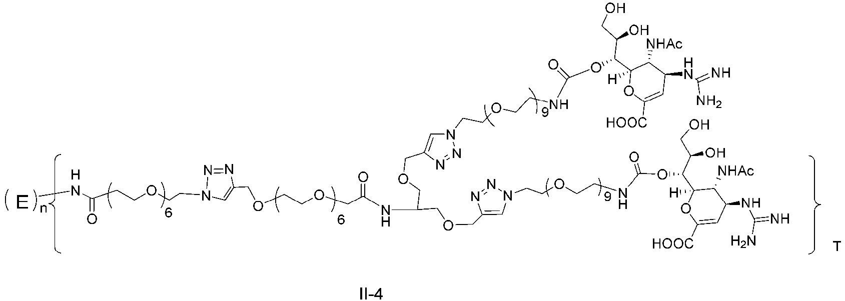 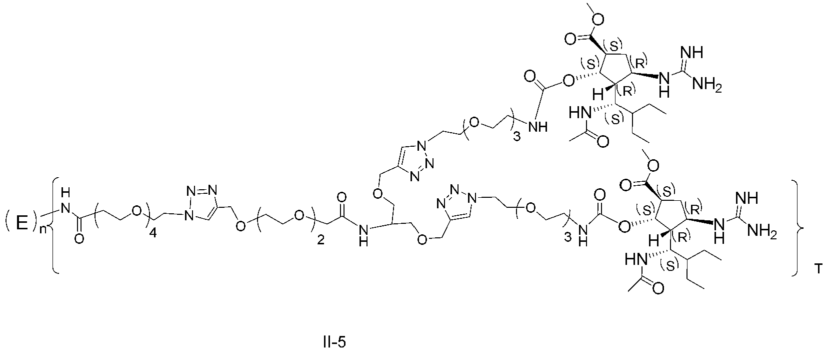 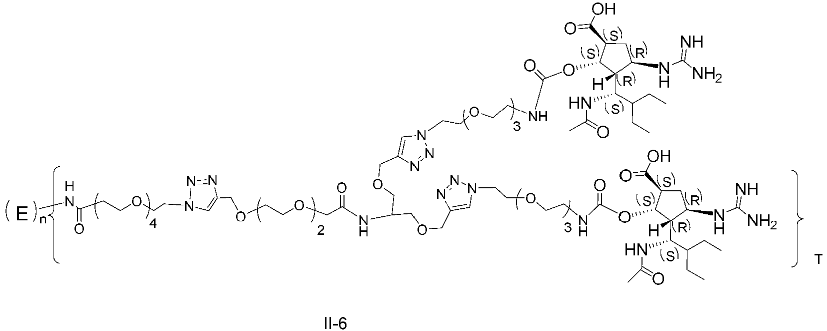 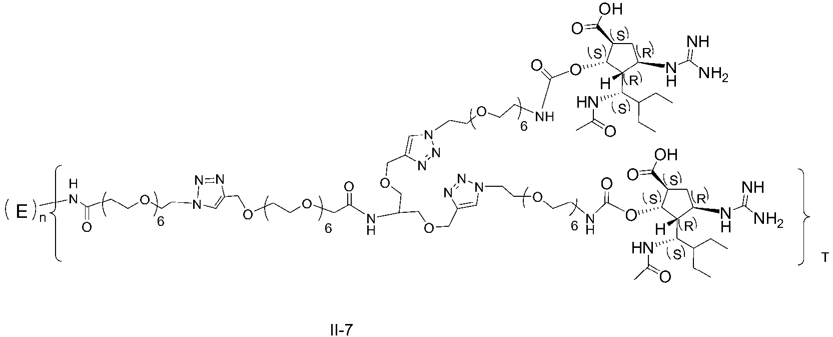 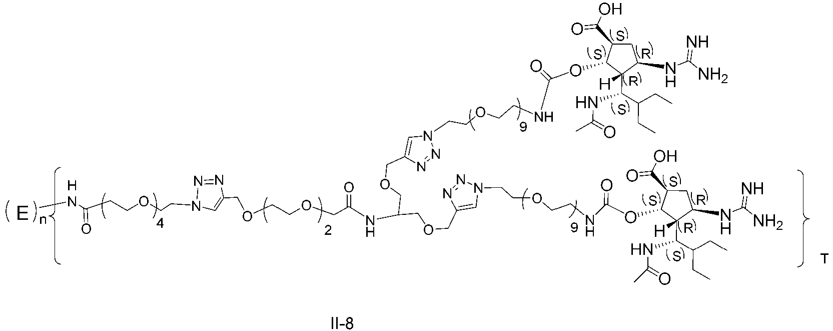 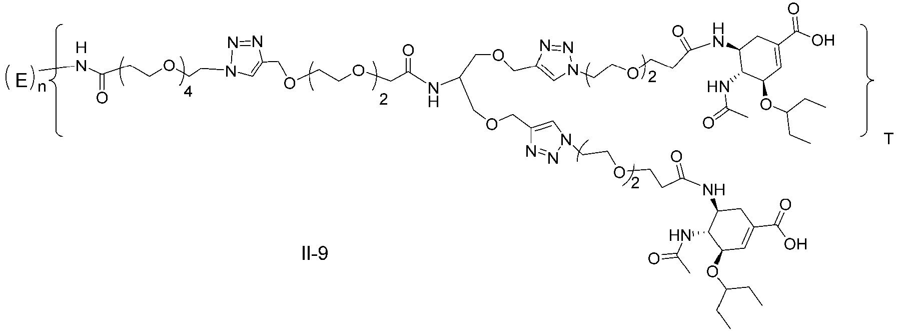 and 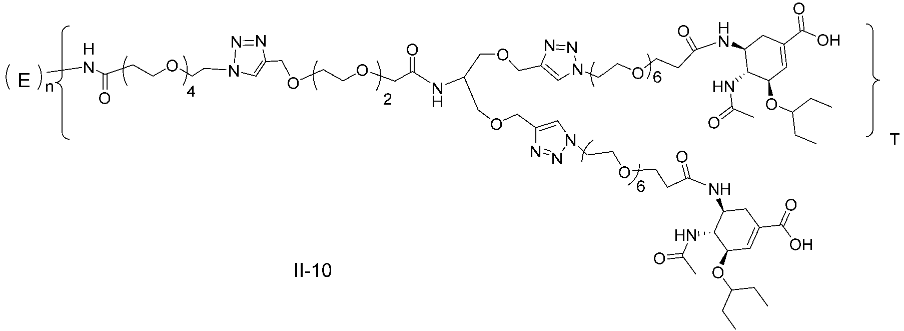 wherein E, n, and T are as defined in any embodiment of the present application.

In another aspect, the present application also provides a compound represented by Formula (IV) or a pharmaceutically acceptable salt, a geometric or optical isomer, a hydrate, a solvate or a polymorph thereof, 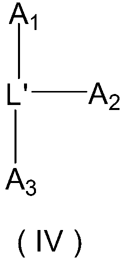
wherein A₁, A₂ and A₃ each independently are selected from the group consisting of Formula (A-I) to Formula (A-XIV), 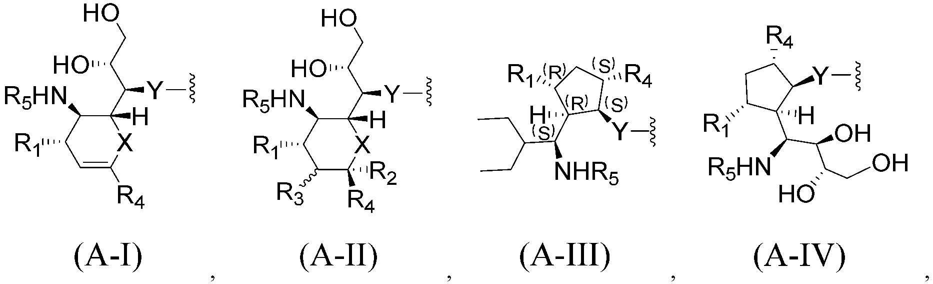 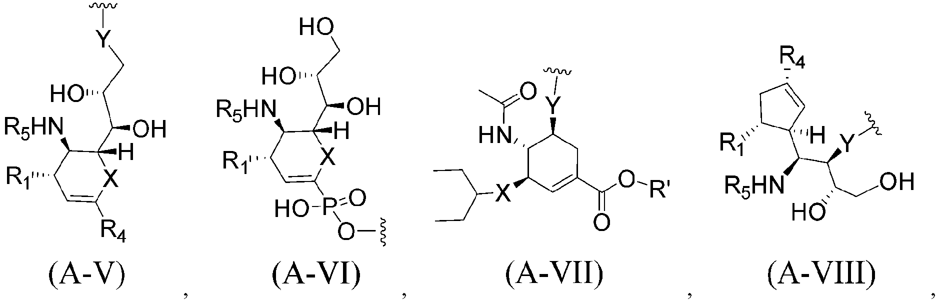 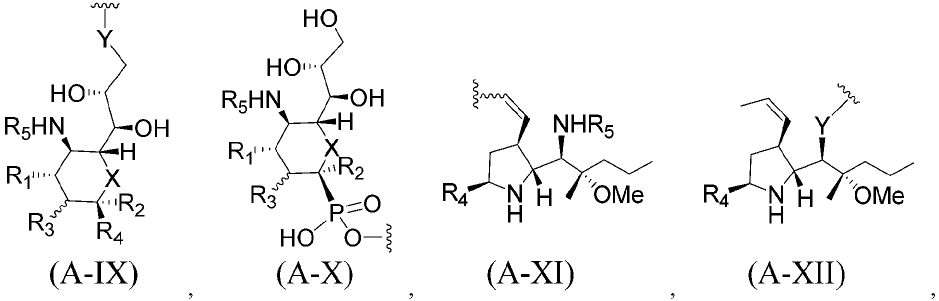 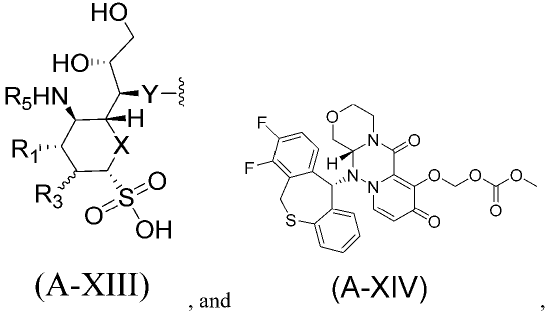
wherein, L may be linked to any possible sites of the compound of Formula (A-XIV);
wherein R₁ is selected from the group consisting of -OH, -NH₂, -NHC(=NH)NH₂ and -NHC(=NH)NHR₆;
R₂ and R₃ each independently are selected from the group consisting of -H, -OH, -F, -Cl and -Br;
R₄ is selected from the group consisting of -C(=O)OH, -P(=O)(OH)₂, -SO₃H, -C(=O)OCH₃ and -C(=O)OCF₃;
R₅ is selected from the group consisting of -C(=O)CH₃, -C(=O)CF₃, and -SO₂CH₃;
X is selected from the group consisting of -O- and -S-;
Y is selected from the group consisting of -O-, -S-, -NR₇-, -OC(=O)NR₇-, -OC(=S)NR₇-, -OC(=O)O-, -OC(=O)-, -NHC(=O)O-, -NHC(=O)-, -NHC(=NH)-, -NHC(=O)NR₇-, -NHC(=NH)NR₇-, -NHC(=S)NR₇-, -NHC(=S)-, -OCH₂C(=O)NR₇-, -NH(SO₂)- and -NH(SO₂)NR₇-;
R' is H or C₁-C₆ alkyl;
R₆ is selected from the group consisting of: 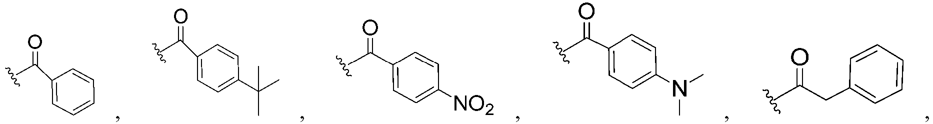 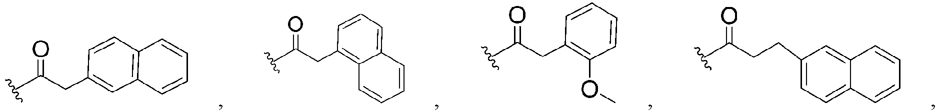 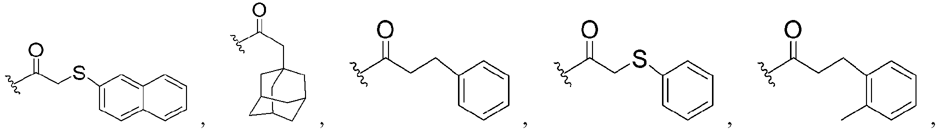 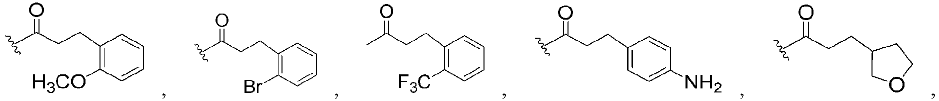 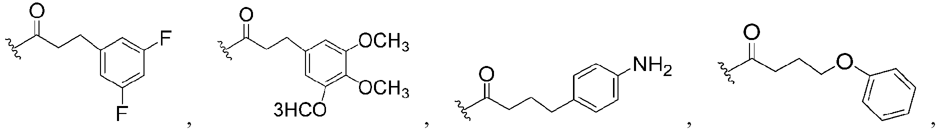 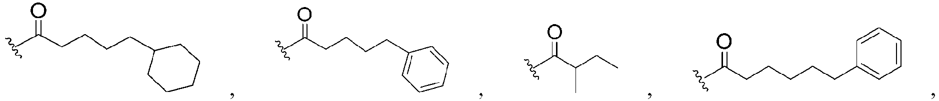 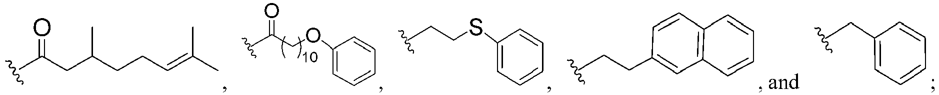
R₇ is selected from the group consisting of H, C₁-C₂₀ alkyl, C₃-C₂₀ cycloalkyl, C₃-C₂₀ heterocycloalkyl, C₅-C₁₅ aryl and C₅-C₁₅ heteroaryl; preferably, R₇ is H; preferably, R₇ is C₁-C₆ alkyl;
L' is a linker covalently linked to each of groups A₁, A₂ and A₃.

In some embodiments, in the compound represented by Formula (IV), A₁, A₂ and A₃ each independently are selected from the group consisting of Formula (A-I) to Formula (A-XIII), 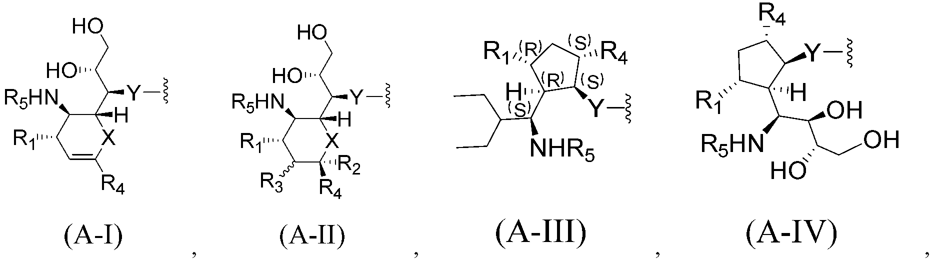 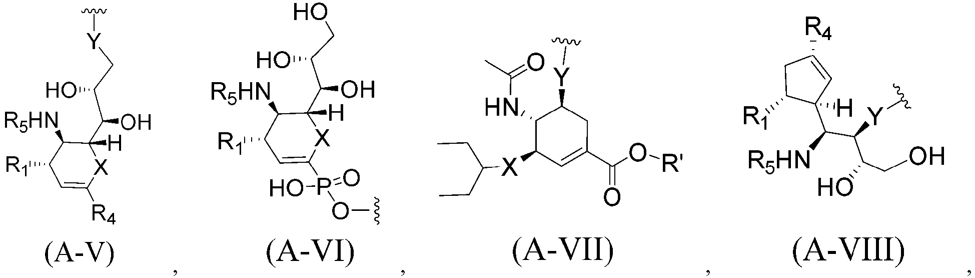 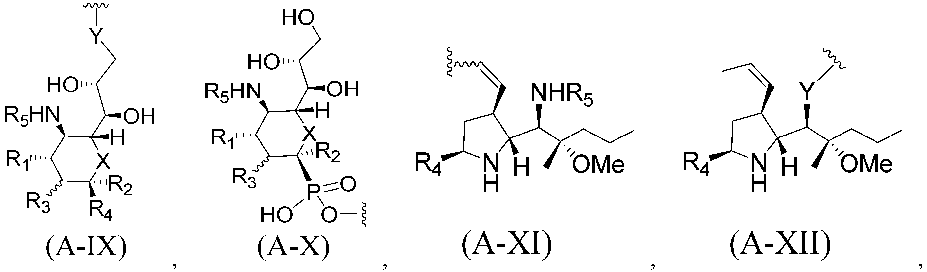 and 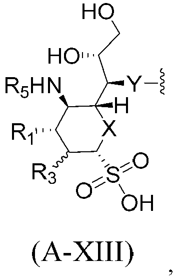 wherein R₁, R₂, R₃, R₄, R₅, R', X, and Y are as defined in any embodiment of the present application.

In some embodiments, in the compound represented by Formula (IV), A₁, A₂, A₃ each independently are selected from the group consisting of Formula (A-I) to Formula (A-IV) and Formula (A-VII), 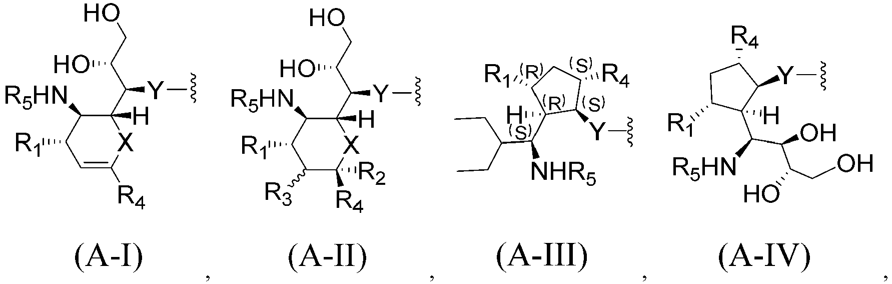 and 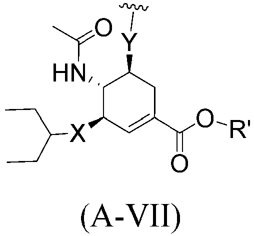 wherein R₁, R₂, R₃, R₄, R₅, R', X, and Y are as defined in any embodiment of the present application.

In some embodiments, in the compound represented by Formula (IV), A₁, A₂ and A₃ each independently are selected from the group consisting of Formula (A-I) to Formula (A-III) and Formula (A-VII), 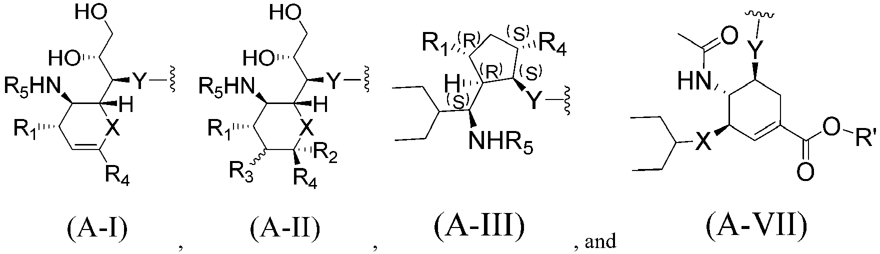 wherein R₁, R₂, R₃, R₄, R₅, R', X, and Y are as defined in any embodiment of the present application.

In some embodiments, in the compound represented by Formula (IV), A₁, A₂ and A₃ each independently are selected from the group consisting of Formula (A-I), Formula (A-III) and Formula (A-VII), 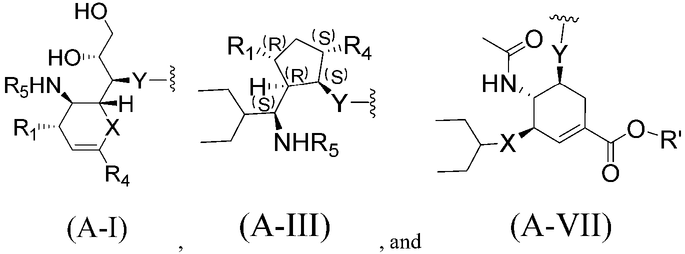 wherein R₁, R₄, R₅, R', X, and Y are as defined in any embodiment of the present application.

In some embodiments, in the compound represented by Formula (IV), R₁ is selected from the group consisting of -OH, -NH₂, and -NHC(=NH)NH₂. In some embodiments, in the compound represented by Formula (IV), R₁ is -NHC(=NH)NH₂.

In some embodiments, in the compound represented by Formula (IV), R₄ is selected from the group consisting of -C(=O)OH, -C(=O)OCH₃ and -C(=O)OCF₃. In some embodiments, in the compound represented by Formula (IV), R₄ is selected from the group consisting of -C(=O)OH and -C(=O)OCH₃.

In some embodiments, in the compound represented by Formula (IV), R₅ is selected from the group consisting of -C(=O)CH₃ and -C(=O)CF₃. In some embodiments, in the compound represented by Formula (IV), R₅ is -C(=O)CH₃.

In some embodiments, in the compound represented by Formula (IV), R' is H or C₁-C₄ alkyl. In some embodiments, in the compound represented by Formula (IV), R' is H, methyl or ethyl. In some embodiments, in the compound represented by Formula (IV), R' is H or ethyl.

In some embodiments, in the compound represented by Formula (IV), X is -O-.

In some embodiments, in the compound represented by Formula (IV), Y is selected from the group consisting of -O-, -S-, and -NH-. In some embodiments, in the compound represented by Formula (IV), Y is selected from the group consisting of -O- and -NH-.

In some embodiments, the compound represented by Formula (IV) has a structure represented by Formula (IV-I), 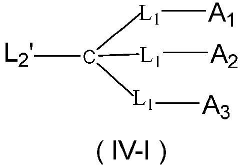
wherein A₁, A₂ and A₃ are as defined in any embodiment of the present application,
L₁ is selected from the group consisting of: 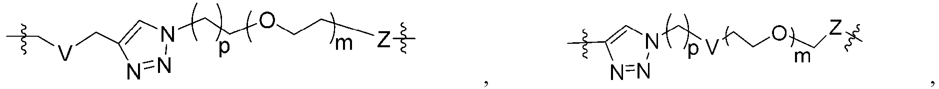 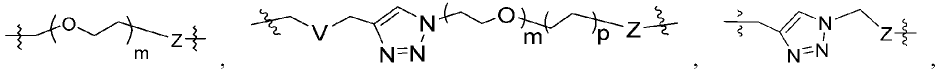 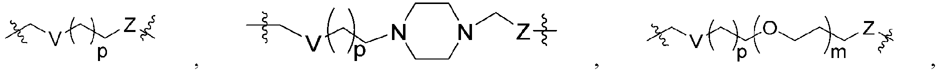 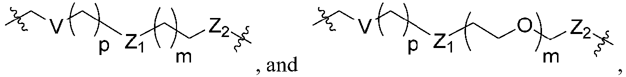
L₂' is selected from the group consisting of: 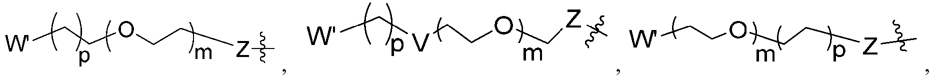 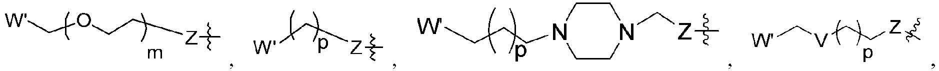 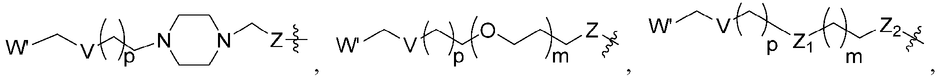 and 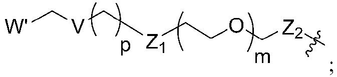
wherein V is -CH₂-, -O- or -S-; Z is -C(=O)-NH-, -CH₂-C(=O)-NH-, -NH-C(=O)-, -CH₂CH₂-C(=O)-, - N(CH₃)-C(=O)-, -N(CH₂CH₃)-C(=O)-, -O-C(=O)-, -C(=O)-, -CH₂- or 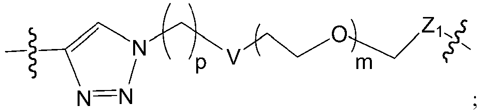 Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-; W' is 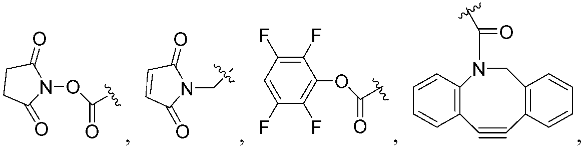 HC≡C-**,** -C(=O)OH, -NH₂ or N₃-; m and p each independently are an integer between 1 and 20.

In some embodiments, in the compound represented by Formula (IV) L₁ is selected from the group consisting of: 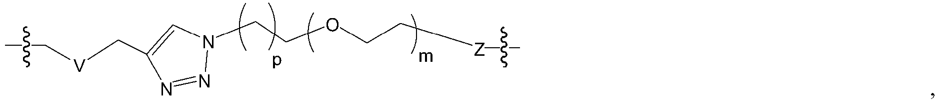 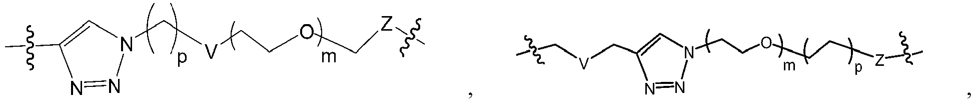 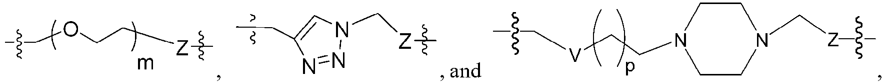 wherein V, p, m, and Z are as defined in any embodiment of the present application.

In some embodiments, in the compound represented by Formula (IV), L₁ is 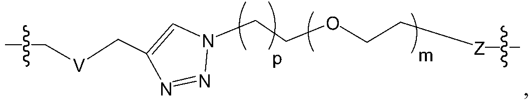 wherein V, p, m, and Z are as defined in any embodiment of the present application.

In some embodiments, in the compound represented by Formula (IV), L₂' is 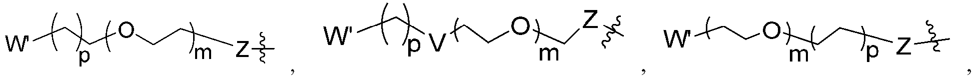 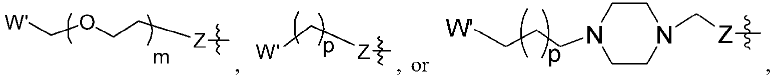 wherein W', V, p, m, and Z are as defined in any embodiment of the present application.

In some embodiments, in the compound represented by Formula (IV), L₂' is 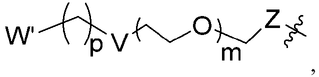 wherein W', V, p, m, and Z are as defined in any embodiment of the present application.

In some embodiments, in the compound represented by Formula (IV), L₂' is 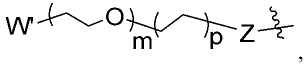 wherein W', p, m, and Z are as defined in any embodiment of the present application.

In some embodiments, in the compound represented by Formula (IV), V is -CH₂- or -O-. In some embodiments, in the compound represented by Formula (IV), V is -O-.

In some embodiments, in the compound represented by Formula (IV), Z is -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-.

In some embodiments, in the compound represented by Formula (IV), Z is 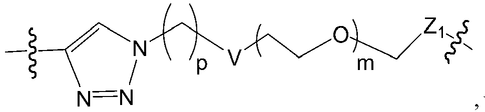 wherein p, V, m, and Z₁ are as defined in any embodiment of the present application.

In some embodiments, in the compound represented by Formula (IV), Z is -C(=O)-NH-, -CH₂-C(=O)-NH-, -NH-C(=O)-, -CH₂CH₂-C(=O)-, -N(CH₃)-C(=O)-, -N(CH₂CH₃)-C(=O)- or -C(=O)-. In some embodiments, in the compound represented by Formula (IV), Z is -NH-C(=O)- or -C(=O)-. In some embodiments, in the compound represented by Formula (IV), Z is -NH-C(=O)-.

In some embodiments, in the compound represented by Formula (IV), Z₁ is -C(=O)-NH-, -CH₂-C(=O)-NH-, -NH-C(=O)- or -CH₂CH₂-C(=O)-. In some embodiments, in the compound represented by Formula (IV), Z₁ is - C(=O)-NH- or -CH₂-C(=O)-NH-. In some embodiments, in the compound represented by Formula (IV), Z₁ is - C(=O)-NH-.

In some embodiments, in the compound represented by Formula (IV), W' is HC≡C-, -C(=O)OH, - NH₂ or N₃-. In some embodiments, in the compound represented by Formula (IV), W' is HC≡C- . In some embodiments, in the compound represented by Formula (IV), W' is

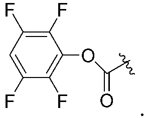

In some embodiments, in the compound represented by Formula (IV), p is an integer between 1 and 10. In some embodiments, in the compound represented by Formula (IV), p is an integer between 1 and 6 (such as 1, 2, 3, 4, 5, 6).

In some embodiments, in the compound represented by Formula (IV), m is an integer between 1 and 12. In some embodiments, in the compound represented by Formula (IV), m is an integer between 1 and 10 (such as 1, 2, 3, 4, 5, 6, 7, 8, 9).

In some embodiments, the compound represented by Formula (IV) has a structure represented by Formula (IV-I-1), Formula (IV-I-2), or Formula (IV-I-3): 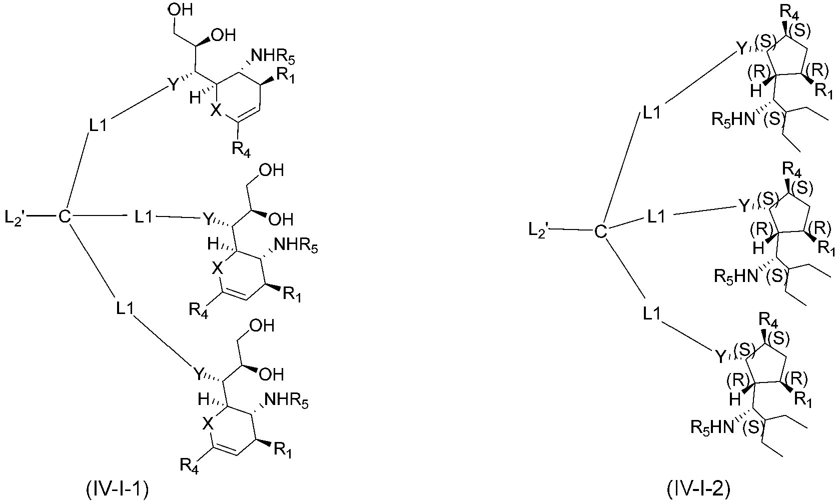 and 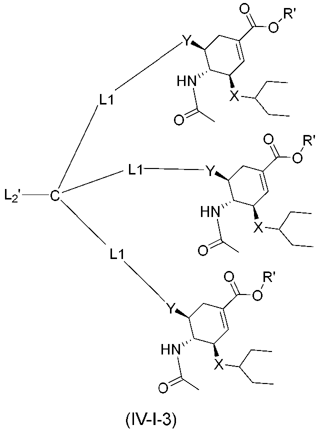 wherein R₁, R₄, R₅, R', X, Y, L₁, and L₂' are as defined in any embodiment of the present application.

In some embodiments, the compound represented by Formula (IV) has a structure represented by Formula (IV-I-1-1), Formula (IV-I-2-1), Formula (IV-I-2-2) or Formula (IV-I-3-1): 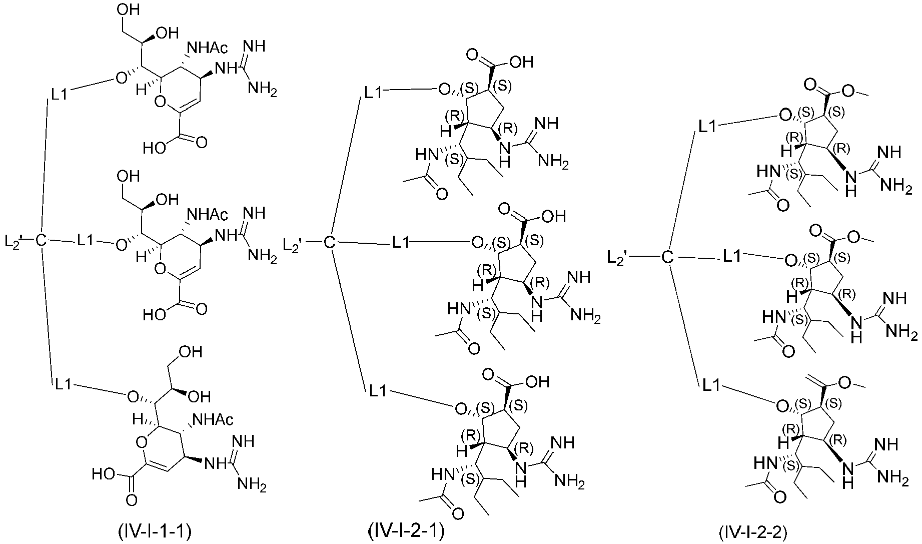 and 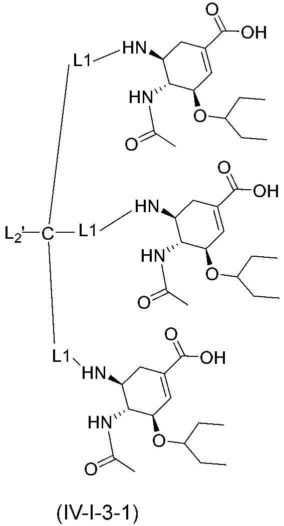 wherein L₁ and L₂' are as defined in any embodiment of the present application.

In some embodiments, the compound represented by Formula (IV) is selected from the group consisting of:

| Intermediates | Structures |
|---|---|
| Int-11 | 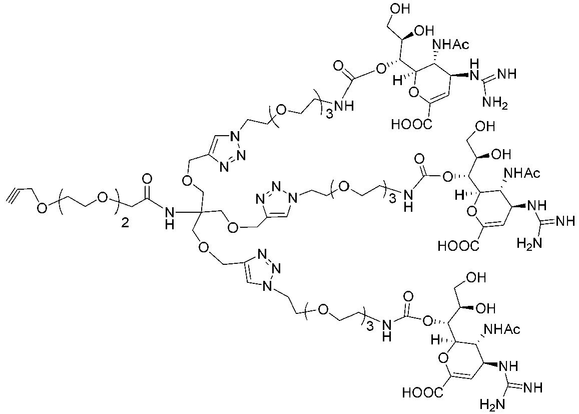 |
| Int-12 | 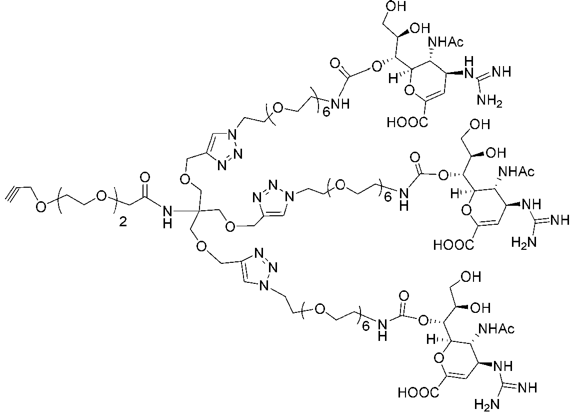 |
| Int-13 | 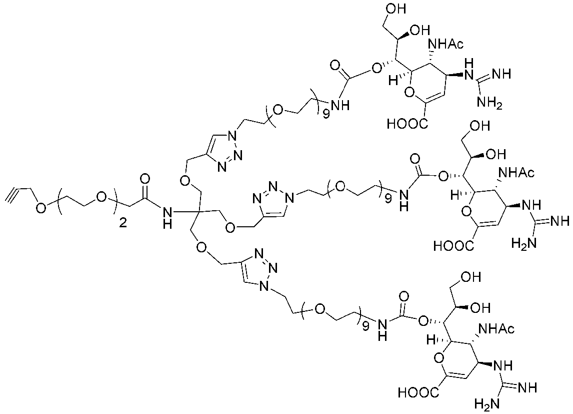 |
| Int-14 | 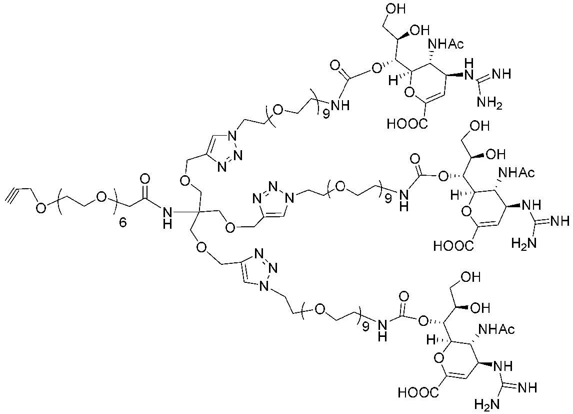 |
| Int-15 | 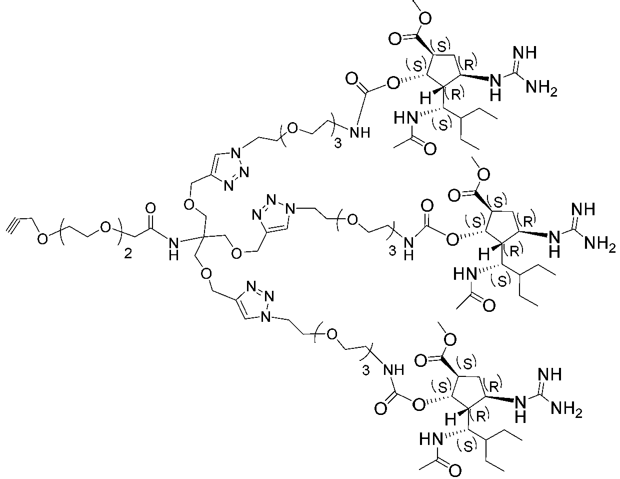 |
| Int-16 | 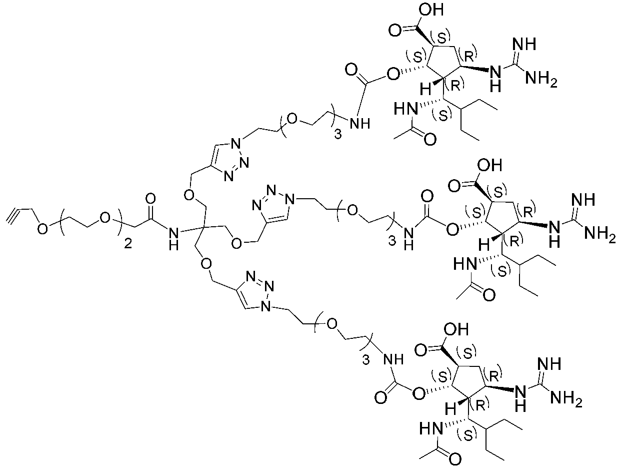 |
| Int-17 | 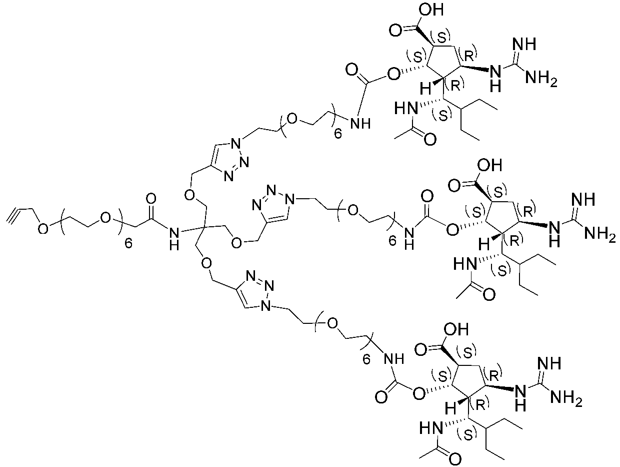 |
| Int-18 | 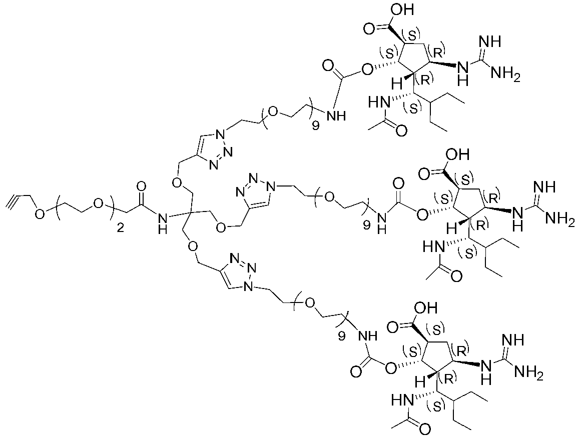 |
| Int-19 | 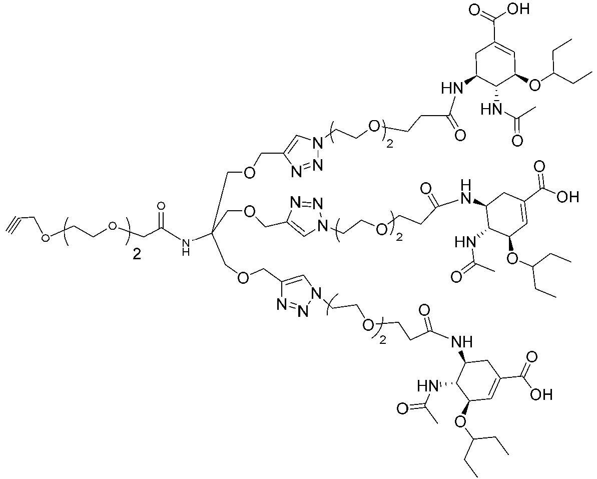 |
| Int-20 | 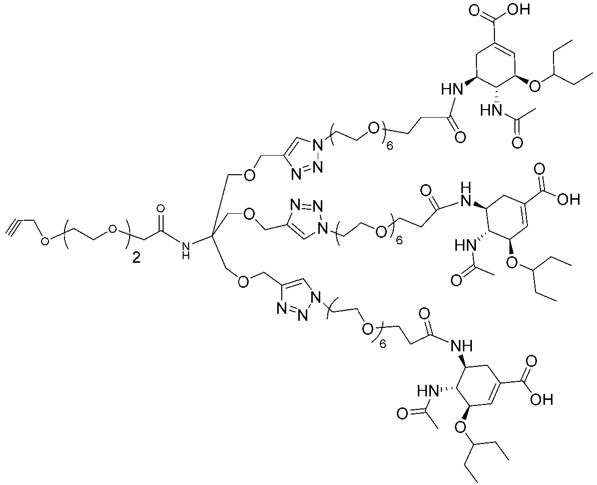 |
| Int-16-A | 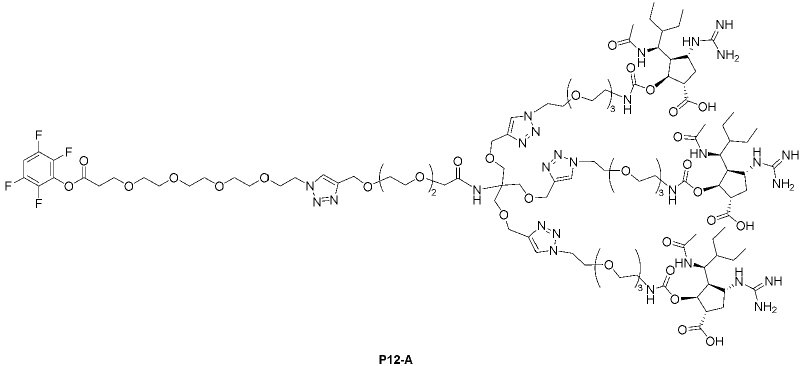 |

In another aspect, the present application also provides a compound represented by Formula (V) or a pharmaceutically acceptable salt, a geometric or optical isomer, a hydrate, a solvate or a polymorph thereof, 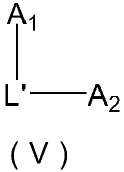
wherein A₁ and A₂ each independently are selected from the group consisting of Formula (A-I) to Formula (A-XIV), 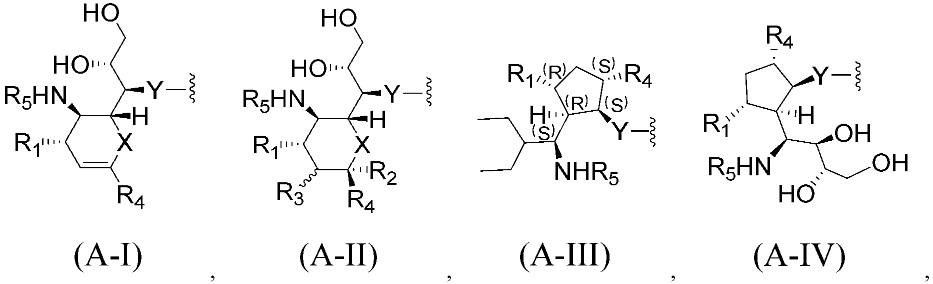 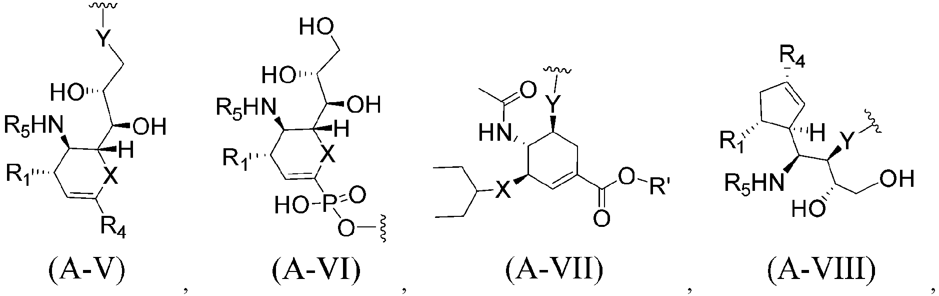 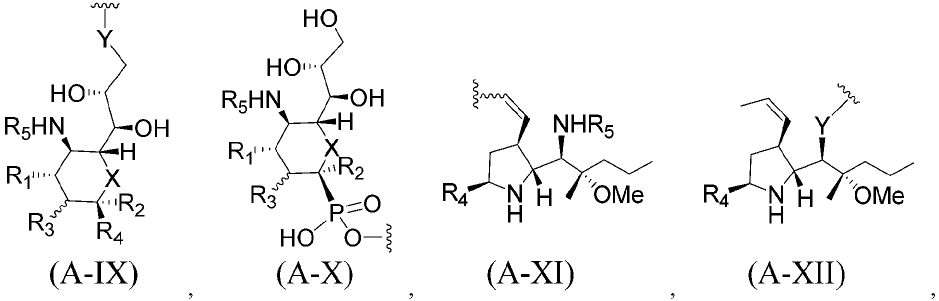 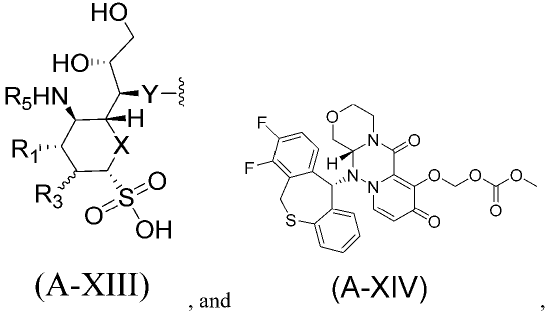
wherein, L may be linked to any possible sites of the compound of Formula (A-XIV); wherein R₁ is selected from the group consisting of -OH, -NH₂, -NHC(=NH)NH₂ and -NHC(=NH)NHR₆;
R₂ and R₃ each independently are selected from the group consisting of -H, -OH, -F, -Cl and -Br;
R₄ is selected from the group consisting of -C(=O)OH, -P(=O)(OH)₂, -SO₃H, -C(=O)OCH₃ and -C(=O)OCF₃;
R₅ is selected from the group consisting of -C(=O)CH₃, -C(=O)CF₃, and -SO₂CH₃;
X is selected from the group consisting of -O- and -S-;
Y is selected from the group consisting of -O-, -S-, -NR₇-, -OC(=O)NR₇-, -OC(=S)NR₇-, -OC(=O)O-, -OC(=O)-, -NHC(=O)O-, -NHC(=O)-, -NHC(=NH)-, -NHC(=O)NR₇-, -NHC(=NH)NR₇-, -NHC(=S)NR₇-, -NHC(=S)-, -OCH₂C(=O)NR₇-, -NH(SO₂)- and -NH(SO₂)NR₇-;
R' is H or C₁-C₆ alkyl;
R₆ is selected from the group consisting of 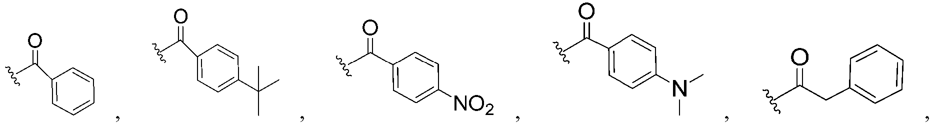 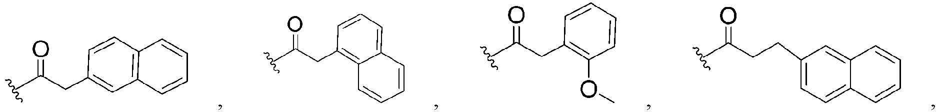 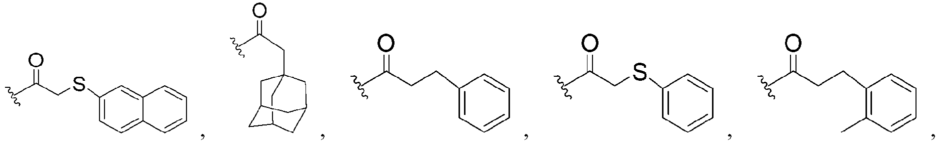 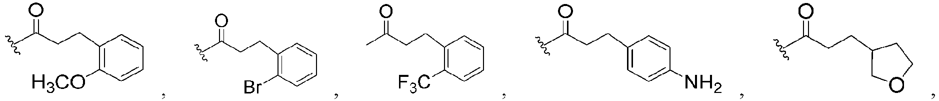 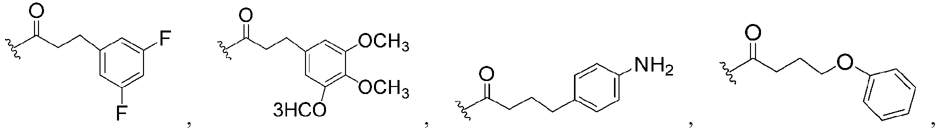 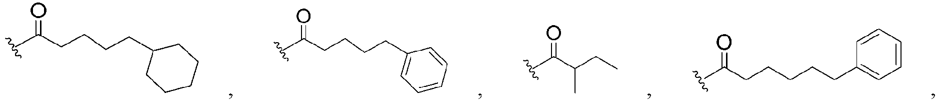 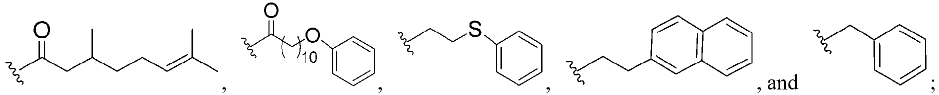
R₇ is selected from the group consisting of H, C₁-C₂₀ alkyl, C₃-C₂₀ cycloalkyl, C₃-C₂₀ heterocycloalkyl, C₅-C₁₅ aryl and C₅-C₁₅ heteroaryl; preferably, R₇ is H; preferably, R₇ is C₁-C₆ alkyl;
L' is a linker covalently linked to each of groups A₁ and A₂.

In some embodiments, in the compound represented by Formula (V), A₁ and A₂ each independently are selected from the group consisting of Formula (A-I) to Formula (A-XIII), 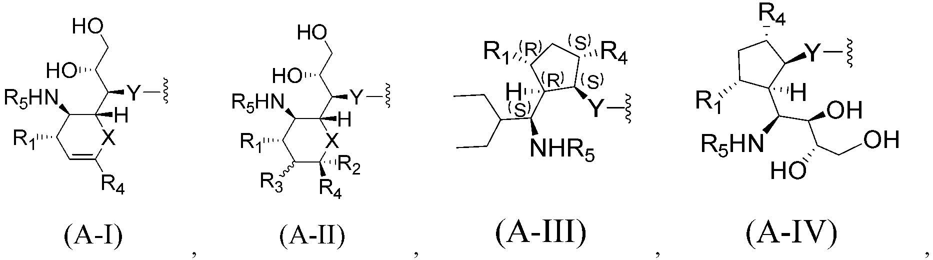 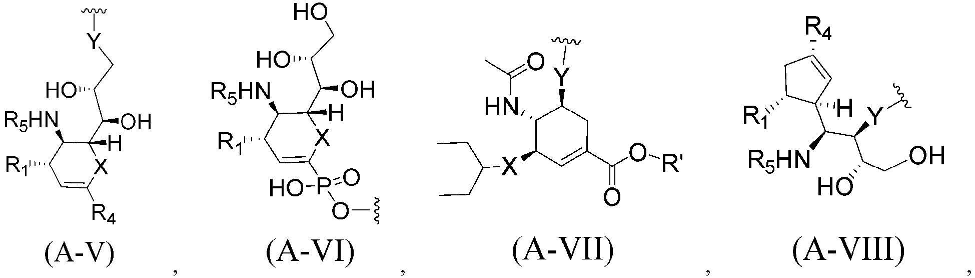 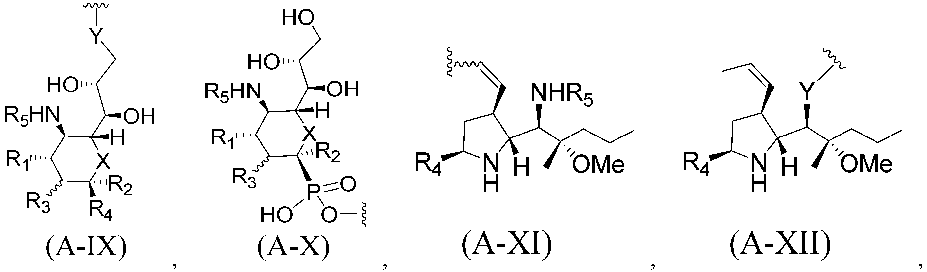 and 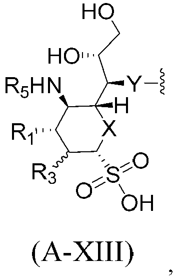 wherein R₁, R₂, R₃, R₄, R₅, R', X, and Y are as defined in any embodiment of the present application.

In some embodiments, in the compound represented by Formula (V), A₁ and A₂ each independently are selected from the group consisting of Formula (A-I) to Formula (A-IV) and Formula (A-VII), 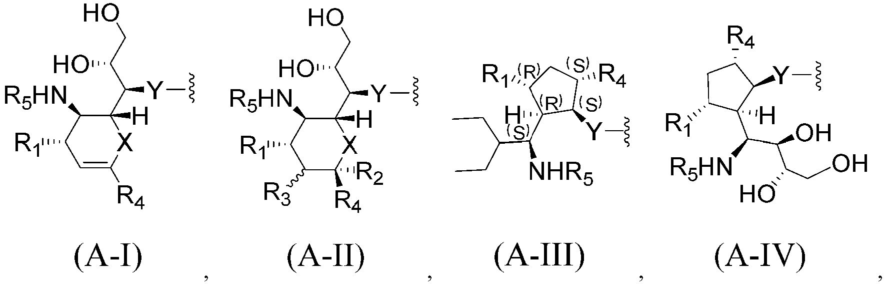 and 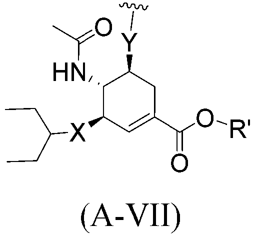 wherein R₁, R₂, R₃, R₄, R₅, R', X, and Y are as defined in any embodiment of the present application.

In some embodiments, in the compound represented by Formula (V), A₁ and A₂ each independently are selected from the group consisting of Formula (A-I) to Formula (A-III) and Formula (A-VII), 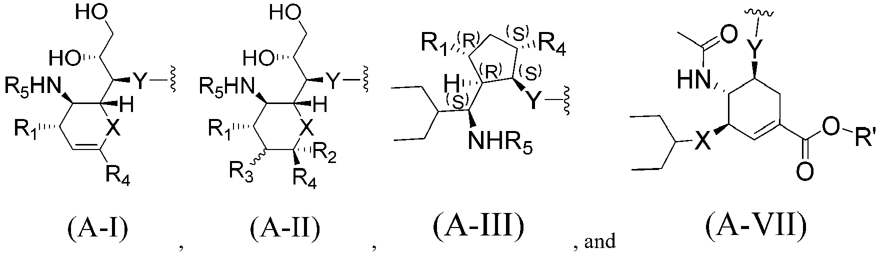 wherein R₁, R₂, R₃, R₄, R₅, R', X, and Y are as defined in any embodiment of the present application.

In some embodiments, in the compound represented by Formula (V), A₁ and A₂ each independently are selected from the group consisting of Formula (A-I), Formula (A-III) and Formula (A-VII), 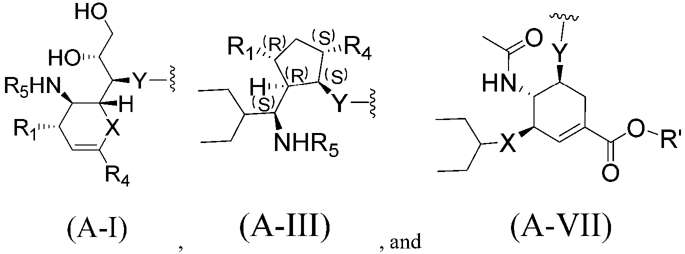 wherein R₁, R₄, R₅, R', X, and Y are as defined in any embodiment of the present application.

In some embodiments, in the compound represented by Formula (V), R₁ is selected from the group consisting of -OH, -NH₂, and -NHC(=NH)NH₂. In some embodiments, in the compound represented by Formula (V), R₁ is -NHC(=NH)NH₂.

In some embodiments, in the compound represented by Formula (V), R₄ is selected from the group consisting of -C(=O)OH, -C(=O)OCH₃ and -C(=O)OCF₃. In some embodiments, in the compound represented by Formula (V), R₄ is selected from the group consisting of -C(=O)OH and -C(=O)OCH₃.

In some embodiments, in the compound represented by Formula (V), R₅ is selected from the group consisting of -C(=O)CH₃ and -C(=O)CF₃. In some embodiments, in the compound represented by Formula (V), R₅ is -C(=O)CH₃.

In some embodiments, in the compound represented by Formula (V), R' is H or C₁-C₄ alkyl. In some embodiments, in the compound represented by Formula (V), R' is H, methyl or ethyl. In some embodiments, in the compound represented by Formula (V), R' is H or ethyl.

In some embodiments, in the compound represented by Formula (V), X is -O-.

In some embodiments, in the compound represented by Formula (V), Y is selected from the group consisting of -O-, -S-, and -NH-. In some embodiments, in the compound represented by Formula (V), Y is selected from the group consisting of -O- and -NH-.

In some embodiments, the compound represented by Formula (V) has a structure represented by Formula (V-I), 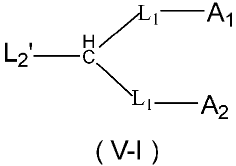
wherein A₁ and A₂ are as defined in any embodiment of the present application,
L₁ is selected from the group consisting of: 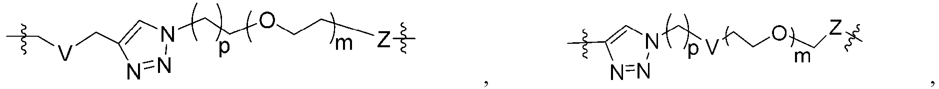 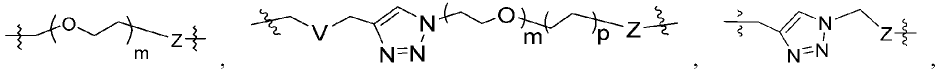 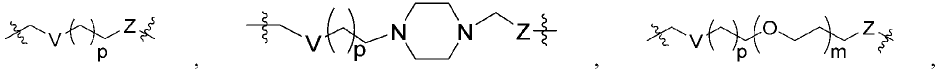 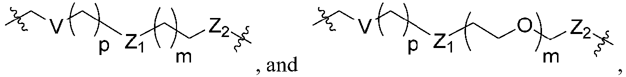
L₂' is selected from the group consisting of: 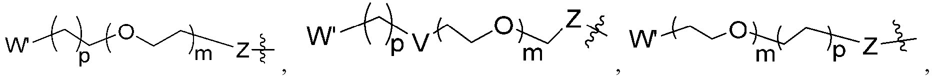 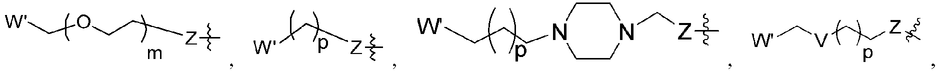 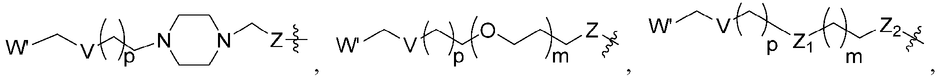 and 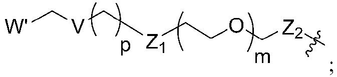
wherein V is -CH₂-, -O- or -S-; Z is -C(=O)-NH-, -CH₂-C(=O)-NH-, -NH-C(=O)-, -CH₂CH₂-C(=O)-, - N(CH₃)-C(=O)-, -N(CH₂CH₃)-C(=O)-, -O-C(=O)-, -C(=O)- or -CH₂-; Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-; W' is 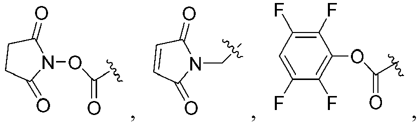 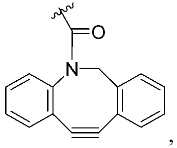 HC≡C-, -C(=O)OH, -NH₂ or N₃-; m and p each independently are an integer between 1 and 20.

In some embodiments, in the compound represented by Formula (V), L₁ is selected from the group consisting of: 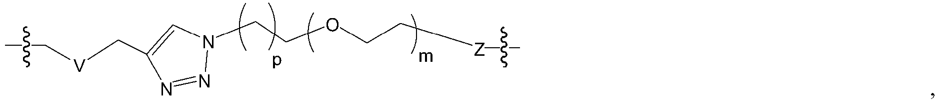 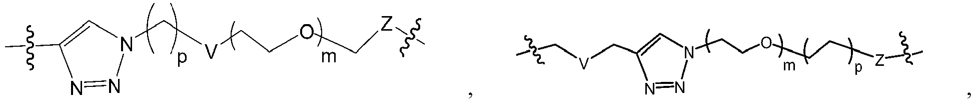 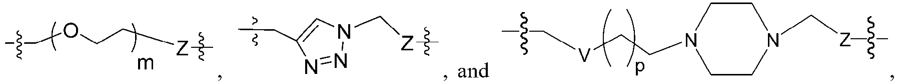 wherein V, p, m, and Z are as defined in any embodiment of the present application.

In some embodiments, in the compound represented by Formula (V), L₁ is 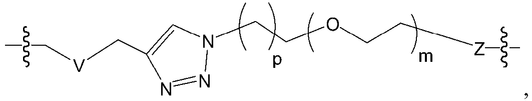 wherein V, p, m, and Z are as defined in any embodiment of the present application.

In some embodiments, in the compound represented by Formula (V), L₂' is 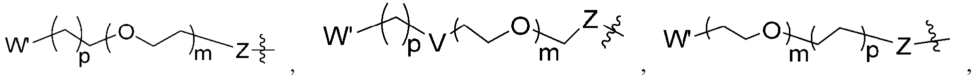 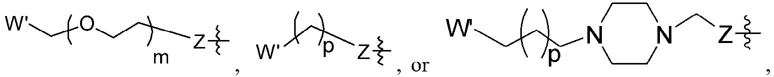 wherein W', V, p, m, and Z are as defined in any embodiment of the present application.

In some embodiments, in the compound represented by Formula (V), L₂' is 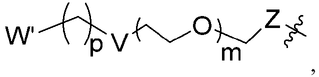 wherein W', V, p, m, and Z are as defined in any embodiment of the present application.

In some embodiments, in the compound represented by Formula (V), V is -CH₂- or -O-. In some embodiments, in the compound represented by Formula (V), V is -O-.

In some embodiments, in the compound represented by Formula (V), Z is -C(=O)-NH-, -CH₂-C(=O)-NH-, -NH-C(=O)-, -CH₂CH₂-C(=O)-, -N(CH₃)-C(=O)-, -N(CH₂CH₃)-C(=O)- or -C(=O)-. In some embodiments, in the compound represented by Formula (V), Z is -NH-C(=O)- or -C(=O)-.

In some embodiments, in the compound represented by Formula (V), W' is HC≡C-, -C(=O)OH, - NH₂ or N₃-. In some embodiments, in the compound represented by Formula (V), W' is HC≡C-.

In some embodiments, in the compound represented by Formula (V), p is an integer between 1 and 10. In some embodiments, in the compound represented by Formula (V), p is an integer between 1 and 6 (such as 1, 2, 3, 4, 5, 6).

In some embodiments, in the compound represented by Formula (V), m is an integer between 1 and 12. In some embodiments, in the compound represented by Formula (V), m is an integer between 1 and 10 (such as 1, 2, 3, 4, 5, 6, 7, 8, 9).

In some embodiments, the compound represented by Formula (V) has a structure represented by Formula (V-I-1), Formula (V-I-2), or Formula (V-I-3): 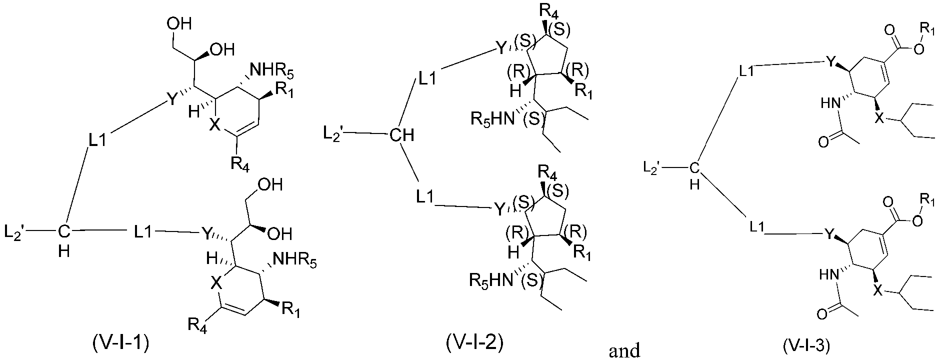 wherein R₁, R₄, R₅, R', X, Y, L₁, and L₂' are as defined in any embodiment of the present application.

In some embodiments, the compound represented by Formula (V) has a structure represented by Formula (V-I-1-1), Formula (V-I-2-1), Formula (V-I-2-2) or Formula (V-I-3-1): 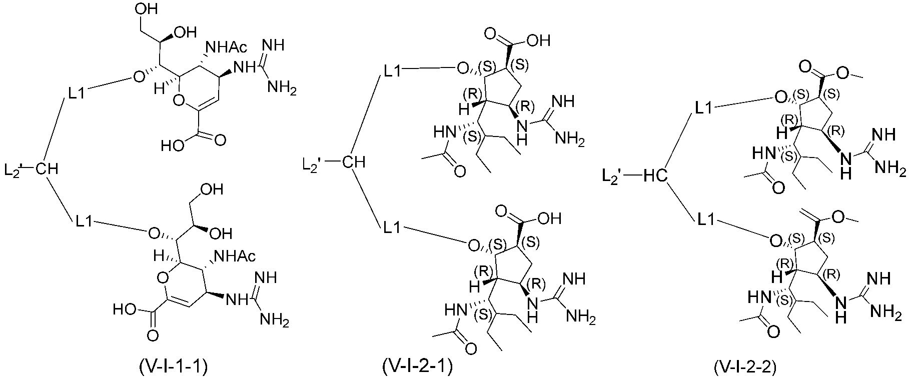 and 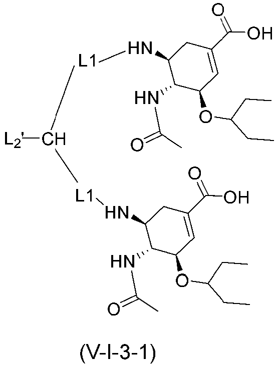 wherein Li and L₂' are as defined in any embodiment of the present application.

In some embodiments, the compound represented by Formula (V) is selected from the group consisting of:

| Intermediates | Structures |
|---|---|
| Int-1 | 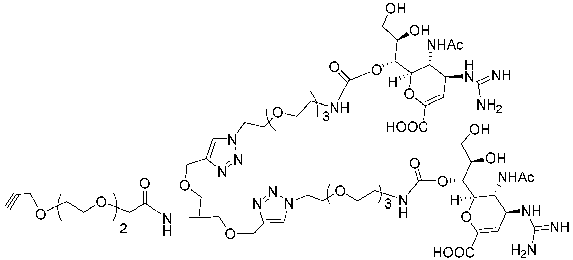 |
| Int-2 | 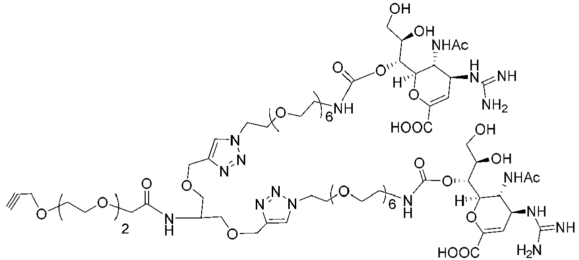 |
| Int-3 | 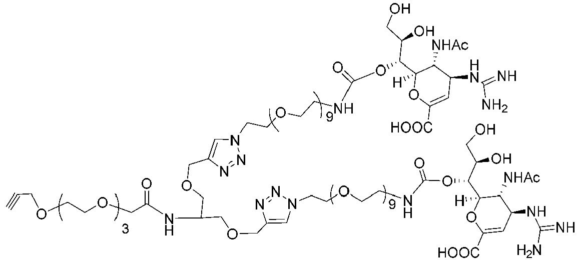 |
| Int-4 | 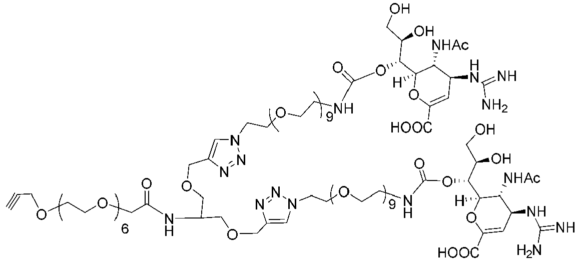 |
| Int-5 | 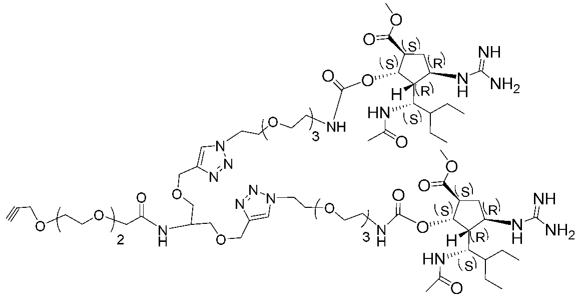 |
| Int-6 | 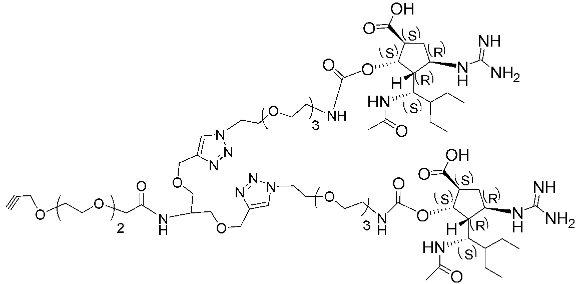 |
| Int-7 | 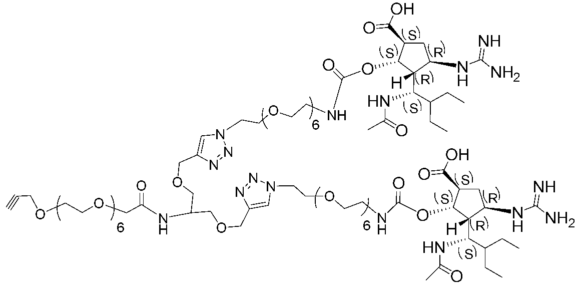 |
| Int-8 | 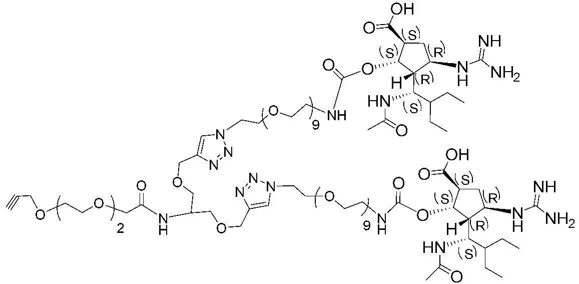 |
| Int-9 | 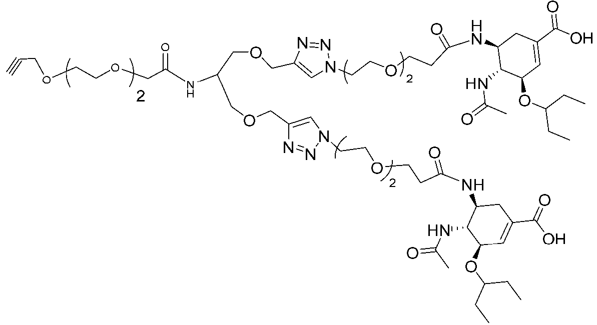 |
| Int-10 | 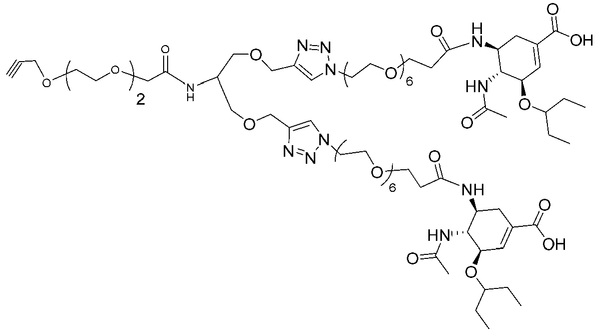 |

In another aspect, the present application also provides a pharmaceutical composition, comprising the conjugate represented by Formula (I) or (II) or a pharmaceutically acceptable salt, a geometric or optical isomer, a hydrate, a solvate or a polymorph thereof, and a pharmaceutically acceptable carrier and/or excipient.

In another aspect, the present application also provides a pharmaceutical composition, comprising the compound represented by Formula (IV) or (V) or a salt, a geometric or optical isomer, a hydrate, a solvate or a polymorph thereof, and a pharmaceutically acceptable carrier and/or excipient.

In another aspect, the present application also provides the use of the conjugate represented by Formula (I) or (II) or a pharmaceutically acceptable salt, a geometric or optical isomer, a hydrate, a solvate or a polymorph thereof in the manufacture of a medicament for treating or preventing a viral infection.

In another aspect, the present application also provides the use of the compound represented by Formula (IV) or (V) or a salt, a geometric or optical isomer, a hydrate, a solvate or a polymorph thereof in the manufacture of a medicament for treating or preventing a viral infection.

In another aspect, the present application also provides the use of the pharmaceutical composition in the manufacture of a medicament for treating or preventing a viral infection.

In another aspect, the present application also provides a method for treating a subject having a viral infection or suspected of having a viral infection, comprising administering to the subject an effective amount of the conjugate represented by Formula (I) or (II) or a pharmaceutically acceptable salt, a geometric or optical isomer, a hydrate, a solvate or a polymorph thereof according to the present application.

In another aspect, the present application also provides a method for treating a subject having a viral infection or suspected of having a viral infection, comprising administering to the subject an effective amount of the compound represented by Formula (IV) or (V) or a salt, a geometric or optical isomer, a hydrate, a solvate or a polymorph thereof according to the present application.

In another aspect, the present application also provides a method for prophylactically treating a viral infection in a subject in need thereof, comprising administering to the subject an effective amount of the conjugate of Formula (I) or Formula (II) or a pharmaceutically acceptable salt, a geometric or optical isomer, a hydrate, a solvate or a polymorph thereof according to the present application.

In some embodiments, the viral infection is an infection caused by influenza virus or parainfluenza virus. In some embodiments, the viral infection is an infection caused by influenza A, B or C viruses or parainfluenza virus. In some embodiments, the viral infection is an infection caused by avian influenza.

In some embodiments, the subject may be further treated with an antiviral agent, wherein the antiviral agent is selected from the group consisting of oseltamivir, zanamivir, peramivir, lanimivir, amantadine, rimantadine, and baloxavir marboxil.

In some embodiments, the subject is immunocompromised. In some embodiments, the subject has been diagnosed with humoral immunodeficiency, T cell deficiency, neutropenia, asplenia, or complement deficiency. In some embodiments, the subject is receiving or is about to receive immunosuppressive therapy. In some embodiments, the subject has been diagnosed with a disease that causes immunosuppression. In some embodiments, the disease is cancer or acquired immunodeficiency syndrome. In some embodiments, the cancer is leukemia, lymphoma, or multiple myeloma. In some embodiments, the subject has been subject to or is about to be subjected to hematopoietic stem cell transplantation. In some embodiments, the subject has been subjected to or is about to be subjected to organ transplant.

In some embodiments, the conjugate as represented by Formula (I) or Formula (II) according to the present application is capable of binding one or more targets (e.g., antigens). In some embodiments, the target is viral (e.g., influenza) protein, such as neuraminidase or hemagglutinin.

In some embodiments, the conjugate of Formula (I) or Formula (II) or a pharmaceutically acceptable salt, a geometric or optical isomer, a hydrate, a solvate or a polymorph thereof or the composition is administrated intramuscularly, intravenously, intradermally, intraarterially, intraperitoneally, intralesionally, intracranially, intraarticularly, intraprostatically, intrapleurally, intratracheally, intranasally, intravitreally, intravaginally, intrarectally, topically, intratumorally, peritoneally, subcutaneously, subconjunctival, intracapsularly, transmucosally, intrapericardially, intraumbilically, intraocularly, orally, topically, by inhalation, by injection, or by infusion.

In another aspect, the present application also provides the use of the compound represented by Formula (IV) or (V) or a salt, a geometric or optical isomer, a hydrate, a solvate or a polymorph thereof as an intermediate in the preparation of a conjugate. The compound represented by Formula (IV) or (V), as an intermediate, may be conjugated to an Fc domain or an Fc domain monomer (e.g., via a linker) by any suitable method known to those skilled in the art, including any method as described or exemplified in the present application. In some embodiments, the conjugate (e.g., the conjugate represented by Formula (I) or Formula (II)) comprises E, wherein the E is an Fc domain monomer (when n is 2, E dimerizes to form an Fc domain, and each Fc domain monomer independently has a sequence as set forth in any one of SEQ ID NO: 1-68). In preferred embodiments, one or more nitrogen atoms of one or more surface-exposed lysine residues of the E are covalently conjugated to a linker (e.g., PEG2-PEG20 linker). The linker conjugated to the E may be functionalized such that it can react to form a covalent bond with any intermediate as described herein (e.g., the compound represented by Formula (IV) or Formula (V)). In preferred embodiments, the E is conjugated to an azide-functionalized linker and the intermediate (e.g., the compound represented by Formula (IV) or Formula (V)) is alkyne-functionalized. The E is conjugated to the intermediate via the azide group of the linker and the alkyne group of the intermediate (e.g., via click chemistry) to form the conjugate of the present application, such as the conjugate represented by Formula (I) or Formula (II).

In some embodiments, in the conjugate of the present application, each E is an Fc domain monomer, comprising a hinge domain, a CH2 antibody constant domain, and a CH3 antibody constant domain. The Fc domain monomer may be of immunoglobulin antibody isotype IgG. A variant Fc domain monomer also may be derived from any immunoglobulin isotype (e.g., IgG1). The variant Fc domain monomer may be of any immunoglobulin antibody allotype (e.g. IGHG1*01 *(i.e.,* G1m(za)), IGHG1*07 *(i.e.,* G1m(zax)), IGHG1*04 (*i.e.,* G1m(zav)), IGHG1*03 (G1m(f)), IGHG1*08 *(i.e.,* G1m(fa)), IGHG2*01, IGHG2*06 or IGHG2*02) (as described, for example, in Vidarsson et al, IgG subclasses and allotypes: from structure to effector function. Frontiers in Immunology. 5(520): 1-17 (2014)). The variant Fc domain monomer may also be of any species, e.g., human, murine, or mouse. A dimer of the variant Fc domain monomers is a variant Fc domain that can bind to an Fc receptor, which is a receptor located on the surface of leukocytes.

In some embodiments, the Fc domain monomer comprises the quadruple mutation C220S/M252Y/S254T/T256E as compared with a wild-type Fc domain monomer. In some embodiments, the Fc domain monomer comprises the quadruple mutation C220S/V309D/Q311H/N434S as compared with a wild-type Fc domain monomer. In other embodiments, the Fc domain monomer comprises C220S mutation as compared with a wild-type Fc domain monomer. The mutations are defined with reference to the amino acid sequence of a wild-type Fc monomer, such as wild-type human IgG1.

In some embodiments, the Fc domain monomer in the conjugate according to the present application may comprise substitution, addition and/or deletion of one or more amino acid as compared with the Fc domain monomer having any one of the sequences as set forth in SEQ ID NO:1-68. In some embodiments, the Fc domain monomer may comprise substitution, addition and/or deletion of one or more amino acid as compared with the Fc domain monomer having any one of the sequences as set forth in SEQ ID NO:1-29, 31-52, or 56-58. In some embodiments, the Asn297 in the Fc domain monomer in the conjugate according to the present application may be substituted with Ala in order to prevent N-linked glycosylation (see, e.g., SEQ ID NO:4, wherein the substitution of Asn297 with Ala is labeled with (*)).

In some embodiments, the Fc domain monomer or Fc domain in the conjugate according to the present application is an aglycosylated Fc domain monomer or Fc domain (e.g., an aglycosylated Fc domain monomer or Fc domain that retains engagement with an Fc receptor (e.g., FcRn)). For example, the Fc domain is an aglycosylated IgG1 variant that retains engagement with the Fc receptor (e.g., an IgG1 with substitution of amino acids at N297 and/or T299 in the glycosylation motif). Exemplary aglycosylated Fc domains and methods for preparing the aglycosylated Fc domains are known in the art, as described, for example, in Sazinsky S. L. et al., Aglycosylated immunoglobulin G1 variants productively engage activating Fc receptors, PNAS, 2008,105(51):20167-20172.

In some embodiments, the C-terminal Lys447 of the Fc domain monomer in the conjugate according to the present application may be present or absent without affecting the structure or stability of the Fc region, such as any one of SEQ ID NOs:1-29 and 31-52 that do not comprise the C-terminal Lys residue. In some embodiments, the N-terminal Asn of the Fc domain monomer in the conjugate according to the present application may be present or absent without affecting the structure or stability of the variant Fc domain monomer, such as any one of SEQ ID NOs:1 -29, 31-52, and 56-58 that do not comprise the N-terminal Asn residue.

In some embodiments, the Fc domain monomer in the conjugate according to the present application comprises an additional moiety attached to the N-terminus or C-terminus of the Fc domain monomer, such as a purification peptide (e.g., a hexahistidine peptide (HHHHHH (SEQ ID NO:59))) or a signal sequence (e.g., IL2 signal sequence MYRMQLLSCIALSLALVTNS (SEQ ID NO:60)). In some embodiments, the Fc domain monomer in the conjugate does not contain any type of antibody variable regions, such as VH, VL, a complementarity determining region (CDR), or a hypervariable region (HVR)

In some embodiments, the Fc domain monomer in the conjugate according to the present application has a sequence with a sequence identity of at least 70% (such as 70%, 75%, 80%, 85%, 90%, 95%, 96%, 97%, 98%, 99% or 100%) as compared with any one of the sequences as set forth in SEQ ID NO: 1-68 as shown below.

In some embodiments, the Fc domain monomer has a sequence as set forth in any one of SEQ ID NOs 1-68 as shown below.
SEQ ID NO:1: adult IgG1 Fc with Cys to Ser substitution (#), YTE triple mutation (bold and underlined), X₁ being Asp or Glu, and X₂ being Leu or Met, N-terminal Fab residue being underlined, hinge residue being italic 
NVNHKPSNTKVDKKVEPKSS(#)DKTHTCPPCPAPELLGGPSVF
LFPPKPKDTLYITREPEVTCVVVDVSHEDPEVKFNWYVDGVEVH
NAKTKPREEQYNSTYRVVSVLTVLHQDWLNGKEYKCKVSNKALP
APIEKTISKAKGQPREPQVYTLPPSRX1EX2TKNQVSLTCLVKGFYP
SDIAVEWESNGQPENNYKTTPPVLDSDGSFFLYSKLTVDKSRWQQ
GNVFSCSVMHEALHNHYTQKSLSLSPGK
SEQ ID NO:2: adult IgG1 Fc with Cys to Ser substitution (#), YTE triple mutation (bold and underlined), allotype G1m(fa) (bold italic), N-terminal Fab residue being underlined, hinge residue being italic 
NVNHKPSNTKVDKKVEPKSS(#)DKTHTCPPCPAPELLGGPSVF
LFPPKPKDTLYITREPEVTCVVVDVSHEDPEVKFNWYVDGVEVH
NAKTKPREEQYNSTYRVVSVLTVLHQDWLNGKEYKCKVSNKALP
APIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHNHYTQKSLSLSPGK
SEQ ID NO:3: adult IgG1 Fc with Cys to Ser substitution (#), YTE triple mutation (bold and underlined), allotype G1m(f) (bold italic), N-terminal Fab residue being underlined, hinge residue being italic 
NVNHKPSNTKVDKKVEPKSS(#)DKTHTCPPCPAPELLGGPSVF
LFPPKPKDTLYITREPEVTCVVVDVSHEDPEVKFNWYVDGVEVH
NAKTKPREEQYNSTYRVVSVLTVLHQDWLNGKEYKCKVSNKALP
APIEKTISKAKGQPREPQVYTLPPSREEMTKNQVSLTCLVKGFYPS
DIAVEWESNGQPENNYKTTPPVLDSDGSFFLYSKLTVDKSRWQQG
NVFSCSVMHEALHNHYTQKSLSLSPGK
SEQ ID NO:4: adult IgG1 Fc with Cys to Ser substitution (#), YTE triple mutation (bold and underlined), Asn to Ala substitution (*), X₁ being Asp or Glu, and X₂ being Leu or Met, N-terminal Fab residue being underlined, hinge residue being italic 
NVNHKPSNTKVDKKVEPKSS(#)DKTHTCPPCPAPELLGGPSVF
LFPPKPKDTLYITREPEVTCVVVDVSHEDPEVKFNWYVDGVEVH
NAKTKPREEQYA(*)STYRVVSVLTVLHQDWLNGKEYKCKVSNKA
LPAPIEKTISKAKGQPREPQVYTLPPSRX1EX2TKNQVSLTCLVKGF
YPSDIAVEWESNGQPENNYKTTPPVLDSDGSFFLYSKLTVDKSRW
QQGNVFSCSVMHEALHNHYTQKSLSLSPGK
SEQ ID NO:5: adult IgG1 Fc with Cys to Ser substitution (#), YTE triple mutation (bold and underlined), allotype G1m(fa) (bold italic), Asn to Ala substitution (*), N-terminal Fab residue being underlined, hinge residue being italic 
NVNHKPSNTKVDKKVEPKSS(#)DKTHTCPPCPAPELLGGPSVF
LFPPKPKDTLYITREPEVTCVVVDVSHEDPEVKFNWYVDGVEVH
NAKTKPREEQYA(*)STYRVVSVLTVLHQDWLNGKEYKCKVSNKA
LPAPIEKTISKAKGQPREPQVYTLPPSRDELTKNQVSLTCLVKGFYP
SDIAVEWESNGQPENNYKTTPPVLDSDGSFFLYSKLTVDKSRWQQ
GNVFSCSVMHEALHNHYTQKSLSLSPGK
SEQ ID NO:6: adult IgG1 Fc with Cys to Ser substitution (#), YTE triple mutation (bold and underlined), allotype G1m(f) (bold italic), Asn to Ala substitution(*), N-terminal Fab residue being underlined, hinge residue being italic 
NVNHKPSNTKVDKKVEPKSS(#)DKTHTCPPCPAPELLGGPSVF
LFPPKPKDTLYITREPEVTCVVVDVSHEDPEVKFNWYVDGVEVH
NAKTKPREEQYA(*)STYRVVSVLTVLHQDWLNGKEYKCKVSNKA
LPAPIEKTISKAKGQPREPQVYTLPPSREEMTKNQVSLTCLVKGFY
PSDIAVEWESNGQPENNYKTTPPVLDSDGSFFLYSKLTVDKSRWQ
QGNVFSCSVMHEALHNHYTQKSLSLSPGK
SEQ ID NO:7: adult IgG1 Fc with Cys to Ser substitution(#), YTE triple mutation (bold and underlined), X₆ being Asp or Glu, and X₇ being Leu or Met, Z₁ being Asn or absent, Z₂ being Asn or Ala, Z₃ being Lys or absent, N-terminal Fab residue being underlined, hinge residue being italic 
Z1VNHKPSNTKVDKKVEPKSS(#)DKTHTCPPCPAPELLGGPSV
FLFPPKPKDTLYITREPEVTCVVVDVSHEDPEVKFNWYVDGVEV
HNAKTKPREEQYZ2STYRVVSVLTVLHQDWLNGKEYKCKVSNKA
LPAPIEKTISKAKGQPREPQVYTLPPSRX6EX7TKNQVSLTCLVKGF
YPSDIAVEWESNGQPENNYKTTPPVLDSDGSFFLYSKLTVDKSRW
QQGNVFSCSVMHEALHNHYTQKSLSLSPGZ3
SEQ ID NO:8: adult IgG1 Fc with Cys to Ser substitution (#), YTE triple mutation (bold and underlined), allotype G1m(fa) (bold italic), N-terminal Fab residue being underlined, hinge residue being italic 
VNHKPSNTKVDKKVEPKSS(#)DKTHTCPPCPAPELLGGPSVFL
FPPKPKDTLYITREPEVTCVVVDVSHEDPEVKFNWYVDGVEVHN
AKTKPREEQYNSTYRVVSVLTVLHQDWLNGKEYKCKVSNKALPA
PIEKTISKAKGQPREPQVYTLPPSRDELTKNQVSLTCLVKGFYPSDI
AVEWESNGQPENNYKTTPPVLDSDGSFFLYSKLTVDKSRWQQGN
VFSCSVMHEALHNHYTQKSLSLSPGK
SEQ ID NO:9: adult IgG1 Fc with Cys to Ser substitution (#), YTE triple mutation (bold and underlined), allotype G1m(f) (bold italic), N-terminal Fab residue being underlined, hinge residue being italic 
VNHKPSNTKVDKKVEPKSS(#)DKTHTCPPCPAPELLGGPSVFL
FPPKPKDTLYITREPEVTCVVVDVSHEDPEVKFNWYVDGVEVHN
AKTKPREEQYNSTYRVVSVLTVLHQDWLNGKEYKCKVSNKALPA
PIEKTISKAKGQPREPQVYTLPPSREEMTKNQVSLTCLVKGFYPSDI
AVEWESNGQPENNYKTTPPVLDSDGSFFLYSKLTVDKSRWQQGN
VFSCSVMHEALHNHYTQKSLSLSPGK
SEQ ID NO: 10: adult IgG1 Fc with Cys to Ser substitution (#), YTE triple mutation (bold and underlined), allotype G1m(fa) (bold italic), N-terminal Fab residue being underlined, hinge residue being italic 
NVNHKPSNTKVDKKVEPKSS(#)DKTHTCPPCPAPELLGGPSVF
LFPPKPKDTLYITREPEVTCVVVDVSHEDPEVKFNWYVDGVEVH
NAKTKPREEQYNSTYRVVSVLTVLHQDWLNGKEYKCKVSNKALP
APIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHNHYTQKSLSLSPG
SEQ ID NO: 11: adult IgG1 Fc with Cys to Ser substitution (#), YTE triple mutation (bold and underlined), allotype G1m(f) (bold italic), N-terminal Fab residue being underlined, hinge residue being italic 
NVNHKPSNTKVDKKVEPKSS(#)DKTHTCPPCPAPELLGGPSVF
LFPPKPKDTLYITREPEVTCVVVDVSHEDPEVKFNWYVDGVEVH
NAKTKPREEQYNSTYRVVSVLTVLHQDWLNGKEYKCKVSNKALP
APIEKTISKAKGQPREPQVYTLPPSREEMTKNQVSLTCLVKGFYPS
DIAVEWESNGQPENNYKTTPPVLDSDGSFFLYSKLTVDKSRWQQG
NVFSCSVMHEALHNHYTQKSLSLSPG
SEQ ID NO: 12: adult IgG1 Fc with Cys to Ser substitution (#), YTE triple mutation (bold and underlined), allotype G1m(fa) (bold italic), N-terminal Fab residue being underlined, hinge residue being italic 
VNHKPSNTKVDKKVEPKSS(#)DKTHTCPPCPAPELLGGPSVFL
FPPKPKDTLYITREPEVTCVVVDVSHEDPEVKFNWYVDGVEVHN
AKTKPREEQYNSTYRVVSVLTVLHQDWLNGKEYKCKVSNKALPA
PIEKTISKAKGQPREPQVYTLPPSRDELTKNQVSLTCLVKGFYPSDI
AVEWESNGQPENNYKTTPPVLDSDGSFFLYSKLTVDKSRWQQGN
VFSCSVMHEALHNHYTQKSLSLSPG
SEQ ID NO: 13: adult IgG1 Fc with Cys to Ser substitution (#), YTE triple mutation (bold and underlined), allotype G1m(f) (bold italic), N-terminal Fab residue being underlined, hinge residue being italic 
VNHKPSNTKVDKKVEPKSS(#)DKTHTCPPCPAPELLGGPSVFL
FPPKPKDTLYITREPEVTCVVVDVSHEDPEVKFNWYVDGVEVHN
AKTKPREEQYNSTYRVVSVLTVLHQDWLNGKEYKCKVSNKALPA
PIEKTISKAKGQPREPQVYTLPPSREEMTKNQVSLTCLVKGFYPSDI
AVEWESNGQPENNYKTTPPVLDSDGSFFLYSKLTVDKSRWQQGN
VFSCSVMHEALHNHYTQKSLSLSPG
SEQ ID NO: 14: adult IgG1 Fc with Cys to Ser substitution (#), YTE triple mutation (bold and underlined), allotype G1m(fa) (bold italic), Asn to Ala substitution (*), N-terminal Fab residue being underlined, hinge residue being italic 
VNHKPSNTKVDKKVEPKSS(#)DKTHTCPPCPAPELLGGPSVFL
FPPKPKDTLYITREPEVTCVVVDVSHEDPEVKFNWYVDGVEVHN
AKTKPREEQYA(*)STYRVVSVLTVLHQDWLNGKEYKCKVSNKAL
PAPIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHNHYTQKSLSLSPGK
SEQ ID NO: 15: adult IgG1 Fc with Cys to Ser substitution (#), YTE triple mutation (bold and underlined), allotype G1m(f) (bold italic), Asn to Ala substitution (*), N-terminal Fab residue being underlined, hinge residue being italic 
VNHKPSNTKVDKKVEPKSS(#)DKTHTCPPCPAPELLGGPSVFL
FPPKPKDTLYITREPEVTCVVVDVSHEDPEVKFNWYVDGVEVHN
AKTKPREEQYA(*)STYRVVSVLTVLHQDWLNGKEYKCKVSNKAL
PAPIEKTISKAKGQPREPQVYTLPPSREEMTKNQVSLTCLVKGFYP
SDIAVEWESNGQPENNYKTTPPVLDSDGSFFLYSKLTVDKSRWQQ
GNVFSCSVMHEALHNHYTQKSLSLSPGK
SEQ ID NO: 16: adult IgG1 Fc with Cys to Ser substitution (#), YTE triple mutation (bold and underlined), allotype G1m(fa) (bold italic), Asn to Ala substitution (*), N-terminal Fab residue being underlined, hinge residue being italic 
NVNHKPSNTKVDKKVEPKSS(#)DKTHTCPPCPAPELLGGPSVF
LFPPKPKDTLYITREPEVTCVVVDVSHEDPEVKFNWYVDGVEVH
NAKTKPREEQYA(*)STYRVVSVLTVLHQDWLNGKEYKCKVSNKA
LPAPIEKTISKAKGQPREPQVYTLPPSRDELTKNQVSLTCLVKGFYP
SDIAVEWESNGQPENNYKTTPPVLDSDGSFFLYSKLTVDKSRWQQ
GNVFSCSVMHEALHNHYTQKSLSLSPG
SEQ ID NO: 17: adult IgG1 Fc with Cys to Ser substitution (#), YTE triple mutation (bold and underlined), allotype G1m(f) (bold italic), Asn to Ala substitution(*), N-terminal Fab residue being underlined, hinge residue being italic 
NVNHKPSNTKVDKKVEPKSS(#)DKTHTCPPCPAPELLGGPSVF
LFPPKPKDTLYITREPEVTCVVVDVSHEDPEVKFNWYVDGVEVH
NAKTKPREEQYA(*)STYRVVSVLTVLHQDWLNGKEYKCKVSNKA
LPAPIEKTISKAKGQPREPQVYTLPPSREEMTKNQVSLTCLVKGFY
PSDIAVEWESNGQPENNYKTTPPVLDSDGSFFLYSKLTVDKSRWQ
QGNVFSCSVMHEALHNHYTQKSLSLSPG
SEQ ID NO: 18: adult IgG1 Fc with Cys to Ser substitution (#), YTE triple mutation (bold and underlined), allotype G1m(fa) (bold italic), Asn to Ala substitution (*), N-terminal Fab residue being underlined, hinge residue being italic 
VNHKPSNTKVDKKVEPKSS(#)DKTHTCPPCPAPELLGGPSVFL
FPPKPKDTLYITREPEVTCVVVDVSHEDPEVKFNWYVDGVEVHN
AKTKPREEQYA(*)STYRVVSVLTVLHQDWLNGKEYKCKVSNKAL
PAPIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHNHYTQKSLSLSPG
SEQ ID NO: 19: adult IgG1 Fc with Cys to Ser substitution (#), YTE triple mutation (bold and underlined), allotype G1m(f) (bold italic), Asn to Ala substitution(*), N-terminal Fab residue being underlined, hinge residue being italic 
VNHKPSNTKVDKKVEPKSS(#)DKTHTCPPCPAPELLGGPSVFL
FPPKPKDTLYITREPEVTCVVVDVSHEDPEVKFNWYVDGVEVHN
AKTKPREEQYA(*)STYRVVSVLTVLHQDWLNGKEYKCKVSNKAL
PAPIEKTISKAKGQPREPQVYTLPPSREEMTKNQVSLTCLVKGFYP
SDIAVEWESNGQPENNYKTTPPVLDSDGSFFLYSKLTVDKSRWQQ
GNVFSCSVMHEALHNHYTQKSLSLSPG
SEQ ID NO:20: adult IgG1 Fc with Cys to Ser substitution (#), X₄ being Asp or Glu, X₅ being Leu or Met; Z₁ being Asn or absent, Z₃ being Lys or absent, N-terminal Fab residue being underlined, hinge residue being italic 
Z1VNHKPSNTKVDKKVEPKSS(#)DKTHTCPPCPAPELLGGPSV
FLFPPKPKDTLMISRTPEVTCVVVDVSHEDPEVKFNWYVDGVEVH
NAKTKPREEQYNSTYRVVSVLTVLHQDWLNGKEYKCKVSNKALP
APIEKTISKAKGQPREPQVYTLPPSRX4EX5TKNQVSLTCLVKGFYP
SDIAVEWESNGQPENNYKTTPPVLDSDGSFFLYSKLTVDKSRWQQ
GNVFSCSVMHEALHNHYTQKSLSLSPGZ3
SEQ ID NO:21: adult IgG1 Fc with Cys to Ser substitution (#), allotype G1m(fa) (bold italic), N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYNSTYRVVSVLTVLHQDWLNGKEYKCKVSNKALP
APIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHNHYTQKSLSLSPGK
SEQ ID NO:22: adult IgG1 Fc with Cys to Ser substitution (#), allotype G1m(f) (bold italic), N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYNSTYRVVSVLTVLHQDWLNGKEYKCKVSNKALP
APIEKTISKAKGQPREPQVYTLPPSREEMTKNQVSLTCLVKGFYPS
DIAVEWESNGQPENNYKTTPPVLDSDGSFFLYSKLTVDKSRWQQG
NVFSCSVMHEALHNHYTQKSLSLSPGK
SEQ ID NO:23: adult IgG1 Fc with Cys to Ser substitution (#), X₁ being Met or Tyr, X₂ being Ser or Thr, X₃ being Thr or Glu, X₆ being Asp or Glu, X₇ being Leu or Met, Z₁ being Asn or absent, Z₂ being Asn or Ala, and Z₃ being Lys or absent, N-terminal Fab residue being underlined, hinge residue being italic 
Z1VNHKPSNTKVDKKVEPKSS(#)DKTHTCPPCPAPELLGGPSV
FLFPPKPKDTLX1IX2RX3PEVTCVVVDVSHEDPEVKFNWYVDGVE
VHNAKTKPREEQYZ2STYRVVSVLTVLHQDWLNGKEYKCKVSNK
ALPAPIEKTISKAKGQPREPQVYTLPPSRX6EX7TKNQVSLTCLVKG
FYPSDIAVEWESNGQPENNYKTTPPVLDSDGSFFLYSKLTVDKSRW
QQGNVFSCSVMHEALHNHYTQKSLSLSPGZ3
SEQ ID NO:24: adult IgG1 Fc with Cys to Ser substitution (#), X₁ being Met or Tyr, X₂ being Ser or Thr, X₃ being Thr or Glu, X₆ being Asp or Glu, X₇ being Leu or Met, and Z₂ being Asn or Ala, N-terminal Fab residue being underlined, hinge residue being italic 
VNHKPSNTKVDKKVEPKSS(#)DKTHTCPPCPAPELLGGPSVFL
FPPKPKDTLX1IX2RX3PEVTCVVVDVSHEDPEVKFNWYVDGVEVH
NAKTKPREEQYZ2STYRVVSVLTVLHQDWLNGKEYKCKVSNKAL
PAPIEKTISKAKGQPREPQVYTLPPSRX6EX7TKNQVSLTCLVKGFY
PSDIAVEWESNGQPENNYKTTPPVLDSDGSFFLYSKLTVDKSRWQ
QGNVFSCSVMHEALHNHYTQKSLSLSPGK
SEQ ID NO:25: adult IgG1 Fc with Cys to Ser substitution (#), X₁ being Met or Tyr, X₂ being Ser or Thr, X₃ being Thr or Glu, and Z₂ being Asn or Ala, N-terminal Fab residue being underlined, hinge residue being italic 
VNHKPSNTKVDKKVEPKSS(#)DKTHTCPPCPAPELLGGPSVFL
FPPKPKDTLX1IX2RX3PEVTCVVVDVSHEDPEVKFNWYVDGVEVH
NAKTKPREEQYZ2STYRVVSVLTVLHQDWLNGKEYKCKVSNKAL
PAPIEKTISKAKGQPREPQVYTLPPSREEMTKNQVSLTCLVKGFYP
SDIAVEWESNGQPENNYKTTPPVLDSDGSFFLYSKLTVDKSRWQQ
GNVFSCSVMHEALHNHYTQKSLSLSPGK
SEQ ID NO:26: adult IgG1 Fc with Cys to Ser substitution (#), X₁ being Met or Tyr, X₂ being Ser or Thr, X₃ being Thr or Glu, and Z₂ being Asn or Ala, N-terminal Fab residue being underlined, hinge residue being italic 
VNHKPSNTKVDKKVEPKSS(#)DKTHTCPPCPAPELLGGPSVFL
FPPKPKDTLX1IX2RX3PEVTCVVVDVSHEDPEVKFNWYVDGVEVH
NAKTKPREEQYZ2STYRVVSVLTVLHQDWLNGKEYKCKVSNKAL
PAPIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHNHYTQKSLSLSPGK
SEQ ID NO:27: adult IgG1 Fc with Cys to Ser substitution (#), X₁ being Met or Tyr, X₂ being Ser or Thr, X₃ being Thr or Glu, X₆ being Asp or Glu, X₇ being Leu or Met, and Z₂ being Asn or Ala, N-terminal Fab residue being underlined, hinge residue being italic 
VNHKPSNTKVDKKVEPKSS(#)DKTHTCPPCPAPELLGGPSVFL
FPPKPKDTLX1IX2RX3PEVTCVVVDVSHEDPEVKFNWYVDGVEVH
NAKTKPREEQYZ2STYRVVSVLTVLHQDWLNGKEYKCKVSNKAL
PAPIEKTISKAKGQPREPQVYTLPPSRX6EX7TKNQVSLTCLVKGFY
PSDIAVEWESNGQPENNYKTTPPVLDSDGSFFLYSKLTVDKSRWQ
QGNVFSCSVMHEALHNHYTQKSLSLSPG
SEQ ID NO:28: adult IgG1 Fc with Cys to Ser substitution (#), X₁ being Met or Tyr, X₂ being Ser or Thr, X₃ being Thr or Glu, and Z₂ being Asn or Ala, N-terminal Fab residue being underlined, hinge residue being italic 
VNHKPSNTKVDKKVEPKSS(#)DKTHTCPPCPAPELLGGPSVFL
FPPKPKDTLX1IX2RX3PEVTCVVVDVSHEDPEVKFNWYVDGVEVH
NAKTKPREEQYZ2STYRVVSVLTVLHQDWLNGKEYKCKVSNKAL
PAPIEKTISKAKGQPREPQVYTLPPSREEMTKNQVSLTCLVKGFYP
SDIAVEWESNGQPENNYKTTPPVLDSDGSFFLYSKLTVDKSRWQQ
GNVFSCSVMHEALHNHYTQKSLSLSPG
SEQ ID NO:29: adult IgG1 Fc with Cys to Ser substitution (#), X₁ being Met or Tyr, X₂ being Ser or Thr, X₃ being Thr or Glu, and Z₂ being Asn or Ala, N-terminal Fab residue being underlined, hinge residue being italic 
VNHKPSNTKVDKKVEPKSS(#)DKTHTCPPCPAPELLGGPSVFL
FPPKPKDTLX1IX2RX3PEVTCVVVDVSHEDPEVKFNWYVDGVEVH
NAKTKPREEQYZ2STYRVVSVLTVLHQDWLNGKEYKCKVSNKAL
PAPIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHNHYTQKSLSLSPG
SEQ ID NO:30: adult IgG1 Fc (bold) with mouse heavy chain MigG Vh signal sequence, Cys to Ser substitution (#), allotype G1m(fa) (bold italic), N-terminal Fab residue being underlined, hinge residue being italic 
MGWSCIILFLVATATGVHSNVNHKPSNTKVDKKVEPKSS(#)
DKTHTCPPCPAPELLGGPSVFLFPPKPKDTLMISRTPEVTCVVVDV
SHEDPEVKFNWYVDGVEVHNAKTKPREEQYNSTYRVVSVLTVLH
QDWLNGKEYKCKVSNKALPAPIEKTISKAKGQPREPQVYTLPPSR
DELTKNQVSLTCLVKGFYPSDIAVEWESNGQPENNYKTTPPVLDS
DGSFFLYSKLTVDKSRWQQGNVFSCSVMHEALHNHYTQKSLSLSP
GK
SEQ ID NO:31: adult IgG1 Fc with Z₁ being Asn or absent, Z₃ being Lys or absent, J₁ being Cys or Ser, and X₁ being Met or Tyr, X₂ being Ser or Thr, X₃ being Thr or Glu, Z₃ being Asn or Ala, X₄ being Leu or Asp, X₅ being Gln or His, X₆ being Asp or Glu, and X₇ being Leu or Met, X₈ being Met or Leu, and X₉ being Asn or Ser, Z₂ being Asn or Ala, N-terminal Fab residue being underlined, hinge residue being italic 
Z1VNHKPSNTKVDKKVEPKSJ1DKTHTCPPCPAPELLGGPSVFL
FPPKPKDTLX1IX2RX3PEVTCVVVDVSHEDPEVKFNWYVDGVEVH
NAKTKPREEQYZ2STYRVVSVLTVX4HX5DWLNGKEYKCKVSNKA
LPAPIEKTISKAKGQPREPQVYTLPPSRX6EX7TKNQVSLTCLVKGF
YPSDIAVEWESNGQPENNYKTTPPVLDSDGSFFLYSKLTVDKSRW
QQGNVFSCSVX8HEALHX9HYTQKSLSLSPGZ3
SEQ ID NO:32: adult IgG1 Fc with Cys to Ser substitution (#), Z₁ being Asn or absent, Z₃ being Lys or absent, and Z₂ being Asn or Ala, X₄ being Leu or Asp, X₅ being Gln or His, X₆ being Asp or Glu, X₇ being Leu or Met, X₈ being Met or Leu, and X₉ being Asn or Ser, N-terminal Fab residue being underlined, hinge residue being italic 
Z1VNHKPSNTKVDKKVEPKSS(#)DKTHTCPPCPAPELLGGPSV
FLFPPKPKDTLMISRTPEVTCVVVDVSHEDPEVKFNWYVDGVEVH
NAKTKPREEQYZ2STYRVVSVLTVX4HX5DWLNGKEYKCKVSNKA
LPAPIEKTISKAKGQPREPQVYTLPPSRX6EX7TKNQVSLTCLVKGF
YPSDIAVEWESNGQPENNYKTTPPVLDSDGSFFLYSKLTVDKSRW
QQGNVFSCSVX8HEALHX9HYTQKSLSLSPGZ3
SEQ ID NO:33: adult IgG1 Fc with Cys to Ser substitution (#), DHS triple mutation (bold and underlined), Z₁ being Asn or absent, Z₃ being Lys or absent, and Z₂ being Asn or Ala, X₆ being Asp or Glu, and X₇ being Leu or Met, X₈ is Met or Leu, and X₉ being Asn or Ser, N-terminal Fab residue being underlined, hinge residue being italic 
Z1VNHKPSNTKVDKKVEPKSS(#)DKTHTCPPCPAPELLGGPSV
FLFPPKPKDTLMISRTPEVTCVVVDVSHEDPEVKFNWYVDGVEVH
NAKTKPREEQYZ2STYRVVSVLTVDHHDWLNGKEYKCKVSNKAL
PAPIEKTISKAKGQPREPQVYTLPPSRX6EX7TKNQVSLTCLVKGFY
PSDIAVEWESNGQPENNYKTTPPVLDSDGSFFLYSKLTVDKSRWQ
QGNVFSCSVX8HEALHX9HYTQKSLSLSPGZ3
SEQ ID NO:34: adult IgG1 Fc with Cys to Ser substitution (#), DHS triple mutation (bold and underlined), Z₂ being Asn or Ala, X₆ being Asp or Glu, and X₇ being Leu or Met, N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYZ2STYRVVSVLTVDHHDWLNGKEYKCKVSNKAL
PAPIEKTISKAKGQPREPQVYTLPPSRX6EX7TKNQVSLTCLVKGFY
PSDIAVEWESNGQPENNYKTTPPVLDSDGSFFLYSKLTVDKSRWQ
QGNVFSCSVMHEALHSHYTQKSLSLSPGK
SEQ ID NO:35: adult IgG1 Fc with Cys to Ser substitution (#), DHS triple mutation (bold and underlined), X₆ being Asp or Glu and X₇ being Leu or Met, N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYNSTYRVVSVLTVDHHDWLNGKEYKCKVSNKAL
PAPIEKTISKAKGQPREPQVYTLPPSRX6EX7TKNQVSLTCLVKGFY
PSDIAVEWESNGQPENNYKTTPPVLDSDGSFFLYSKLTVDKSRWQ
QGNVFSCSVMHEALHSHYTQKSLSLSPGK
SEQ ID NO:36: adult IgG1 Fc with Cys to Ser substitution (#), DHS triple mutation (bold and underlined), allotype G1m(fa) (bold italic), N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYNSTYRVVSVLTVDHHDWLNGKEYKCKVSNKAL
PAPIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHSHYTQKSLSLSPGK
SEQ ID NO:37: adult IgG1 Fc with Cys to Ser substitution (#), DHS triple mutation (bold and underline) 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYNSTYRVVSVLTVDHHDWLNGKEYKCKVSNKAL
PAPIEKTISKAKGQPREPQVYTLPPSREEMTKNQVSLTCLVKGFYP
SDIAVEWESNGQPENNYKTTPPVLDSDGSFFLYSKLTVDKSRWQQ
GNVFSCSVMHEALHSHYTQKSLSLSPGK
SEQ ID NO:38: adult IgG1 Fc with Cys to Ser substitution (#), DHS triple mutation (bold and underlined), allotype G1m(fa) (bold italic), N-terminal Fab residue being underlined, hinge residue being italic 
VNHKPSNTKVDKKVEPKSS(#)DKTHTCPPCPAPELLGGPSVFL
FPPKPKDTLMISRTPEVTCVVVDVSHEDPEVKFNWYVDGVEVHN
AKTKPREEQYNSTYRVVSVLTVDHHDWLNGKEYKCKVSNKALP
APIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHSHYTQKSLSLSPGK
SEQ ID NO:39: adult IgG1 Fc with Cys to Ser substitution (#), DHS triple mutation (bold and underlined), allotype G1m(f) (bold italic), N-terminal Fab residue being underlined, hinge residue being italic 
VNHKPSNTKVDKKVEPKSS(#)DKTHTCPPCPAPELLGGPSVFL
FPPKPKDTLMISRTPEVTCVVVDVSHEDPEVKFNWYVDGVEVHN
AKTKPREEQYNSTYRVVSVLTVDHHDWLNGKEYKCKVSNKALP
APIEKTISKAKGQPREPQVYTLPPSREEMTKNQVSLTCLVKGFYPS
DIAVEWESNGQPENNYKTTPPVLDSDGSFFLYSKLTVDKSRWQQG
NVFSCSVMHEALHSHYTQKSLSLSPGK
SEQ ID NO:40: adult IgG1 Fc with Cys to Ser substitution (#), DHS triple mutation (bold and underlined), allotype G1m(fa) (bold italic), N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYNSTYRVVSVLTVDHHDWLNGKEYKCKVSNKAL
PAPIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHSHYTQKSLSLSPGK
SEQ ID NO:41: adult IgG1 Fc with Cys to Ser substitution (#), DHS triple mutation(bold and underlined) 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYNSTYRVVSVLTVDHHDWLNGKEYKCKVSNKAL
PAPIEKTISKAKGQPREPQVYTLPPSREEMTKNQVSLTCLVKGFYP
SDIAVEWESNGQPENNYKTTPPVLDSDGSFFLYSKLTVDKSRWQQ
GNVFSCSVMHEALHSHYTQKSLSLSPGK
SEQ ID NO:42: adult IgG1 Fc with Cys to Ser substitution (#), DHS triple mutation (bold and underlined), allotype G1m(fa) (bold italic), N-terminal Fab residue being underlined, hinge residue being italic 
VNHKPSNTKVDKKVEPKSS(#)DKTHTCPPCPAPELLGGPSVFL
FPPKPKDTLMISRTPEVTCVVVDVSHEDPEVKFNWYVDGVEVHN
AKTKPREEQYNSTYRVVSVLTVDHHDWLNGKEYKCKVSNKALP
APIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHSHYTQKSLSLSPG
SEQ ID NO:43: adult IgG1 Fc with Cys to Ser substitution (#), DHS triple mutation (bold and underlined), allotype G1m(f) (bold italic), N-terminal Fab residue being underlined, hinge residue being italic 
VNHKPSNTKVDKKVEPKSS(#)DKTHTCPPCPAPELLGGPSVFL
FPPKPKDTLMISRTPEVTCVVVDVSHEDPEVKFNWYVDGVEVHN
AKTKPREEQYNSTYRVVSVLTVDHHDWLNGKEYKCKVSNKALP
APIEKTISKAKGQPREPQVYTLPPSREEMTKNQVSLTCLVKGFYPS
DIAVEWESNGQPENNYKTTPPVLDSDGSFFLYSKLTVDKSRWQQG
NVFSCSVMHEALHSHYTQKSLSLSPG
SEQ ID NO:44: adult IgG1 Fc with Cys to Ser substitution (#), Asn to Ala substitution (*), DHS triple mutation (bold and underlined), X₆ being Asp or Glu and X₇ being Leu or Met, N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYA(*)STYRVVSVLTVDHHDWLNGKEYKCKVSNK
ALPAPIEKTISKAKGQPREPQVYTLPPSRX6EX7TKNQVSLTCLVKG
FYPSDIAVEWESNGQPENNYKTTPPVLDSDGSFFLYSKLTVDKSRW
QQGNVFSCSVMHEALHSHYTQKSLSLSPGK
SEQ ID NO:45: adult IgG1 Fc with Cys to Ser substitution (#), Asn to Ala substitution (*), DHS triple mutation (bold and underlined), allotype G1m(fa) (bold italic), N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYA(*)STYRVVSVLTVDHHDWLNGKEYKCKVSNK
ALPAPIEKTISKAKGQPREPQVYTLPPSRDELTKNQVSLTCLVKGF
YPSDIAVEWESNGQPENNYKTTPPVLDSDGSFFLYSKLTVDKSRW
QQGNVFSCSVMHEALHSHYTQKSLSLSPGK
SEQ ID NO:46: adult IgG1 Fc with Cys to Ser substitution (#), Asn to Ala substitution (*), DHS triple mutation (bold and underlined), allotype G1m(f) (bold italic), N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYA(*)STYRVVSVLTVDHHDWLNGKEYKCKVSNK
ALPAPIEKTISKAKGQPREPQVYTLPPSREEMTKNQVSLTCLVKGF
YPSDIAVEWESNGQPENNYKTTPPVLDSDGSFFLYSKLTVDKSRW
QQGNVFSCSVMHEALHSHYTQKSLSLSPGK
SEQ ID NO:47: adult IgG1 Fc with Cys to Ser substitution (#), Asn to Ala substitution (*), DHS triple mutation (bold and underlined), allotype G1m(fa) (bold italic), N-terminal Fab residue being underlined, hinge residue being italic 
VNHKPSNTKVDKKVEPKSS(#)DKTHTCPPCPAPELLGGPSVFL
FPPKPKDTLMISRTPEVTCVVVDVSHEDPEVKFNWYVDGVEVHN
AKTKPREEQYA(*)STYRVVSVLTVDHHDWLNGKEYKCKVSNKAL
PAPIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHSHYTQKSLSLSPGK
SEQ ID NO:48: adult IgG1 Fc with Cys to Ser substitution (#), Asn to Ala substitution (*), DHS triple mutation (bold and underlined), allotype G1m(f) (bold italic), N-terminal Fab residue being underlined, hinge residue being italic 
VNHKPSNTKVDKKVEPKSS(#)DKTHTCPPCPAPELLGGPSVFL
FPPKPKDTLMISRTPEVTCVVVDVSHEDPEVKFNWYVDGVEVHN
AKTKPREEQYA(*)STYRVVSVLTVDHHDWLNGKEYKCKVSNKAL
PAPIEKTISKAKGQPREPQVYTLPPSREEMTKNQVSLTCLVKGFYP
SDIAVEWESNGQPENNYKTTPPVLDSDGSFFLYSKLTVDKSRWQQ
GNVFSCSVMHEALHSHYTQKSLSLSPGK
SEQ ID NO:49: adult IgG1 Fc with Cys to Ser substitution (#), Asn to Ala substitution (*), DHS triple mutation (bold and underlined), allotype G1m(fa) (bold italic), N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYA(*)STYRVVSVLTVDHHDWLNGKEYKCKVSNK
ALPAPIEKTISKAKGQPREPQVYTLPPSRDELTKNQVSLTCLVKGF
YPSDIAVEWESNGQPENNYKTTPPVLDSDGSFFLYSKLTVDKSRW
QQGNVFSCSVMHEALHSHYTQKSLSLSPGK
SEQ ID NO:50: adult IgG1 Fc with Cys to Ser substitution (#), Asn to Ala substitution (*), DHS triple mutation (bold and underlined), allotype G1m(f) (bold italic), N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYA(*)STYRVVSVLTVDHHDWLNGKEYKCKVSNK
ALPAPIEKTISKAKGQPREPQVYTLPPSREEMTKNQVSLTCLVKGF
YPSDIAVEWESNGQPENNYKTTPPVLDSDGSFFLYSKLTVDKSRW
QQGNVFSCSVMHEALHSHYTQKSLSLSPGK
SEQ ID NO:51: adult IgG1 Fc with Cys to Ser substitution (#), Asn to Ala substitution (*), DHS triple mutation (bold and underlined), allotype G1m(fa) (bold italic), N-terminal Fab residue being underlined, hinge residue being italic 
VNHKPSNTKVDKKVEPKSS(#)DKTHTCPPCPAPELLGGPSVFL
FPPKPKDTLMISRTPEVTCVVVDVSHEDPEVKFNWYVDGVEVHN
AKTKPREEQYA(*)STYRVVSVLTVDHHDWLNGKEYKCKVSNKAL
PAPIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHSHYTQKSLSLSPG
SEQ ID NO:52: adult IgG1 Fc with Cys to Ser substitution (#), Asn to Ala substitution (*), DHS triple mutation (bold and underlined), allotype G1m(f) (bold italic), N-terminal Fab residue being underlined, hinge residue being italic 
VNHKPSNTKVDKKVEPKSS(#)DKTHTCPPCPAPELLGGPSVFL
FPPKPKDTLMISRTPEVTCVVVDVSHEDPEVKFNWYVDGVEVHN
AKTKPREEQYA(*)STYRVVSVLTVDHHDWLNGKEYKCKVSNKAL
 
PAPIEKTISKAKGQPREPQVYTLPPSREEMTKNQVSLTCLVKGFYP
SDIAVEWESNGQPENNYKTTPPVLDSDGSFFLYSKLTVDKSRWQQ
GNVFSCSVMHEALHSHYTQKSLSLSPG
SEQ ID NO:53: adult IgG1 Fc with N-terminal ISAMVRS amino acid residue added (italic), C-terminal G4S linker (italic), and C-terminal myc tag (underlined), allotype G1m(f) (bold italic) 
ISAMVRSDKTHTCPPCPAPELLGGPSVFLFPPKPKDTLMISRTPE
VTCVVVDVSHEDPEVKFNWYVDGVEVHNAKTKPREEQYNSTYR
VVSVLTVLHQDWLNGKEYKCKVSNKALPAPIEKTISKAKGQPREP
QVYTLPPSREEMTKNQVSLTCLVKGFYPSDIAVEWESNGQPENNY
KTTPPVLDSDGSFFLYSKLTVDKSRWQQGNVFSCSVMHEALHNH
YTQKSLSLSPGGGGGSEQKLISEEDL
SEQ ID NO:54: adult IgG1 Fc with N-terminal ISAMVRS amino acid residue added (italic), allotype G1m(fa) (bold italic) 
KTHTCPPCPAPELLGGPSVFLFPPKPKDTLMISRTPEVTCVVV
DVSHEDPEVKFNWYVDGVEVHNAKTKPREEQYNSTYRVVSVLT
VLHQDWLNGKEYKCKVSNKALPAPIEKTISKAKGQPREPQVYTLP
PSRDELTKNQVSLTCLVKGFYPSDIAVEWESNGQPENNYKTTPPVL
DSDGSFFLYSKLTVDKSRWQQGNVFSCSVMHEALHNHYTQKSLS
LSPG
SEQ ID NO:55: adult IgG1 Fc with N-terminal amino acid residue added (italic), hinge residue (italic), allotype G1m(fa) (bold italic) 
EPKSS(#)DKTHTCPPCPAPELLGGPSVFLFPPKPKDTLMISRTPE
VTCVVVDVSHEDPEVKFNWYVDGVEVHNAKTKPREEQYNSTYR
VVSVLTVLHQDWLNGKEYKCKVSNKALPAPIEKTISKAKGQPREP
QVYTLPPSRDELTKNQVSLTCLVKGFYPSDIAVEWESNGQPENNY
KTTPPVLDSDGSFFLYSKLTVDKSRWQQGNVFSCSVMHEALHNH
YTQKSLSLSPG
SEQ ID NO:56: adult IgG1 Fc with Cys to Ser substitution (#), allotype G1m(fa) (bold italic), N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYNSTYRVVSVLTVLHQDWLNGKEYKCKVSNKALP
APIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVMHEALHNHYTQKSLSLSPG
SEQ ID NO:57: adult IgG1 Fc with Cys to Ser substitution (#), allotype G1m(f) (bold italic), N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYNSTYRVVSVLTVLHQDWLNGKEYKCKVSNKALP
APIEKTISKAKGQPREPQVYTLPPSREEMTKNQVSLTCLVKGFYPS
DIAVEWESNGQPENNYKTTPPVLDSDGSFFLYSKLTVDKSRWQQG
NVFSCSVMHEALHNHYTQKSLSLSPG
SEQ ID NO:58: adult IgG1 Fc with Cys to Ser substitution (#), M428L, N434S (bold/underlined), allotype G1m(fa) (bold italic), N-terminal Fab residue being underlined, hinge residue being italic 
NVNHKPSNTKVDKKVEPKSS(#)DKTHTCPPCPAPELLGGPSVF
LFPPKPKDTLMISRTPEVTCVVVDVSHEDPEVKFNWYVDGVEVH
NAKTKPREEQYNSTYRVVSVLTVLHQDWLNGKEYKCKVSNKALP
APIEKTISKAKGQPREPQVYTLPPSRDELTKNQVSLTCLVKGFYPS
DIAVEWESNGQPENNYKTTPPVLDSDGSFFLYSKLTVDKSRWQQG
NVFSCSVLHEALHSHYTQKSLSLSPG
SEQ ID NO:59: adult IgG1 with mouse heavy chain MigG1 signal sequence (bold), Cys to Ser substitution (#),M428L, N434S mutations (bold/underlined), C-terminal G4S (italic) and C-terminal IgA peptide (underlined), and allotype G1m(fa) (bold italic) 
MGWSCIILFLVATATGVHSEPKSS(#)DKTHTCPPCPAPELLG
GPSVFLFPPKPKDTLMISRTPEVTCVVVDVSHEDPEVKFNWYVDG
VEVHNAKTKPREEQYNSTYRVVSVLTVLHQDWLNGKEYKCKVS
NKALPAPIEKTISKAKGQPREPQVYTLPPSRDELTKNQVSLTCLVK
GFYPSDIAVEWESNGQPENNYKTTPPVLDSDGSFFLYSKLTVDKS
RWQQGNVFSCSVLHEALHSHYTQKSLSLSPGGGGGSQRNPRLRLI
RRHPTLRIPPI
SEQ ID NO:60: adult IgG1 Fc with Z₁ being Cys or Ser, and X₁ being Met or Trp, X₂ being Ser or Thr, X₃ being Thr or Glu, X₄ being Asp or Glu, and X₅ being Leu or Met, X₆ being Met or Leu, and X₇ being Asn or Ser 
NVNHKPSNTKVDKKVEPKSZ1DKTHTCPPCPAPELLGGPSVFLFPPKPKDTLX1IX2RX3PEVTCVV
VDVSHEDPEVKFNWYVDGVEVHNAKTKPREEQYNSTYRVVSVLTVLHQDWLNGKEYKCKVSNKAL
PAPIEKTISKAKGQPREPQVYTLPPSRX4EX5TKNQVSLTCLVKGFYPSDIAVEWESNGQPENNYKTTPP
VLDSDGSFFLYSKLTVDKSRWQQGNVFSCSVX6HEALHX7HYTQKSLSLSPGK
SEQ ID NO:61: adult IgG1 Fc with Cys to Ser substitution (#), and X₁ being Met or Trp, X₂ being Ser or Thr, X₃ being Thr or Glu, X₄ being Asp or Glu, and X₅ being Leu or Met, X₆ being Met or Leu, and X₇ being Asn or Ser 
NVNHKPSNTKVDKKVEPKSS(#)DKTHTCPPCPAPELLGGPSVFLFPPKPKDTLX1IX2RX3PEVTCV
VVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRX4EX5TKNQVSLTCLVKGFYPSDIAVEWESNGQPENNYKTT
PPVLDSDGSFFLYSKLTVDKSRWQQGNVFSCSVX6HEALHX7HYTQKSLSLSPGK
SEQ ID NO:62: adult IgG1 Fc with Cys to Ser substitution (#), X₄ being Asp or Glu, and X₅ being Leu or Met 
NVNHKPSNTKVDKKVEPKSS(#)DKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLNGKEYKCKVSNKALP
APIEKTISKAKGQPREPQVYTLPPSRX4EX5TKNQVSLTCLVKGFYPSDIAVEWESNGQPENNYKTTPPV
LDSDGSFFLYSKLTVDKSR WQQGNVFSCSVMHEALHNHYTQKSLSLSPGK
SEQ ID NO:63: adult IgG1 Fc with Cys to Ser substitution (#), allotype G1m(f) (bold italic) 
NVNHKPSNTKVDKKVEPKSS(#)DKTHTCPPCPAPELLGGPSV
FLFPPKPKDTLMISRTPEVTCVVVDVSHEDPEVKFNWYVDGVEV
HNAKTKPREEQYNSTYRVVSVLTVLHQDWLNGKEYKCKVSNKA
LPAPIEKTISKAKGQPREPQVYTLPPSREEMTKNQVSLTCLVKGFY
PSDIAVEWESNGQPENNYKTTPPVLDSDGSFFLYSKLTVDKSRWQ
QGNVFSCSVMHEALHNHYTQKSLSLSPGK
SEQ ID NO:64: adult IgG1 Fc with Cys to Ser substitution (#), allotype G1m(fa) (bold italic) 
NVNHKPSNTKVDKKVEPKSS(#)DKTHTCPPCPAPELLGGPSV
FLFPPKPKDTLMISRTPEVTCVVVDVSHEDPEVKFNWYVDGVEV
HNAKTKPREEQYNSTYRVVSVLTVLHQDWLNGKEYKCKVSNKA
LPAPIEKTISKAKGQPREPQVYTLPPSRDELTKNQVSLTCLVKGFY
PSDIAVEWESNGQPENNYKTTPPVLDSDGSFFLYSKLTVDKSRWQ
QGNVFSCSVMHEALHNHYTQKSLSLSPGK
SEQ ID NO:65: adult IgG1 Fc with Cys to Ser substitution (#), M428L, N434S mutations (bold/underlined), allotype G1m(fa) (bold italic) 
NVNHKPSNTKVDKKVEPKSS(#)DKTHTCPPCPAPELLGGPSV
FLFPPKPKDTLMISRTPEVTCVVVDVSHEDPEVKFNWYVDGVEV
HNAKTKPREEQYNSTYRVVSVLTVLHQDWLNGKEYKCKVSNKA
LPAPIEKTISKAKGQPREPQVYTLPPSRDELTKNQVSLTCLVKGFY
PSDIAVEWESNGQPENNYKTTPPVLDSDGSFFLYSKLTVDKSRWQ
QGNVFSCSVLHEALHSHYTQKSLSLSPGK
SEQ ID NO:66: adult IgG1 Fc with Cys to Ser substitution (#), M428L, N434S mutations (bold/underlined), allotype G1m(f) (bold italic) 
NVNHKPSNTKVDKKVEPKSS(#)DKTHTCPPCPAPELLGGPSV
FLFPPKPKDTLMISRTPEVTCVVVDVSHEDPEVKFNWYVDGVEV
HNAKTKPREEQYNSTYRVVSVLTVLHQDWLNGKEYKCKVSNKA
LPAPIEKTISKAKGQPREPQVYTLPPSREEMTKNQVSLTCLVKGFY
PSDIAVEWESNGQPENNYKTTPPVLDSDGSFFLYSKLTVDKSRWQ
QGNVFSCSVLHEALHSHYTQKSLSLSPGK
SEQ ID NO:67: adult IgG1 Fc with Cys to Ser substitution (#), YTE triple mutation (bold/underlined), allotype G1m(fa) (bold italic) 
NVNHKPSNTKVDKKVEPKSS(#)DKTHTCPPCPAPELLGGPSV
FLFPPKPKDTLYITREPEVTCVVVDVSHEDPEVKFNWYVDGVEV
HNAKTKPREEQYNSTYRVVSVLTVLHQDWLNGKEYKCKVSNKA
LPAPIEKTISKAKGQPREPQVYTLPPSRDELTKNQVSLTCLVKGFY
PSDIAVEWESNGQPENNYKTTPPVLDSDGSFFLYSKLTVDKSRWQ
QGNVFSCSVMHEALHNHYTQKSLSLSPGK
SEQ ID NO:68: adult IgG1 Fc with Cys to Ser substitution (#), YTE triple mutation (bold and underlined), allotype G1m(f) (bold italic)

NVNHKPSNTKVDKKVEPKSS(#)DKTHTCPPCPAPELLGGPSV
FLFPPKPKDTLYITREPEVTCVVVDVSHEDPEVKFNWYVDGVEV
HNAKTKPREEQYNSTYRVVSVLTVLHQDWLNGKEYKCKVSNKA
LPAPIEKTISKAKGQPREPQVYTLPPSREEMTKNQVSLTCLVKGFY
PSDIAVEWESNGQPENNYKTTPPVLDSDGSFFLYSKLTVDKSRWQ
QGNVFSCSVMHEALHNHYTQKSLSLSPGK

In some embodiments, in the conjugate of the present application, each E is a naturally occurring albumin or a variant thereof, such as an engineered variant of a naturally occurring albumin. The variant comprises polymorphisms, fragments such as domains and subdomains, and fusion proteins. The albumin may comprise an albumin sequence obtained from any source. Preferably, the source is a mammal, such as human or bovine. Most preferably, the albumin is human serum albumin (HSA) or a variant thereof. The human serum albumin includes any albumin having an amino acid sequence naturally occurring in humans and variants thereof. An albumin coding sequence can be obtained by methods known to those skilled in the art for isolating and sequencing cDNA corresponding to the human gene. The albumin of the present application may comprise the amino acid sequence of human serum albumin (HSA) as set forth in SEQ ID NO 69 or SEQ ID NO 70, or the amino acid sequence of mouse serum albumin (MSA) as set forth in SEQ ID NO 71, or a variant or fragment thereof, preferably a functional variant or fragment thereof. The fragment or variant may or may not be functional, or may retain the albumin function to some extent. For example, the fragment or variant may retain the ability to bind an albumin receptor (such as HSA or MSA) at least 10%, 20%, 30%, 40%, 50%, 60%, 70%, 80%, 90%, 100%, or 105% of the ability of the parent albumin (e.g., the parent albumin from which the fragment or variant is derived). The relative binding capacity can be determined by methods known in the art, such as surface plasmon resonance.
SEQ ID NO:69 (Human Serum Albumin (HSA), Variant 1)
SEQ ID NO:70 (Human Serum Albumin (HSA), Variant 2) 
RGVFRRDAHKSEVAHRFKDLGEENFKALVLIAFAQYLQQCPFEDHVKLVNEVTEFAKTCVADES
AENCDKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELRDEGKAS
SAKQRLKCASLQKFGERAFKAWAVARLSQRFPKAEFAEVSKLVTDLTKVHTECCHGDLLECADDRAD
LAKYICENQDSISSKLKECCEKPLLEKSHCIAEVENDEMPADLPSLAADFVESKDVCKNYAEAKDVFL
GMFLYEYARRHPDYSVVLLLRLAKTYETTLEKCCAAADPHECYAKVFDEFKPLVEEPQNLIKQNCELF
EQLGEYKFQNALLVRYTKKVPQVSTPTLVEVSRNLGKVGSKCCKHPEAKRMPCAEDYLSVVLNQLC
VLHEKTPVSDRVTKCCTESLVNRRPCFSALEVDETYVPKEFNAETFTFHADICTLSEKERQIKKQTALV
ELVKHKPKATKEQLKAVMDDFAAFVEKCCKADDKETCFAEEGKKLVAASQAALGL
SEQ ID NO:71 (Mouse Serum Albumin (MSA))

RGVFRREAHKSEIAHRYNDLGEQHFKGLVLIAFSQYLQKCSYDEHAKLVQEVTDFAKTCVADES
AANCDKSLHTLFGDKLCAIPNLRENYGELADCCTKQEPERNECFLQHKDDNPSLPPFERPEAEAMCTS
FKENPTTFMGHYLHEVARRHPYFYAPELLYYAEQYNEILTQCCAEADKESCLTPKLDGVKEKALVSSV
RQRMKCSSMQKFGERAFKAWAVARLSQTFPNADFAEITKLATDLTKVNKECCHGDLLECADDRAELA
KYMCENQATISSKLQTCCDKPLLKKAHCLSEVEHDTMPADLPAIAADFVEDQEVCKNYAEAKDVFLG
TFLYEYSRRHPDYSVSLLLRLAKKYEATLEKCCAEANPPACYGTVLAEFQPLVEEPKNLVKTNCDLYE
KLGEYGFQNAILVRYTQKAPQVSTPTLVEAARNLGRVGTKCCTLPEDQRLPCVEDYLSAILNRVCLLH

EKTPVSEHVTKCCSGSLVERRPCFSALTVDETYVPKEFKAETFTFHSDICTLPEKEKQIKKQTALAELVK
HKPKATAEQLKTVMDDFAQFLDTCCKAADKDTCFSTEGPNLVTRCKDALA

In some embodiments of any aspect according to the present application, E (e.g., each E) comprises the amino acid sequence as set forth in SEQ ID NO:1. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO: 1.

In some embodiments of any aspect according to the present application, E (e.g., each E) comprises the amino acid sequence as set forth in SEQ ID NO:2. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:2.

In some embodiments of any aspect according to the present application, E (e.g., each E) comprises the amino acid sequence as set forth in SEQ ID NO:3. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:3.

In some embodiments of any aspect according to the present application, E (e.g., each E) comprises the amino acid sequence as set forth in SEQ ID NO:4. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:4.

In some embodiments of any aspect according to the present application, E (e.g., each E) comprises the amino acid sequence as set forth in SEQ ID NO:5. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:5.

In some embodiments of any aspect according to the present application, E (e.g., each E) comprises the amino acid sequence as set forth in SEQ ID NO:6. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:6.

In some embodiments of any aspect according to the present application, E (e.g., each E) comprises the amino acid sequence as set forth in SEQ ID NO:7. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:7.

In some embodiments of any aspect according to the present application, E (e.g., each E) comprises the amino acid sequence as set forth in SEQ ID NO:8. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:8.

In some embodiments of any aspect according to the present application, E (e.g., each E) comprises the amino acid sequence as set forth in SEQ ID NO:9. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:9.

In some embodiments of any aspect according to the present application, E (e.g., each E) comprises the amino acid sequence as set forth in SEQ ID NO:10. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO: 10.

In some embodiments of any aspect according to the present application, E (e.g., each E) comprises the amino acid sequence as set forth in SEQ ID NO:11. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO: 11.

In some embodiments of any aspect according to the present application, E (e.g., each E) comprises the amino acid sequence as set forth in SEQ ID NO:12. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO: 12.

In some embodiments of any aspect according to the present application, E (e.g., each E) comprises the amino acid sequence as set forth in SEQ ID NO:13. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:13.

In some embodiments of any aspect according to the present application, E (e.g., each E) comprises the amino acid sequence as set forth in SEQ ID NO:14. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO: 14.

In some embodiments of any aspect according to the present application, E (e.g., each E) comprises the amino acid sequence as set forth in SEQ ID NO:15. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:15.

In some embodiments of any aspect according to the present application, E (e.g., each E) comprises the amino acid sequence as set forth in SEQ ID NO:16. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO: 16.

In some embodiments of any aspect according to the present application, E (e.g., each E) comprises the amino acid sequence as set forth in SEQ ID NO:17. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO: 17.

In some embodiments of any aspect according to the present application, E (e.g., each E) comprises the amino acid sequence as set forth in SEQ ID NO:18. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:18.

In some embodiments of any aspect according to the present application, E (e.g., each E) comprises the amino acid sequence as set forth in SEQ ID NO:19. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO: 19.

In some embodiments of any aspect according to the present application, E (e.g., each E) comprises the amino acid sequence as set forth in SEQ ID NO:20. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:20.

In some embodiments of any aspect according to the present application, E (e.g., each E) comprises the amino acid sequence as set forth in SEQ ID NO:21. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:21.

In some embodiments of any aspect according to the present application, E (e.g., each E) comprises the amino acid sequence as set forth in SEQ ID NO:22. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:22.

In some embodiments of any aspect according to the present application, E (e.g., each E) comprises the amino acid sequence as set forth in SEQ ID NO:23. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:23.

In some embodiments of any aspect according to the present application, E (e.g., each E) comprises the amino acid sequence as set forth in SEQ ID NO:24. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:24.

In some embodiments of any aspect according to the present application, E (e.g., each E) comprises the amino acid sequence as set forth in SEQ ID NO:25. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:25.

In some embodiments of any aspect according to the present application, E (e.g., each E) comprises the amino acid sequence as set forth in SEQ ID NO:26. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:26.

In some embodiments of any aspect according to the present application, E (e.g., each E) comprises the amino acid sequence as set forth in SEQ ID NO:27. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:27.

In some embodiments of any aspect according to the present application, E (e.g., each E) comprises the amino acid sequence as set forth in SEQ ID NO:28. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:28.

In some embodiments of any aspect according to the present application, E (e.g., each E) comprises the amino acid sequence as set forth in SEQ ID NO:29. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:29.

In some embodiments of any aspect according to the present application, E (e.g., each E) comprises the amino acid sequence as set forth in SEQ ID NO:30. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:30.

In some embodiments of any aspect according to the present application, E (e.g., each E) comprises the amino acid sequence as set forth in SEQ ID NO:31. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:31.

In some embodiments of any aspect according to the present application, E (e.g., each E) comprises the amino acid sequence as set forth in SEQ ID NO:32. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:32.

In some embodiments of any aspect according to the present application, E (e.g., each E) comprises the amino acid sequence as set forth in SEQ ID NO:33. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:33.

In some embodiments of any aspect according to the present application, E (e.g., each E) comprises the amino acid sequence as set forth in SEQ ID NO:34. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:34.

In some embodiments of any aspect according to the present application, E (e.g., each E) comprises the amino acid sequence as set forth in SEQ ID NO:35. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:35.

In some embodiments of any aspect according to the present application, E (e.g., each E) comprises the amino acid sequence as set forth in SEQ ID NO:36. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:36.

In some embodiments of any aspect according to the present application, E (e.g., each E) comprises the amino acid sequence as set forth in SEQ ID NO:37. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:37.

In some embodiments of any aspect according to the present application, E (e.g., each E) comprises the amino acid sequence as set forth in SEQ ID NO:38. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:38.

In some embodiments of any aspect according to the present application, E (e.g., each E) comprises the amino acid sequence as set forth in SEQ ID NO:39. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:39.

In some embodiments of any aspect according to the present application, E (e.g., each E) comprises the amino acid sequence as set forth in SEQ ID NO:40. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:40.

In some embodiments of any aspect according to the present application, E (e.g., each E) comprises the amino acid sequence as set forth in SEQ ID NO:41. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:41.

In some embodiments of any aspect according to the present application, E (e.g., each E) comprises the amino acid sequence as set forth in SEQ ID NO:42. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:42.

In some embodiments of any aspect according to the present application, E (e.g., each E) comprises the amino acid sequence as set forth in SEQ ID NO:43. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:43.

In some embodiments of any aspect according to the present application, E (e.g., each E) comprises the amino acid sequence as set forth in SEQ ID NO:44. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:44.

In some embodiments of any aspect according to the present application, E (e.g., each E) comprises the amino acid sequence as set forth in SEQ ID NO:45. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:45.

In some embodiments of any aspect according to the present application, E (e.g., each E) comprises the amino acid sequence as set forth in SEQ ID NO:46. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:46.

In some embodiments of any aspect according to the present application, E (e.g., each E) comprises the amino acid sequence as set forth in SEQ ID NO:47. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:47.

In some embodiments of any aspect according to the present application, E (e.g., each E) comprises the amino acid sequence as set forth in SEQ ID NO:48. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:48.

In some embodiments of any aspect according to the present application, E (e.g., each E) comprises the amino acid sequence as set forth in SEQ ID NO:49. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:49.

In some embodiments of any aspect according to the present application, E (e.g., each E) comprises the amino acid sequence as set forth in SEQ ID NO:50. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:50.

In some embodiments of any aspect according to the present application, E (e.g., each E) comprises the amino acid sequence as set forth in SEQ **ID** NO:51. **In** some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:51.

**In** some embodiments of any aspect according to the present application, E (e.g., each E) comprises the amino acid sequence as set forth in SEQ ID NO:52. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:52.

In some embodiments of any aspect according to the present application, E (e.g., each E) comprises the amino acid sequence as set forth in SEQ ID NO:53. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:53.

In some embodiments of any aspect according to the present application, E (e.g., each E) comprises the amino acid sequence as set forth in SEQ ID NO:54. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:54.

In some embodiments of any aspect according to the present application, E (e.g., each E) comprises the amino acid sequence as set forth in SEQ ID NO:55. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:55.

In some embodiments of any aspect according to the present application, E (e.g., each E) comprises the amino acid sequence as set forth in SEQ ID NO:56. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:56.

In some embodiments of any aspect according to the present application, E (e.g., each E) comprises the amino acid sequence as set forth in SEQ ID NO:57. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:57.

In some embodiments of any aspect according to the present application, E (e.g., each E) comprises the amino acid sequence as set forth in SEQ ID NO:58. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:58.

In some embodiments of any aspect according to the present application, E (e.g., each E) comprises the amino acid sequence as set forth in SEQ ID NO:59. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:59.

In some embodiments of any aspect according to the present application, E (e.g., each E) comprises the amino acid sequence as set forth in SEQ ID NO:60. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:60.

In some embodiments of any aspect according to the present application, E (e.g., each E) comprises the amino acid sequence as set forth in SEQ ID NO:61. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:61.

In some embodiments of any aspect according to the present application, E (e.g., each E) comprises the amino acid sequence as set forth in SEQ ID NO:62. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:62.

In some embodiments of any aspect according to the present application, E (e.g., each E) comprises the amino acid sequence as set forth in SEQ ID NO:63. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:63.

In some embodiments of any aspect according to the present application, E (e.g., each E) comprises the amino acid sequence as set forth in SEQ ID NO:64. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:64.

In some embodiments of any aspect according to the present application, E (e.g., each E) comprises the amino acid sequence as set forth in SEQ ID NO:65. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:65.

In some embodiments of any aspect according to the present application, E (e.g., each E) comprises the amino acid sequence as set forth in SEQ ID NO:66. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:66.

In some embodiments of any aspect according to the present application, E (e.g., each E) comprises the amino acid sequence as set forth in SEQ ID NO:67. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:67.

In some embodiments of any aspect according to the present application, E (e.g., each E) comprises the amino acid sequence as set forth in SEQ ID NO:68. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:68.

In some embodiments of any aspect according to the present application, E (e.g., each E) comprises the amino acid sequence as set forth in SEQ ID NO:69. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:69.

In some embodiments of any aspect according to the present application, E (e.g., each E) comprises the amino acid sequence as set forth in SEQ ID NO:70. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:70.

In some embodiments of any aspect according to the present application, E (e.g., each E) comprises the amino acid sequence as set forth in SEQ ID NO:71. In some embodiments, E comprises an amino acid sequence with a sequence identity of at least 70%, 75%, 80%, 85%, 90%, 95%, 96%, 97%, 98%, 99% or 100% as compared with the sequence as set forth in SEQ ID NO:71.

### Definitions

As used herein, the term "half maximal inhibitory concentration" refers to the concentration of a drug required for 50% inhibition *in vitro.*

As used herein, the term "viral infection" means the pathogenic growth of a virus (e.g., influenza virus) in a host organism (e.g., a mouse). A viral infection may be any condition in which the presence of a viral population is impairing the body of a host organism. Accordingly, a subject is "suffering from" a viral infection when an excessive viral population is present in or on the body of the host organism, or when the presence of the viral population is damaging cells or other tissues of the subject.

As used herein, the term "neuraminidase inhibitor" refers to a compound that reduces the activity of influenza virus neuraminidases (e.g., those from influenza A, B or C virus). Viral neuraminidase inhibitors known to those skilled in the art include zanamivir, peramivir, oseltamivir, and analogs thereof. The neuraminidase inhibitors according to the present application include zanamivir, peramivir, oseltamivir, and analogs thereof, such as neuraminidase inhibitors represented by Formulas (A-I) to (A-XIII) in the conjugate.

As used herein, the term "Fc domain monomer" refers to a polypeptide chain comprising at least one hinge domain and the second and third antibody constant domains (CH2 and CH3), or functional fragments thereof (e.g., fragments capable of: (i) dimerizing with another Fc domain monomer to form an Fc domain, and (ii) binding to an Fc receptor). The Fc domain monomer may be of any immunoglobulin antibody isotype, including IgG, IgE, IgM, IgA, or IgD (e.g., IgG). Additionally, the Fc domain monomer may be of an IgG subtype, including IgG1, IgG2a, IgG2b, IgG3, or IgG4 (e.g., IgG1). The Fc domain monomer does not comprise any portion of an immunoglobulin that can serve as an antigen-recognition region, such as variable domains or a complementarity determining regions (CDRs). The Fc domain monomer in the conjugate according to the present application may comprise one or more alterations (e.g., substitution, addition, or deletion of 1 to 10, 1 to 8, 1 to 6, 1 to 4 amino acids) as compared with the wild-type Fc domain monomer sequence, which alters the interaction between the Fc domain and the Fc receptor. Examples of suitable alterations are known in the art. In some embodiments, a human Fc domain monomer (e.g., an IgG heavy chain, such as IgG1) comprises a region extending from any one of Asn208, Glu216, Asp221, Lys222, or Cys226 to the heavy chain carboxy-terminus at Lys 447. The presence or absence of the C-terminal Lys447 of the Fc region does not affect the structure or stability of the Fc region. Unless otherwise specified herein, the numbering of the amino acid residues in the IgG or Fc domain monomer follows the EU numbering system for antibodies, also known as the Kabat EU index, as described, for example, in Kabat et al, Sequences of Proteins of Immunological Interest, 5th edition, Public Health Service, National Institutes of Health, Bethesda, MD, 1991.

As used herein, the term "Fc domain" refers to a dimer of two Fc domain monomers, which is capable of binding an Fc receptor. In a wild-type Fc domain, the two Fc domain monomers dimerize via the interaction between two CH3 antibody constant domains, and in some embodiments, one or more disulfide bonds form between the hinge domains of the two dimerized Fc domain monomers.

As used herein, the term "covalent linking" refers to the connection between two moieties in a conjugate via a covalent bond formed between two atoms in the two moieties of the conjugate.

As used herein, the term "Fc-binding peptide" refers to a polypeptide having an amino acid sequence with 5 to 50 (e.g., 5 to 40, 5 to 30, 5 to 20, 5 to 15, 5 to 10, 10 to 50, 10 to 30, or 10 to 20) amino acid residues, which has affinity for Fc domain (e.g., any Fc domain described in the present application) and has the function of binding the Fc domain. The Fc-binding peptides may be of various origins, such as synthetics, human, mouse, or rat. The Fc-binding peptides of the present application include Fc-binding peptides that have been engineered to contain one or more (e.g., two, three, four, or five) solvent-exposed lysine residues, which may provide sites for conjugation to a compound of the present application (e.g., to a dimer or trimer of the neuraminidase inhibitor represented by Formula (IV) or Formula (V), e.g., via a linker). Most preferably, the Fc-binding peptides contain a single solvent-exposed lysine, thereby enabling site-specific conjugation of the compound of the present application. An Fc-binding peptide may comprise only naturally occurring amino acid residues, or may also comprise one or more non-naturally occurring amino acid residues. The non-naturally occurring amino acid residues (e.g., side chains of non-naturally occurring amino acid residues) can be used as a conjugation site for the compound of the present application (e.g., a dimer or trimer of the neuraminidase inhibitor represented by formula (IV) or formula (V), e.g., via a linker). The Fc-binding peptide of the present application may be linear or cyclic. The Fc-binding peptides of the present application include any Fc-binding peptide known to those skilled in the art.

As used herein, the term "albumin" refers to a polypeptide comprising an amino acid corresponding to a naturally occurring albumin (e.g., human serum albumin) or a variant thereof (such as an engineered variant of the naturally occurring albumin). The variants of albumin comprise polymorphisms, fragments such as domains and subdomains, and fusion proteins (e.g., fusion albumin with a C-terminal or N-terminal polypeptide linker). Preferably, the albumin has the amino acid sequence of human serum albumin (HSA) or a variant or fragment thereof, most preferably a functional variant or fragment thereof. The albumins of the present application comprise proteins with a sequence identity of at least 70%, 75%, 80%, 85%, 90%, 91%, 92%, 93%, 94%, 95%, 96%, 97%, 98%, 99% or 100% as compared with any one of the sequences as set forth in SEQ ID NOs 69-71. The albumins of the present application comprise albumins that have been engineered to contain one or more (e.g., two, three, four, or five) solvent-exposed lysine residues, which can provide sites for conjugation of the compound of the present application (e.g., to a dimer or trimer of the neuraminidase inhibitor represented by Formula (IV) or Formula (V), e.g., via a linker). Most preferably, the albumin contains a single solvent-exposed lysine, thereby enabling site-specific conjugation of the compound of the present application (a dimer or trimer of the neuraminidase inhibitor represented by Formula (IV) or Formula (V)). The albumin may comprise only naturally occurring amino acid residues, or may also comprise one or more non-naturally occurring amino acid residues. The non-naturally occurring amino acid residues (e.g., side chains of non-naturally occurring amino acid residues) can be used as a conjugation site for the compound of the present application (e.g., a dimer or trimer of the neuraminidase inhibitor represented by formula (IV) or formula (V), e.g., via a linker).

As used herein, the term "albumin-binding peptide" refers to a polypeptide having an amino acid sequence with 5 to 50 (such as 5 to 40, 5 to 30, 5 to 20, 5 to 15, 5 to 10, 10 to 50, 10 to 30, or 10 to 20) amino acid residues, which has affinity for albumin (e.g., any albumin described in the present application) and has the function of binding the albumin. Preferably, the albumin-binding peptide is conjugated to naturally occurring serum albumin, most preferably human serum albumin. The albumin-binding peptides may be of various origins, such as synthetics, human, mouse, or rat. The albumin-binding peptides of the present application include albumin-binding peptides that have been engineered to contain one or more (e.g., two, three, four, or five) solvent-exposed lysine residues, which can provide sites for conjugation to a compound of the present application (e.g., to a dimer or trimer of the neuraminidase inhibitor represented by Formula (IV) or Formula (V), e.g., via a linker). Most preferably, the albumin-binding peptides contain a single solvent-exposed lysine, thereby enabling site-specific conjugation of the compound of the present application (a dimer or trimer of the neuraminidase inhibitor represented by Formula (IV) or Formula (V)). The albumin-binding peptide may comprise only naturally occurring amino acid residues, or may also comprise one or more non-naturally occurring amino acid residues. The non-naturally occurring amino acid residues (e.g., side chains of non-naturally occurring amino acid residues) can be used as a conjugation site for the compounds of the present application (a dimer or trimer of the neuraminidase inhibitor represented by Formula (IV) or Formula (V)). The albumin-binding peptide of the present application may be linear or cyclic. The albumin-binding peptides of the present application include any Fc-binding peptide known to those skilled in the art.

As used herein, a "surface-exposed amino acid" or a "solvent-exposed amino acid", such as a surface-exposed lysine, refers to an amino acid that is accessible to the solvent surrounding the protein. The surface-exposed amino acid may be naturally occurring, or introduced via engineered modifications (e.g., substitution or insertion) of the protein. In some embodiments, the surface-exposed amino acid is an amino acid whose substitution does not substantially alter the three-dimensional structure of the protein.

As used herein, the terms "linker" or "L" refer to a covalent linking between two or more than two components in a compound (such as, between the two neuraminidase inhibitors in the conjugate as described herein, between the neuraminidase inhibitor and an Fc domain monomer or an Fc domain or albumin in the conjugate as described herein, and between a multimer of the two neuraminidase inhibitors and an Fc domain monomer or an Fc domain or albumin in the conjugate as described herein).

In some embodiments, the compound as described herein may contain a linker having a trivalent structure (e.g., a trivalent linker). The trivalent linker has four arms, wherein each arm is covalently linked to a component of the compound (e.g., a first arm bound to a first neuraminidase inhibitor (e.g., A₁ in the conjugate represented by Formula (I)), a second arm bound to a second neuraminidase inhibitor (e.g., A₂ in the conjugate represented by Formula (I)), a third arm bound to a third neuraminidase inhibitor (e.g., A₃ in the conjugate represented by Formula (I)), and a fourth arm bound to an Fc protein or albumin).

As used herein, the term "amino acid" means naturally occurring amino acids and non-naturally occurring amino acids.

As used herein, the term "naturally occurring amino acid" means amino acids including Ala, Arg, Asn, Asp, Cys, Gln, Glu, Gly, His, Ile, Leu, Lys, Met, Phe, Pro, Ser, Thr, Trp, Tyr, and Val.

As used herein, the term "T" refers to the average number of neuraminidase inhibitor dimers or neuraminidase inhibitor trimers conjugated to the Fc domain monomer, Fc domain or albumin of the conjugate, which may range from 1 to 20 (e.g., T is 1 to 2, 1 to 3, 1 to 4, 1 to 5, 5 to 10, 10 to 15, or 15 to 20).

As used herein, the term "pharmaceutical composition" refers to a medicinal or pharmaceutical formulation comprising at least one active ingredient (e.g., any one of the conjugates represented by Formula (I) and Formula (II), and the compounds represented by Formula (IV) and Formula (V)) and one or more carriers and/or excipients so that the active ingredient is suitable for the intended route of administration.

As used herein, the term "about" can be understood to be within +/-10%, +/-9%, +/-8%, +/-7%, +/-6%, +/-5%, +/-4%, +/-3%, +/-2%, +/-1%, +/-0.5%, +/-0.4%, +/-0.3%, +/-0.2% or +/-0.1% of the mentioned value. For example, "about 10%" refers to a value between 9% and 11%. Unless otherwise apparent from the context, all numerical values provided herein are modified by the term "about".

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison × 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by, for example, using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, and with a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and with a gap weight of 16, 14, 12, 10, 8, 6, or 4 and a length weight of 1, 2, 3, 4, 5, or 6.

As used herein, the term "conservative substitution" refers to amino acid substitutions which would not disadvantageously affect or change the essential properties of a protein/polypeptide comprising the amino acid sequence. For example, a conservative substitution may be introduced by standard techniques known in the art such as site-directed mutagenesis and PCR-mediated mutagenesis. Conservative amino acid substitutions include substitutions wherein an amino acid residue is substituted with another amino acid residue having a similar side chain, for example, a residue physically or functionally similar (such as, having similar size, shape, charge, chemical property including the capability of forming covalent bond or hydrogen bond, etc.) to the corresponding amino acid residue. The families of amino acid residues having similar side chains have been defined in the art. These families include amino acids having basic side chains (for example, lysine, arginine and histidine), amino acids having acidic side chains (for example, aspartic acid and glutamic acid), amino acids having uncharged polar side chains (for example, glycine, asparagine, glutamine, serine, threonine, tyrosine, cysteine, and tryptophan), amino acids having nonpolar side chains (for example, alanine, valine, leucine, isoleucine, proline, phenylalanine, and methionine), amino acids having β-branched side chains (such as threonine, valine, and isoleucine) and amino acids having aromatic side chains (for example, tyrosine, phenylalanine, tryptophan, and histidine). Therefore, generally a conservative substitution refers to a substitution of a corresponding amino acid residue with another amino acid residue from the same side-chain family. Methods for identifying amino acid conservative substitutions are well known in the art (see, for example, Brummell et al., Biochem. 32: 1180-1187 (1993); Kobayashi et al., Protein Eng. 12(10): 879-884 (1999); and Burks et al., Proc. Natl. Acad. Sci. Set USA 94: 412-417 (1997), which are incorporated herein by reference).

As used herein, the term "subject" may be a human, non-human primates or other mammal, including, but not being limited to, dog, cat, horse, cattle, pig, turkey, goat, fish, monkey, chicken, rat, mouse, and sheep.

As used herein, the term "effective amount" refers to an amount that is sufficient to achieve a desired therapeutic effect, for example, an amount that achieves the alleviation of symptoms associated with diseases to be treated. In addition, it should be pointed out that the dosage and administration regimen of the conjugate or compound of the present application depends on a variety of factors including the patient's age, weight, gender, natural health status and nutritional status, activity intensity, administration time and metabolic rate of conjugate or compounds, severity of the disease, and subjective judgment of attending physicians. The preferred administration dosage range is between 0.001 mg/kg body weight/day and 1000 mg/kg body weight/day.

As used herein, the term "pharmaceutically acceptable carrier and/or excipient" refers to a carrier and/or excipient that is pharmacologically and/or physiologically compatible with subjects and active ingredients, which is well known in the art (see, e.g., Remington's Pharmaceutical Sciences, Edited by Gennaro AR, 19th ed. Pennsylvania: Mack Publishing Company, 1995). Pharmaceutically acceptable carriers and/or excipients include, but are not limited to, pH regulators, surfactants, ionic strength enhancers, diluents, osmotic pressure maintaining agents, absorption-delaying agents, preservatives, and stabilizers. For example, the pH regulators include, but are not limited to, phosphate buffers. The surfactants include, but are not limited to, cationic, anionic or nonionic surfactants, such as Tween-80. The ionic strength enhancers include, but are not limited to, sodium chloride. The preservatives include, but are not limited to, various antibacterial and antifungal agents, such as parabens, chlorobutanol, phenol, sorbic acid, and the like. The osmotic pressure maintaining agents include, but are not limited to, sugars, NaCl, and analogues thereof. The absorption-delaying agents include, but are not limited to, monostearates and gelatin. The diluents include, but are not limited to, water, aqueous buffers (e.g., buffered saline), alcohols and polyols (e.g., glycerol), and the like. The preservatives include, but are not limited to, various antibacterial agents and antifungal agents, such as thiomersal, 2-phenoxyethanol, parabens, chlorobutanol, phenol, sorbic acid and the like. The stabilizers have the meanings as commonly understood by those skilled in the art, and they can stabilize the desired activities of active ingredients in a drug, including, but being not limited to, sodium glutamate, gelatin, SPGA, saccharides (such as sorbitol, mannitol, starch, sucrose, lactose, dextran, or glucose), amino acids (such as glutamic acid, or glycine), proteins (such as dried whey, albumin, or casein) or degradation products thereof (such as lactalbumin hydrolysate), and the like.

### Brief Description of the Drawings

FIG. 1 and FIG. 2 show that the conjugates of the present application exhibit good NA enzyme inhibitory activity;
FIGS. 3 and 4 show that the conjugates of the present application exhibit good NA enzyme inhibitory activity against drug-resistant strain;
FIG. 5 shows that the conjugates of the present application have good plasma half-life and can maintain drug concentrations above the effective level for an extended period;
FIG. 6 shows that the conjugates of the present application can maintain ADCC effect comparable to that of Fc;
FIG. 7 shows that a single administration of the conjugates of the present application can effectively inhibit viral replication at different virus challenge doses;
FIG. 8 shows that the conjugates of the application can effectively alleviate body weight loss in the severe influenza DBA/2 mouse model;
FIG. 9 to FIG. 10 show that the negative control group as well as small molecule drugs oseltamivir and zanamivir fail to exert protective effects on a severe influenza DBA/2 mouse model;
FIG. 11 shows the survival curve results of the conjugates of the present application versus the severe influenza DBA/2 mouse model, demonstrating that the conjugates of the present application have good protective effect on DBA/2 mice suffered from severe influenza;
FIG. 12 and FIG. 13 show the HPLC chromatogram and mass spectrum (MS) of conjugate I-1 prepared in one example of the present application, respectively;
FIG. 14 and FIG. 15 show the HPLC chromatogram and mass spectrum (MS) of conjugate I-5 prepared in one example of the present application, respectively;
FIG. 16 and FIG. 17 show the HPLC chromatogram and mass spectrum (MS) of conjugate I-6 prepared in one example of the present application, respectively;
FIG. 18 and FIG. 19 show the HPLC chromatogram and mass spectrum (MS) of conjugate II-1 prepared in one example of the present application, respectively;
FIG. 20 and FIG. 21 show the HPLC chromatogram and mass spectrum (MS) of conjugate II-6 prepared in one example of the present application, respectively;
FIG. 22 and FIG. 23 show the HPLC chromatogram and mass spectrum (MS) of conjugate I-9 prepared in one example of the present application, respectively;
FIG. 24 and FIG. 25 show HPLC chromatogram and mass spectrum (MS) of conjugate I-6 prepared in another example of the present application, respectively;
FIGS. 26 to FIG. 29 show the inhibition curves of the conjugates of the present application against A/Puerto Rico/8/1934 (H1N1-PR8), A/Victoria/2570/2019 (H1N1), A/Darwin/9/2021 (H3N2), and B/Austria/1359417/2021 (BV) viruses, respectively;
FIG. 30 shows that therapeutic administration of the conjugates of the present application can effectively alleviate body weight loss in the H1N1-PR8-infected BALB/c mouse model;
FIG. 31 shows that therapeutic administration of the conjugates of the present application exerts protective effects and improves the survival rate of the H1N1-PR8-infected BALB/c mouse model;
FIG. 32 shows that therapeutic administration of the conjugates of the present application can effectively inhibit the replication of H1N1-PR8 virus in the BALB/c mouse model, **** indicates a significance level of P < 0.0001;
FIG. 33 shows that prophylactic administration of the conjugates of the present application can effectively alleviate body weight loss in the H1N1-PR8-infected BALB/c mouse model;
FIG. 34 shows that prophylactic administration of the conjugates of the present application exerts protective effects and improves the survival rate of the H1N1-PR8-infected BALB/c mouse model;
FIG. 35 shows that prophylactic administration of the conjugates of the present application can effectively inhibit the replication of H1N1-PR8 virus in the BALB/c mouse model, **** indicates a significance level of P < 0.0001.

### Detailed Description of the Preferred Embodiment

### Example 1 Synthesis of Divalent Scaffold

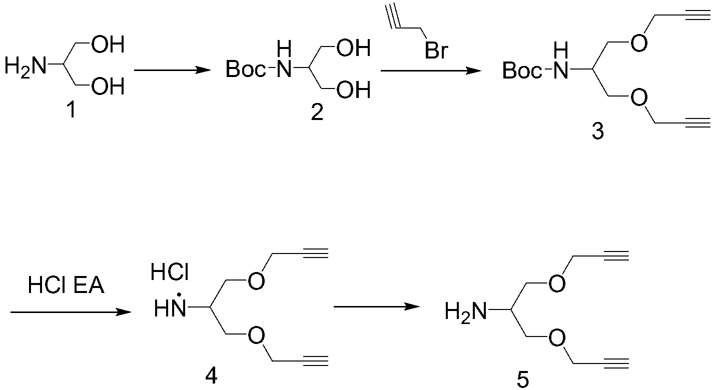

### 1) Preparation of Compound 2:

2-Amino-1,3-propanediol (5 g, 54.9 mmol) was dissolved in 150 ml of anhydrous ethanol. Di-tert-butyl dicarbonate (Boc₂O, 12 g, 54.98 mmol, dissolved in 100 ml anhydrous ethanol) was added, and the mixture was stirred at room temperature, and reacted for 6 hours. The reaction solution was concentrated to dryness using a rotary evaporator, the product was recrystallized from n-heptane to obtain a white solid. After filtration and vacuum drying, a solid was obtained (7 mg, 90% yield). [M+H]⁺=192.2

### 2) Preparation of Compound 3:

Under the condition of an ice bath, KOH (7.87 g, 140.18 mmol) was dispersed in 20ml of ultra-dry DMF, to obtain a DMF solution containing KOH. Compound 2 (5 g, 26.2 mmol) was dissolved in a mixed solvent of 20ml THF and 8ml DMF, and then the resulting solution was slowly added to the DMF solution containing KOH. 3-Bromopropyne (12.46 g, 104.7 mmol) was dropwise added to the reaction solution, and the solution was turned brown and reacted at 0 °C for 7 h. The reaction solution was extracted with 100 ml of H₂O and 140 ml of DCM, respectively, and the extraction operation was repeated twice. The DCM extracts were combined, then were washed with saturated brine, and left to stand over anhydrous sodium sulfate for 8 h. After purification by column chromatography and vacuum drying, a brown-yellow solid was obtained (4 g, 50.2% yield), [M+H]⁺=268.31

### 3) Preparation of Compound 4:

Compound 3 (2 g, 7.49 mmol) was dissolved in 15 ml of HCl in ethyl acetate, and the mixture was stirred at room temperature for 3 h, the reaction solution was concentrated to dryness by a rotary evaporator, to obtain a brown oily liquid. Since the reaction solution contains no other impurities, the crude product was used directly in the next step.

### 4) Preparation of Compound 5:

The brown oily liquid (Compound 4) obtained in the previous step was dissolved in 2 ml of MeOH, to which 15 ml of aqueous NaHCO₃ solution was added, and after 2 hours of reaction, the pH of the reaction solution was determined to be 7. The reaction solution was extracted with 50 ml of DCM and 30 ml of H₂O, respectively, and the extraction operation was repeated twice. After the DCM extracts were combined, the resulting mixture was washed with saturated NaCl and dried overnight over anhydrous sodium sulfate. After purification by column chromatography and vacuum drying, 1.26 g of yellow oil were obtained (85% yield). ¹H NMR (600 MHz, DMSO-*d*₆) δ 4.13 (d, 4H), 3.42 (t, 2H), 3.38 (dd, 2H), 3.29 (dd, 2H), 2.97 (tt, 1H), 1.55 (s, 2H). [M+H]⁺=168.01

### Example 2 Synthesis of Trivalent Scaffold

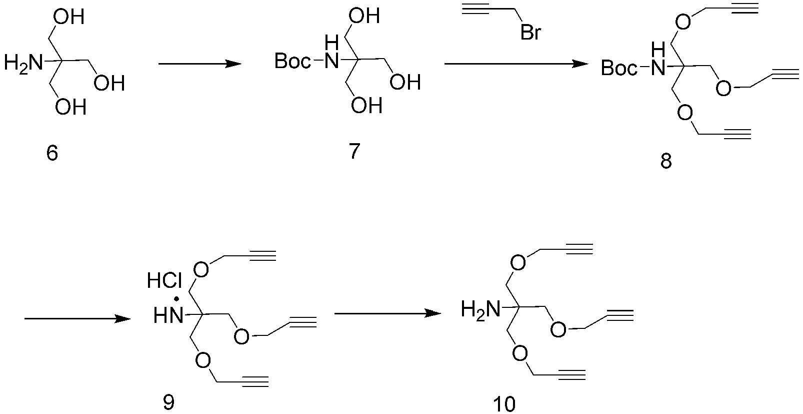

### 1) Preparation of Compound 7:

Tris(hydroxymethyl)aminomethane (5 g, 41.27 mmol) was dissolved in a mixed solution of 30 ml methanol and 30 ml t-butanol to obtain a suspension. A solution of di-tert-butyl dicarbonate (Boc₂O, 11.75 g, 53.84 mmol) in tert-butanol was added dropwise. The mixture was stirred at room temperature, and a white precipitate gradually formed in the reaction solution. After 8 h, the reaction solution was concentrated to dryness with a rotary evaporator to obtain a white solid. Ethyl acetate was added to the solid, the mixture was cooled to 0 °C and reacted for 2 h. After filtration and vacuum drying, a solid was obtained (8.20 mg, 90% yield). [M+H]⁺=222.80

### 2) Preparation of Compound 8:

Under the condition of an ice bath, KOH (7.87 g, 140.18 mmol) was dispersed in 20 ml of ultra-dry DMF, to obtain a DMF solution containing KOH. N-Boc-tris(hydroxymethyl)aminomethane (Compound 7, 5 g, 22.61 mmol) was dissolved in a mixed solvent of 20ml THF and 8ml DMF, and then the resulting solution was slowly added to the DMF solution containing KOH. 3-Bromopropyne (16.68 g, 140.18) was dropwise added to the reaction solution, and the solution was turned brown and reacted at 0 °C for 6 h. The reaction solution was extracted with 100 ml of H₂O and 140 ml of DCM, respectively, and the extraction operation was repeated twice. The DCM extracts were combined, then were washed with saturated brine, and left to stand over anhydrous sodium sulfate for 8 h. 3.6 g of a brown-yellow solid were obtained after purification by column chromatography and vacuum drying (47.5% yield), [M+H]⁺=336.29

### 3) Preparation of Compound 9:

Compound 8 (1 g, 2.98 mmol) was dissolved in 15 ml of HCll in ethyl acetate, and the mixture was stirred at room temperature, and after 3 h, the reaction solution was concentrated to dryness by a rotary evaporator, to obtain a brown oily liquid. Since the reaction solution contains no other impurities, the crude product was used directly in the next step.

### 4) Preparation of Compound 10:

The brown oily liquid (Compound 9) obtained in the previous step was dissolved with 2 ml of MeOH, to which 15 ml of aqueous NaHCO₃ solution was added, and after 2 hours of reaction, the pH of the reaction solution was determined to be 7. The reaction solution was extracted with 50 ml of DCM and 30 ml of H₂O, respectively, and the extraction operation was repeated twice. After the DCM extracts were combined, the resulting mixture was washed with saturated NaCl and dried overnight over anhydrous sodium sulfate. 0.58 g of a yellow oily substance were obtained after purification by column chromatography and vacuum drying (83% yield), ¹H NMR (600 MHz, DMSO-d₆) δ 4.14 (d, 6H), 3.42 (t, 3H), 3.32 (s, 6H), 1.47 (s, 2H). [M+H]⁺=236.12

### Example 3 Synthesis of Zanamivir Dimer Derivative

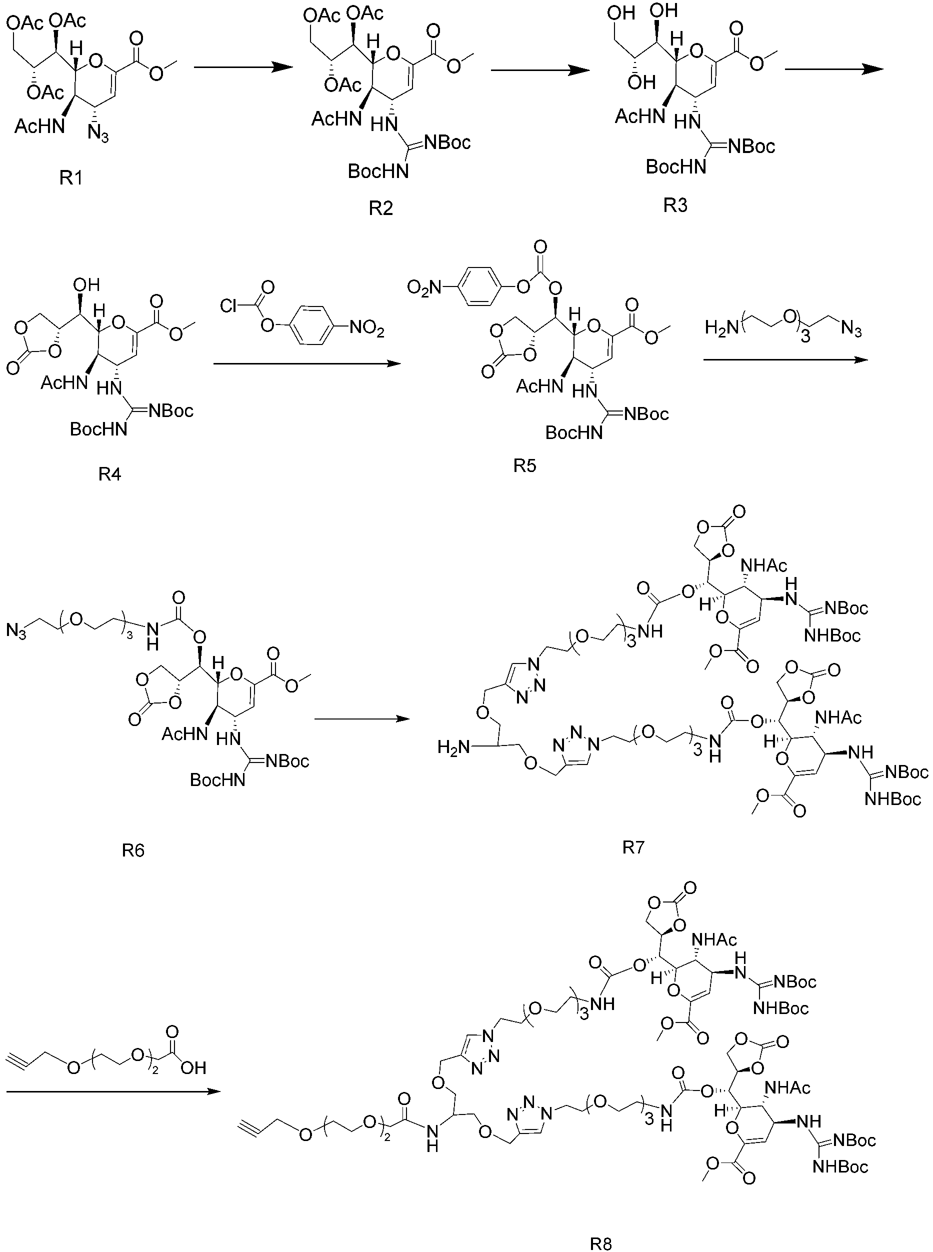

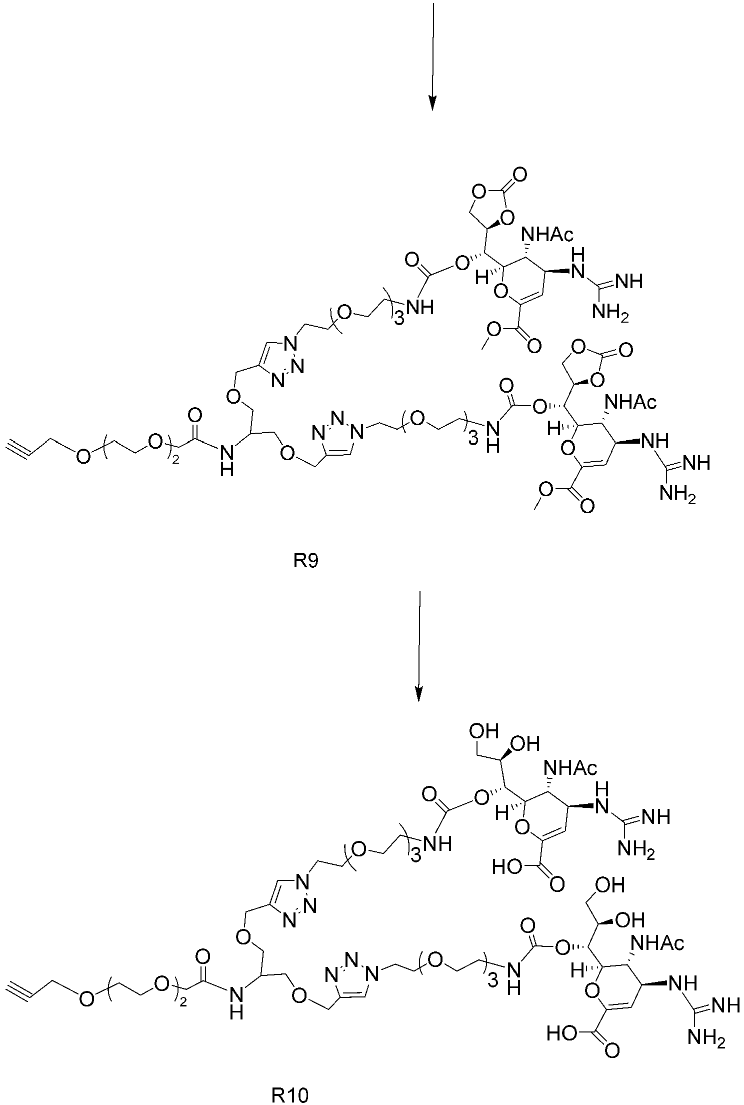

### 1) Preparation of Compound R2:

Methyl 5-acetamido-7,8,9-O-triacetyl-2,6-anhydrous-4-azido-3,4,5-trideoxy-D-glycerol-D-galacto-non-2-enoate (Compound R1, 2.28 g, 5.0 mmol) was dissolved in anhydrous THF (15 mL), and the solution was cooled to about 13 °C. After 20 minutes, triphenylphosphine (1.58g, 6mmol) was added in several portions. The resulting mixture was stirred at about 13 °C to room temperature for 2 hours, followed by dropwise addition of a solution of LiOH (12 mg, 0.5 mmol) in water (1 mL). After stirring for 28 hours, N,N'-bis-boc-1-amidinopyrazole (1.63 g, 5.25 mmol) and 4-dimethylaminopyridine (122.2 mg, 1 mmol) were added to the reaction mixture. The reaction was stirred for 1.5 days. The mixture was then diluted with a 1:1 mixture of ethyl acetate and hexanes (100 mL) and extracted with water (30 mL). The aqueous layer was back extracted with ethyl acetate (30 mL). The organic layers were combined and concentrated by a rotary evaporator. The residue was purified by C₁₈ reverse-phase column chromatography (150 g, 25% to 70% acetonitrile in water). The collected fractions were concentrated at room temperature by a rotary evaporator. A turbid aqueous solution was formed, and most of the product were deposited on a flask in the form of a gel. The solution was then extracted with ethyl acetate (150 mL). The organic layers were used to redissolve the gel material. They were then dried over Na₂SO₄, concentrated by a rotary evaporator, and further dried under high vacuum to obtain the title compound as a white foam. Yield: 3 g, 90.2%. Ions found by LCMS: [M+H]+=673.2.

### 2) Preparation of Compound R3:

Compound R2 (3 g, 4.46 mmol) was dissolved in MeOH (15 mL) and cooled in an ice water bath, to which a 0.5 M solution of sodium methoxide in MeOH (13 mL, 6.5 mmol) was added slowly, and they were stirred for 1 hour, followed by carefully adjusting the pH to 7 to 7.5 by dropwise addition of a 1,4-dioxane solution of hydrochloric acid (4 M, 2 mL, Innochem, cat# A 18256). The solvent was removed by a rotary evaporator at a temperature not higher than room temperature. The residue was diluted with a 2:1 mixture (150mL) of ethyl acetate and hexane, and the obtained solution was extracted with water (20 mL). The aqueous layer was back extracted with ethyl acetate (30 mL). The organic layers were combined, dried over anhydrous Na₂SO₄, concentrated by a rotary evaporator and then dried under vacuum. The product was used in the subsequent step without further purification. Yield: 2.7 g, 98.4%. Ions found by LCMS: [M+H]+=547.26.

### 3) Preparation of Compound R4:

Compound R3 (1 g, 1.82 mmol) was dissolved in anhydrous DCM (6 mL) and cooled in an ice water bath, then to which 4-dimethylaminopyridine (221.26 mg, 1.82mmol) and DIPEA (728 mg, 5.6 mmol) were added. Next, p-nitrophenyl chloroformate (537.75 mg, 3.05 mmol) was added dropwise to the reaction solution. Then, the ice water bath was removed, and the reaction solution was stirred for 3 h with monitoring by LCMS (additional p-nitrophenyl chloroformate may be supplemented if necessary). After the reaction was completed, water (10 mL) was added to quench the reaction, and the organic layer was separated and concentrated by a rotary evaporator, followed by purification via C₁₈ reverse phase chromatography (100 g, 20% to 70% acetonitrile in water). Acetonitrile was removed from the collected fractions by a rotary evaporator at room temperature, and then the residue was extracted with a 1:1 mixture (120 mL) of ethyl acetate and hexane. The aqueous layer was extracted repeatedly with ethyl acetate (30 mL). The organic phases were combined and dried for 8 h over anhydrous Na₂SO₄. After concentration by a rotary evaporator and vacuum drying, a white solid product was obtained. Yield: 712 mg, 68.34 %. ¹H NMR (600 MHz, DMSO-d₆) δ 11.39 (s, 1H), 8.26 (d, 2H), 8.15 (d, 1H) , 5.80 (d, 1H) , 5.71 (d, 1H), 4.98 (m, 1H) , 4.88 (m, 1H) , 4.59 (m, 1H) , 4.55 (t, 1H) , 4.17 (dd, 1H) , 4.10 (m, 1H) , 4.01 (m, 1H) , 3.73 (s, 3H), 1.85 (s, 3H), 1.47 (s, 9H) , 1.40 (s, 9H). [M+H]⁺=573.24.

### 4) Preparation of Compound R5:

Compound R4 (700 mg, 1.22 mmol) was dissolved in 5 ml of ultra-dry DCM and cooled in an ice water bath, to which p-nitrophenyl chloroformate (294.6 mg, 1.46 mmol) was added, and then dimethylaminopyridine (123.2 mg, 0.61 mmol) was added, and under nitrogen protection, the ice water bath was removed, and the mixture was stirred and reacted for 3 h. Additional p-nitrophenyl chloroformate (201.8 mg, 1 mmol) was supplemented, and the reaction continued for 1 h. After the reaction was completed, the solvent was removed by a rotary evaporator and 540 mg of the product were obtained after purification by column chromatography, 60% yield.

### 5) Preparation of Compound R6:

Compound R5 (240 mg, 0.33 mmol) was dissolved in 5 ml of anhydrous DCM, to which azido-triethylene glycol amine (N₃-PEG₃-NH₂, 2-(2-(2-(2-azidoethoxy)ethoxy)ethoxy)ethylamine, 71 mg, 0.33 mmol), 1-hydroxybenzotriazole (HOBT, 43.96 mmg, 0.33 mmol), and N,N-diisopropylethylamine (DIPEA, 84.11 mg, 0.65 mmol) were added sequentially, and under nitrogen protection, the mixture was stirred at room temperature, and reacted for 8 h, to obtain 140 mg of the product following purification with column chromatography, 51% yield.1H NMR (600 MHz, DMSO-d6) δ 7.64 (m, 1H), 7.30 (m, 1H), 4.34 (t, 1H), 3.72 (d, 1H), 3.58 (m, 3H), 3.32 (m, 1H), 2.27 (t, 1H), 1.65 (m, 1H), 1.56 (m, 1H), 1.41 (t, 3H), 1.35 (m, 2H), 1.18 (s, 24H), 0.89 (t, 2H), 0.81 (t, 8H), 0.77 (t, 2H), [M+H]+=817.34

Azido-hexaethylene glycol amine (N₃-PEG₆-NH₂) and azido-nonaethylene glycol amine (N₃-PEG₉-NH₂) were used to replace azido-triethylene glycol amine (N₃-PEG₃-NH₂) respectively, , and Compound R6-1 and Compound R6-2 were prepared using the same method.

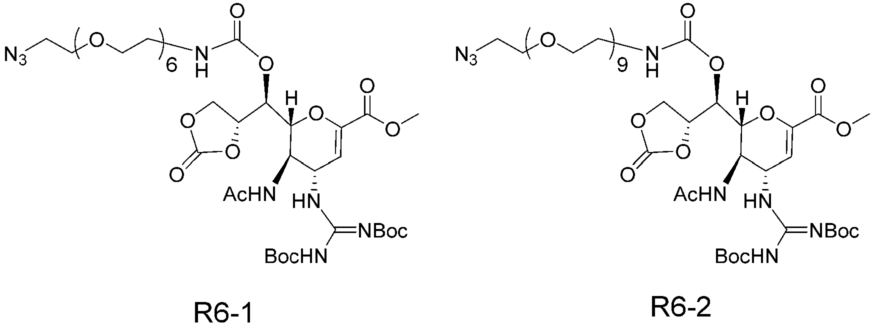

### 6) Preparation of Compound R7:

Compound R6 (140 mg, 0.168 mmol) and Compound 5 (11.29 mg, 0.0672 mmol) were dissolved in 1.5 ml of tetrahydrofuran (THF), respectively, and mixed with stirring. Aqueous solutions of CuSO₄·5H₂O (6.294 mg, 0.025 mmol) and sodium ascorbate (Na·VC, 9.98mg, 0.05mmol) were added to the reaction solution sequentially, and after reaction at room temperature for 3 h, the reaction solution was extracted with DCM, and finally purification by column chromatography was carried out to obtain 100 mg of the product, 33% yield. [M+H]⁺=1780.3
Compound R6-1 and Compound R6-2 were used to replace Compound R6, respectively, and Compound R7-1 and Compound R7-2 were prepared using the same method.

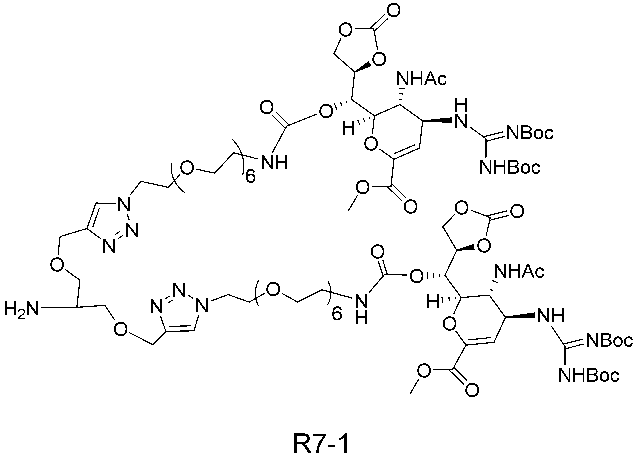

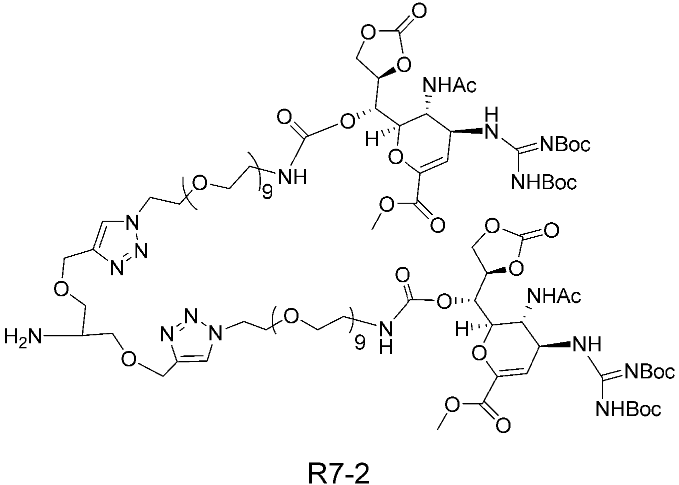

### 7) Preparation of Compound R8:

The starting materials prop-2-yn-1-yloxy-PEG₂-COOH (2-(2-(2-(prop-2-yn-1-yloxy)ethoxy)ethoxy)acetic acid, 14.68 mg, 0.168 mmol) and 1-(3-dimethylaminopropyl)-3-ethylcarbodiimide (EDCI, 28.31 mg, 0.145 mmol), HOBT (19.6 mg, 0.145 mmol), DMAP (2.96 mg, 0.024 mmol), and DIPEA (31.27 mg, 0.242 mmol) were added sequentially to 3 ml of ultra-dry DMF, and the mixture was stirred at room temperature for 30 minutes under argon protection, then compound R7 (80 mg, 0.048 mmol) was added, and stirring was continued at room temperature under argon protection,. After 12 hours of reaction, 60 mg of the product were obtained following purification with column chromatography. 63% yield. [M+H]⁺=1984.6

Compound R7-1 was used to replace Compound R7, and Compound R8-1 was prepared using the same method.

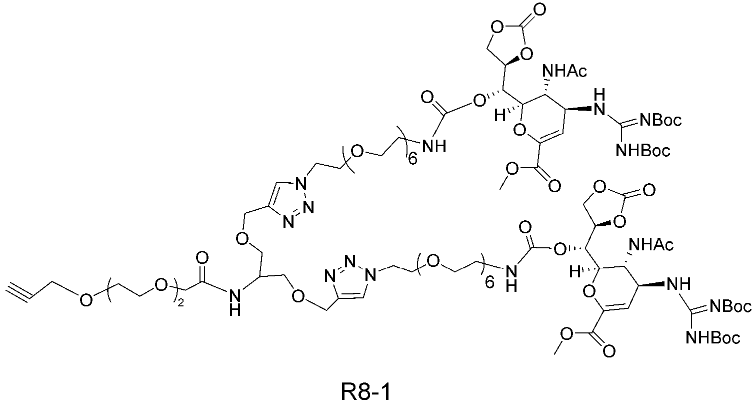

Prop-2-yn-1-yloxy-PEG₃-COOH was used to replace prop-2-yn-1-yloxy-PEG₂-COOH, Compound R7-2 was used to replace Compound R7, and Compound R8-2 was prepared using the same method.

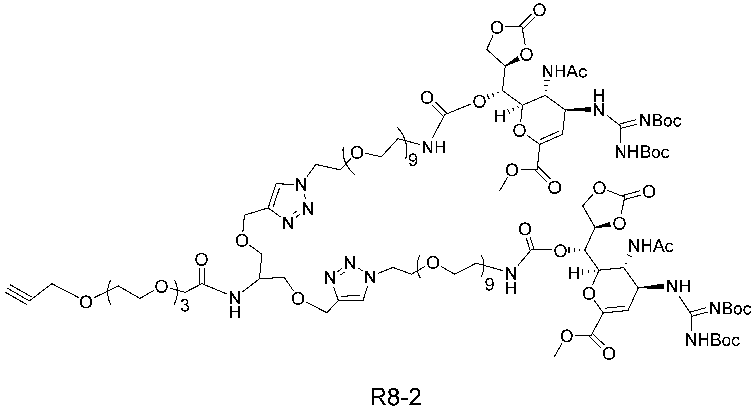

Prop-2-yn-1-yloxy-PEG₆-COOH was used to replace prop-2-yn-1-yloxy-PEG₂-COOH, Compound R7-2 was used to replace Compound R7, and Compound R8-3 was prepared using the same method.

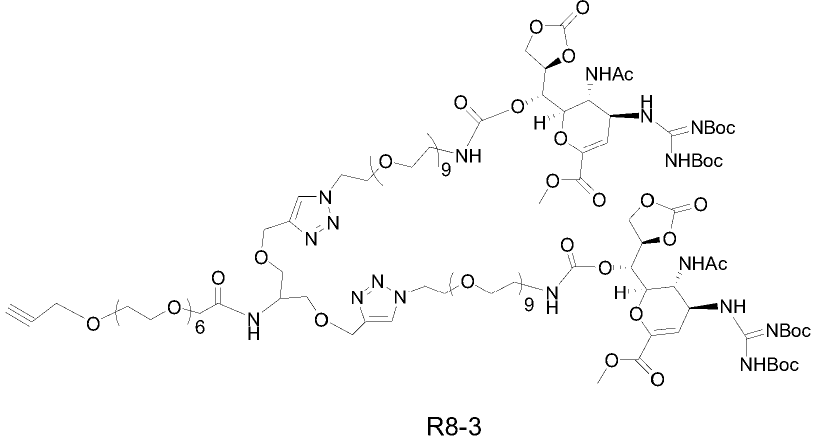

### 8) Preparation of Compound R9:

Compound R8 (60 mg, 0.03 mmol) was dissolved in 2 ml of ultra-dry DMF, then 2 ml of trifluoroacetic acid (TFA) was added, and the resulting mixture was stirred at room temperature and reacted for 4 h. The TFA was removed by a rotary evaporator and 46 mg of the product were obtained following purification with C₁₈ reverse phase chromatography, 97% yield. [M+H]⁺=1584.5

The same method was used to prepare Compound R9-1 to Compound R9-3.

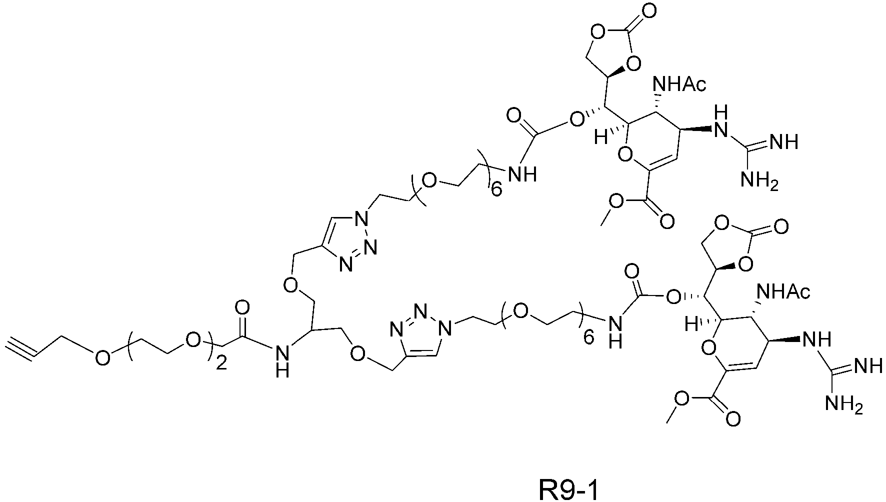

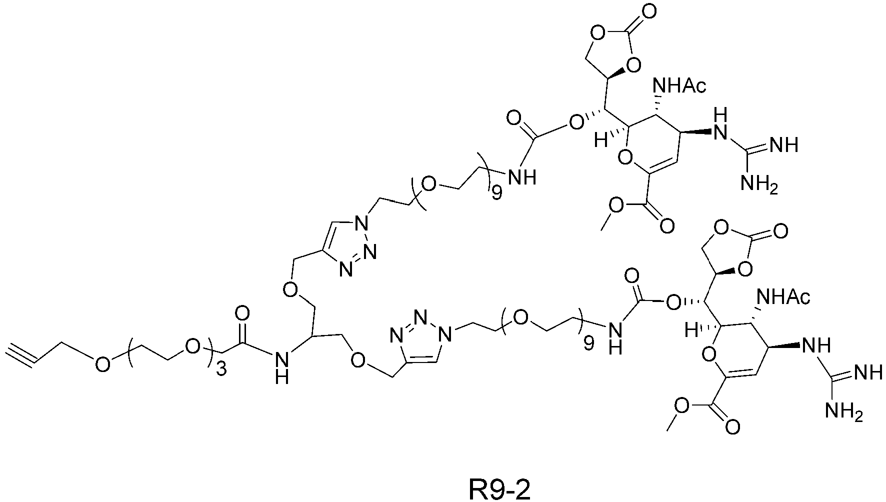

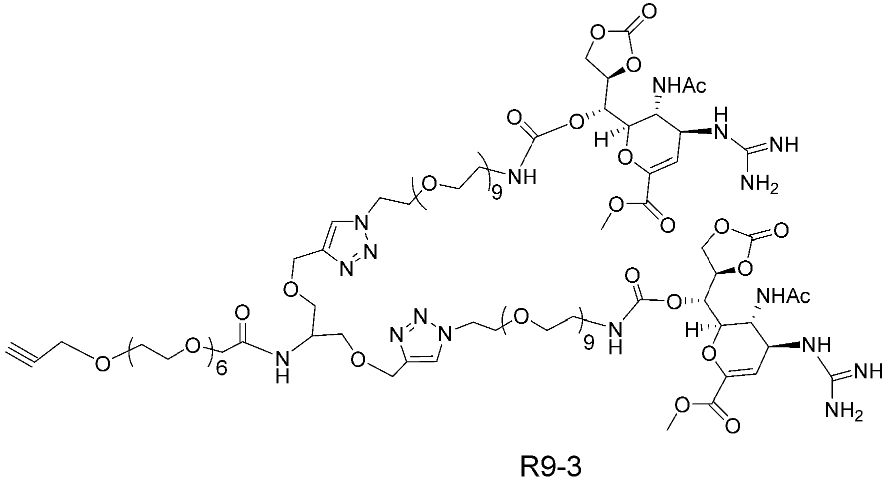

### 9) Preparation of Compound R10

Compound R9 (46 mg, 0.029 mmol) was dissolved in 1.5 ml of methanol, then 200 ul of double distilled water were added. The pH of the mixture was adjusted to 12 to 13 with 10 mol/L of aqueous NaOH solution, and the mixture was stirred at room temperature for 4 h. The pH of the reaction system was adjusted to 4 with 4 M hydrochloric acid in 1,4-dioxane, and finally 40 mg of the product were obtained following purification with C₁₈ reverse phase chromatography, 91.7% yield. [M+H]⁺=1503.9

The same method was used to prepare Compound R10-1 to Compound R10-3.

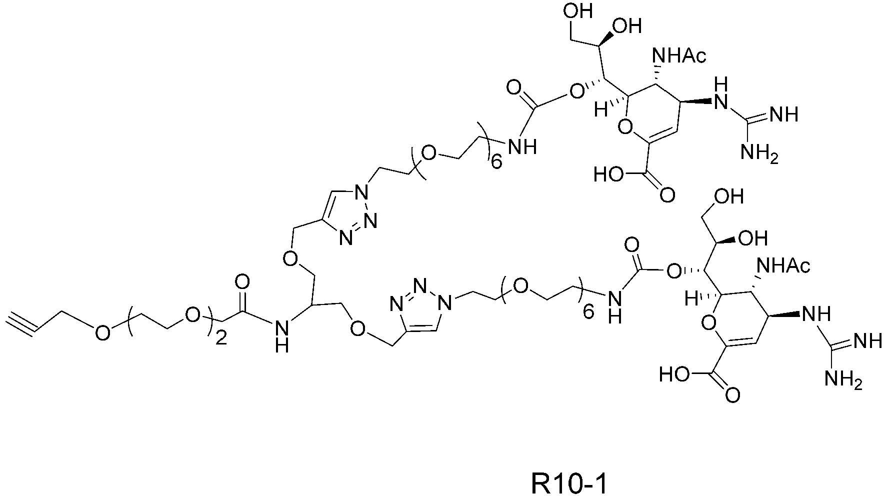

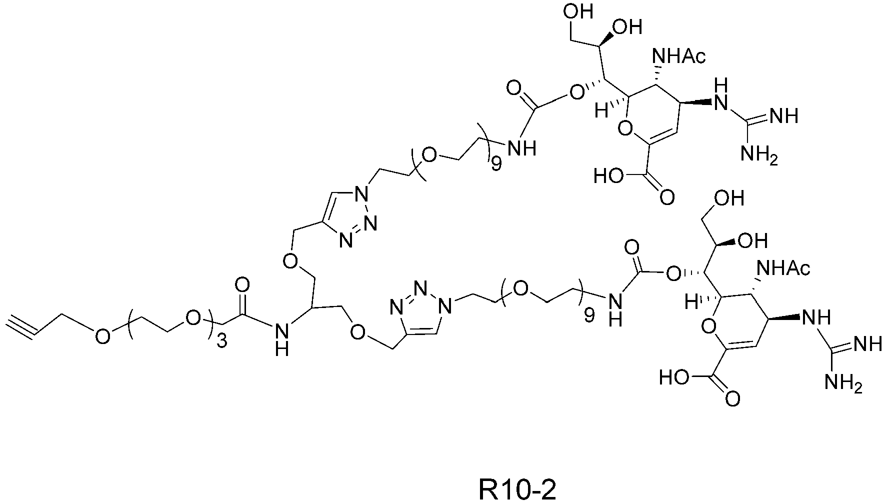

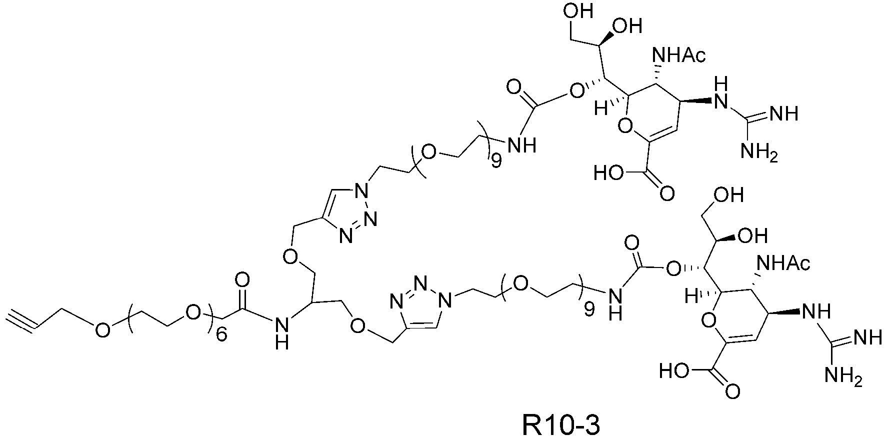

### Example 4 Synthesis of Zanamivir Trimer Derivative

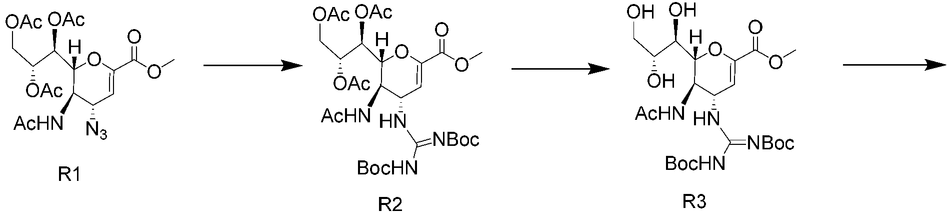

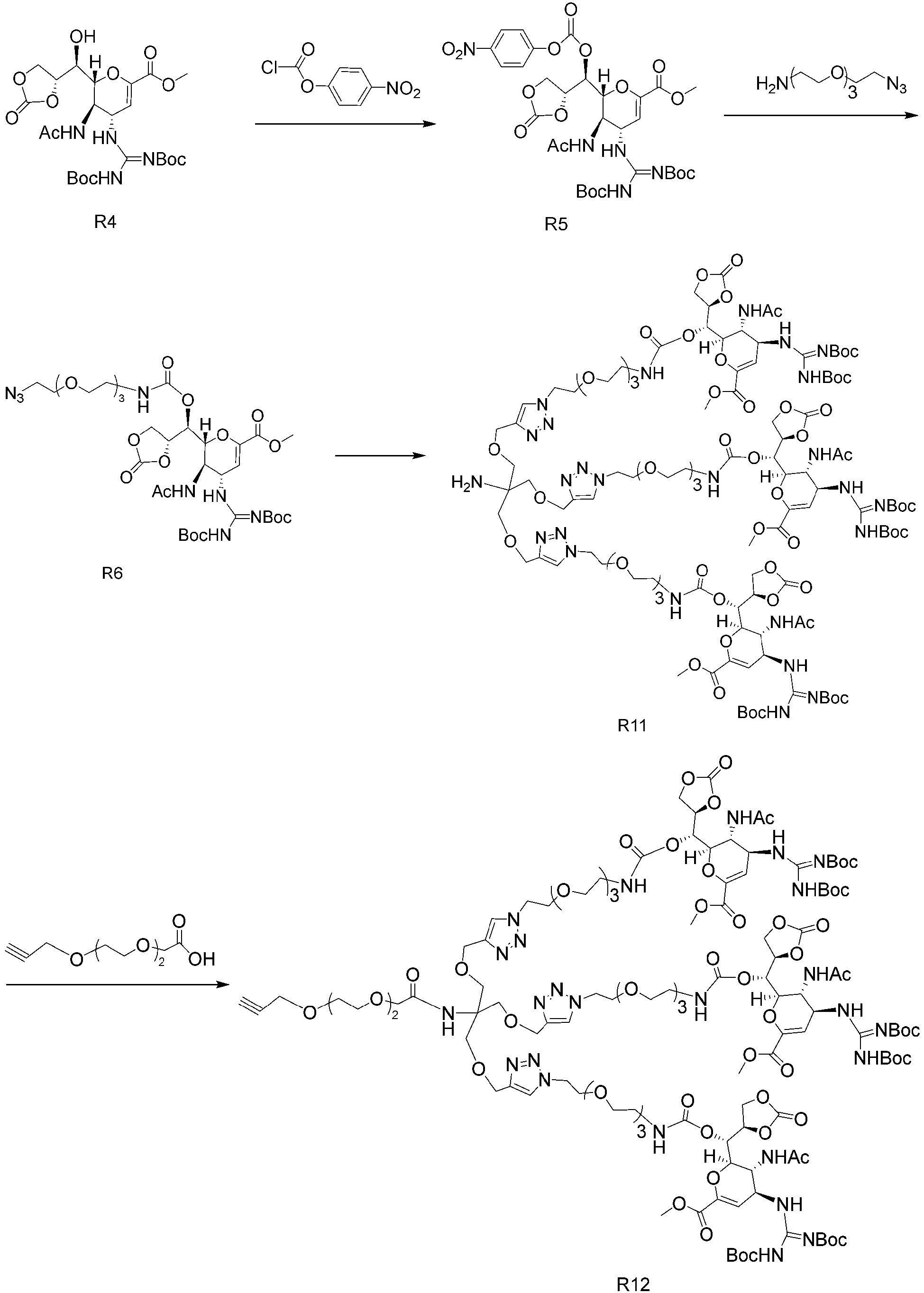

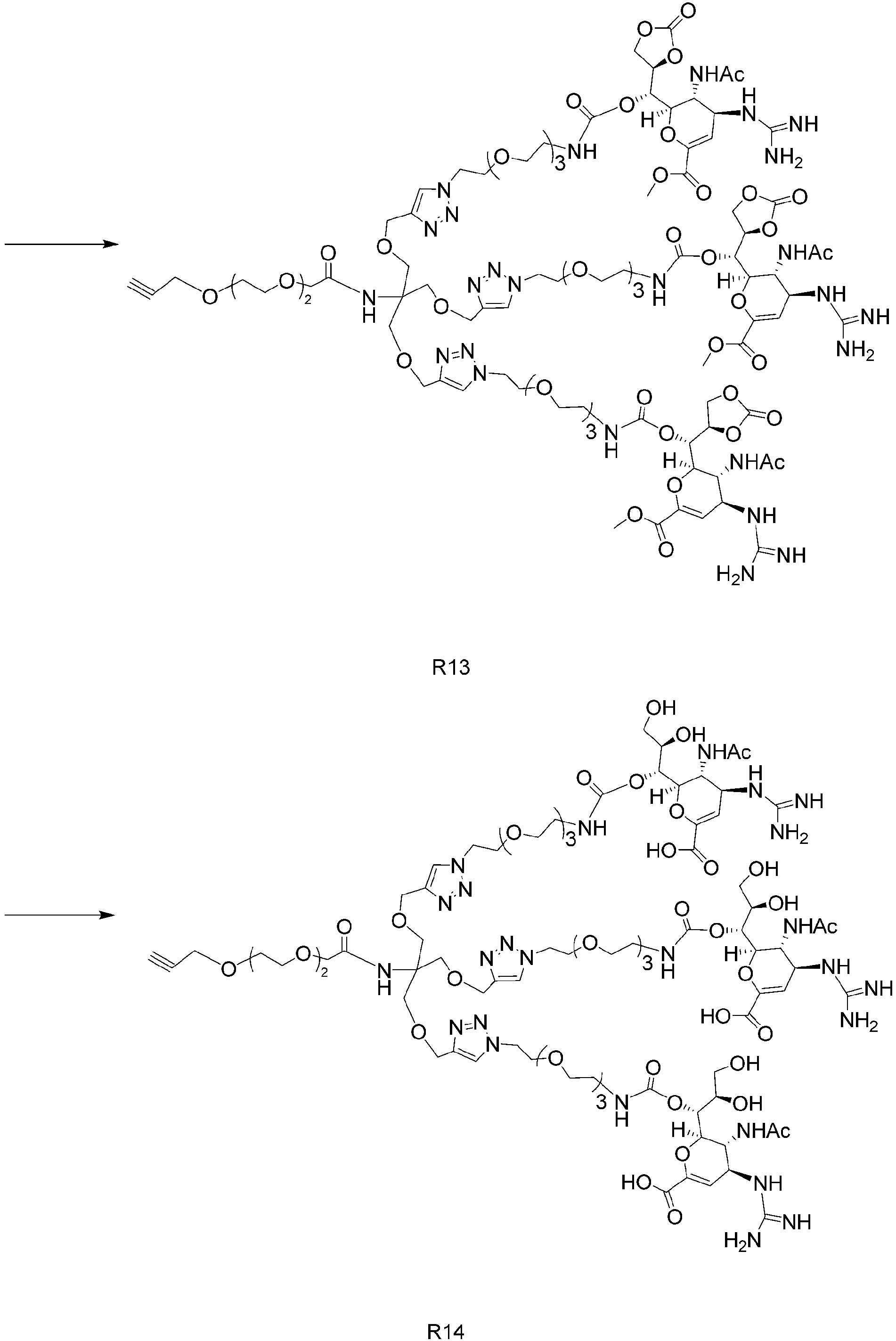

### 1) Preparation of Compound R11:

Compound R6 (140 mg, 0.168 mmol) and Compound 10 (12.34 mg, 0.0525 mmol) were dissolved in 1.5 ml of tetrahydrofuran (THF), respectively, and mixed with stirring. Aqueous solutions of CuSO₄·5H₂O (6.294 mg, 0.025 mmol) and sodium ascorbate (Na·VC, 9.98 mg, 0.05 mmol) were added to the reaction solution sequentially, and after 3 hours of reaction at room temperature, the reaction solution was extracted with DCM, and finally purification by column chromatography was carried out to obtain 106 mg of the product, 25.5% yield. ¹H NMR (600 MHz, CDCl3) δ 11.38 (s, 2H), 8.43 (d, 3H), 7.88 (s, 2H), 5.97 (d, 2H), 5.68 (s, 2H), 5.62 (t, 2H), 5.17 (m, 3H), 5.12 (s, 2H), 4.66 (m, 12H), 4.54 (m, 8H), 4.18 (q, 3H), 3.89 (t, 6H), 3.78 (s, 8H), 3.68 (s, 3H), 3.57 (m, 32H), 3.31 (m, 2H), 3.23 (m, 3H), 3.31 (m, 2H), 3.23 (m, 3H), 1.91 (s, 7H), 1.47 (s, 54H), 1.29 (d, 4H), 1.26 (d, 6H), 0.87 (m, 3H).[M+2H]⁺=1343.51

Compound R6-1 and Compound R6-2 were used to replace Compound R6 respectively, and Compound R11-1 and Compound R11-2 were prepared using the same method.

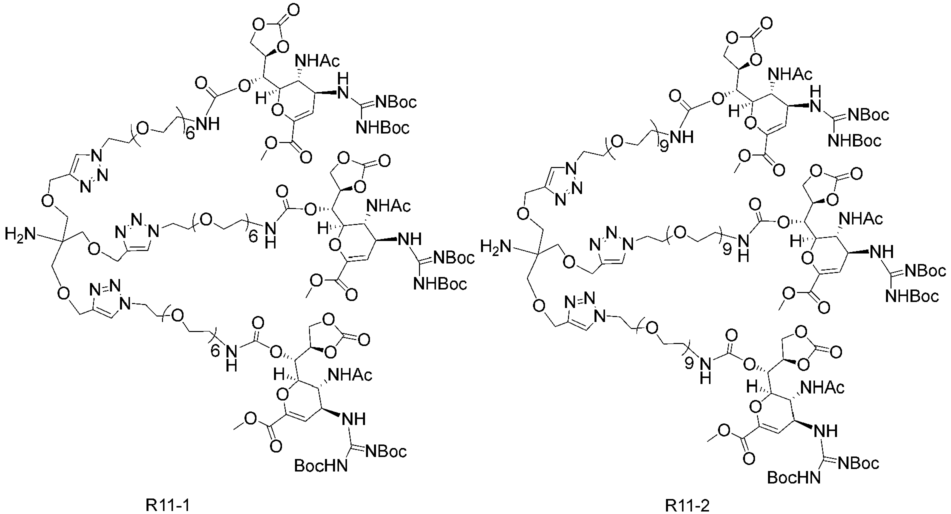

### 2) Preparation of Compound R12:

The starting materials prop-2-yn-1-yloxy-PEG₂-COOH (2-(2-(2-(prop-2-yn-1-yloxy)ethoxy)ethoxy) acetic acid, 9.83 mg, 0.0486 mmol), EDCI (18.96 mg, 0.0972 mmol), HOBT (13.127 mg, 0.0972 mmol), DMAP (1.979 mg, 0.0162 mmol), and DIPEA (20.94 mg, 0.1621 mmol) were sequentially added to 3ml of ultra-dry DMF, and the mixture was stirred at room temperature for 30 min under argon protection, then Compound R11 (80mg, 0.03mmol) were added, and stirring was continued at room temperature under argon protection. After 12 hours of reaction, 82 mg of the product were obtained following purification with column chromatography, 95.3% yield. ¹H NMR (600 MHz, CDCl3) δ 11.38 (s, 1H), 9.11 (s, 2H), 8.45 (d, 2H), 7.82 (dt, 2H), 7.79 (s, 1H), 7.67 (dt, 1H), 7.37 (m, 3H), 6.89 (m, 2H), 5.94 (d, 2H), 5.64 (t, 2H), 5.57 (m, 1H), 5.21 (t, 2H), 5.02 (m, 2H), 4.65 (m, 4H), 4.56 (m, 9H), 4.48 (dd, 2H), 4.17 (d, 1H), 4.14 (d, 1H), 3.88 (t, 6H), 3.78 (d, 11H), 3.66 (m, 2H), 3.63 (m, 3H), 3.59 (m, 21H), 3.52 (m, 3H), 3.18 (m, 4H), 3.34 (m, 3H), 3.26 (dd, 2H), 1.90 (s, 6H), 1.46 (t, 54H), 1.43 (d, 3H), 1.37 (s, 1H), 1.33 (s, 1H), 1.28 (d, 4H), 1.27 (s, 2H), 1.25 (s, 13H), 1.88 (t, 4H), 0.84 (m, 2H). [M+3H]⁺=957.42

Compound R11-1 was used to replace Compound R11, and Compound R12-1 was prepared using the same method.

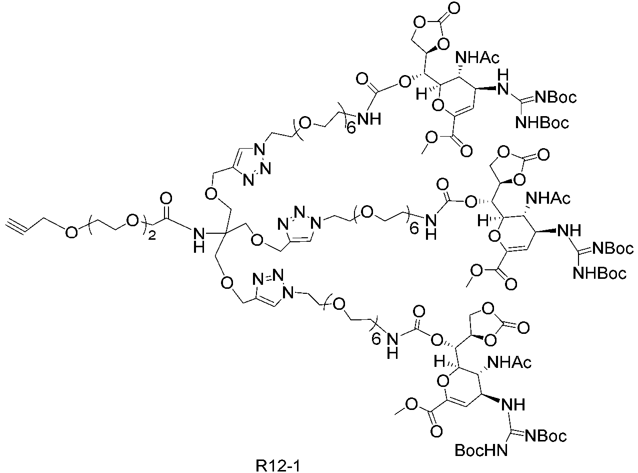

Compound R11-2 was used to replace Compound R11, and Compound R12-2 was prepared using the same method.

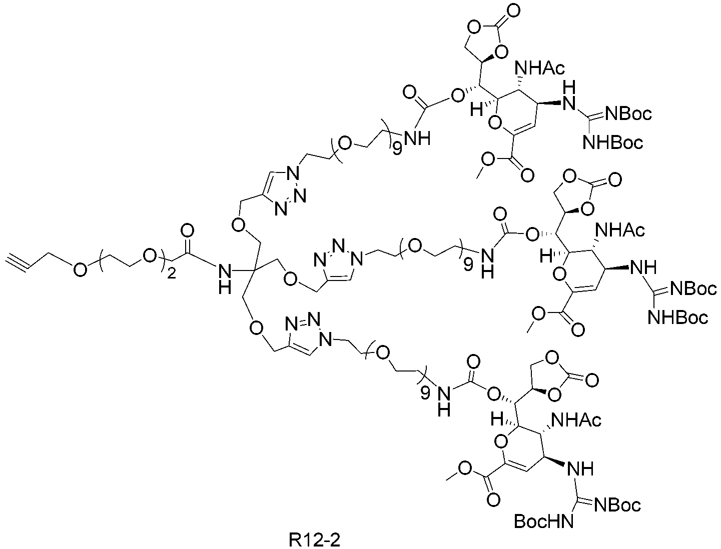

Prop-2-yn-1-yloxy-PEG₆-COOH was used to replace prop-2-yn-1-yloxy-PEG₂-COOH, and Compound R11-2 was used to replace Compound R11, and Compound R12-3 was prepared using the same method.

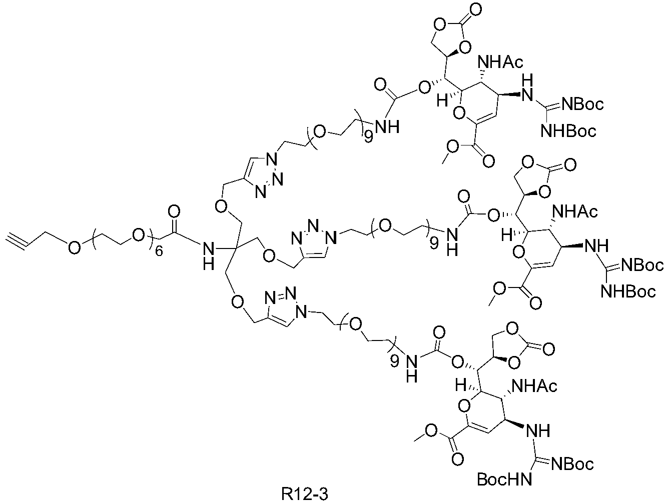

### 3) Preparation of Compound R13:

Compound R12 (60 mg, 0.028 mmol) was dissolved in 2 ml of ultra-dry DMF, then 2 ml of trifluoroacetic acid TFA were added, and the resulting mixture was stirred at room temperature and reacted for 4 h. The TFA was removed by a rotary evaporator, and 41 mg of the product were obtained following purification with C₁₈ reverse phase chromatography, 64.5% yield. [M+H]⁺=2268.7

Compound R13-1 to Compound R13-3 were prepared using the same method.

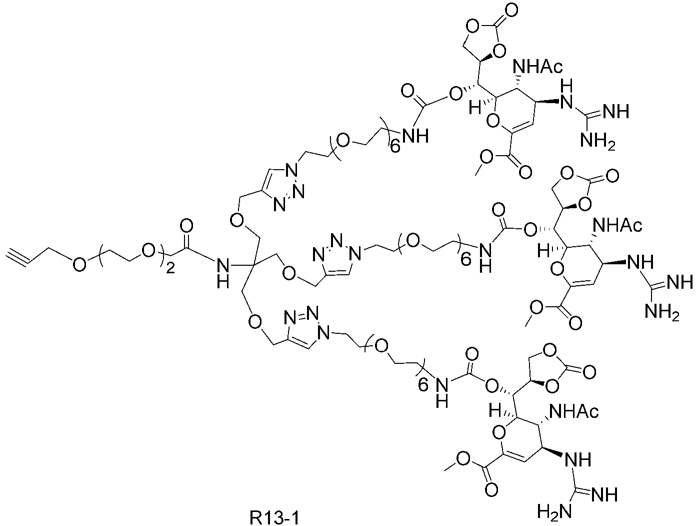

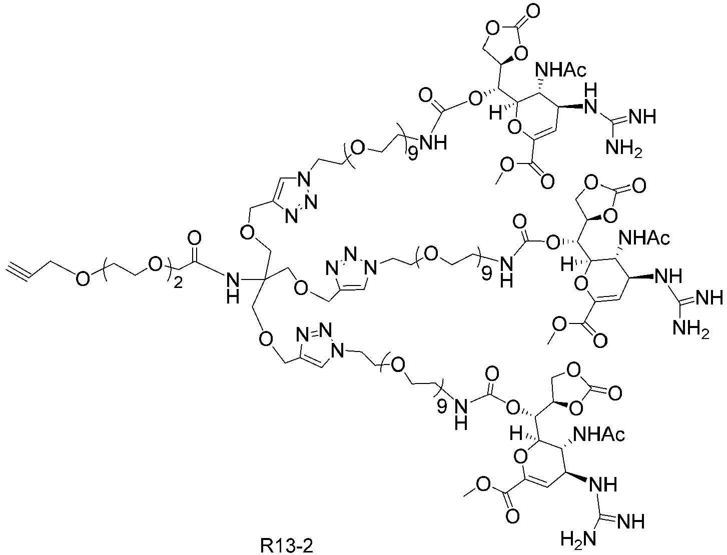

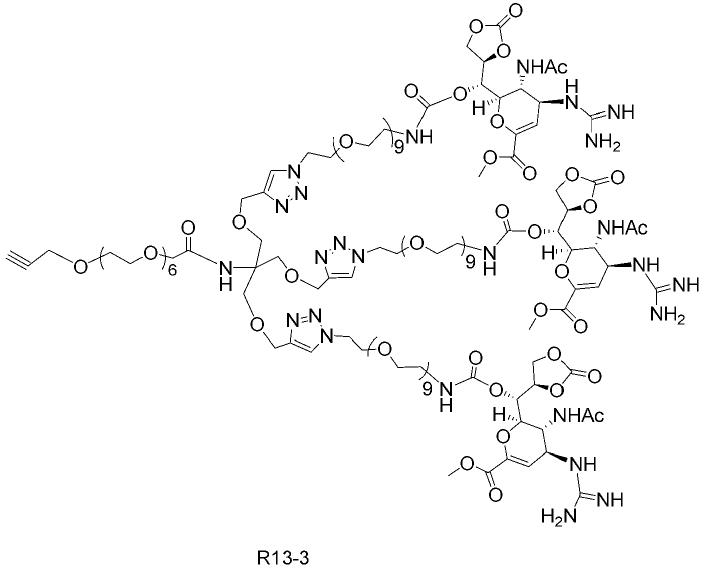

### 4) Preparation of Compound R14

Compound R13 (41 mg, 0.018 mmol) was dissolved in 1.5 ml of methanol, then 200 ul of double distilled water were added. The pH of the mixture was adjusted to 12 to 13 with 10 mol/L of aqueous NaOH solution, and then the mixture was stirred at room temperature for 4 h. The pH of the reaction system was adjusted to 4 with 4 M hydrochloric acid in 1,4-dioxane solution, and finally 32 mg of the product were obtained following purification with C₁₈ reverse phase chromatography, 82.8% yield. [M+H]⁺=2148.9

Compound R14-1 to Compound R14-3 were prepared using the same method.

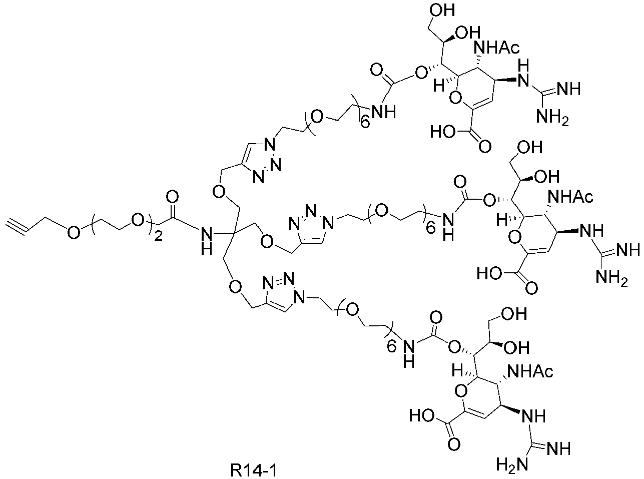

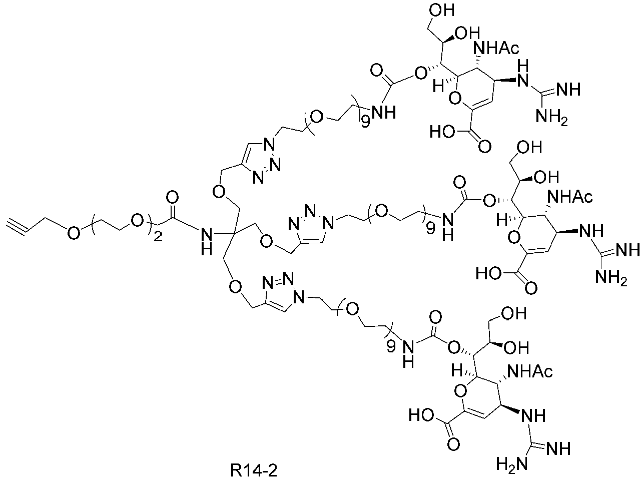

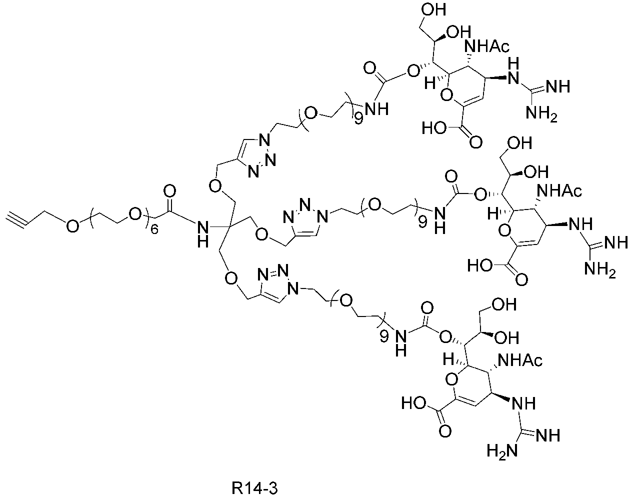

### Example 5 Synthesis of Peramivir Dimer Derivative

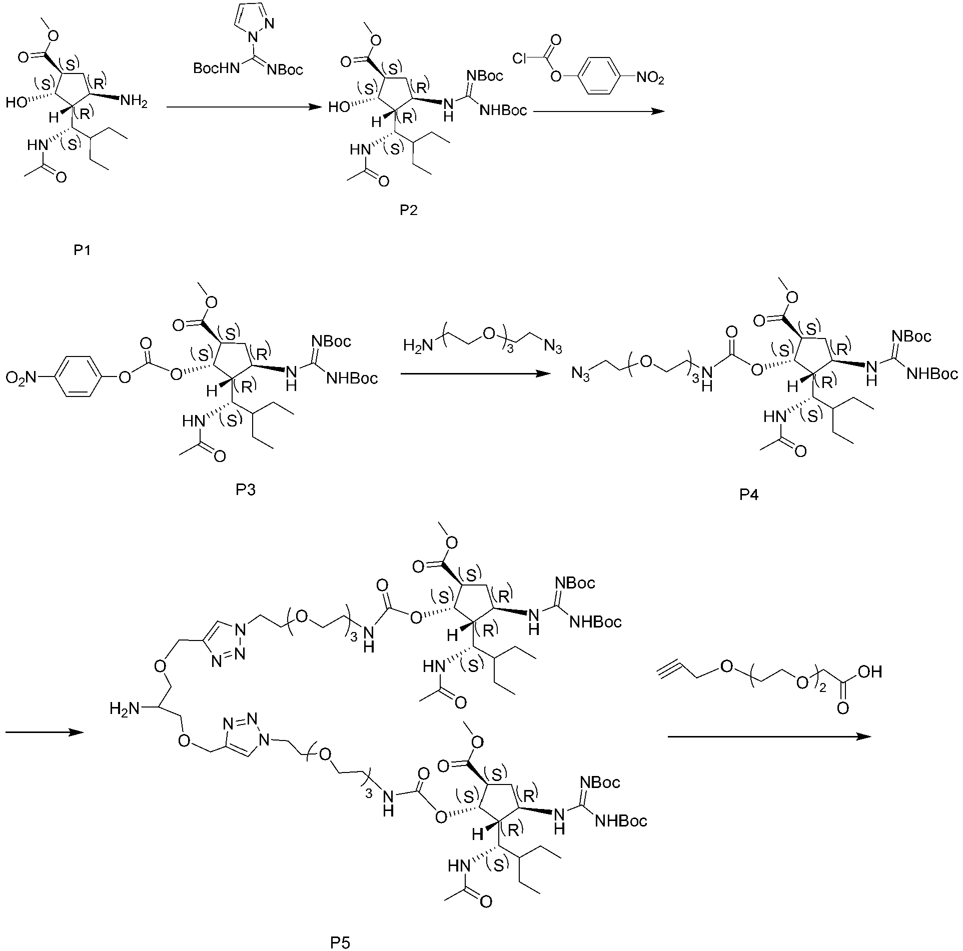

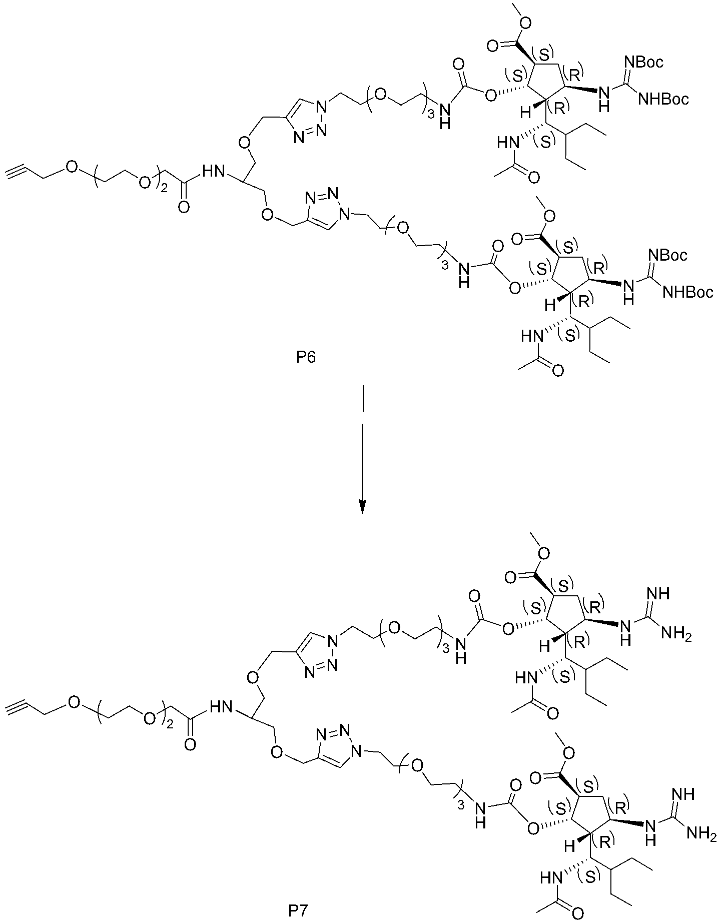

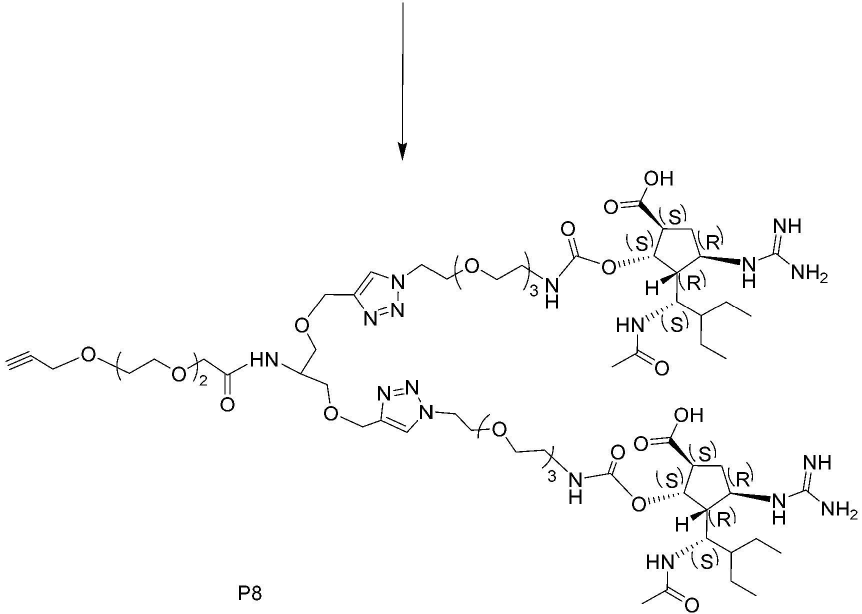

### 1) Preparation of Compound P2:

Compound P1 (5 g, 16.7 mmol) was added to DCM (100 ml), to which N,N'-bis(tert-butoxycarbonyl)-1H-pyrazole-1-carboxamidine (7.77 g, 25 mmol), diisopropylethylamine (DIEA, 4.3 g, 33.3 mmol) and dimethylaminopyridine (DMAP, 0.1 g) were added, and the resulting mixture was stirred and reacted for 24 h at room temperature with monitoring by TLC (DCM: MeOH=10:1). After Compound P1 was reacted completely, water (100ml) was added with to the reaction solution, the resulting mixture was stirred for 10 min, and left to stand to separate the aqueous phase, and the organic phase was further washed with 100 ml of water. The organic phases were collected, dried over anhydrous sodium sulfate, and filtered, and the filtrate was collected, concentrated and purified by column chromatography (eluent: DCM/MeOH=50:1); the target product was collected to obtain 5.8 g of Compound P2, 66% yield.

### 2) Preparation of Compound P3:

Compound P2 (100 mg, 0.184 mmol) was dissolved in 3ml of anhydrous DCM, and the resulting mixture was stirred in an ice water bath, to which p-nitrophenyl chloroformate (55.7 mg, 0.276 mmol) was added, and then dimethylaminopyridine (DMAP, 45.03 mg, 0.368 mmol) were added, and under nitrogen protection, the ice water bath was removed, the mixture was continuously stirred and reacted for 6 h. Additional p-nitrophenyl chloroformate (27.5 mg, 0.184 mmol) was supplemented, and the reaction continued for 6 h. After the reaction was completed, the solvent was removed by a rotary evaporator, and 98 mg of the product were obtained following purification with column chromatography, 75.3% yield.

### 3) Preparation of Compound P4:

Compound P3 (60 mg, 0.0848 mmol) was dissolved in 5 ml of anhydrous DCM, to which azido-triethylene glycol amine (N₃-PEG₃-NH₂, 2-(2-(2-(2-azido-ylethoxy)ethoxy)ethoxy)ethanamine, 27.783 mg, 0.127 mmol), HOBT (11.47, 0.0848 mmol), and DIPEA (21.94 mg, 0.1697 mmol) were added sequentially, and under nitrogen protection, the resulting mixture was stirred at room temperature. After 8 hours of reaction, 62 mg of the product were obtained following purification with column chromatography, 93.0% yield, [M+H]⁺=787.3

Azido-hexaethylene glycol amine (N₃-PEG₆-NH₂) or azido-nonaethylene glycol amine (N₃-PEG₉-NH₂) was used to replace azido-triethylene glycol amine (N₃-PEG₃-NH₂), and Compound P4-1 and Compound P4-2 were prepared using the same method.

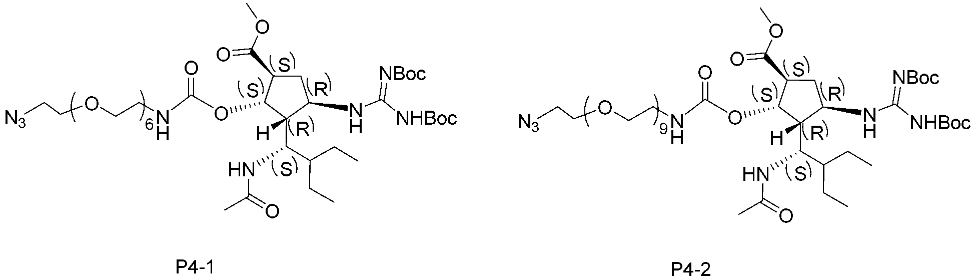

### 4) Preparation of Compound P5:

Compound P4 (60 mg, 0.076 mmol) and Compound 5 (5.77 mg, 0.0345 mmol) were dissolved in 1.5 ml of tetrahydrofuran (THF), respectively, and mixed with stirring. Aqueous solutions of CuSO₄·5H₂O (9.57 mg, 0.038 mmol) and sodium ascorbate (Na·VC, 15.12 mg, 0.076 mmol) were added to the reaction solution sequentially, and after 3 hours of reaction at room temperature, the reaction solution was extracted with DCM, and finally purification by column chromatography was carried out to obtain 54 mg of the product, 40.8% yield.¹H NMR (600 MHz, CDCl₃) δ 11.43 (s, 2H), 8.56 (d, 2H), 8.24 (m, 2H), 7.97 (s, 1H), 6.14 (t, 2H), 5.18 (dd, 2H), 4.68 (s, 3H), 4.56 (t, 4H), 4.39 (m, 2H), 4.24 (m, 2H), 3.89 (t, 4H), 3.80 (s, 2H), 3.72 (s, 6H), 3.61 (m, 17H), 3.54 (t, 5H), 3.33 (m, 5H), 2.96 (m, 2H), 2.50 (m, 2H), 2.36 (q, 2H), 2.05 (d, 6H), 1.83 (m, 2H), 1.48 (d, 36H), 1.41 (d, 3H), 1.28 (s, 2H), 1.25 (s, 4H), 1.11 (m, 3H), 1.05 (m, 2H), 0.85 (m, 14H), [M+H]⁺=1741.0540

Compound P4-1 and Compound P4-2 were used to replace Compound P4, respectively, and Compound P5-1 and Compound P5-2 were prepared using the same method.

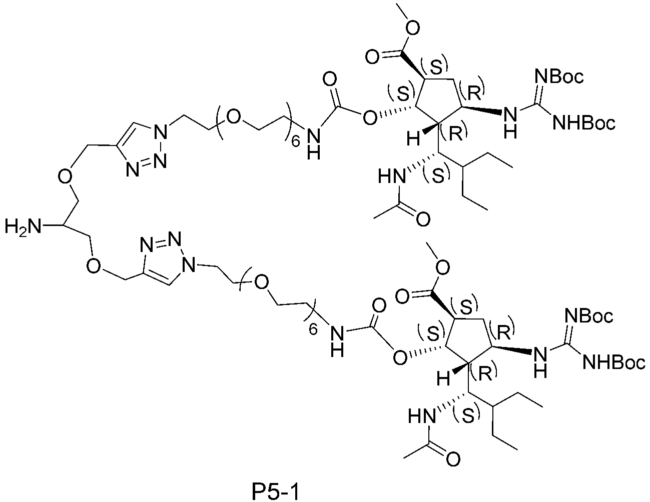

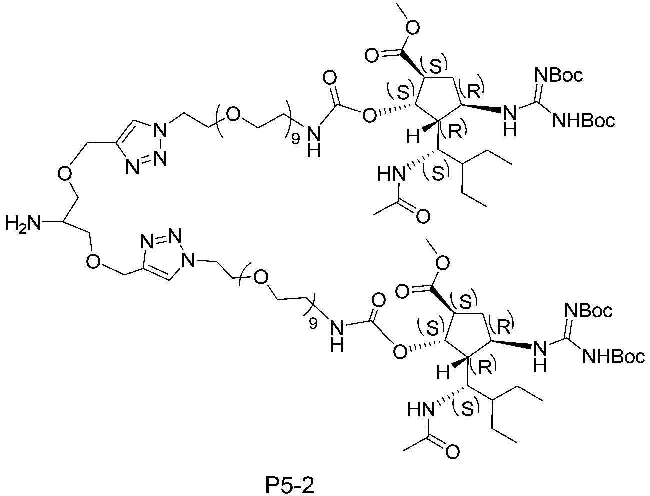

### 5) Preparation of Compound P6:

The starting materials prop-2-yn-1-yloxy-PEG2-COOH (2-(2-(2-(prop-2-yn-1-yloxy)ethoxy)ethoxy) acetic acid, 4.68 mg, 0.023 mmol), EDCI (8.868 mg, 0.046 mmol), HOBT (6.245 mg, 0.046 mmol), DMAP (0.942 mg, 0.008 mmol), and DIPEA (9.96 mg, 0.077 mmol) were added sequentially to 3 ml of ultra-dry DMF, and the resulting mixture was stirred at room temperature for 30 minutes under argon protection, then Compound P5 (40 mg, 0.023 mmol) was added, and under argon protection, the resulting mixture was continuously stirred at room temperature. After 12 hours of reaction, 43 mg of the product were obtained following purification with column chromatography, 97.2% yield. [M+H]⁺=1925.4

Compound P5-1 was used to replace Compound P5, and prop-2-yn-1-yloxy-PEG₆-COOH was used to replace prop-2-yn-1-yloxy-PEG₂-COOH, and Compound P6-1 was prepared using the same method.

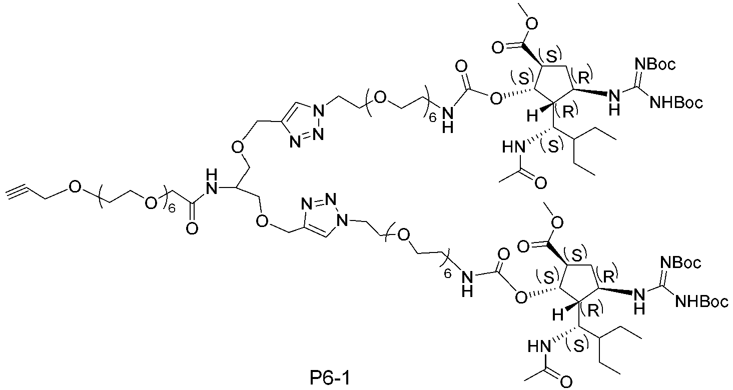

Compound P5-2 was used to replace Compound P5, and Compound P6-2 was prepared using the same method.

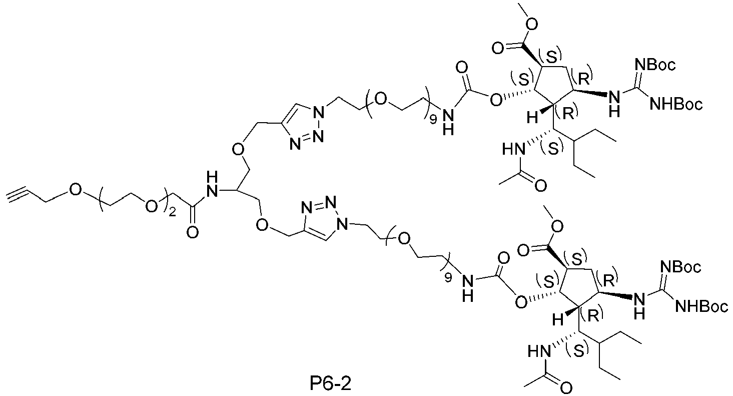

### 6) Preparation of Compound P7:

Compound P6 (40 mg, 0.021 mmol) was dissolved in 2 ml of ultra-dry DMF, then 2 ml of trifluoroacetic acid (TFA) was added, and the resulting mixture was stirred at room temperature and reacted for 4 h. The TFA was removed by rotary evaporation, and 29 mg of the product were obtained after purification by C₁₈ reverse phase chromatography, 92.0% yield. [M+H]⁺=1524.6

Compound P7-1 to Compound P7-2 were prepared using the same method.

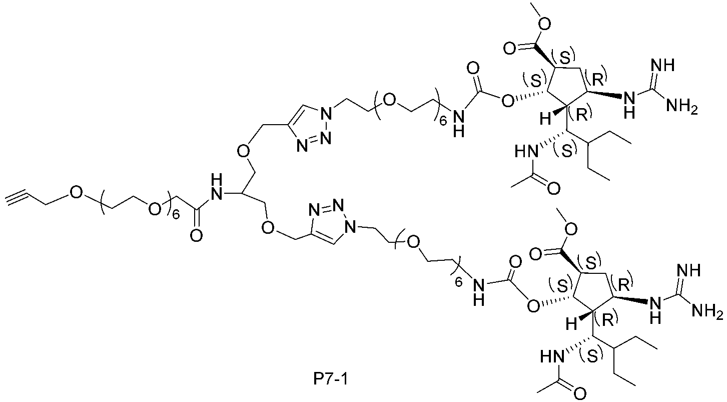

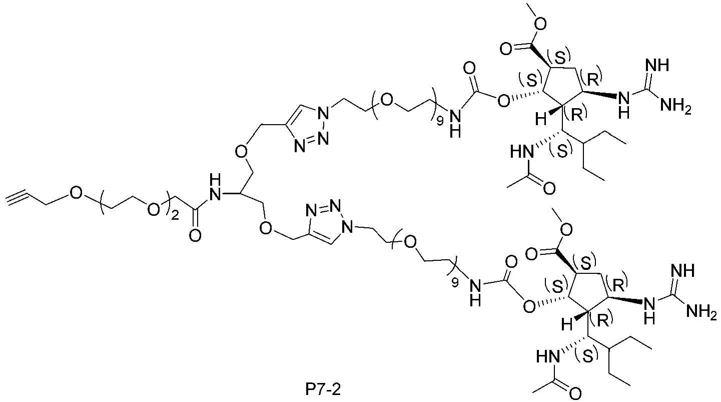

### 7) Preparation of Compound P8

Compound P7 (29 mg, 0.019 mmol) was dissolved in 1.5 ml of methanol, then 200 ul of double distilled water were added. The pH of the mixture was adjusted to12 to 13 with 10 mol/L of aqueous NaOH solution, and the mixture was stirred at room temperature for 4 h. The pH of the reaction system was adjusted to 4 with 4 M hydrochloric acid in 1,4-dioxane solution, and finally 20 mg of the product was obtained following purification with C₁₈ reverse phase chromatography, 70.4% yield. [M+H]⁺=1496.3

Compound P8-1 to Compound P8-2 were prepared using the same method.

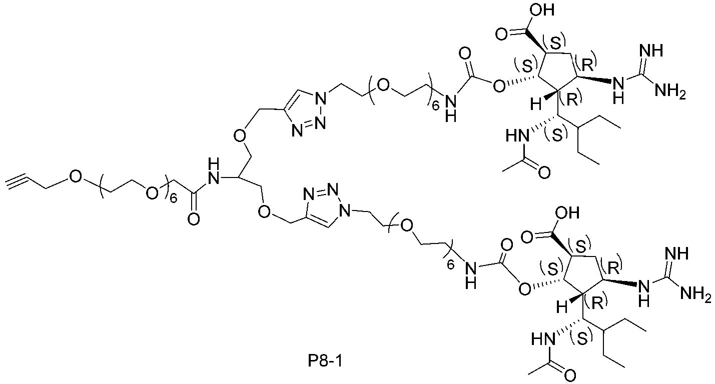

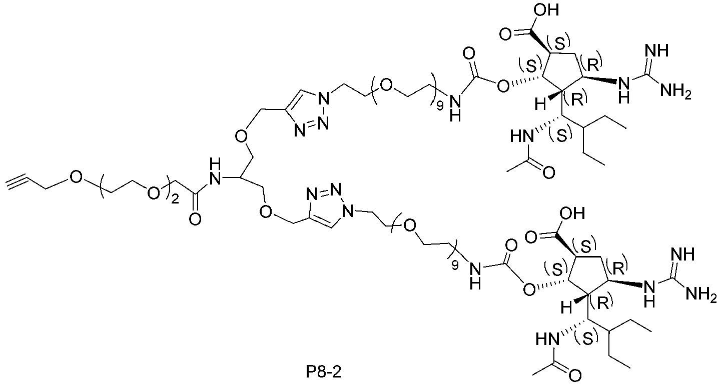

### Example 6 Synthesis of Peramivir Trimer Derivative

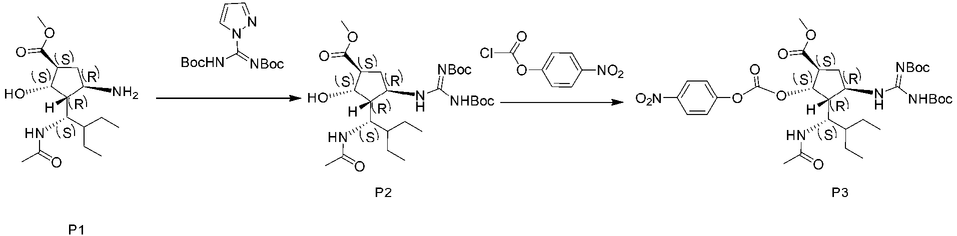

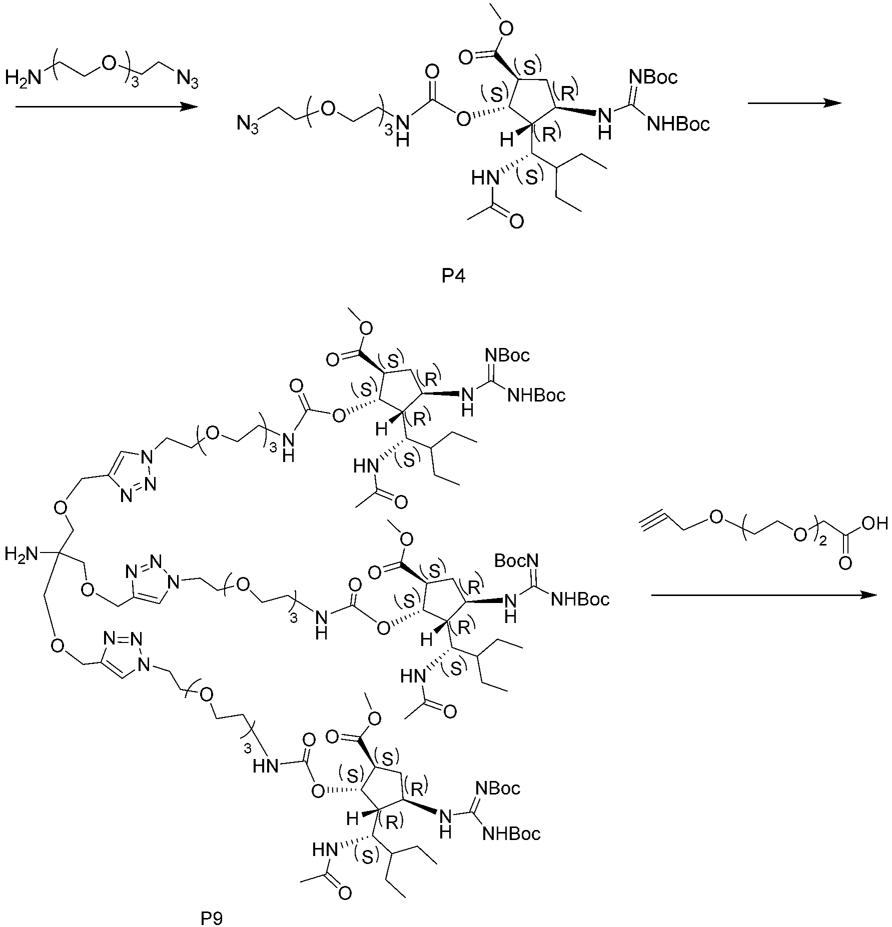

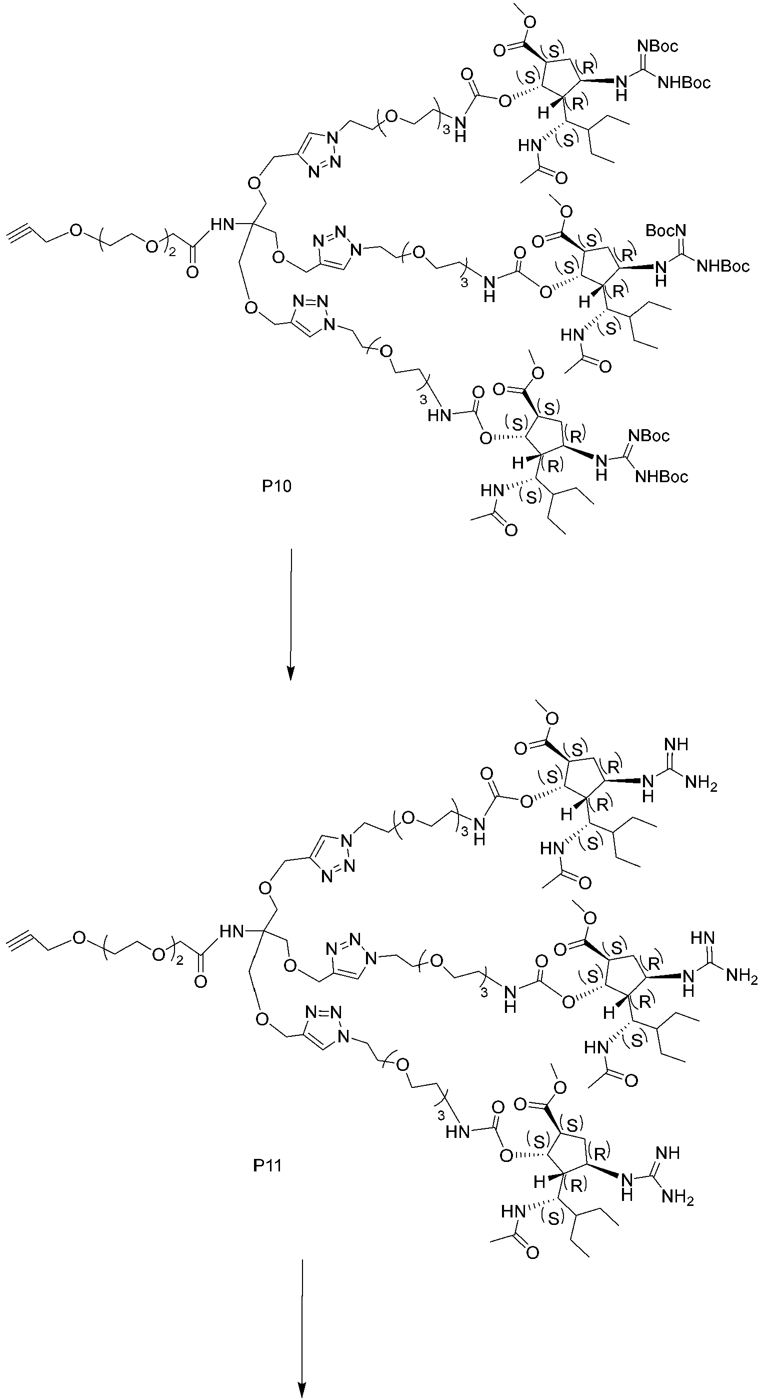

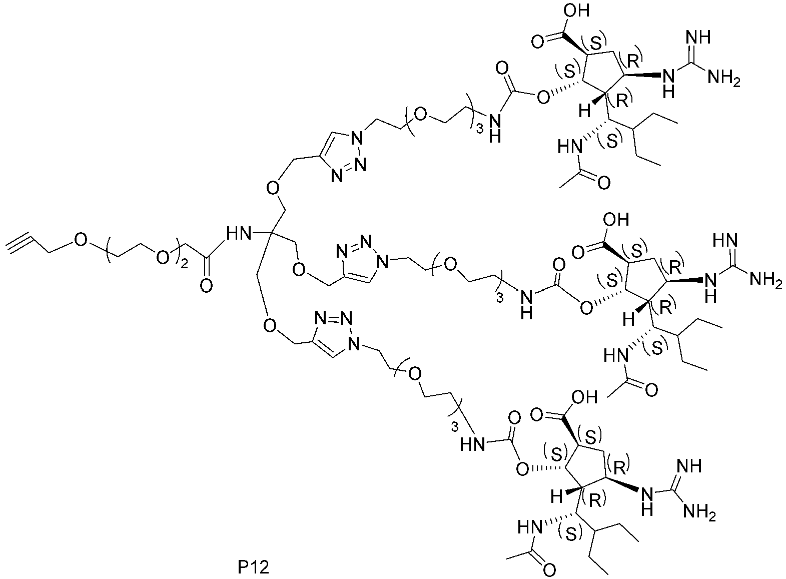

### 1) Preparation of Compound P9:

Compound P4 (300 mg, 0.382 mmol) and Compound 10 (28.06 mg, 0.119 mmol) were dissolved in 1.5 ml of tetrahydrofuran (THF), respectively, and mixed with stirring. Aqueous solutions of CuSO₄·5H₂O (14.29 mg, 0.057 mmol) and sodium ascorbate (Na·VC, 22.68 mg, 0.115 mmol) were added to the reaction solution sequentially, and after 3 hours of reaction at room temperature, the reaction solution was extracted with DCM, and finally purified by column chromatography to obtain 190 mg of the product, 57.5% yield. ¹H NMR (600 MHz, CDCl3) δ 11.43 (s, 3H), 8.55 (d, 3H), 8.20 (d, 2H), 7.85 (s, 2H), 5.91 (t, 2H), 5.15 (dd, 3H), 4.62 (s, 4H), 4.55 (t, 6H), 4.41 (m, 3H), 4.22 (m, 3H), 3.89 (t, 6H), 3.72 (s, 9H), 3.62 (d, 28H), 3.54 (m, 6H), 3.32 (q, 6H), 2.97 (m, 3H), 2.49 (m, 3H), 2.35 (m, 3H), 2.06 (s, 8H), 1.82 (m, 3H), 1.48 (d, 54H), 1.42(s, 2H), 1.38 (m, 4H), 1.28 (s, 3H), 1.25 (s, 8H), 1.13 (m, 3H), 1.04 (m, 3H), 0.84 (m, 20H), [M+2H]⁺=1298.2681
Compound P4-1 and Compound P4-2 were used to replace Compound P4, respectively, and Compound P9-1 and Compound P9-2 were prepared using the same method.

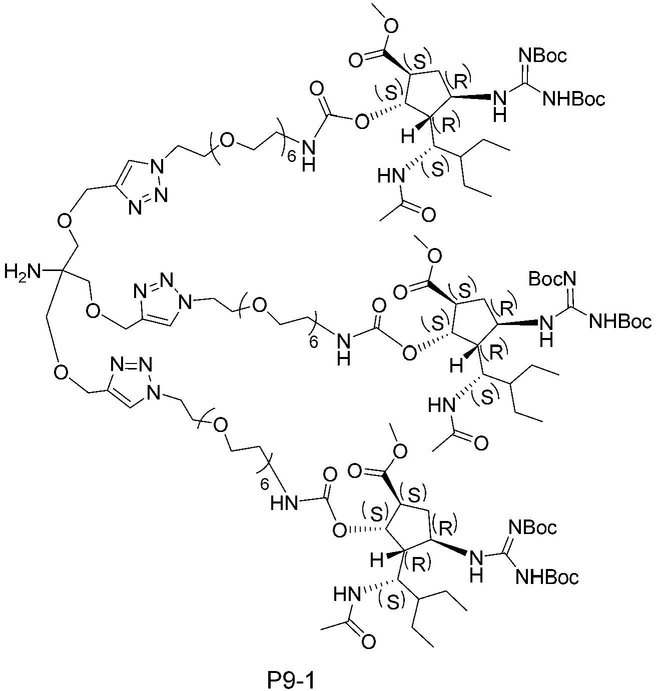

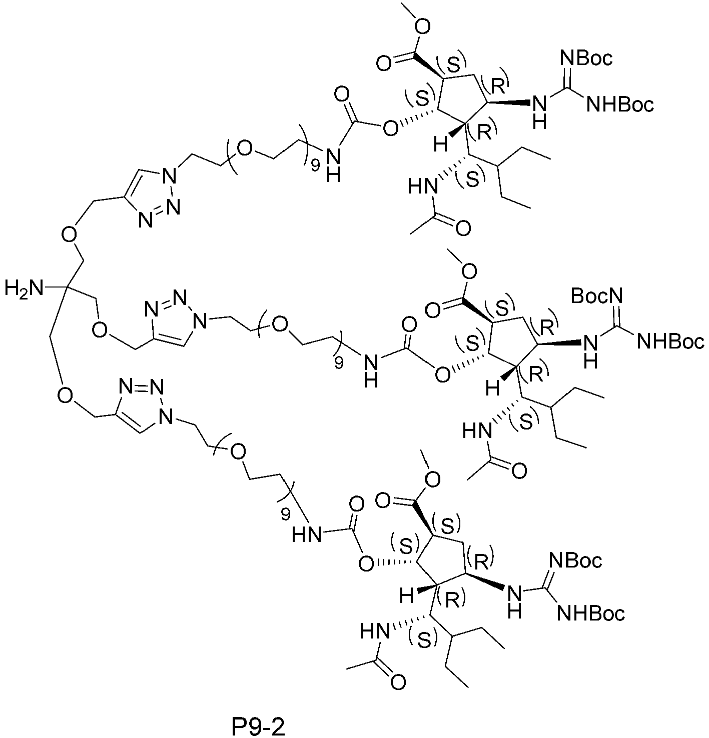

### 2) Preparation of Compound P10:

The starting materials 2-(2-(2-(prop-2-yn-1-yloxy)ethoxy)ethoxy)acetic acid (18.72 mg, 0.093 mmol), EDCI (35.452 mg, 0.185 mmol), HOBT (24.98 mg, 0.185 mmol), DMAP (3.768 mg, 0.032 mmol), and DIPEA (39.8 mg, 0.308 mmol) were sequentially added to 3 ml of ultra-dry DMF, and the resulting mixture was stirred at room temperature for 30 min under argon protection, then Compound P9 (160 mg, 0.062 mmol) were added, and the resulting mixture was continuously stirred at room temperature under argon protection. After 12 hours of reaction, 126 mg of the product were obtained following purification with column chromatography, 73.1% yield. ¹H NMR (600 MHz, CDCl3) δ 11.43 (s, 2H), 8.55 (d, 2H), 8.20 (d, 2H), 7.71(d, 2H), 7.36 (m, 1H), 7.31 (t, 1H), 5.79 (m, 2H), 5.30 (s, 3H), 5.15 (m, 3H), 4.58 (s, 4H), 4.54 (t, 4H), 4.41 (m, 3H), 4.21 (m, 2H), 4.17 (d, 1H), 3.88 (m, 6H), 3.80 (s, 4H), 3.72 (s, 6H), 3.61 (d, 20H), 3.52 (m, 4H), 3.41 (m, 2H), 3.32 (m, 6H), 2.96 (m, 3H), 2.48 (m, 3H), 2.35 (q, 3H), 2.31 (t, 1H), 2.05 (s, 6H), 1.82 (m, 3H), 1.48 (d, 54H), 1.42 (s, 3H), 1.37 (s, 3H), 1.28 (s, 5H), 1.25 (s, 18H), 1.13 (m, 4H), 1.04 (m, 3H), 0.84 (m, 26H), [M+H]⁺=2780.5539

Compound P9-1 was used to replace Compound P9, and prop-2-yn-1-yloxy-PEG₆-COOH was used to replace prop-2-yn-1-yloxy- PEG₂-COOH, and Compound P10-1 was prepared using the same method.

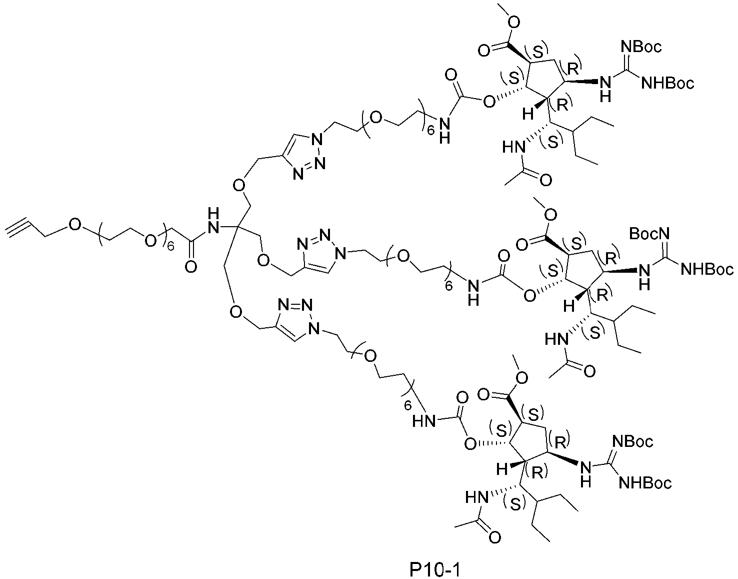

Compound P9-2 was used to replace Compound P9, and Compound P10-2 was prepared using the same method.

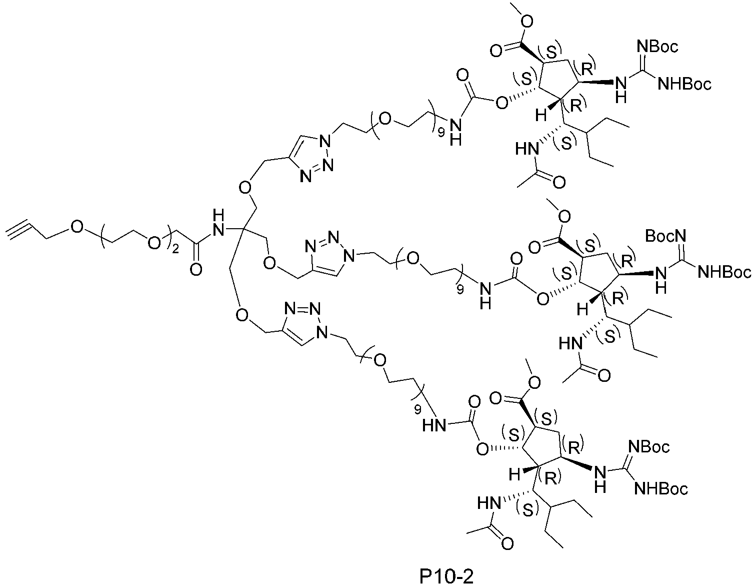

### 3) Preparation of Compound P11:

Compound P10 (120 mg, 0.043 mmol) was dissolved in 2 ml of ultra-dry DMF, then 2 ml of trifluoroacetic acid (TFA) were added, and the resulting mixture was stirred at room temperature and reacted for 4 h. The TFA was removed by a rotary evaporator, and 81 mg of the product were obtained following purification with C₁₈ reverse phase chromatography, 86.5% yield. [M+H]⁺=2179.3

Compound P11-1 and Compound P11-2 were prepared using the same method.

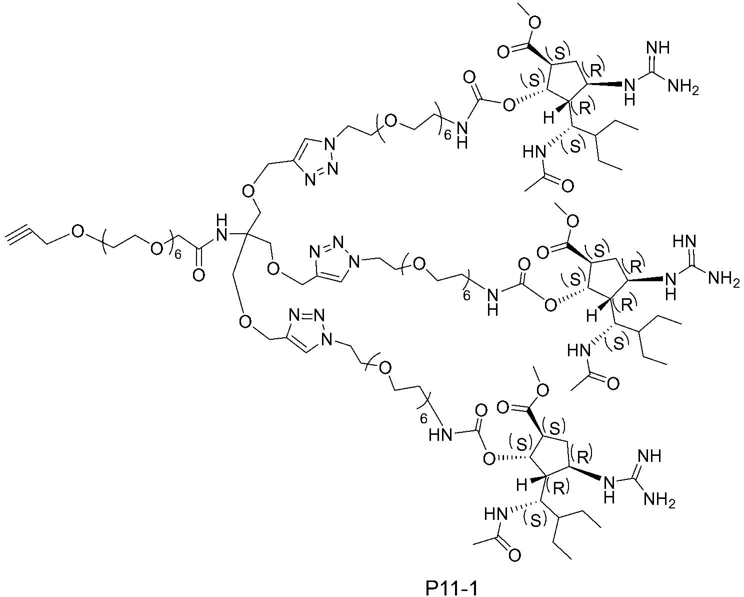

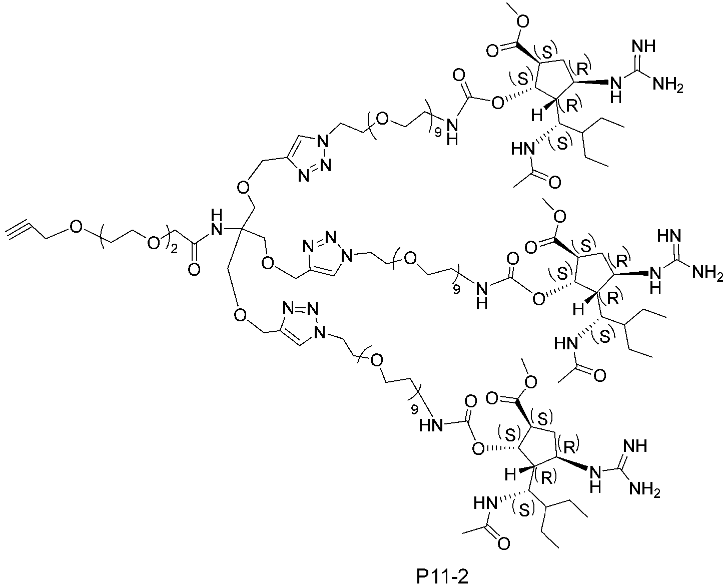

### 4) Preparation of Compound P12

Compound P11 (80 mg, 0.036 mmol) was dissolved in 1.5 ml of methanol, then 200 ul of double distilled water was added. The pH of the mixture was adjusted to 12 to 13 with 10 N aqueous NaOH solution, and the mixture was stirred at room temperature for 4 h. The pH of the reaction was adjusted to 4 with 4N HCl in dioxane, and finally 36 mg of the product were obtained following purification with C₁₈ reverse phase chromatography, 46.8% yield. [M+H]⁺=2137.4

Compound P12-1 and Compound P12-2 were prepared using the same method.

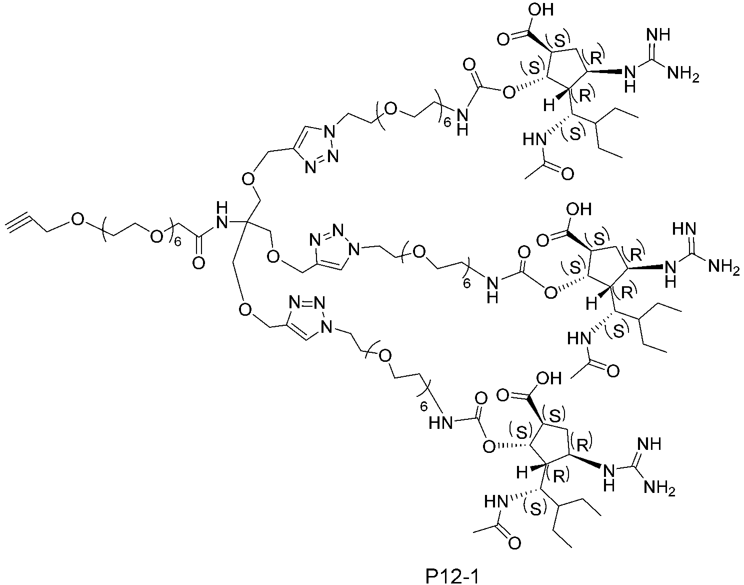

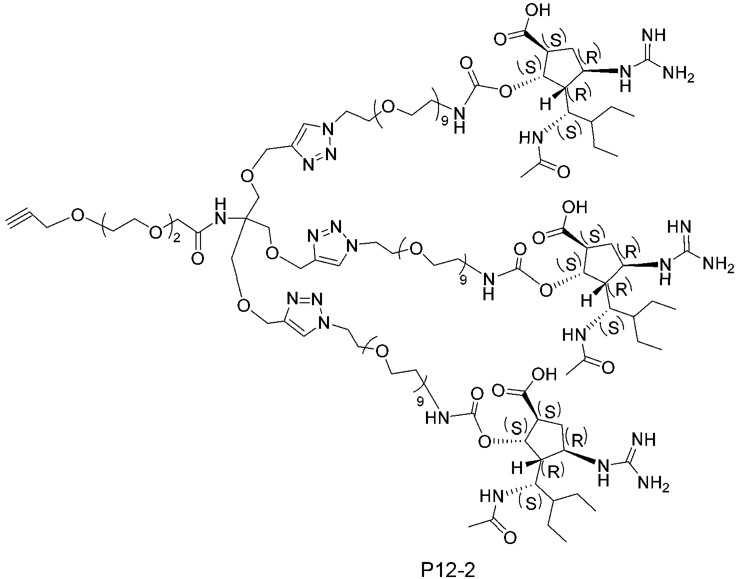

### Example 7 Synthesis of Oseltamivir Dimer Derivative

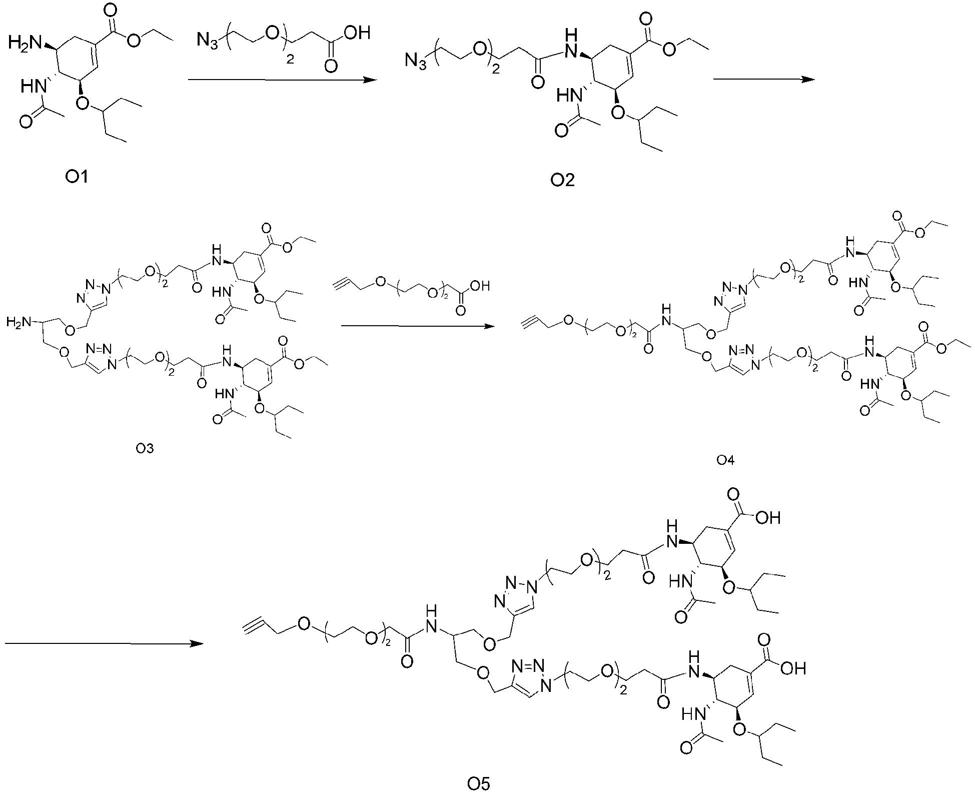

### 1) Preparation of Compound O2

The starting materials N₃-PEG₂-(CH₂)₂-COOH (1.084 g, 5.34 mmol), EDCI (1.53 g, 8.01 mmol), HOBT (1.81 g, 8.01 mmol), and DIPEA (1.378 g, 10.68 mmol) were sequentially added to 10 ml of ultra-dry DMF, and the resulting mixture was stirred at room temperature for 30 min under argon protection, then Compound O1 (2 g, 6.4 mmol) were added, and the resulting mixture was continuously stirred at room temperature under argon protection. After 12 hours of reaction, 1.76 g of the product were obtained following purification with column chromatography, 60.65% yield. [M+H]⁺=498.53

N₃-PEG₆-(CH₂)₂-COOH was used to replace N₃-PEG₂-(CH₂)₂-COOH, and Compound O2-1 was prepared using the same method.

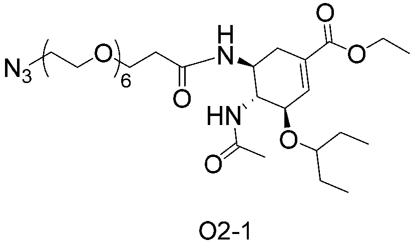

### 2) Preparation of Compound O3

Compound O2 (430 mg, 0.865 mmol) and Compound 5 (57.76 mg, 0.345 mmol) were dissolved in 2 ml of tetrahydrofuran (THF), respectively, and mixed with stirring. Aqueous solutions of CuSO₄·5H₂O (86 mg, 0.345 mmol) and sodium ascorbate (Na·VC, 136.7 mg, 0.69 mmol) were added to the reaction solution sequentially, and after 3 hours of reaction at room temperature, the reaction solution was extracted with DCM, and finally purified by column chromatography to obtain 160 mg of the product, 79.69% yield. [M+H]⁺=1162.48

Compound O2-1 was used to replace Compound O2, and Compound 03-1 was prepared using the same method.

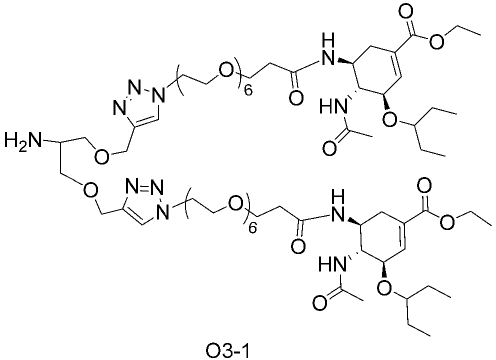

### 3) Preparation of Compound O4:

The starting materials prop-2-yn-1-yloxy-PEG₂-COOH (2-(2-(2-(prop-2-yn-1-yloxy)ethoxy)ethoxy)acetic acid, 41.8 mg, 0.2067 mmol), EDCI (80.62 mg, 0.4134 mmol), HOBT (55.81 mg, 0.4134 mmol), DMAP (8.42 mg, 0.0689 mmol), and DIPEA (89.05 mg, 0.6891 mmol) were sequentially added to 3 ml of ultra-dry DMF, and the mixture was stirred at room temperature for 30 min under argon protection, then Compound O3 (160 mg, 0.1378 mmol) were added, and the resulting the mixture was continuously stirred at room temperature under argon protection. After 12 hours of reaction, 170 mg of the product were obtained following purification with column chromatography, 91.72% yield. [M+H]⁺=1346.22

Compound 03-1 was used to replace Compound O3, and Compound 04-1 was prepared using the same method.

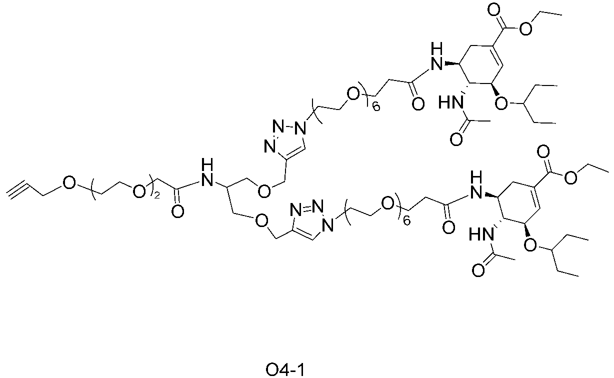

### 4) Preparation of Compound O5:

Compound O4 (170mg, 0.1264mmol) was completely dissolved in 1 ml of methanol solution, then a 5% aqueous NaOH solution was added dropwise slowly, precipitate formed, and then the solution gradually turned clear. After 1.5 hours of reaction, under the condition of an ice water bath, the pH of the reaction solution was adjusted to 4 with 1N diluted hydrochloric acid and then the reaction solution was extracted with n-butanol and purified by column chromatography to obtain 95 mg of the product, 62.58% yield, [M+H]⁺=1202.03

Compound 04-1 was used to replace Compound O4, and Compound 05-1 was prepared using the same method.

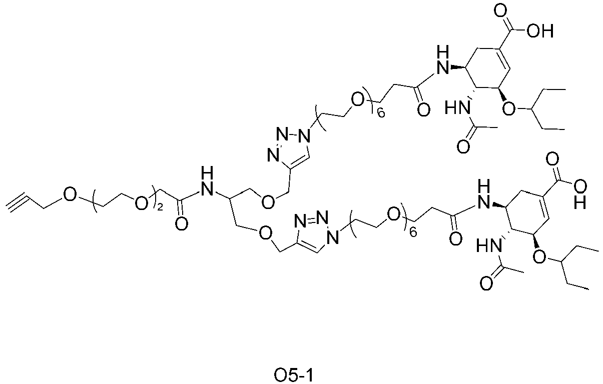

### Example 8. Synthesis of Oseltamivir Trimer Derivative

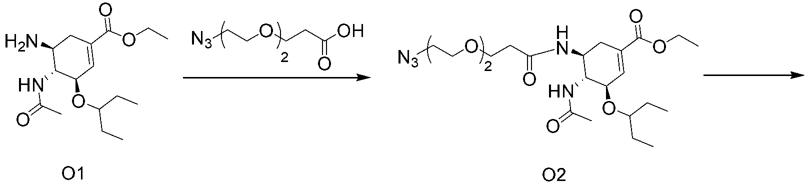

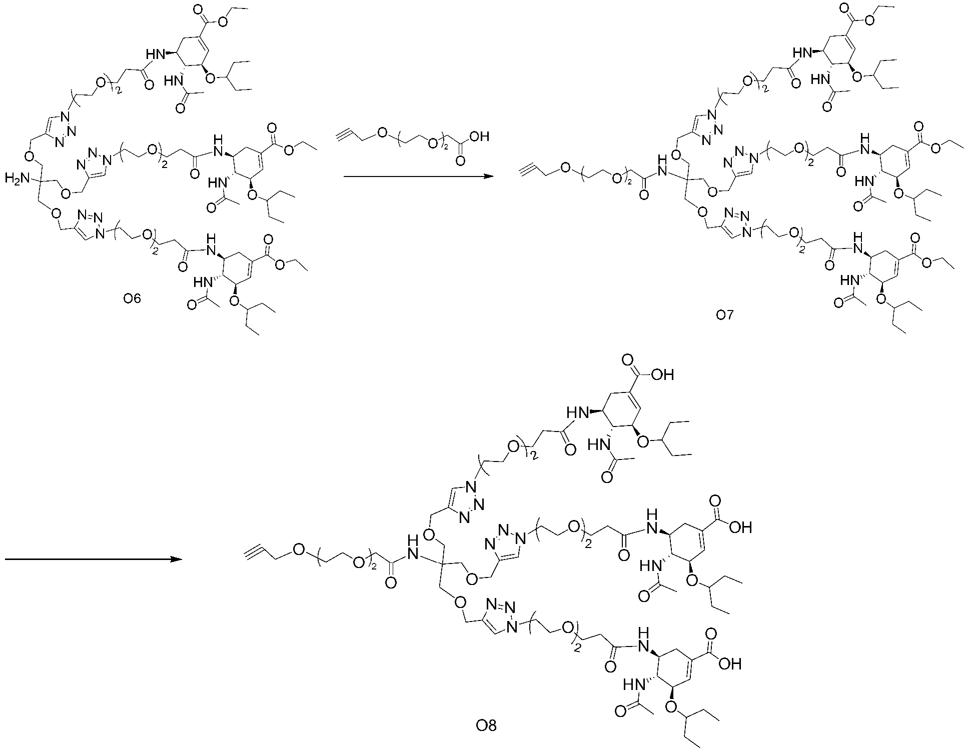

### 1) Preparation of Compound O6

Compound O2 (1.76 g, 3.541 mmol) and Compound 10 (250 mg, 1.063 mmol) were dissolved in 1.5 ml of tetrahydrofuran (THF), respectively, and mixed with stirring. Aqueous solutions of C_{U}SO₄·5H₂O (132.66 mg, 0.53 mmol) and sodium ascorbate (Na·VC, 210.60 mg, 1.06 mmol) were added to the reaction solution sequentially, and after 3 hours of reaction at room temperature, the reaction solution was extracted with DCM, and finally purified by column chromatography to obtain 1.28 g of the product, 62.8% yield. M+H]⁺=1727.34.

Compound O2-1 was used to replace Compound O2, and Compound 06-1 was prepared using the same method.

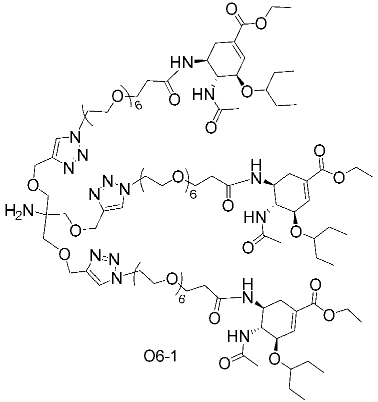

### 2) Preparation of Compound O7:

The starting materials prop-2-yn-1-yloxy-PEG₂-COOH (2-(2-(2-(prop-2-yn-1-yloxy)ethoxy)ethoxy)acetic acid, 57.99 mg, 0.2868 mmol), EDCI (111.85 mg, 0.5736 mmol), HOBT (77.436 mg, 0.5736 mmol), DMAP (11.68 mg, 0.0956 mmol), and DIPEA (123.55 mg, 0.956 mmol) were sequentially added to 3 ml of ultra-dry DMF, and the mixture was stirred at room temperature for 30 min under argon protection, then Compound O6 (330 mg, 0.1912 mmol) were added, and the resulting mixture was continuously stirred at room temperature under argon protection. After 12 hours of reaction, 305 mg of the product were obtained following purification with column chromatography, 89.72% yield. [M+H]⁺=1779.4.

Compound 06-1 is used to replace Compound O6, and Compound 07-1 was prepared using the same method.

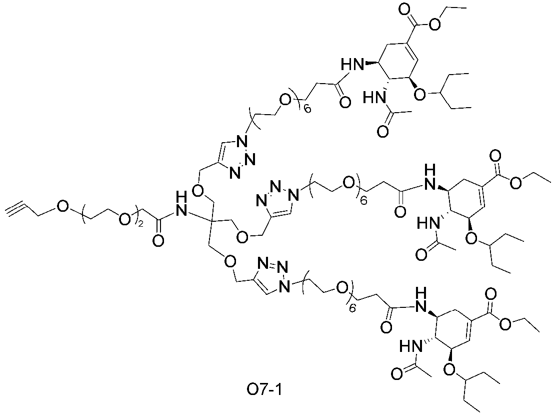

### 3) Preparation of Compound O8:

Compound O7 (150 mg, 0.078 mmol) was completely dissolved in 1 ml of methanol solution, then a 5% aqueous NaOH solution was added slowly dropwise, precipitate formed, and then the solution gradually turned clear. After 1.5 hours of reaction, under the condition of an ice water bath, the pH of the reaction solution was adjusted to 4 with 1N diluted hydrochloric acid, and then the reaction solution was extracted with n-butanol and purified by column chromatography to obtain 95 mg of the product, 71.89% yield, [M+H]⁺=1695.79.

Compound 07-1 was used to replace Compound O7, and Compound 08-1 was prepared using the same method.

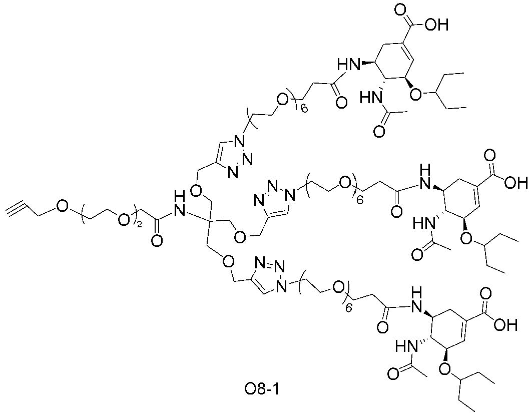

### Example 9 Synthesis I of conjugates

Compound R14 and an Fc protein (with a sequence as set forth in SEQ ID NO:64) were conjugated to form Conjugate I-1

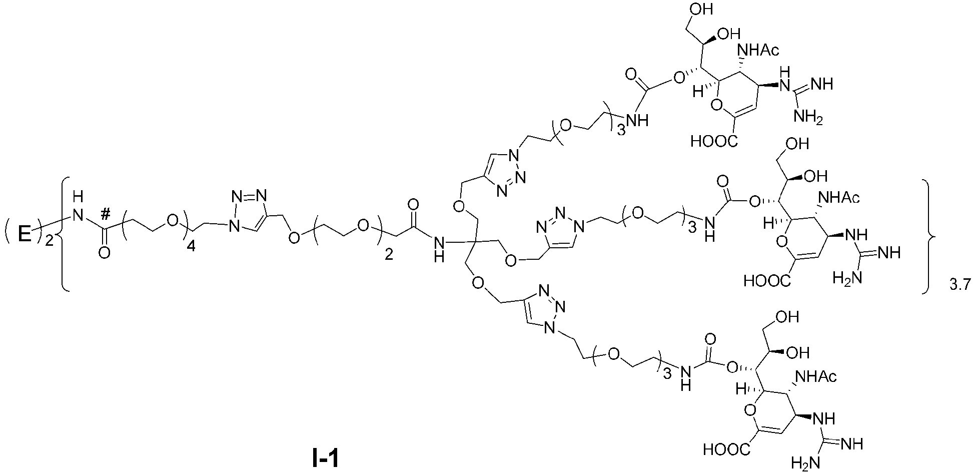

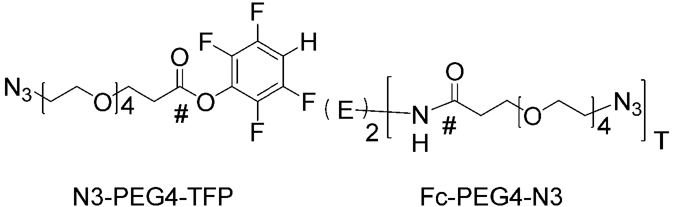

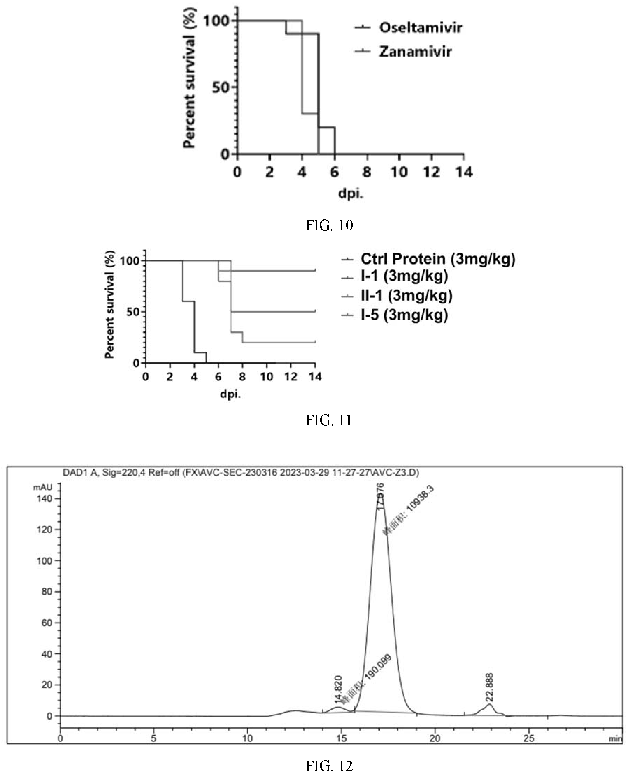

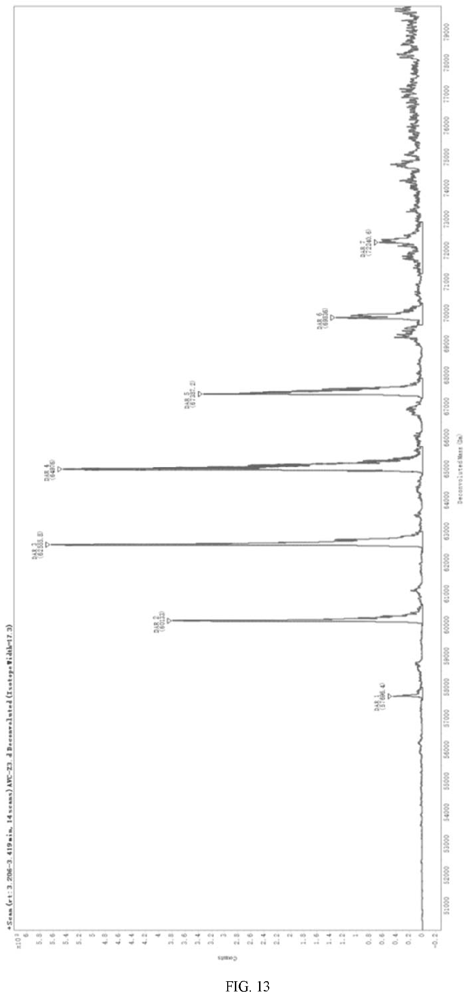

The Fc protein (an Fc domain, with a sequence as set forth in SEQ ID NO:64) was buffer-exchanged into a PBS solution. A 5 mg/ml stock solution of N₃-PEG₄-TFP (manufactured by Hunan Huateng pharmaceutical Co., Ltd.) in DMSO was prepared. 5 mg of FC protein was taken and combined with N₃-PEG₄-TFP (0.278 mg, 0.633 µmol), and the mixture was reacted at room temperature overnight, to conjugate the Fc protein to the site# of N₃-PEG₄-TFP via its N atom. The product was purified by a PD-10 desalting chromatography column, and concentrated by ultrafiltration, to obtain 4.6 mg of Fc-PEG₄-N₃, to which Compound R14 (R14: Fc = 20 eq : 1 eq) was then added, and CuSO₄·5H₂O (0.95 mg, 3.81 µmol), tris(3-hydroxypropyltriazomethyl) amine (THPTA, 1.66 mg, 3.81 µmol) and sodium ascorbate (Na•VC, 2.7 mg, 13.6 µmol) were added sequentially, and the resulting solution was reacted at room temperature for 4 h. Conjugate I-1 was obtained after purification with a PD-10 desalting chromatography column and ultrafiltration concentration, and its HPLC chromatogram was shown in Fig. 12, with a purify of 95.57%; the mass spectrum (MS) was shown in FIG. 13. In Conjugate I-1, E represents the Fc domain monomer (with an amino acid sequence as set forth in SEQ ID NO:64), (E)₂ represents an Fc protein, and T is about 3.7.

Conjugate I-2 was obtained via click reaction between Compound R14-1 and Fc-PEG₄-N₃ using the same method, wherein E represents the Fc domain monomer (with an amino acid sequence as set forth in SEQ ID NO:64), (E)₂ represents the Fc protein, and T is about 3.7.

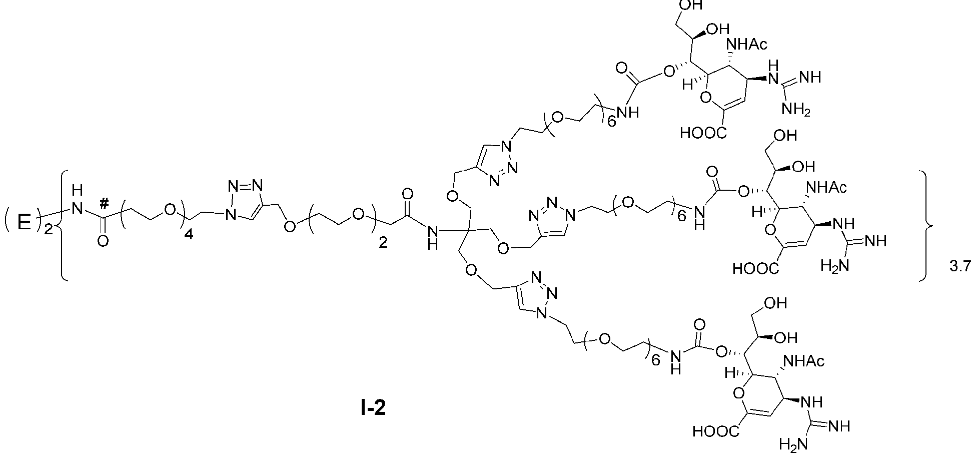

Conjugate I-3 was obtained via click reaction between Compound R14-2 and Fc-PEG₅-N₃ using the same method, wherein E represents the Fc domain monomer (with an amino acid sequence as set forth in SEQ ID NO:64), (E)₂ represents the Fc protein, and T is about 3.7.

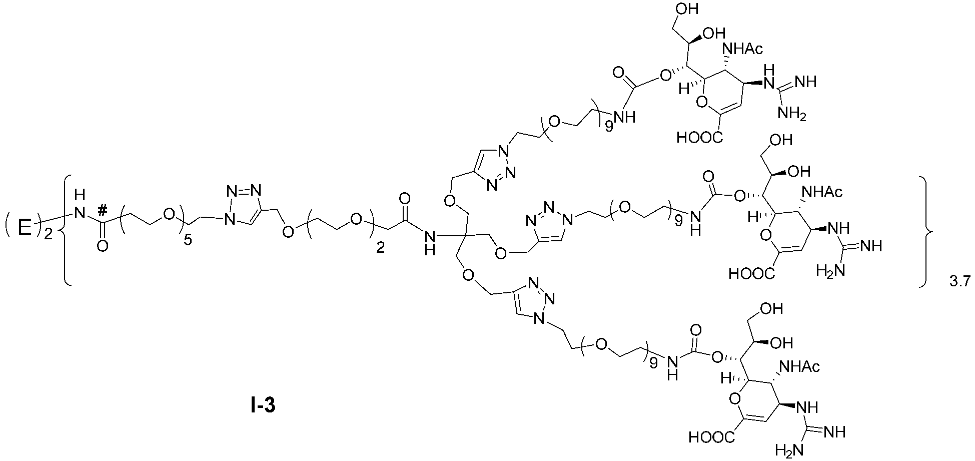

Conjugate I-4 was obtained via click reaction between Compound R14-3 and Fc-PEG₆-N₃ using the same method, wherein E represents the Fc domain monomer (with an amino acid sequence as set forth in SEQ ID NO:64), (E)₂ represents the Fc protein, and T is about 3.7.

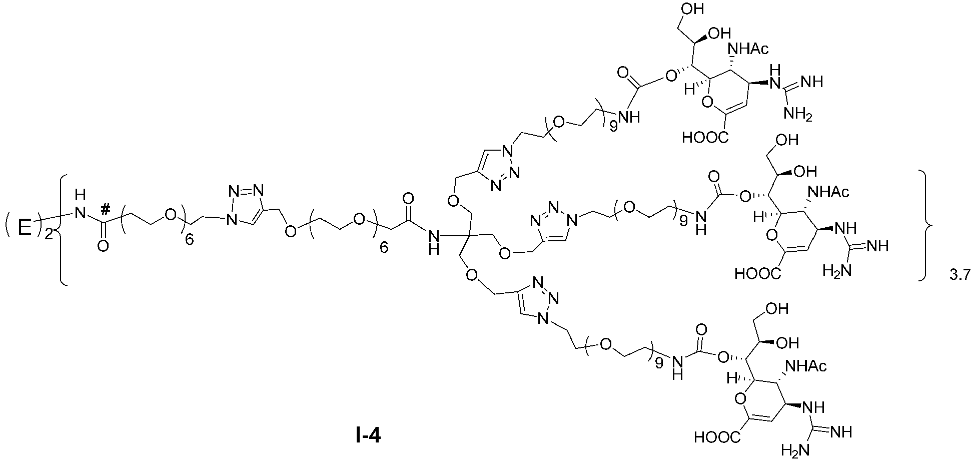

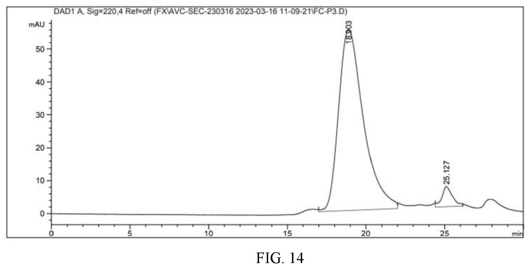

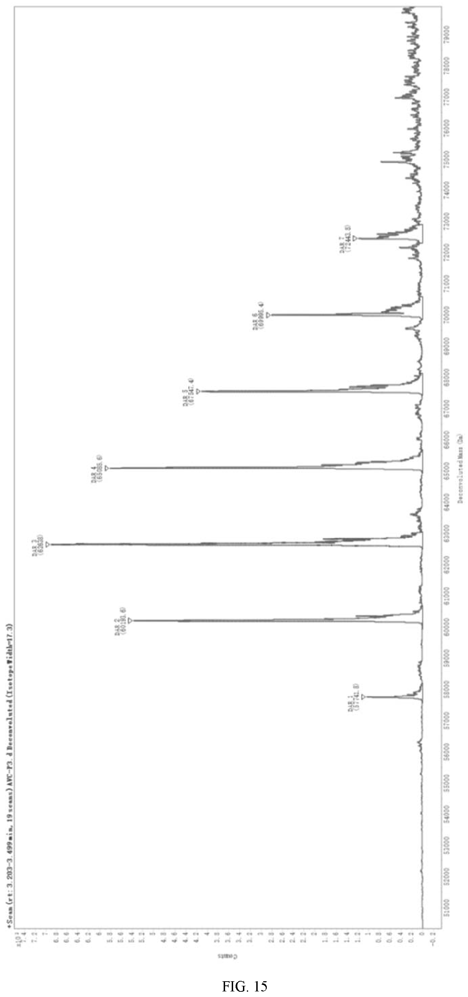

Conjugate I-5 was obtained via click reaction between Compound P11 and Fc-PEG₄-N₃ using the same method, of which the HPLC chromatogram was shown in FIG. 14, with a purity of 95.49%; the mass spectrum (MS) was shown in FIG. 15. In Conjugate I-5, E represents the Fc domain monomer (with an amino acid sequence as set forth in SEQ ID NO:64), (E)₂ represents the Fc protein, and T is about 3.7.

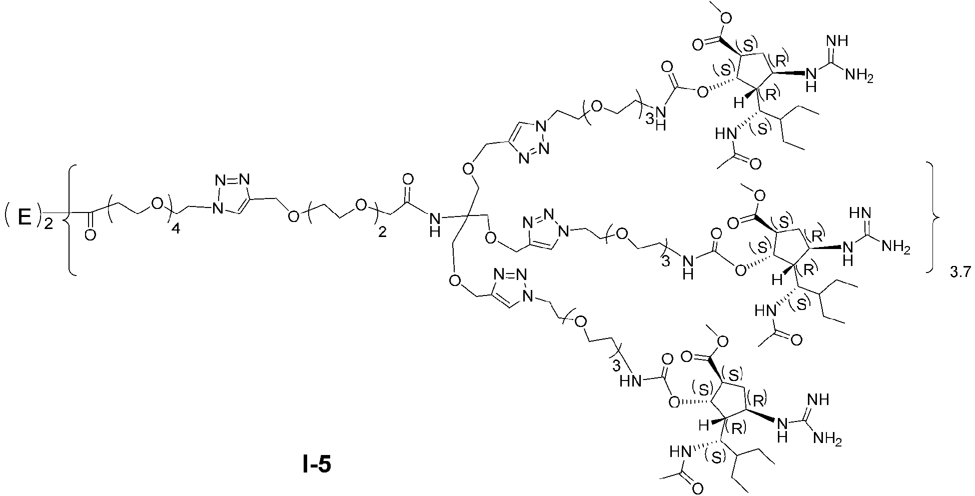

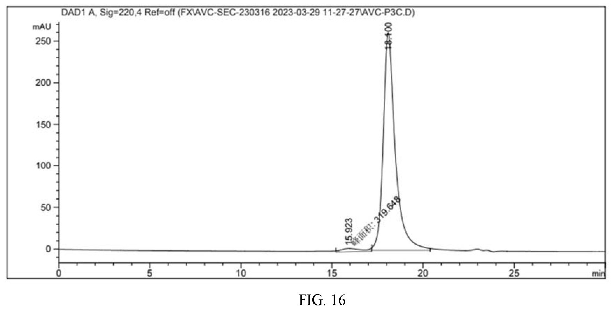

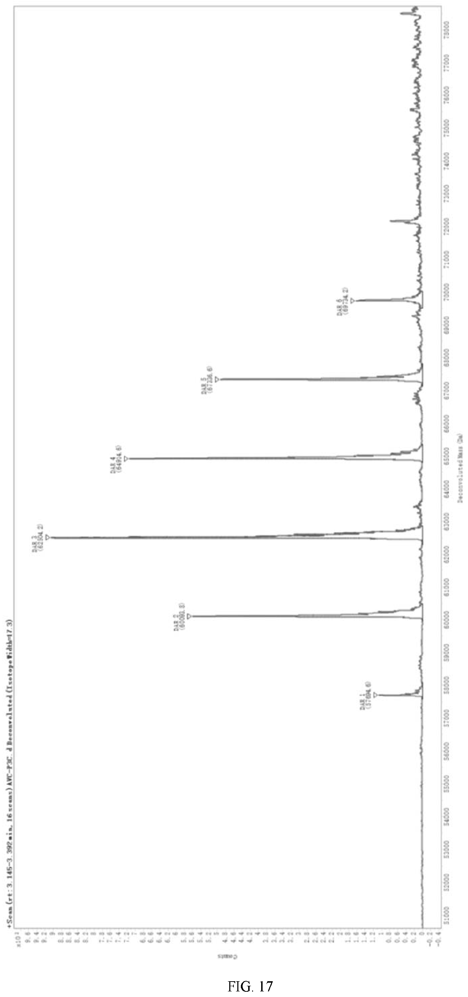

Conjugate I-6 was obtained via click reaction between Compound P12 and Fc-PEG₄-N₃ using the same method, of which the HPLC chromatogram was shown in FIG. 16, with a purity of 97.41%; the mass spectrum (MS) was shown in FIG. 17. In Conjugate I-6, E represents the Fc domain monomer (with an amino acid sequence as set forth in SEQ ID NO:64), (E)₂ represents the Fc protein, and T is about 3.5.

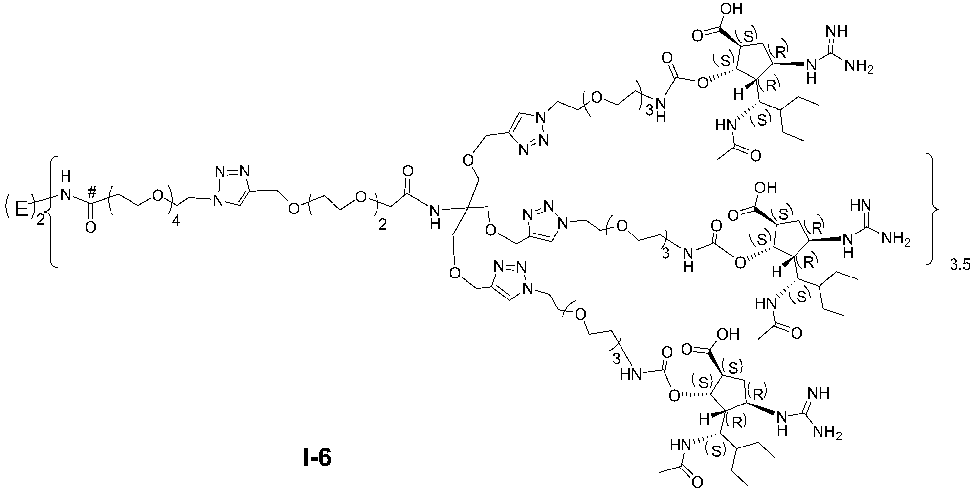

Conjugate I-7 was obtained via click reaction between Compound P12-1 and Fc-PEG₆-N₃ using the same method, wherein E represents the Fc domain monomer (with an amino acid sequence as set forth in SEQ ID NO:64), (E)₂ represents the Fc protein, and T is about 3.5.

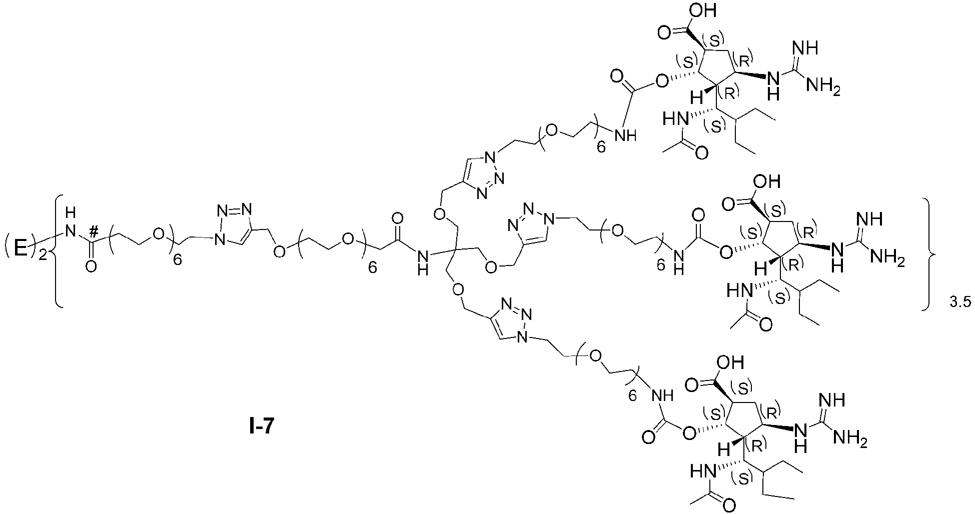

Conjugate I-8 was obtained via click reaction between Compound P12-2 and Fc-PEG₄-N₃ using the same method, wherein E represents the Fc domain monomer (with an amino acid sequence as set forth in SEQ ID NO:64), (E)₂ represents the Fc protein, and T is about 3.5.

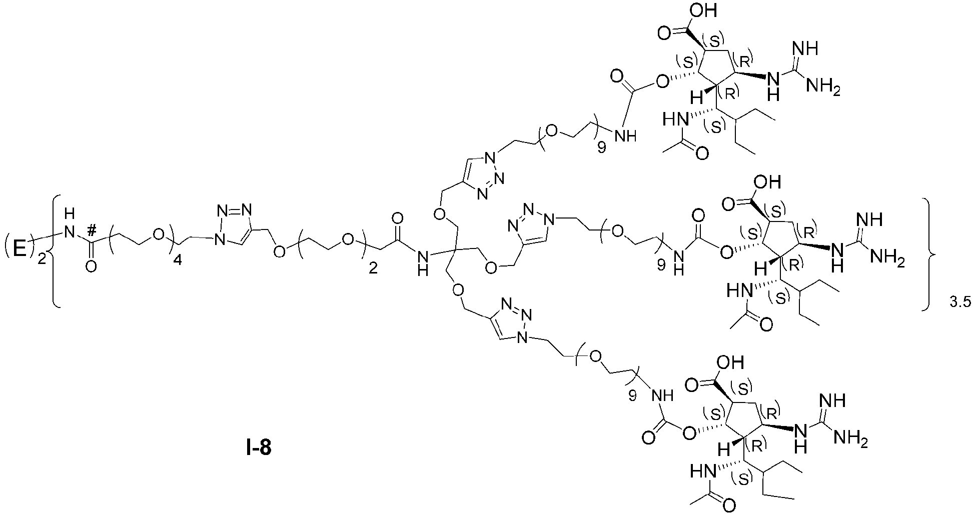

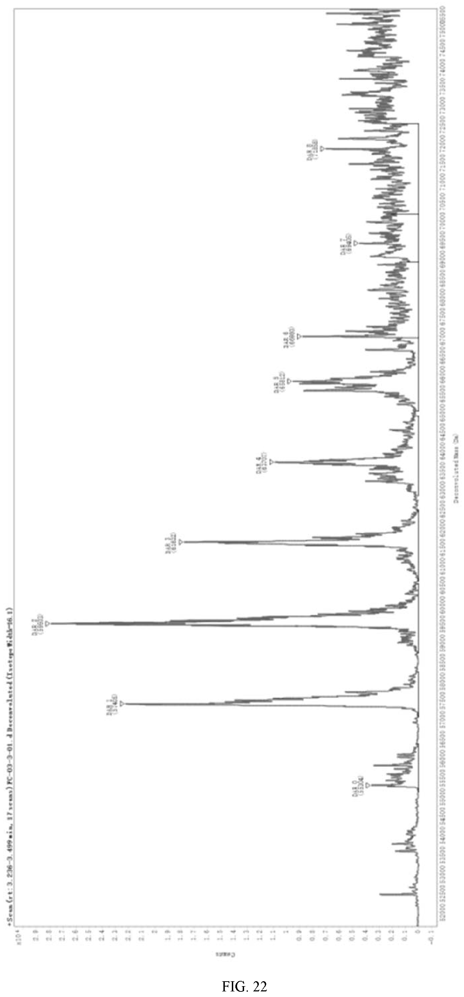

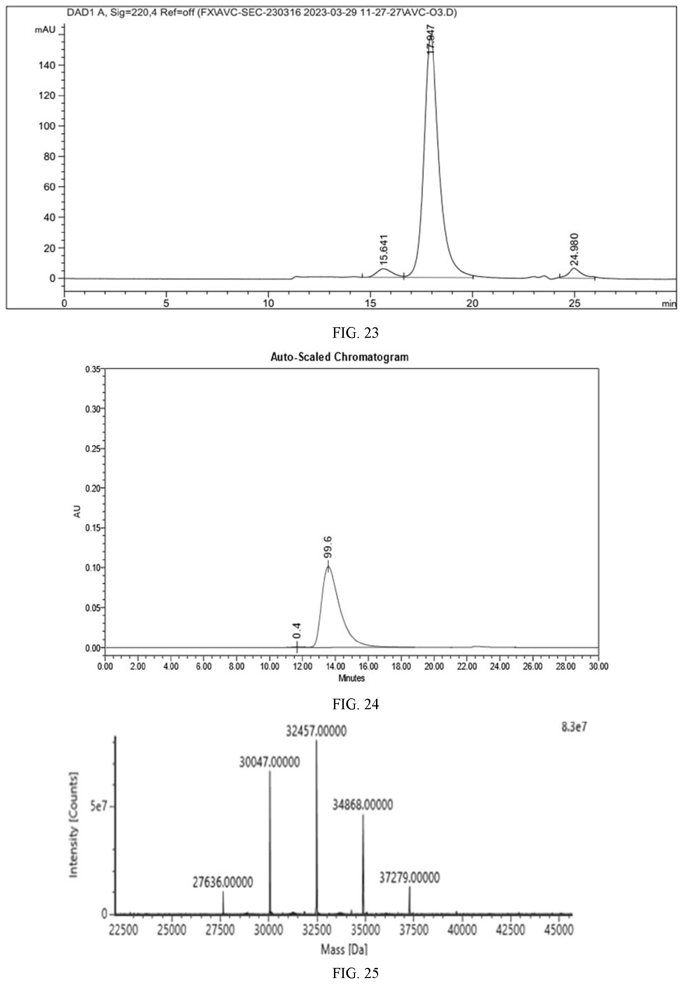

Conjugate I-9 was obtained via click reaction between Compound O8 and Fc-PEG₄-N₃ using the same method, of which the HPLC chromatogram was shown in FIG. 22, with a purity of 93.22%; the mass spectrum (MS) was shown in FIG. 23. In Conjugate I-9, E represents the Fc domain monomer (with an amino acid sequence as set forth in SEQ ID NO:64), (E)₂ represents the Fc protein, and T is about 3.2.

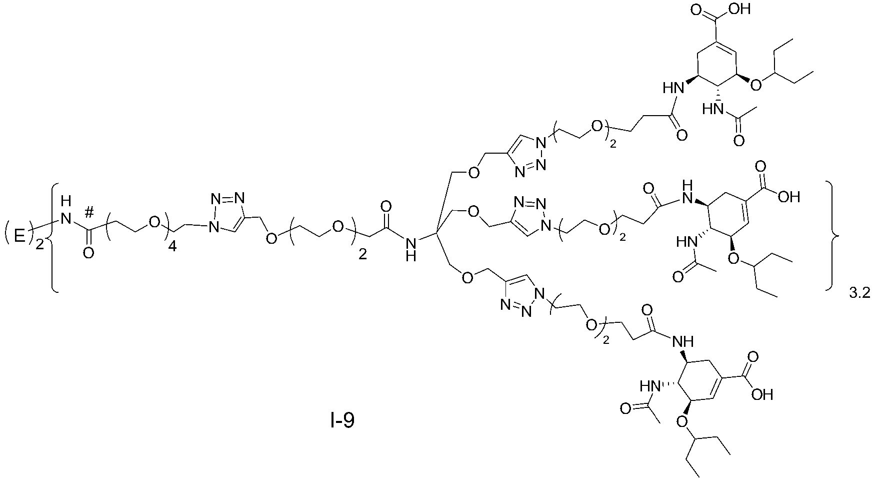

Conjugate I-10 was obtained via click reaction between Compound 08-1 and Fc-PEG₄-N₃ using the same method, wherein E represents the Fc domain monomer (with an amino acid sequence as set forth in SEQ ID NO:64), (E)₂ represents the Fc protein, and T is about 3.2.

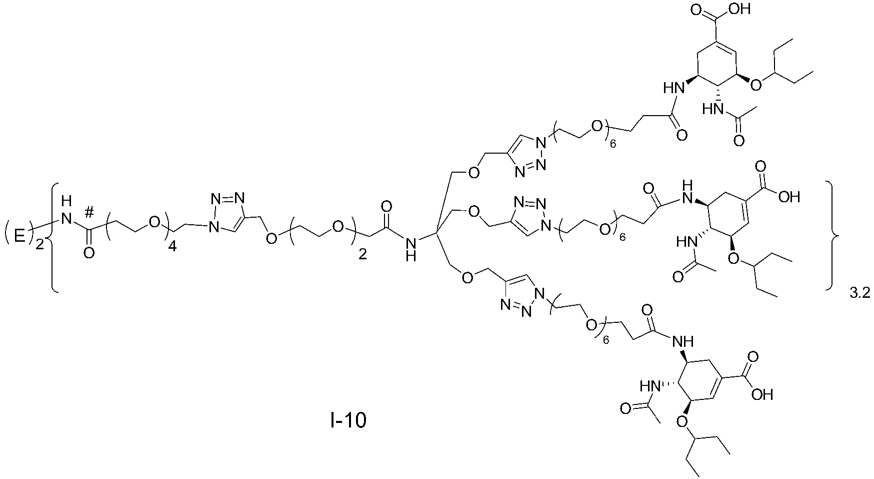

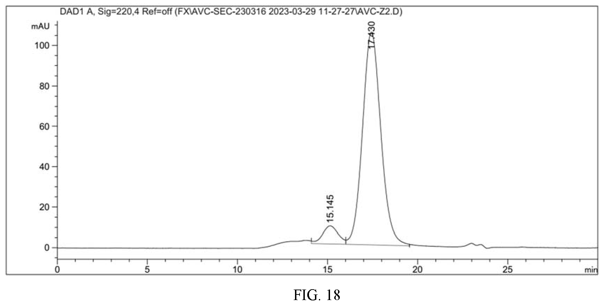

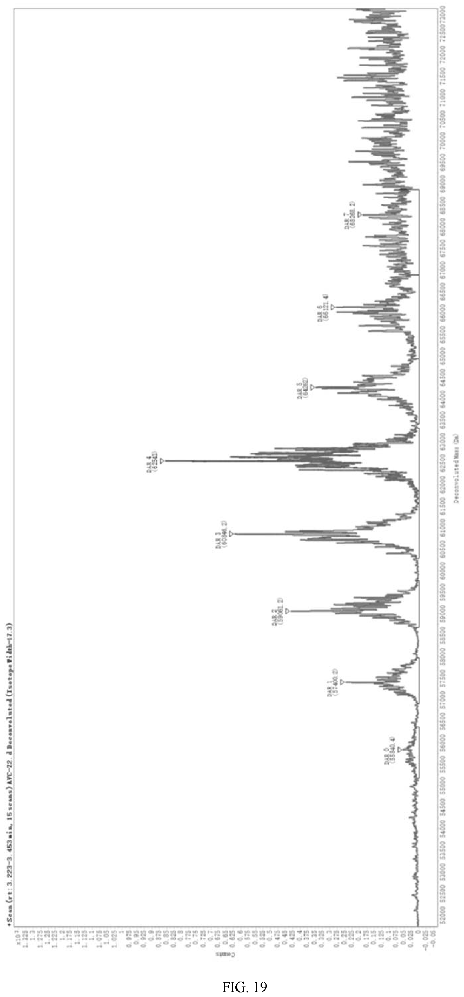

Conjugate II-1 was obtained via click reaction between Compound R10 and Fc-PEG₄-N₃ using the same method, of which the HPLC chromatogram was shown in FIG. 18, with a purity of 93.27%; the mass spectrum (MS) was shown in FIG. 19. In Conjugate II-1, E represents the Fc domain monomer (with an amino acid sequence as set forth in SEQ ID NO:64), (E)₂ represents the Fc protein, and T is about 3.7. 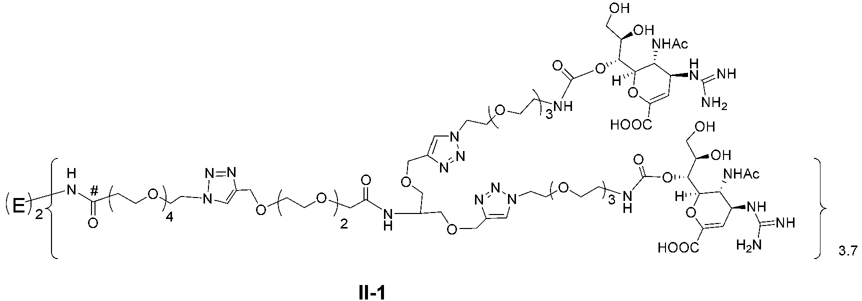 Conjugate II-2 was obtained via click reaction between Compound R10-1 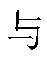 Fc-PEG₄-N₃ using the same method, wherein E represents the Fc domain monomer (with an amino acid sequence as set forth in SEQ ID NO:64), (E)₂ represents the Fc protein, and T is about 3.7.

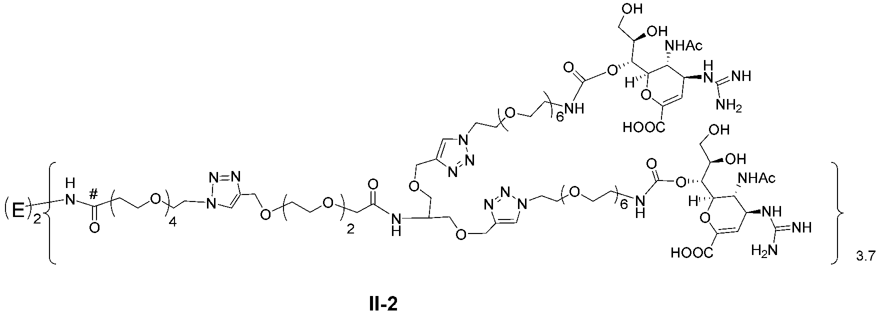

Conjugate II-3 was obtained via click reaction between Compound R10-2 and Fc-PEG₅-N₃ using the same method, wherein E represents the Fc domain monomer (with an amino acid sequence as set forth in SEQ ID NO:64), (E)₂ represents the Fc protein, and T is about 3.7.

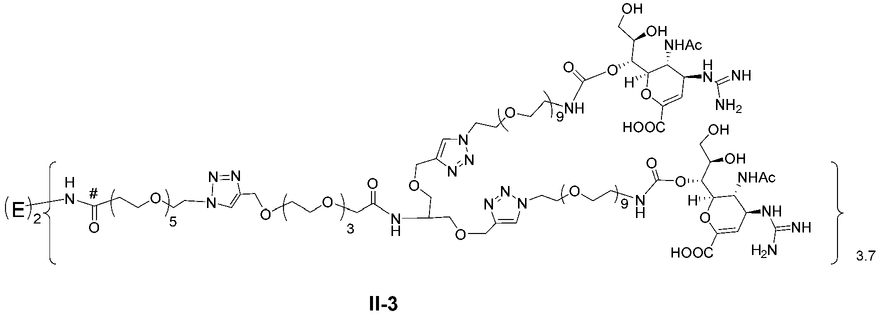

Conjugate II-4 was obtained via click reaction between Compound R10-3 and Fc-PEG₆-N₃ using the same method, wherein E represents the Fc domain monomer (with an amino acid sequence as set forth in SEQ ID NO:64), (E)₂ represents the Fc protein, and T is about 3.7.

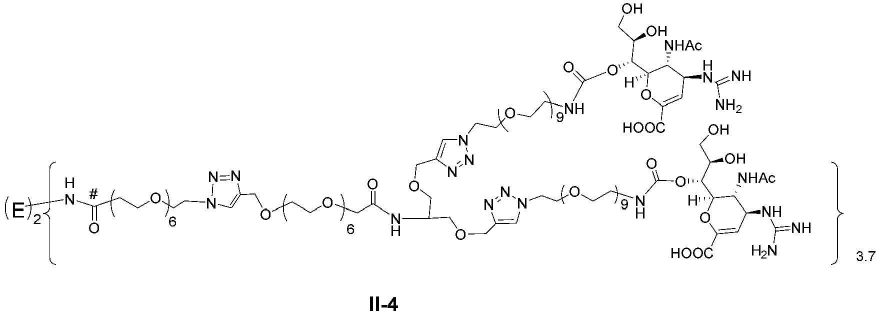

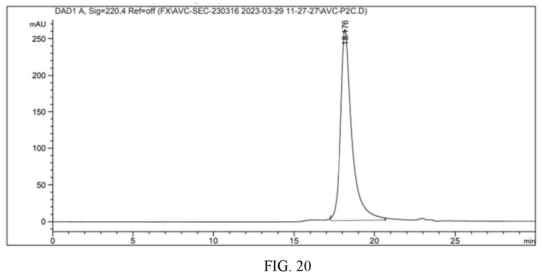

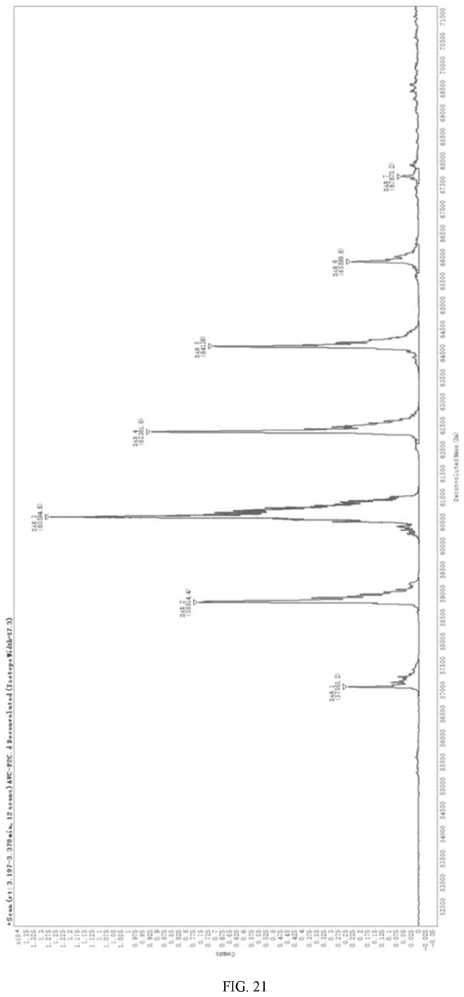

Conjugate II-6 was obtained via click reaction between Compound P8 and Fc-PEG₄-N₃ using the same method, of which the HPLC chromatogram was shown in FIG. 20, with a purity of 100.00%; the mass spectrum (MS) was shown in FIG. 21. In Conjugate II-6, E represents the Fc domain monomer (with an amino acid sequence as set forth in SEQ ID NO:64), (E)₂ represents the Fc protein, and T is about 3.5.

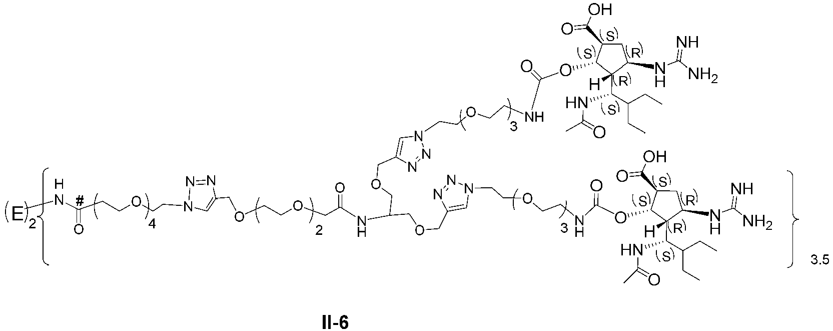

Conjugate II-5 was obtained via click reaction between Compound P7 and Fc-PEG₄-N₃ using the same method, wherein E represents the Fc domain monomer (with an amino acid sequence as set forth in SEQ ID NO:64), (E)₂ represents the Fc protein, and T is about 3.5.

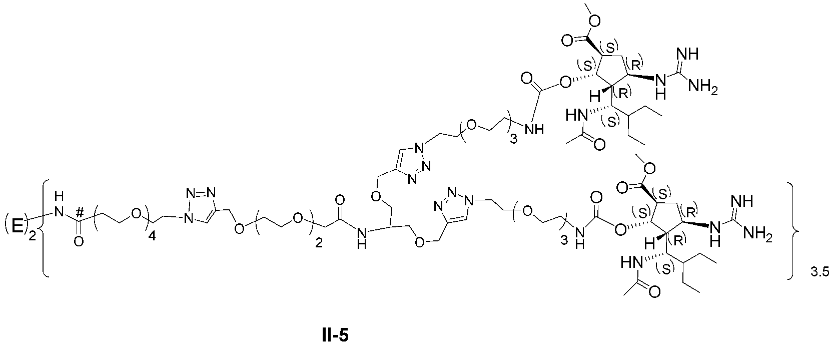

Conjugate II-7 was obtained via click reaction between Compound P8-1 and Fc-PEG₆-N₃ using the same method, wherein E represents the Fc domain monomer (with an amino acid sequence as set forth in SEQ ID NO:64), (E)₂ represents the Fc protein, and T is about 3.5.

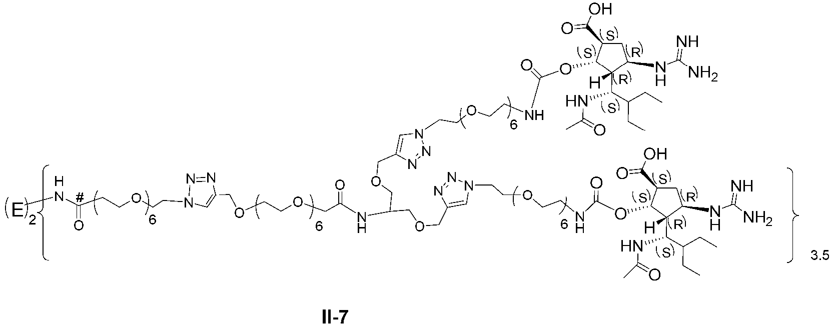

Conjugate II-8 was obtained via click reaction between Compound P8-2 and Fc-PEG₄-N₃ using the same method, wherein E represents the Fc domain monomer (with an amino acid sequence as set forth in SEQ ID NO:64), (E)₂ represents the Fc protein, and T is about 3.5.

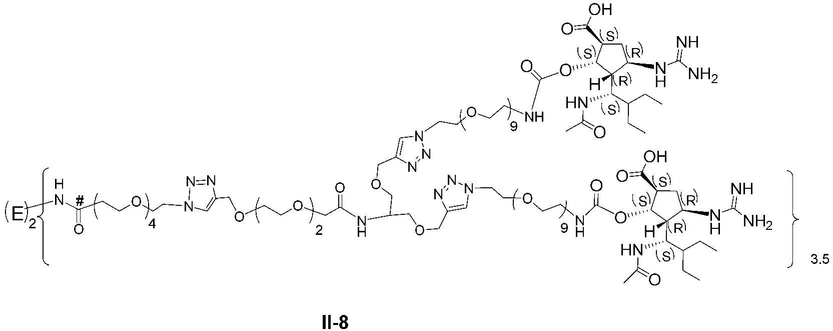

Conjugate II-9 was obtained via click reaction between Compound O5 and Fc-PEG₄-N₃ using the same method, wherein E represents the Fc domain monomer (with an amino acid sequence as set forth in SEQ ID NO:64), (E)₂ represents the Fc protein, and T is about 3.2.

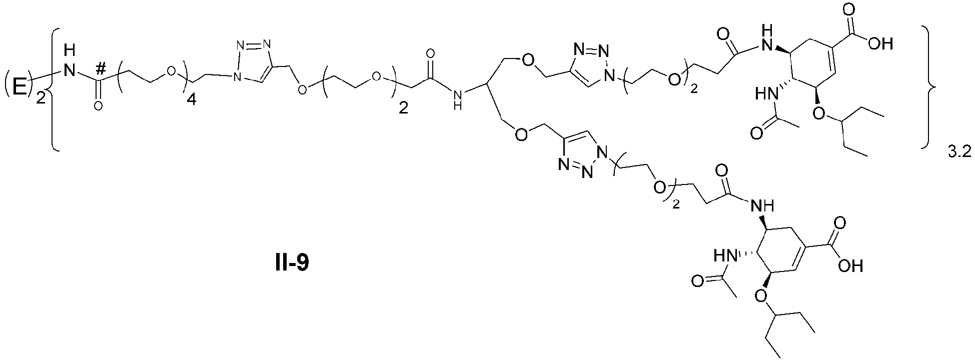

Conjugate II-10 was obtained via click reaction between Compound O5-1 and Fc-PEG₄-N₃ using the same method, wherein E represents the Fc domain monomer (with an amino acid sequence as set forth in SEQ ID NO:64), (E)₂ represents the Fc protein, and T is about 3.2.

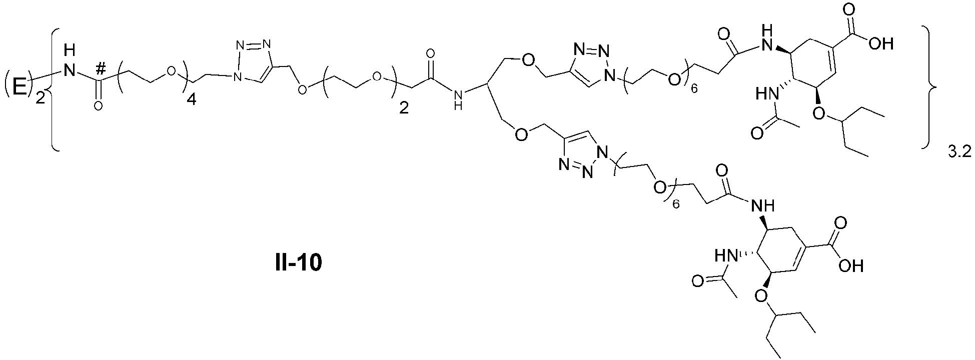

### Example 9-1 Synthesis II of Conjugates

### 1. Preparation of Compound P 12-A

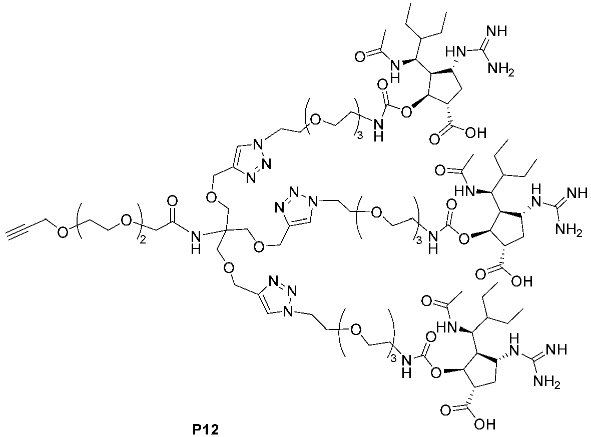

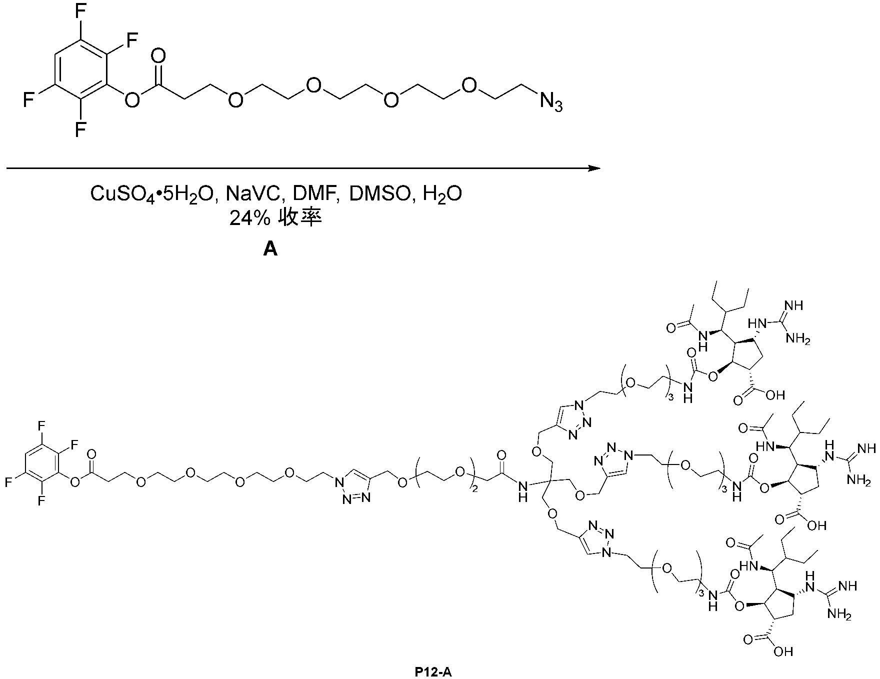

At room temperature, to an mixed solvent of N,N-dimethylformamide (2 mL), dimethylsulfoxide (2 mL) and water (4 mL) in which compound P12 (300 mg, 0.140 mmol), 2,3,5,6-tetrafluorophenyl 1-azido-3,6,9,12-tetraoxypentadecane-15-ate (93 mg, 0.212 mmol) were dissolved, aqueous solutions of CuSO₄·5H₂O (18 mg, 0.072 mmol) and sodium ascorbate (28 mg, 0.141 mmol) were added subsequentially at room temperature. After the addition was complete, the resulting mixture was stirred at room temperature for 30 min. The reaction solution is not treated and directly prepared and separated by HPLC, with the preparation conditions: Shim-pack GIST C18 (20 mm × 250 mm, 5 µm), A: 0.1% TFA in water, B: MeCN; 40 mL/min; 0~3min, 10% B; 3~3.5min, 10%~25% B; 3.5~14min, 25%~60% B; 14~14.5min, 60%~100% B; detector, UV 254 nm. The white solid product P12-A was obtained (98.28 mg, 0.034 mmol, 24.03%).¹H NMR (500 MHz, DMSO-*d₆*) *δ* ppm 12.64 (br s, 3H), 8.01 (s, 3H), 7.63 (d, *J* = 7.6 Hz, 3H), 7.29 (br s, 4H), 7.01 (t, *J* = 6.3 Hz, 3H), 6.82-6.88 (m, 3H), 6.70 (br s, 4H), 5.14-5.20 (m, 3H), 4.45-4.54 (m, 15H), 4.34 (t, *J* = 10.4 Hz, 3H), 3.75-3.85 (m, 14H), 3.66 (s, 6H), 3.35-3.56 (m, 44H), 3.06-3.18 (m, 6H), 3.01 (t, *J* = 6.0 Hz, 2H), 2.57-2.69 (m, 7H), 2.26-2.34 (m, 3H), 1.96-2.04 (m, 2H), 1.78 (s, 9H), 1.52-1.60 (m, 3H), 1.40-1.48 (m, 5H), 1.20-1.32 (m, 12H), 1.01-1.08 (m, 4H), 0.78-0.91 (m, 21H); ESI-MS m/z = 1289.3 [M/2+H]⁺; Calcu. = 2575.3.

### 2. Synthesis of Conjugate I-6

50 mg/mL of a stock solution of P12-A was formulated with DMSO. An Fc protein (10 mg/mL, 37.32 mg, 0.67 µmol) was buffer-exchanged into a 50 mM PBS (pH 7.4) solution, to which 11 eq of a stock solution of small molecule P12-A (50 mg/mL, 19.05 mg, 7.4 µmol) was added, and the amount of DMSO was 10% of the total system. The resulting mixture was shaken at room temperature and reacted overnight, and the sample was buffer-exchanged into a 20 mM histidine buffer solution (pH 5.0) by a 30kD ultrafiltration centrifuge tube (Millipore), to obtain 12.5 mg of Conjugate I-6 in total. The SEC-HPLC chromatogram of Conjugate I-6 was shown in FIG. 24, with a purity of 99.6%; and the mass spectrum (MS) was shown in FIG. 25, with a DAR value of 3.8.

Conjugates I-1 to I-5 and I-7 to I-10 and Conjugates II-1 to II-10 were also synthesized and prepared using the same method.

### Example 10 Evaluation on NA enzyme inhibitory activity

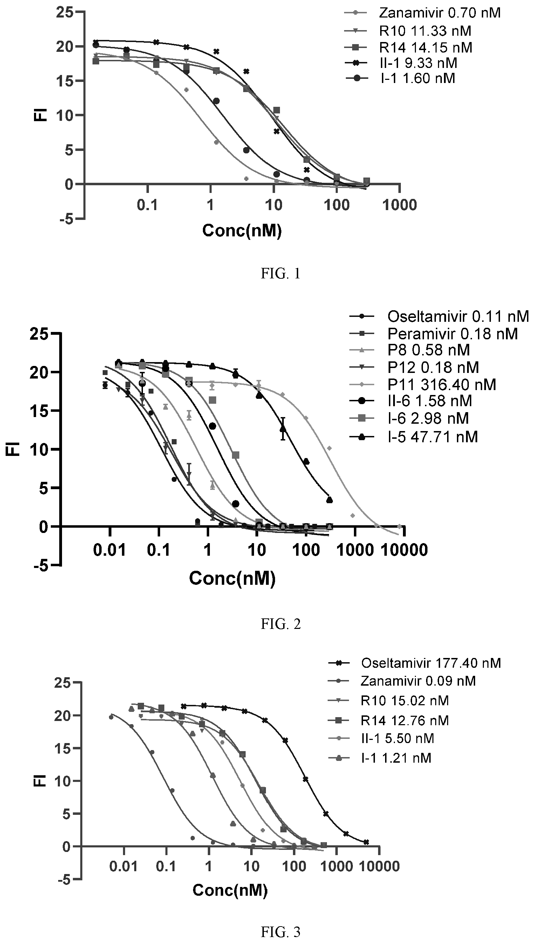

To verify the activity of inhibiting influenza virus at the enzyme level, the half maximal inhibitory concentration (IC₅₀) of the multimeric small molecule derivatives and conjugates provided in the examples of the present application as well as small molecule drugs (zanamivir (purchased from Adamas-beta, Catalog #C63-102157A), peramivir (purchased from Bide Pharmatech Co., Ltd., Catalog #BD317210), and oseltamivir phosphate (purchased from Innochem, Catalog #A90108)) against NA (A/California/04/2009, H1N1, purchased from Sino Biological, Catalog #11058-VNAHC) was tested. The serially diluted drugs were mixed with NA proteins and co-incubated for 30 min at room temperature, then to which a MUNANA substrate (purchased from Sigma, Catalog #M8639) was added, and fluorescence intensity was measured using a microplate reader (Molecular Devices, SpectraMax Gemini EM), the IC₅₀ values were calculated. The results were as shown in FIG. 1 and FIG. 2, the conjugates each exhibited good NA enzyme inhibitory activity.

### Example 11 Evaluation on NA enzyme inhibitory activity against Drug-Resistant Strains

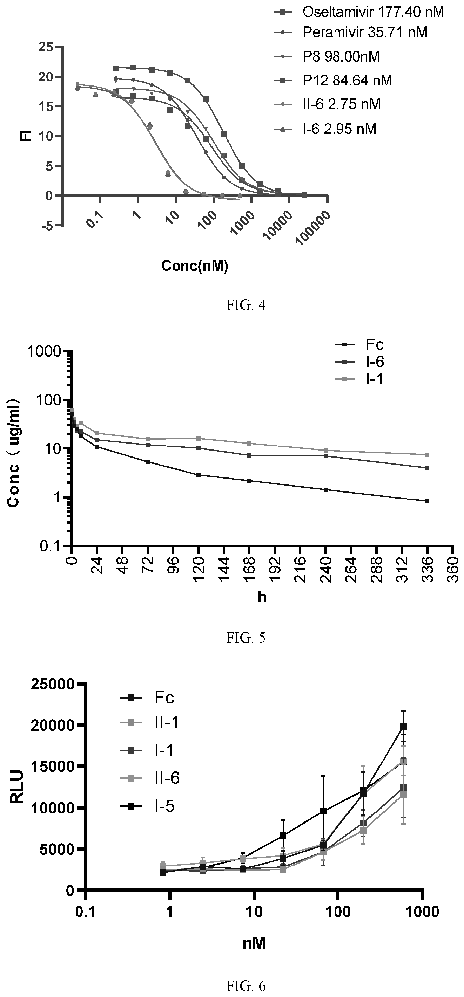

To verify the activity of inhibiting influenza virus at the enzyme level, the half maximal inhibitory concentration (IC₅₀) of the multimeric small molecule derivatives and conjugates provided in the examples of the present application as well as small molecule drugs (zanamivir, peramivir, and oseltamivir phosphate) against NA (A/California/04/2009, H275Y, H1N1, purchased from Sino biological, Catalog #11058-VNAHC) was tested. The serially diluted drugs were mixed with NA proteins and co-incubated for 30 min at room temperature, then to which a MUNANA substrate was added, and fluorescence intensity was measured using a microplate reader, the IC₅₀ values were calculated. The results were as shown in FIG. 3 and FIG. 4, the conjugates each showed good NA enzyme inhibitory activity against drug-resistant strains.

### Example 12 Evaluation on In Vitro Anti-Influenza Virus Activity

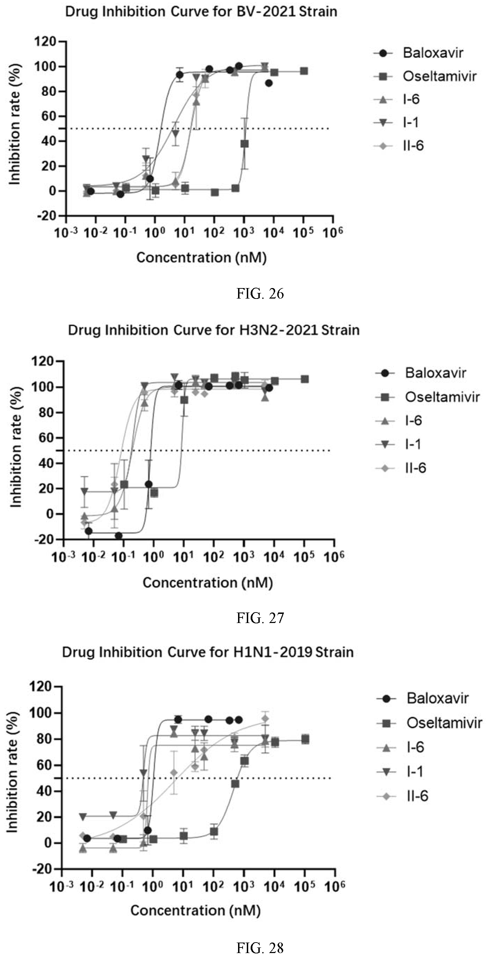

To verify the activity of inhibiting influenza virus at the cellular level, in MDCK cell (kindly provided by Xiamen University) models infected with nine influenza virus strains, including A/Victoria/2570/2009(H1N1) (kindly provided by Xiamen University), A/California/04/2009(H1N1) (kindly provided by Xiamen University), A/Vietnam/2004(H5N1), A/Shanghai/02/2013(H7N9) (kindly provided by Xiamen University), B/Phuket/3073/2013 (BY) (kindly provided by Xiamen University), A/Puerto Rico/8/1934(H1N1-PR8) (purchased from NIBSC, NIBSC16/108), A/Victoria/2570/2019(H1N1) (purchased from NIBSC, NIBSC21/346), A/Darwin/9/2021(H3N2) (purchased from NIBSC, NIBSC21/214), and B/Austria/1359417/2021(BV) (purchased from NIBSC, NIBSC21/224), the anti-viral activity was tested. Various concentrations of the multimeric small molecule derivatives or conjugates provided in the examples of the present application or small molecule drugs (zanamivir, peramivir, oseltamivir) were mixed with the viruses, and incubated at 37 °C for 1 h, the resulting mixture was subsequently added to monolayer MDCK cells with 80% to 90% confluent, after 90 minutes of culture, the cells were washed. Subsequently, the monolayer cells were covered with carboxymethyl cellulose (purchased from Innochem, A05925) to minimize the spread of the viruses and further cultured for two days. After the 2-day culture, the effective drug concentration capable of inhibiting 50% cytopathic effect (CPE) (EC₅₀) was recorded and the results were shown in Tables 1 and 2. The inhibition curves of the drugs against A/Puerto Rico/8/1934(H1N1-PR8), A/Victoria/2570/2019(H1N1), A/Darwin/9/2021(H3N2), and B/Austria/1359417/2021(BV) viruses were shown in FIG. 26 to FIG. 29.

**Table 1**

| Compound | Style | EC₅₀ (nM) | | | | |
|---|---|---|---|---|---|---|
| | | A/Victoria /2570/200 9 (H1N1) | A/Californi a/04/2009 (H1N1) | A/Vietnam/2004 (H5N1) | A/Shanghai/02 /2013 (H7N9) | B/Phuket/ 3073/2013 (BY) |
| Oseltamivir | SM | 125 | 29.62 | 266.67 | 100.02 | |
| Zanamivir | SM | NT | 77.77 | 266.67 | 55.57 | NT |
| Peramivir | SM | NT | 122.22 | 300 | 88.90 | NT |
| R10 | SM | 7.81 | 40.75 | 133.35 | 300 | 31.25 |
| R14 | SM | 1.95 | 40.75 | 133.35 | 366.67 | 7.81 |
| P8 | SM | NT | 40.75 | 233.35 | 6.17 | NT |
| P12 | SM | NT | 29.63 | 100.02 | 6.17 | NT |
| P11 | SM | 500 | 33.33 | 133.35 | 100.02 | 500 |
| II-1 | Conjugate | 7.81 | 6.17 | 88.90 | 166.67 | 31.25 |
| I-1 | Conjugate | 1.95 | 2.05 | 77.77 | 25.93 | 7.81 |
| II-6 | Conjugate | NT | 55.57 | 133.35 | 1.23 | NT |
| I-6 | Conjugate | NT | 1.64 | 44.45 | 1.05 | NT |
| I-5 | Conjugate | 31.25 | 3.29 | 111.10 | 88.90 | 125 |

**Table 2**

| Compound | EC₅₀ (nM) | | | |
|---|---|---|---|---|
| | A/Puerto Rico/8/1934( H1N1-PR8) | A/Victoria/257 0/2019(H1N1) | A/Darwin/9/2 021 (H3N2) | B/Austria/1359 417/2021(BV) |
| Baloxavir marboxil | 0.6105 | 1.0550 | 0.7720 | 1.5255 |
| Oseltamivir | 8588.0077 | 465.2717 | 9.0171 | 1140.8475 |
| I-6 | 0.6021 | 0.6249 | 0.1810 | 15.5709 |
| I-1 | 0.5212 | 0.4609 | 0.1947 | 4.2021 |
| II-6 | 0.0338 | 5.1506 | 0.0761 | 15.4117 |

### Example 13 Pharmacodynamic Analysis

In order to verify whether the conjugates provided in the examples of the present application have a longlasting protective effect, the pharmacokinetics of the conjugates in mice were analyzed. In each group, 4 healthy BALB/c mice (purchased from Vital River) were selected and injected intravenously with 10 mg/kg of I-1, 1-6 or an Fc protein (purchased from Sino Biological, customized, JS 95A). 20 µL blood samples were collected from the mice at designated time points for determination of drug concentrations. Blood samples were collected at different interval time points. An anticoagulant (saturated EDTA-Na2) was immediately added to the collected blood samples, and after the samples were centrifuged, the supernatant was retained for use. A 96-well plate was coated with NA proteins (purchased from Sino Biological, 11058-V08B-(MF14DE2511), I-1 and I-6 at different concentrations were incubated on the plate for 1 hour at room temperature. The concentrations of the drugs in the serum samples were quantified via sandwich ELISA using goat anti-human IgG (Fc) Antibody, Peroxidaselaboratory (SeraCare, CAT: 5220-0279). The results were shown in FIG. 5, indicating that the conjugates exhibit good plasma half-life and could maintain drug concentrations above the effective levels for an extended period.

### Example 14 Analysis on ADCC Effect

Fc has certain biological functions and can activate effector functions such as ADCC and ADCP in vivo to enhance antiviral activity. To verify whether the conjugates had this function, a virus-infected cell model was constructed, which was incubated first for 18 hours to 24 hours under the conditions at 37 °C with 5% CO₂. An ADCC reporter cell line stably expressing the FcγRIIIa receptor was then added, and the cells. Finally, drugs to be tested at different concentrations were added. After incubation for 15 minutes under the conditions at 37 °C with 5% CO₂, the intensity of the fluorescence signal emitted by the ADCC reporter cell line was detected, to determine the intensity of the ADCC effect induced by the conjugates. As shown in FIG. 6, the conjugates provided in the examples of the present application can maintain ADCC effect comparable to that of Fc.

### Example 15 Evaluation of Conjugates for Inhibiting Viral Replication in Mice

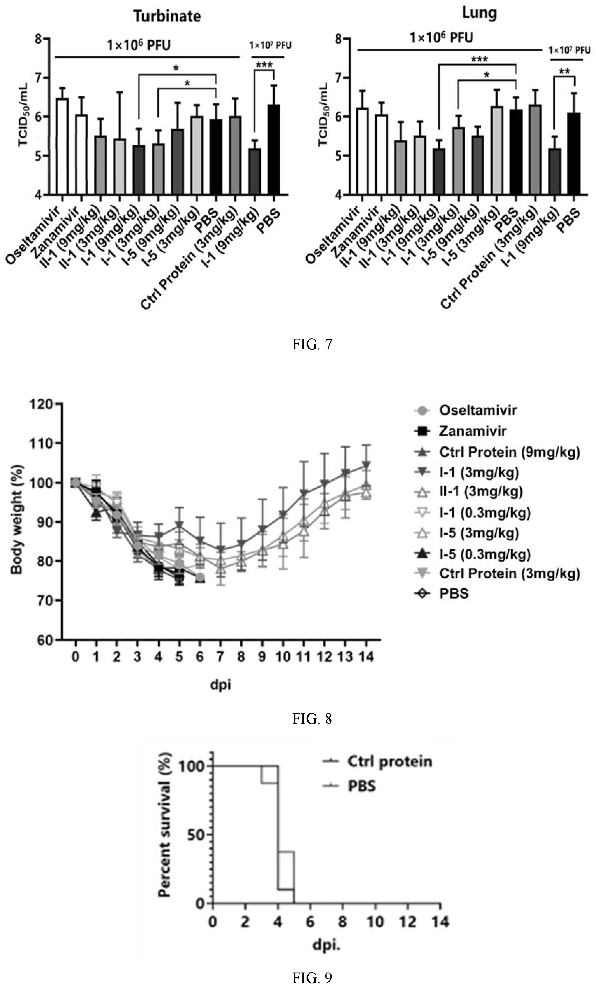

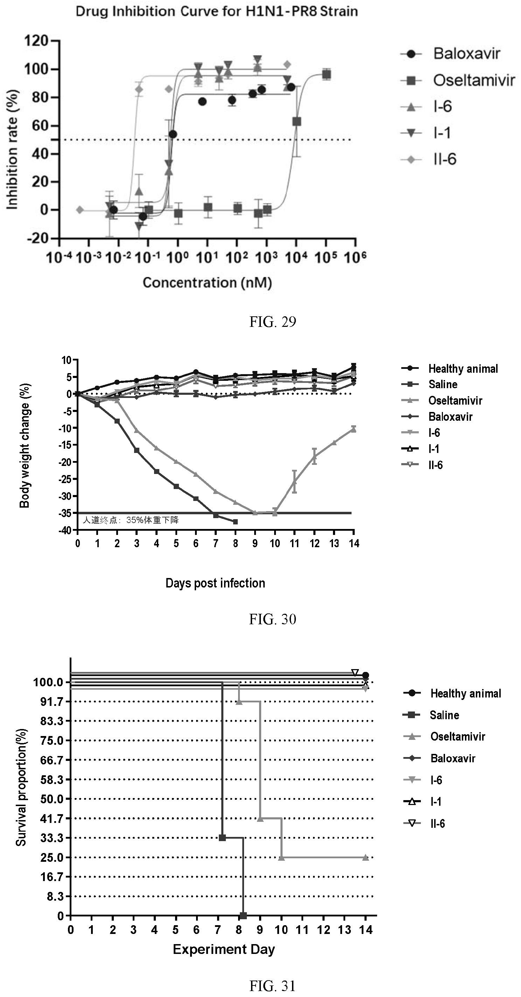

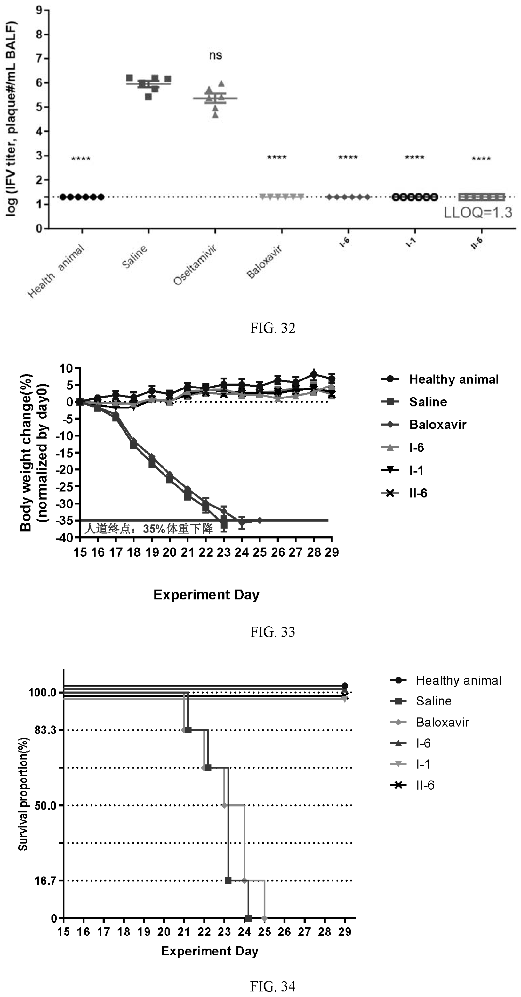

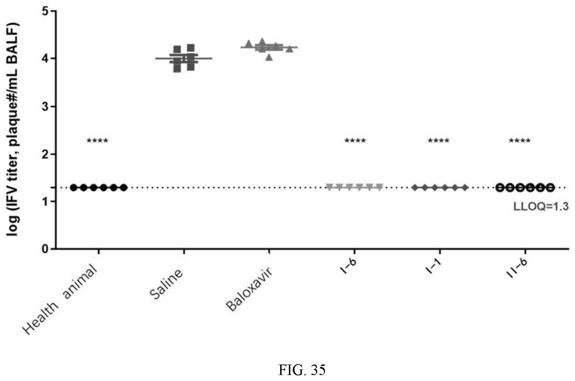

1. In order to study whether the conjugates provided in the examples of the present application could inhibit viral replications in the lung and nasal turbinate of the mice, 6 week-aged BALB/c mice (purchased from Vital River) were intranasally challenged with about 1×10⁶ PFU or about 1×10⁷ PFU of H1N1 influenza virus (A/California/07/2009, kindly provided by Xiamen University). Administration was performed 2 hours post-challenge. Mice were intravenously injected with the conjugate in a single dose (3, 9 mg/kg), PBS or negative control Fc protein (purchased from Sino Biological, customized, JS95A, 3 mg/kg). The positive control zanamivir (5 mg/kg) was intravenously injected 2 hours post-challenge once daily for 3 consecutive days. Oseltamivir phosphate (20 mg/kg) was administered orally 2 hours post-challenge twice daily for 3 consecutive days. All the mice were euthanized on day 4 post-challenge, and the viral loads in the lung and nasal turbinate were quantified. The results were shown in FIG. 7, indicating that the conjugates provided in the examples of the present application could effectively inhibit viral replication at different virus challenge doses.
2. BALB/c mice were challenged with influenza virus (A/Puerto Rico/8/1934(H1N1-PR8)) at a dose of 10×LD₅₀. Administration was performed 2 hours post-challenge. Mice were injected intravenously with the conjugate at a single dose (3 mg/kg), or were administered orally with positive controls oseltamivir (10 mg/kg) or baloxavir marboxil (5 mg/kg). On day 6 post-administration, the viral content in bronchoalveolar lavage fluid was detected, and the body weight and the survival rate were monitored daily. The results were shown in FIG. 30 to FIG. 32, indicating that the therapeutic administration of the conjugates provided in the examples of the present application, such as I-6, I-1, and II-6, could effectively inhibit viral replication with low toxic side effects, the body weight of the mice can increase normally, and the survival rate of the mice was high.
3. BALB/c mice were intravenously injected with the conjugate at a single dose (3 mg/kg), or were administered orally with positive controls oseltamivir (10 mg/kg) or baloxavir marboxil (5 mg/kg). On day 15 post administration, mice were challenged with influenza virus (A/Puerto Rico/8/1934(H1N1-PR8)) at a dose of 10×LD₅₀, the viral content in bronchoalveolar lavage fluid was detected on day 6 post-challenge, and the body weight and the survival rate were monitored daily. The results were shown in FIG. 33 to FIG. 35, indicating that the prophylactic administration of the conjugates provided in the examples of the present application, such as I-6, I-1, and II-6, could effectively inhibit viral replication with low toxic side effects, and the body weight of the mice could increase normally, and the survival rate of the mice was high.

### Example 16 Evaluation on In Vivo Anti-Influenza Virus Activity of Conjugates

In order to adequately compare the antiviral activity of the conjugates provided in the examples of the present application, an influenza-susceptible DBA/2 mouse model (purchased from Vital River) was selected. As compared with the BALB/c mouse model, this model can develop severe influenza symptoms at a low virus challenge dose. 6 Week-aged female DBA/2 mice (10 per group) were selected and intranasally challenged with influenza virus (A/California/07/2009) at a dose of 1×10⁴ PFU. Mice were administrated by a single tail vein injection of the conjugates (I-1, I-5) at doses of 0.3 and 3 mg/kg, PBS or negative control Fc protein (3 mg/kg, 9 mg/kg, purchased from Sino Biological, customized, JS95A) 2 hours post-challenge. Mice were intravenously injected with the positive control zanamivir (5 mg/kg) 2 hours post-challenge once daily for 5 consecutive days, or were orally administered with oseltamivir phosphate (20 mg/kg) 2 hours after challenge twice daily for 5 consecutive days. The body weight and the survival rate (mice with a body weight loss of 25% were considered dead) were monitored daily for 14 days. The results were shown in FIG. 9 to FIG. 12, and in the DBA/2 mouse model, as shown in FIG. 8, the body weight reached the lowest point on day 7 post-challenge, where I-1 (3 mg/kg) could significantly alleviate the weight loss of the mice. All mice in both the PBS group and the Fc protein control group died on day 5 (FIG. 9), and all the mice receiving positive control drugs oseltamivir phosphate and zanamivir died despite high-dose and multiple-dosing regimens (FIG. 10). In contrast, singledose administration of I-1 and I-5 at doses as low as 3 mg/kg achieved good protection rate (FIG. 11), and the I-5 achieved a protection rate as high as 90%, these above results reflected the remarkable antiviral activity of the conjugates in a single dose.

## Claims

1. A conjugate represented by Formula (I) or a pharmaceutically acceptable salt, a geometric or optical isomer, a hydrate, a solvate or a polymorph thereof, 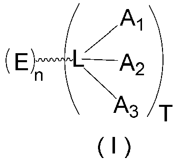
wherein A₁, A₂ and A₃ each independently are selected from the group consisting of Formula (A-I) to Formula (A-XIV), 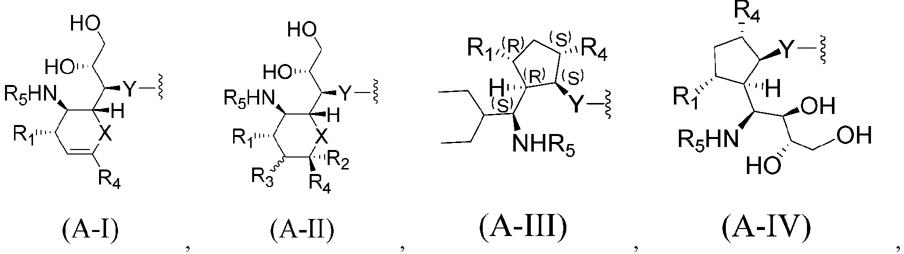 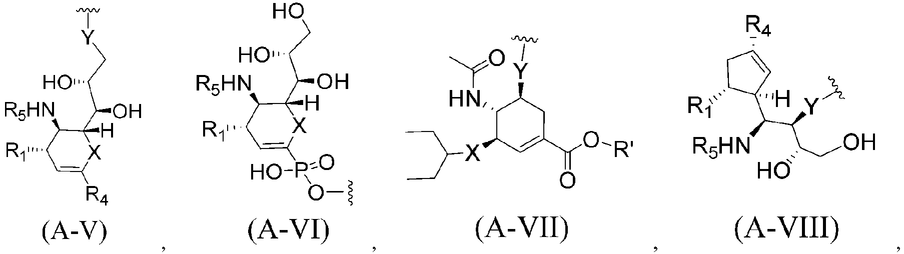 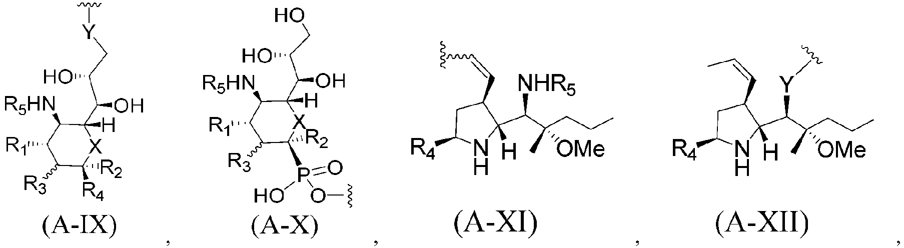 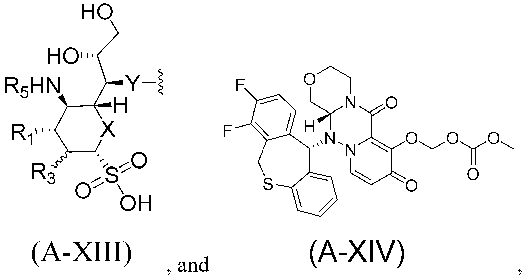
wherein, L may be linked to any possible sites of the compound of Formula (A-XIV);
wherein R₁ is selected from the group consisting of -OH, -NH₂, -NHC(=NH)NH₂ and -NHC(=NH)NHR₆;
R₂ and R₃ each independently are selected from the group consisting of -H, -OH, -F, -Cl and -Br;
R₄ is selected from the group consisting of -C(=O)OH, -P(=O)(OH)₂, -SO₃H, -C(=O)OCH₃ and -C(=O)OCF₃;
R₅ is selected from the group consisting of -C(=O)CH₃, -C(=O)CF₃, and -SO₂CH₃;
X is selected from the group consisting of -O- and -S-;
Y is selected from the group consisting of -O-, -S-, -NR₇-, -OC(=O)NR₇-, -OC(=S)NR₇-, -OC(=O)O-, -OC(=O)-, -NHC(=O)O-, -NHC(=O)-, -NHC(=NH)-, -NHC(=O)NR₇-, -NHC(=NH)NR₇-, -NHC(=S)NR₇-, -NHC(=S)-, -OCH₂C(=O)NR₇-, -NH(SO₂)- and -NH(SO₂)NR₇-;
R' is H or C₁-C₆ alkyl;
R₆ is selected from the group consisting of 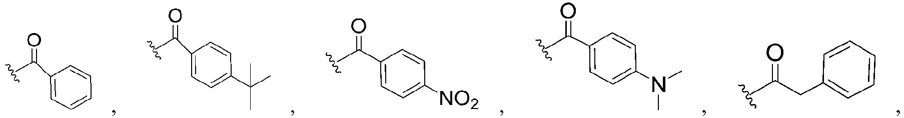 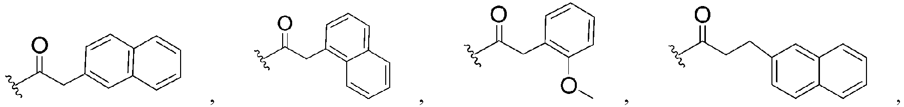 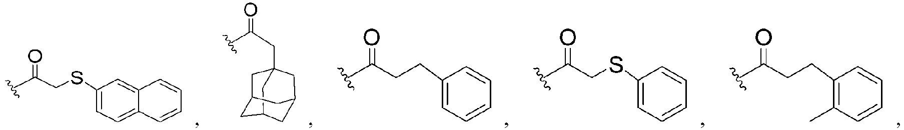 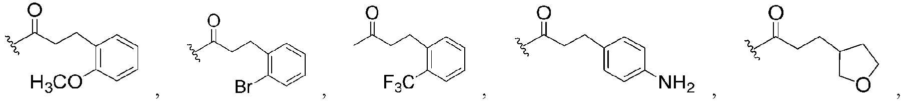 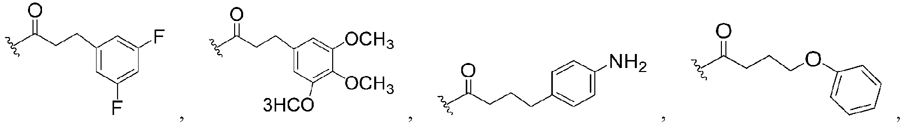 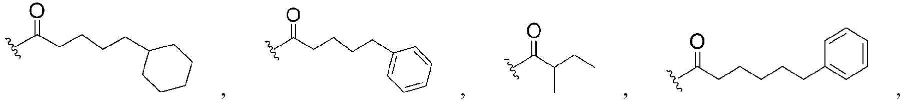 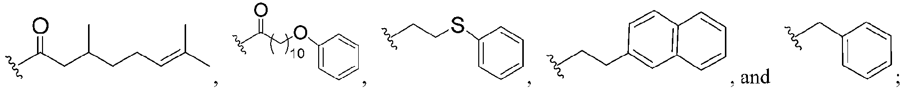
R₇ is selected from the group consisting of H, C₁-C₂₀ alkyl, C₃-C₂₀ cycloalkyl, C₃-C₂₀ heterocycloalkyl, C₅-C₁₅ aryl and C₅-C₁₅ heteroaryl; preferably, R₇ is H; preferably, R₇ is C₁-C₆ alkyl;
each E is a biomacromolecule; n is 1 or 2; T is a number between 1 and 20;
the wavy line linked to each E represents a covalent linking (e.g., via a covalent bond or a linker) of each 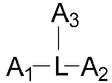 to an N atom in the each E (e.g., an N atom on the side chain amino group of a lysine, arginine, asparagine, or glutamine residue), and L is a linker covalently linked to each of groups E, A1, A2 and A3.

2. The conjugate or a pharmaceutically acceptable salt, a geometric or optical isomer, a hydrate, a solvate or a polymorph thereof according to claim 1, wherein A₁, A₂ and A₃ each independently are selected from the group consisting of Formula (A-I) to Formula (A-XIII), 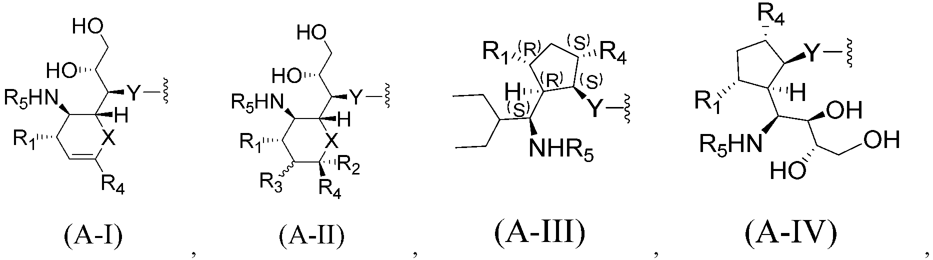 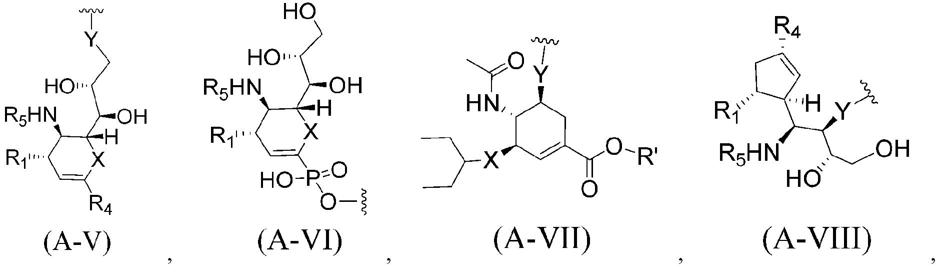 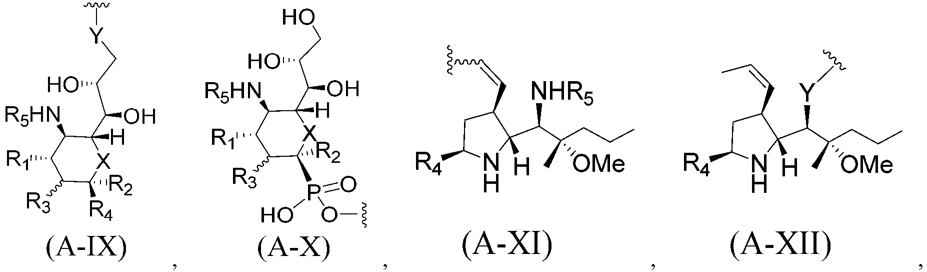 and 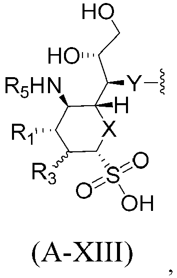
preferably, A₁, A₂ and A₃ each independently are selected from the group consisting of Formula (A-I) to Formula (A-IV) and Formula (A-VII), 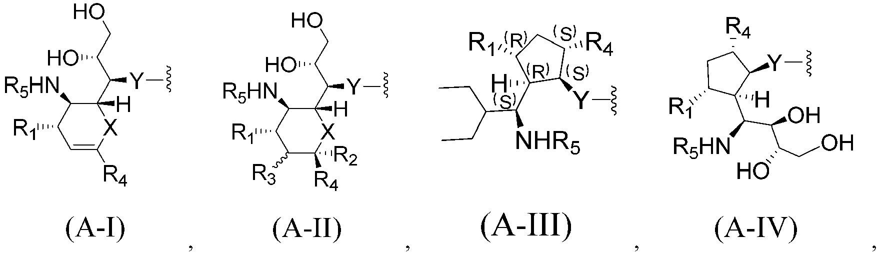 and 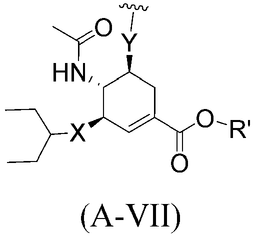
wherein R₁, R₂, R₃, R₄, R₅, R', X, and Y are as defined in claim 1;
each E comprises an Fc domain monomer (e.g., an Fc domain monomer having a sequence as set forth in any one of SEQ ID NO:1-68), albumin (e.g., albumin having a sequence as set forth in any one of SEQ ID NO:69-71), and an albumin-binding peptide, or an Fc-binding peptide; n is 1 or 2, wherein when n is 2 and E is the Fc domain monomer, the two E dimerize to form an Fc domain;
L is a linker covalently linked to each of groups E, A₁, A₂ and A₃;
preferably, A₁, A₂ and A₃ each independently are selected from the group consisting of Formula (A-I) to Formula (A-III) and Formula (A-VII), 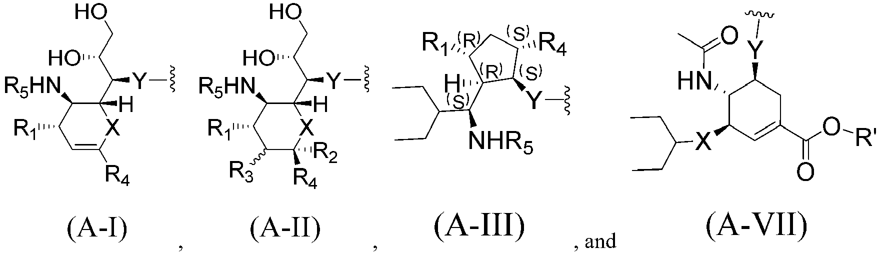
wherein R₁, R₂, R₃, R₄, R₅, R', X, and Y are as above defined;
preferably, A₁, A₂ and A₃ each independently are selected from the group consisting of (A-I), Formula (A-III) and Formula (A-VII), 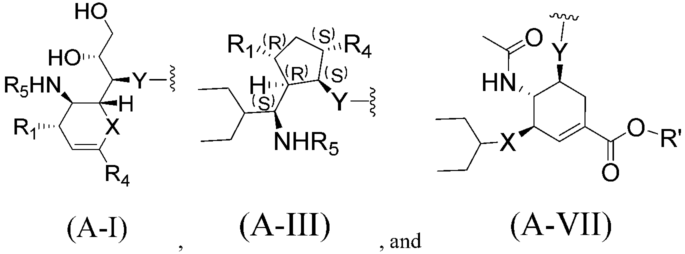
wherein R₁, R₄, R₅, R', X, and Y are as above defined.

3. The conjugate or a pharmaceutically acceptable salt, a geometric or optical isomer, a hydrate, a solvate or a polymorph thereof according to claim 1 or 2, wherein E comprises an Fc domain monomer (e.g., an Fc domain monomer having a sequence as set forth in any one of SEQ ID NO: 1-68); n is 1 or 2, wherein when n is 2, the two E dimerize to form an Fc domain; L is a linker covalently linked to each of groups E, A₁, A₂ and A₃;
preferably, the Fc domain monomer has an amino acid sequence selected from the group consisting of:
i) any one of the sequences as set forth in SEQ ID NO:1-68;
ii) a sequence with substitution, deletion or addition of one or several amino acids (e.g., substitution, deletion or addition of one, two, three, four or five amino acids) as compared with any one of the sequences as set forth in SEQ ID NO: 1-68; and
iii) a sequence with a sequence identity of at least 70% (such as 70%, 75%, 80%, 85%, 90%, 95%, 96%, 97%, 98%, 99% or 100%) as compared with any one of the sequences as set forth in SEQ ID NO: 1-68;
preferably, the substitution as described in (ii) is a conservative substitution;
preferably, E is an Fc domain monomer having a sequence with a sequence identity of at least 70% (such as 70%, 75%, 80%, 85%, 90%, 95%, 96%, 97%, 98%, 99% or 100%) as compared with the sequences as set forth in SEQ ID NO:64;
preferably, E is an Fc domain monomer having a sequence as set forth in SEQ ID NO:64;
preferably, R₁ is selected from the group consisting of -OH, -NH₂, and -NHC(=NH)NH₂; further preferably, R₁ is -NHC(=NH)NH₂;
preferably, R₄ is selected from the group consisting of -C(=O)OH, -C(=O)OCH₃ and -C(=O)OCF₃; further preferably, R₄ is selected from the group consisting of -C(=O)OH, and -C(=O)OCH₃;
preferably, R₅ is selected from the group consisting of -C(=O)CH₃ and -C(=O)CF₃; further preferably, R₅ is -C(=O)CH₃;
preferably, R' is H or C₁-C₄ alkyl; further preferably, R' is H, methyl or ethyl; further preferably, R' is H or ethyl;
preferably, X is -O-;
preferably, Y is selected from the group consisting of -O-, -S-, and -NH-; further preferably, Y is selected from the group consisting of -O- and -NH-.

4. The conjugate or a pharmaceutically acceptable salt, a geometric or optical isomer, a hydrate, a solvate or a polymorph thereof according to any one of claims 1 to 3, wherein the conjugate has a structure represented by Formula (I-II-A), Formula (I-II-B) or Formula (I-II-C), 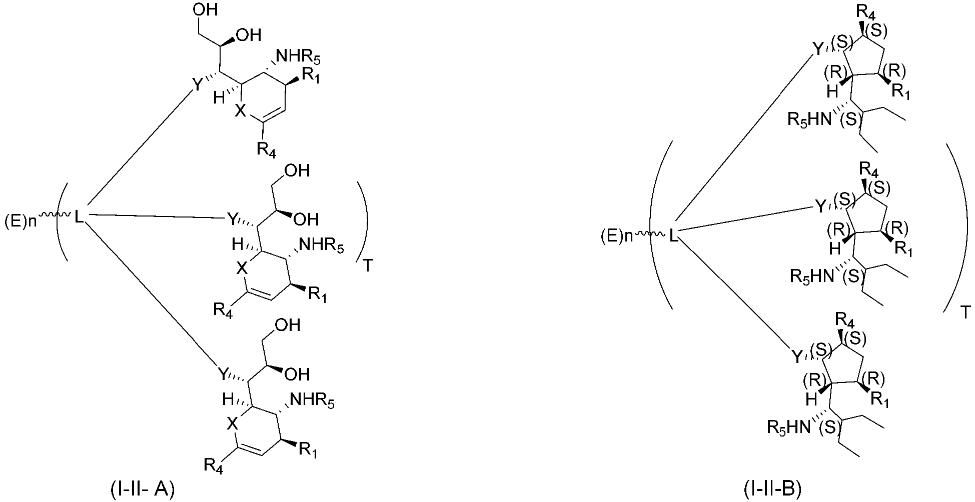 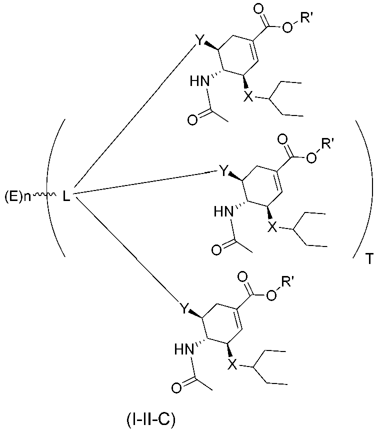 wherein R₁, R₄, R₅, R', X, Y, E, n, L, and T are as defined in any one of claims 1 to 3.

5. The conjugate or a pharmaceutically acceptable salt, a geometric or optical isomer, a hydrate, a solvate or a polymorph thereof according to any one of claims 1 to 4, wherein the conjugate has a structure represented by Formula (I-II-A'), Formula (I-II-B') or Formula (I-II-C'), 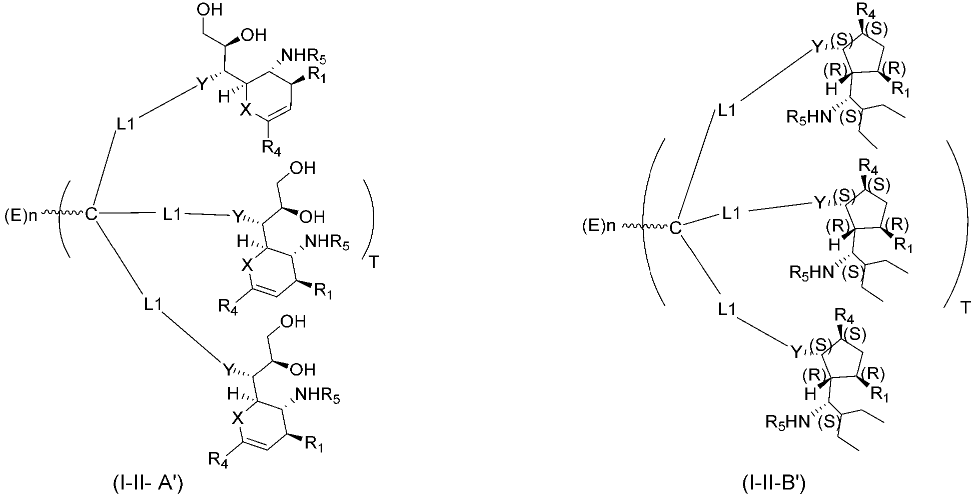 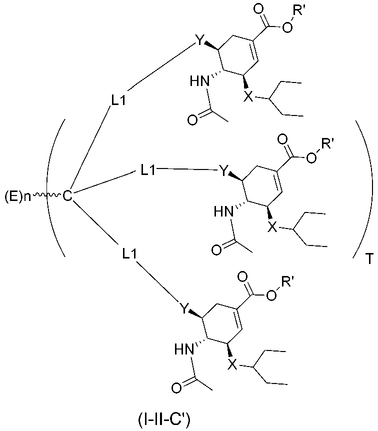
wherein R₁, R₄, R₅, R', X, Y, E, n, and T are as defined in any one of claims 1 to 4;
Li is selected from the group consisting of: 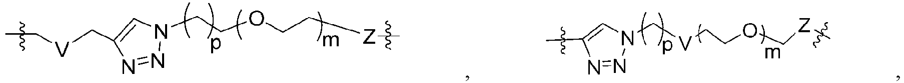 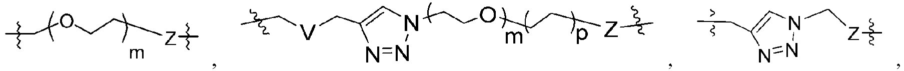 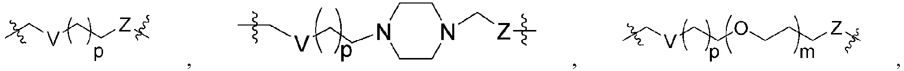 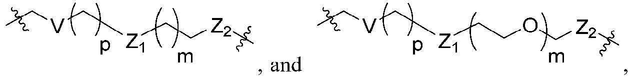 wherein V is -CH₂-, -O- or -S-; Z is - C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-; Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, and - C(=O)- or -CH₂-; m and p each independently are an integer between 1 and 20;
preferably, L₁ is selected from the group consisting of: 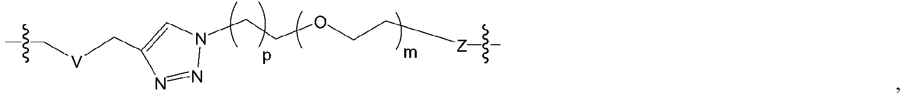 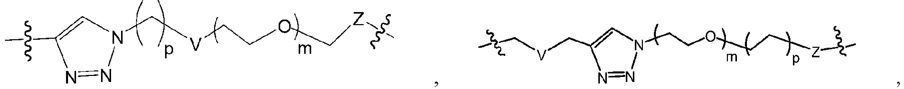 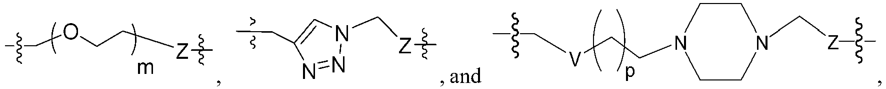 wherein V, p, m, and Z are as above defined;
preferably, L₁ is 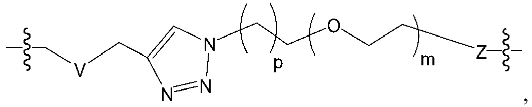 wherein V, p, m, and Z are as above defined;
preferably, V is -CH₂- or -O-; further preferably, V is -O-;
preferably, Z is -C(=O)-NH-, -CH₂-C(=O)-NH-, -NH-C(=O)-, -CH₂CH₂-C(=O)-, -N(CH₃)-C(=O)-, - N(CH₂CH₃)-C(=O)- or -C(=O)-; further preferably, Z is -NH-C(=O)- or -C(=O)-;
preferably, p is an integer between 1 and 10; further preferably, p is an integer between 1 and 6 (such as 1, 2, 3, 4, 5, 6);
preferably, m is an integer between 1 and 12; further preferably, m is an integer between 1 and 10 (such as 1, 2, 3, 4, 5, 6, 7, 8, 9).

6. The conjugate or a pharmaceutically acceptable salt, a geometric or optical isomer, a hydrate, a solvate or a polymorph thereof according to any one of claims 1 to 5, wherein the conjugate has a structure represented by Formula (I-II-A'-1), Formula (I-II-B'-1), Formula (I-II-B'-2) or Formula (I-II-C'-1); 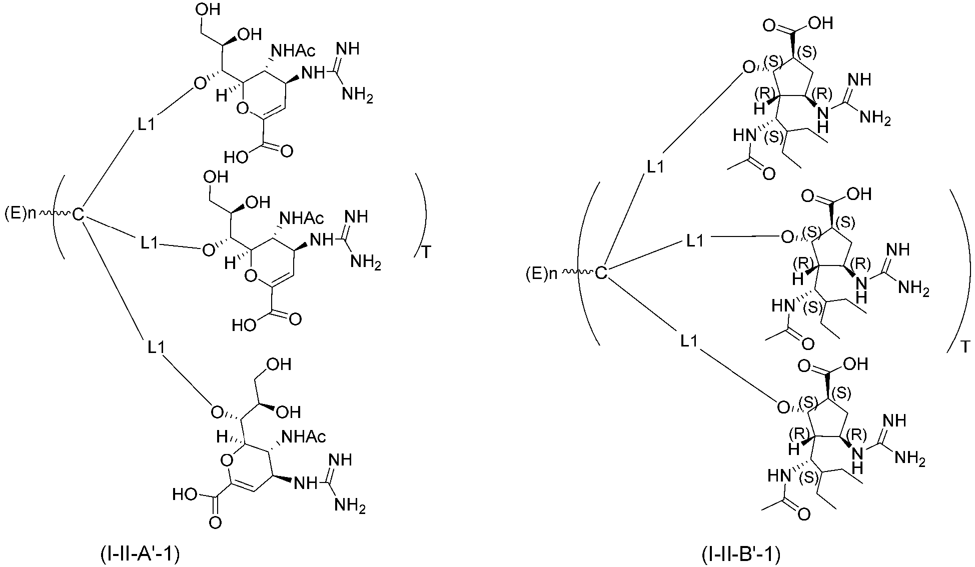 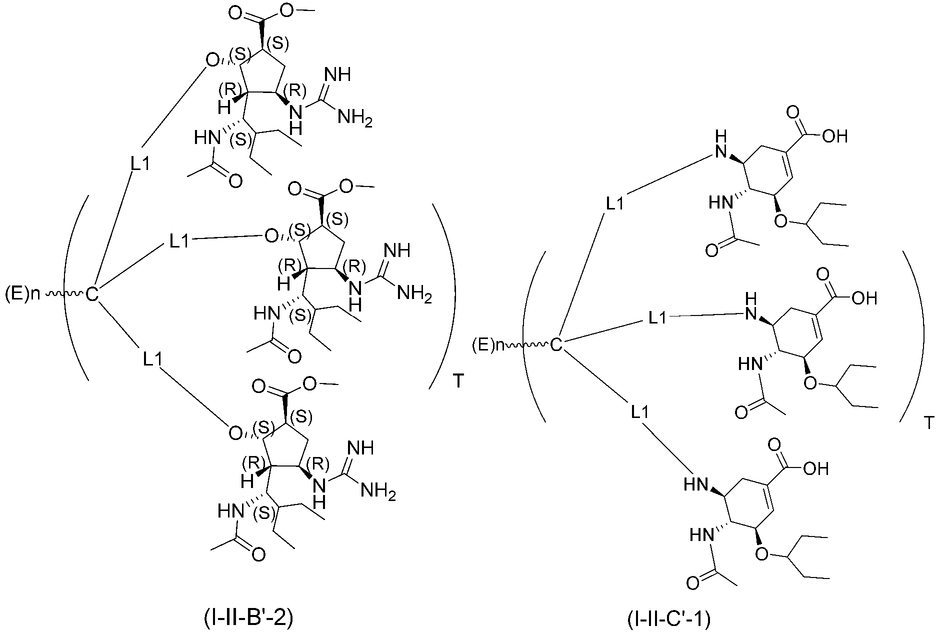 wherein E, n, and T are as defined in any one of claims 1 to 4, and L₁ is as defined in claim 5.

7. The conjugate or a pharmaceutically acceptable salt, a geometric or optical isomer, a hydrate, a solvate or a polymorph thereof according to any one of claims 1 to 6, wherein the conjugate has a structure represented by Formula (I-II-A-2), Formula (I-II-B-3), Formula (I-II-B-4) or Formula (I-II-C-2), 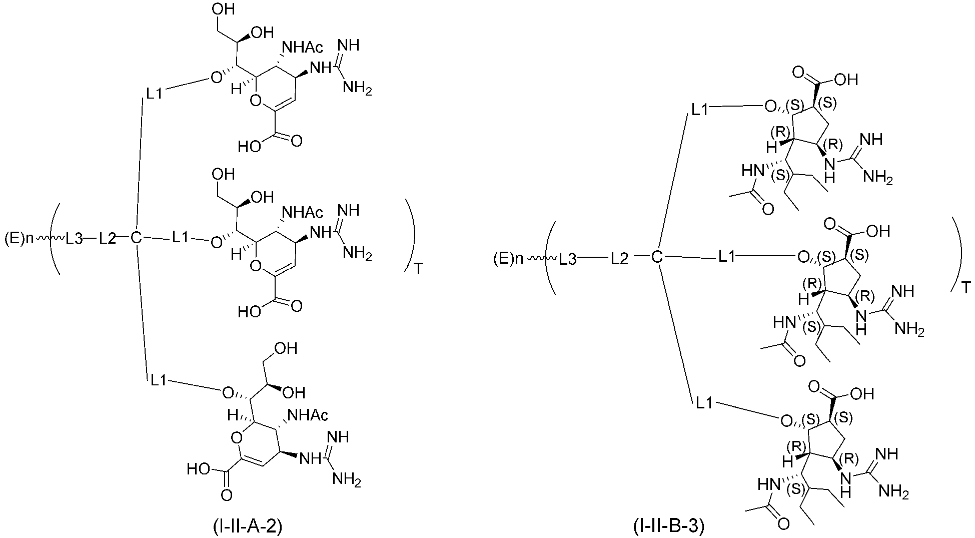 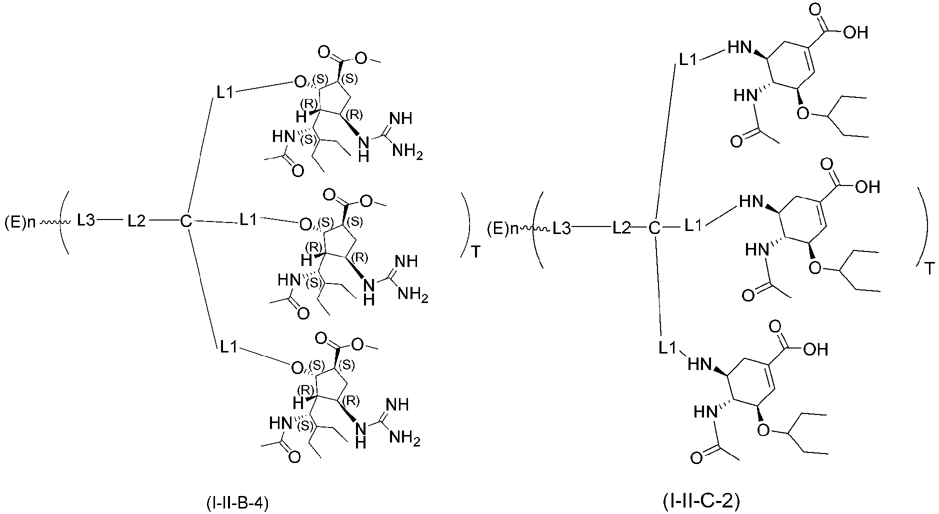
wherein E, n, and T are as defined in any one of claims 1 to 4, and L₁ is as defined in claim 5;
L₂ is selected from the group consisting of: 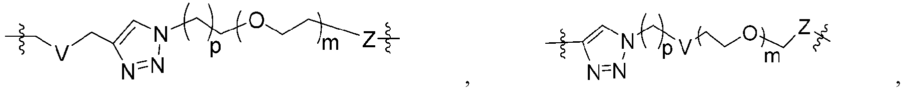 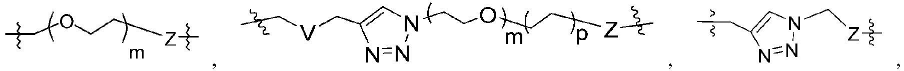 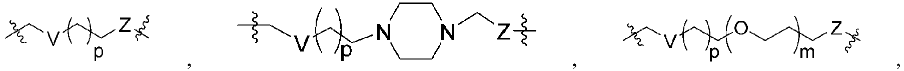 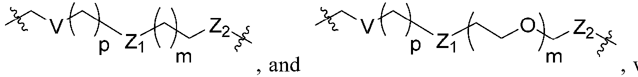 wherein V is -CH₂-, -O- or -S-; Z is - C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-; Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, - C(=O)- or -CH₂-; m and p each independently are an integer between 1 and 20;
preferably, L₂ is selected from the group consisting of: 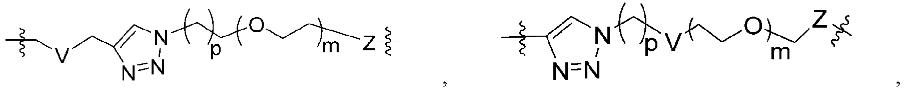 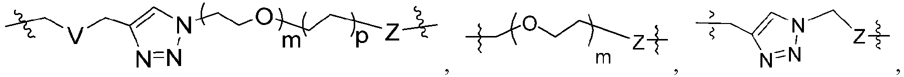 and 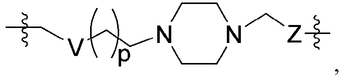 wherein V is -CH₂-, -O- or -S-; Z is -C(=O)-NH-, -CH₂-C(=O)-NH-, - NH-C(=O)-, -CH₂CH₂-C(=O)-, -N(CH₃)-C(=O)-, -N(CH₂CH₃)-C(=O)-, -O-C(=O)-, -C(=O)- or -CH₂-; m and p each independently are an integer between 1 and 20;
L₃ is 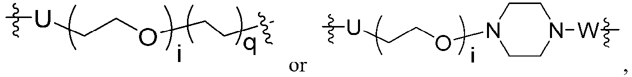 wherein U is -C(=O)-NH-, - NH-C(=O)-, -N(CH₃)-C(=O)-, -N(CH₂CH₃)-C(=O)-, -O-C(=O)-, -C(=O)-, or -CH₂-; W is -CH₂-, -O- or -S-; i and q each independently are an integer between 1 and 20, one end of L₃ is covalently linked to E, and the other end is covalently linked to L₂;
preferably, L₁ is 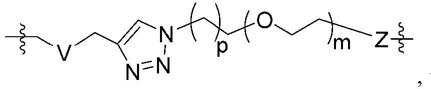 wherein V, p, m, and Z are as above defined; preferably, V is -CH₂- or -O-; further preferably, V is -O-; preferably, Z is -C(=O)-NH-, -CH₂-C(=O)-NH-, -NH-C(=O)-, -CH₂CH₂-C(=O)-, -N(CH₃)-C(=O)-, -N(CH₂CH₃)-C(=O)- or -C(=O)-; further preferably, Z is -NH-C(=O)- or -C(=O)-; preferably, p is an integer between 1 and 10; further preferably, p is an integer between 1 and 6 (such as 1, 2, 3, 4, 5, 6); preferably, m is an integer between 1 and 12; further preferably, m is an integer between 1 and 10 (such as 1, 2, 3, 4, 5, 6, 7, 8, 9);
preferably, L₂ is 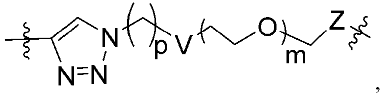 wherein V, p, m, and Z are as above defined; preferably, V is -CH₂- or -O-; further preferably, V is -O-; preferably, Z is -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-; further preferably, Z is -C(=O)-NH-; preferably, p is an integer between 1 and 10; further preferably, p is an integer between 1 and 6 (such as1, 2, 3, 4, 5, 6); preferably, m is an integer between 1 and 12; further preferably, m is an integer between 1 and 10 (such as 1, 2, 3, 4, 5, 6, 7, 8, 9);
preferably, L₃ is 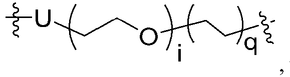 wherein U, i, and q are as above defined; preferably, U is -NH-C(=O)-, -N(CH₃)-C(=O)- or -N(CH₂CH₃)-C(=O)-; further preferably, U is -NH-C(=O)-; preferably, i is an integer between 1 and 12; further preferably, i is an integer between 1 and 10 (such as 1, 2, 3, 4, 5, 6, 7, 8, 9); preferably, q is an integer between 1 and 10; further preferably, q is an integer between 1 and 6 (such as 1, 2, 3, 4, 5, 6);
preferably, T is a number between 1 and 10; further preferably, T is a number between 1 and 9; further preferably, T is a number between 1 and 7; further preferably, T is a number between 1 and 5, further preferably, T is a number between 3 and 7, further preferably, T is a number between 3 and 5.5.

8. The conjugate or a pharmaceutically acceptable salt, a geometric or optical isomer, a hydrate, a solvate or a polymorph thereof according to any one of claims 1 to 7, wherein the conjugate has a structure represented by Formula (I-II-A-3), Formula (I-II-B-5), Formula (I-II-B-6) or Formula (I-II-C-3), 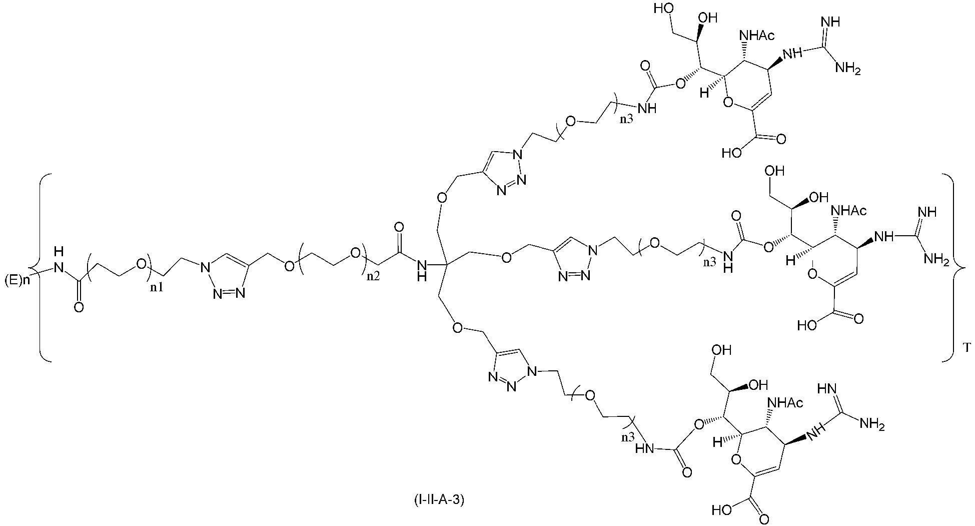 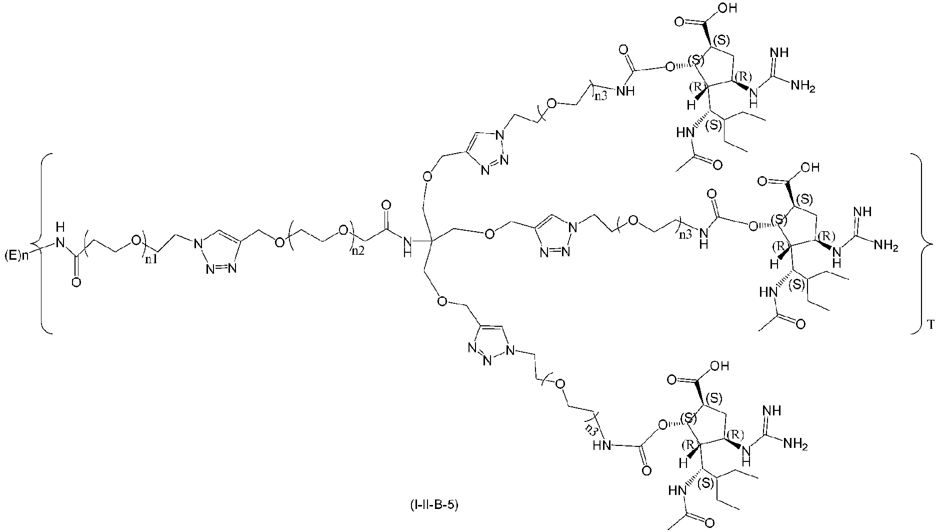 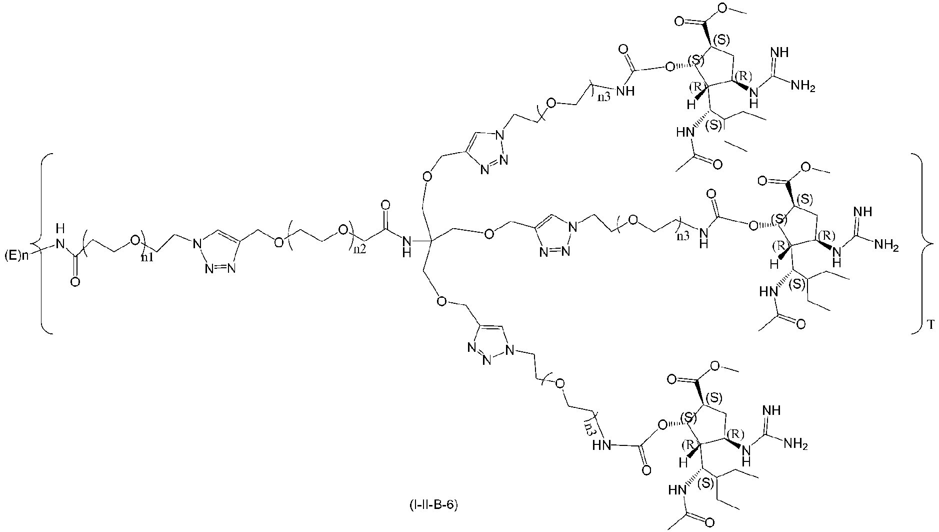 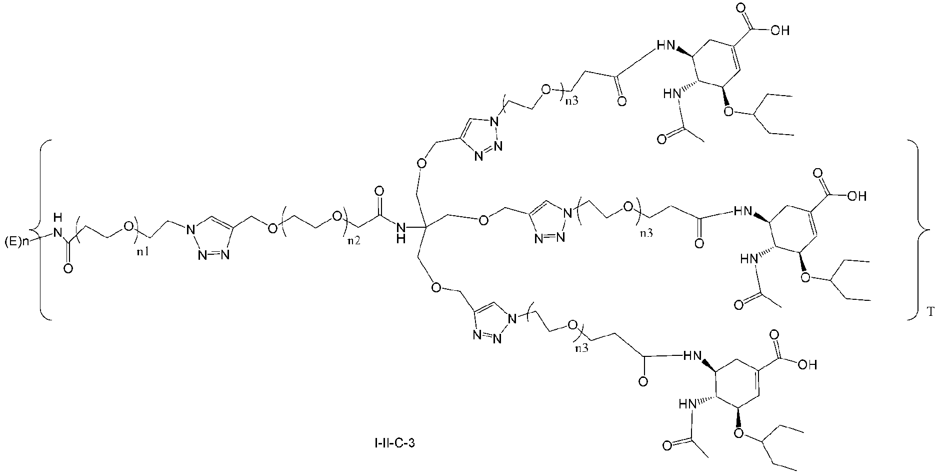
wherein E, n, and T are as defined in any one of claims 1 to 7; n1, n2, and n3 each independently are an integer between 1 and 20;
preferably, n1, n2, and n3 each independently are an integer between 1 and 12; further preferably, n1, n2, and n3 each independently are an integer between 1 and 10 (such as 1, 2, 3, 4, 5, 6, 7, 8, 9).

9. The conjugate or a pharmaceutically acceptable salt, a geometric or optical isomer, a hydrate, a solvate or a polymorph thereof according to any one of claims 1 to 8, wherein the conjugate is selected from the group consisting of: 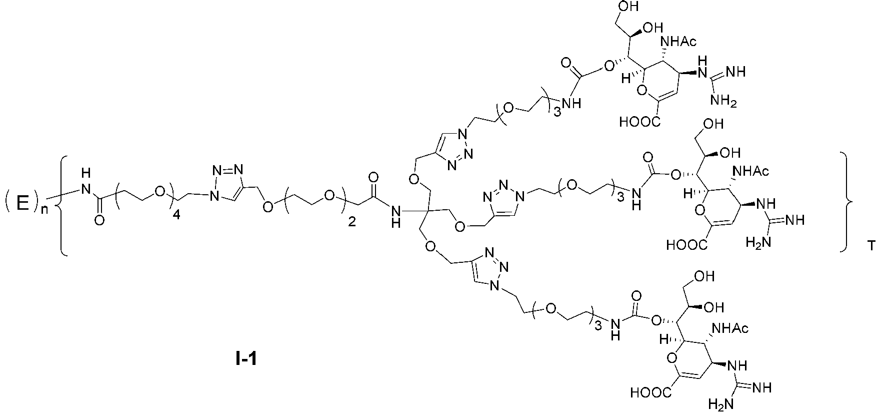 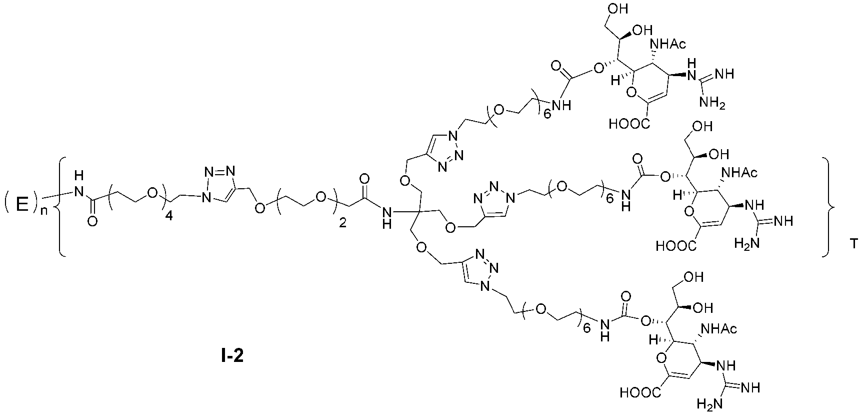 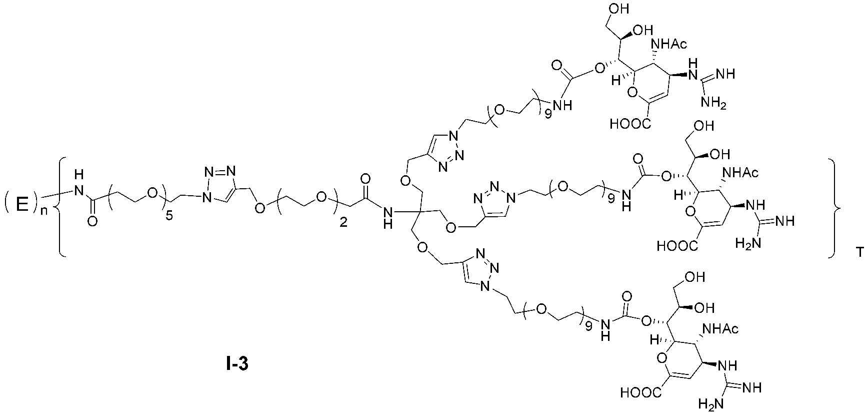 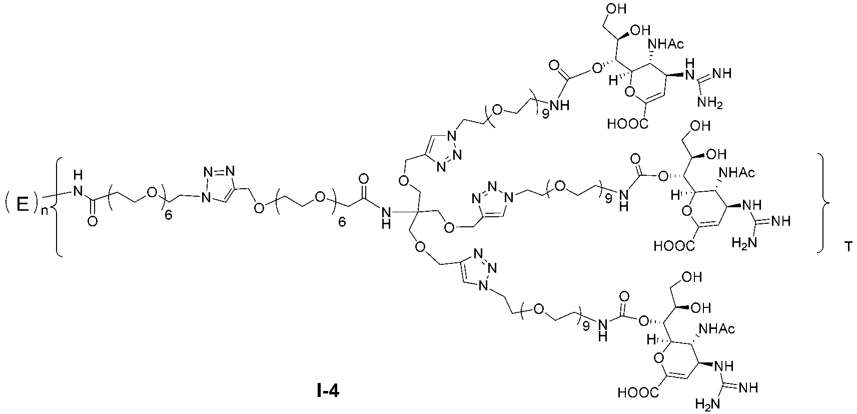 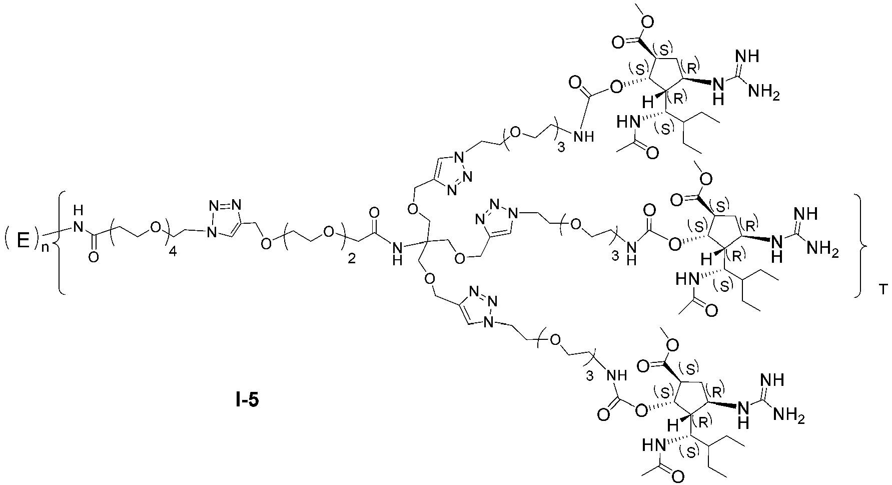 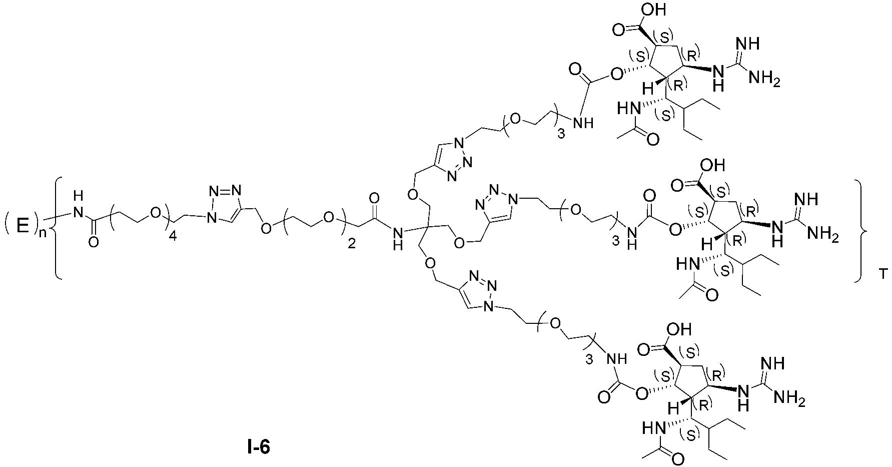 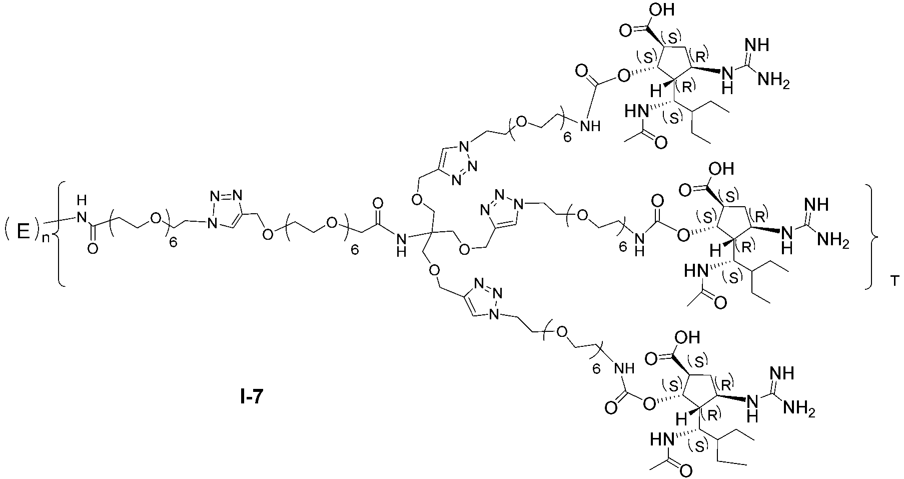 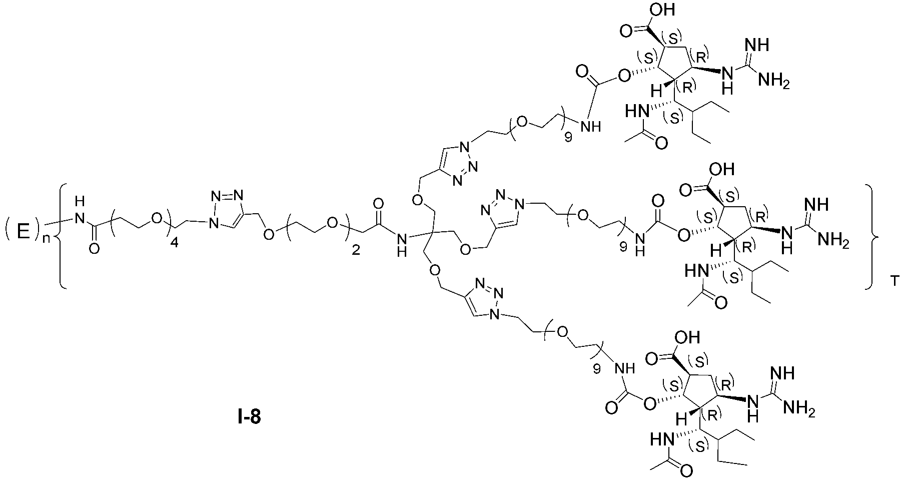 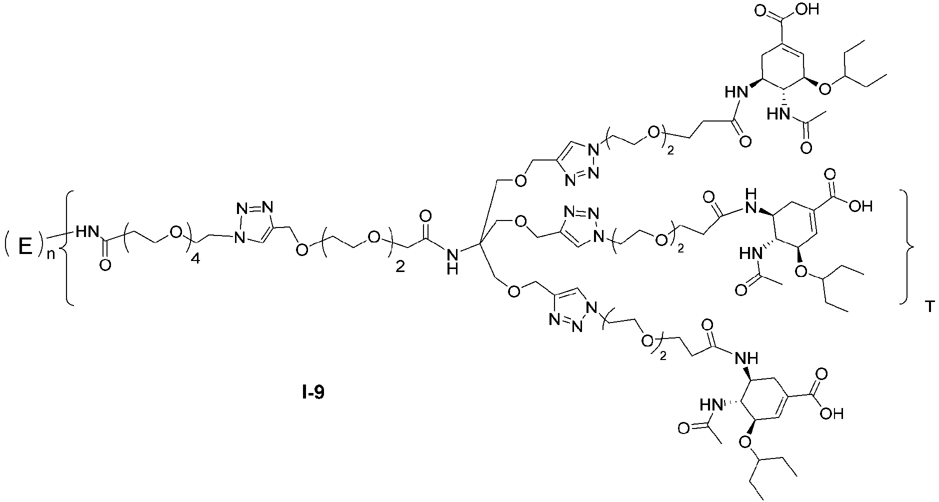 and 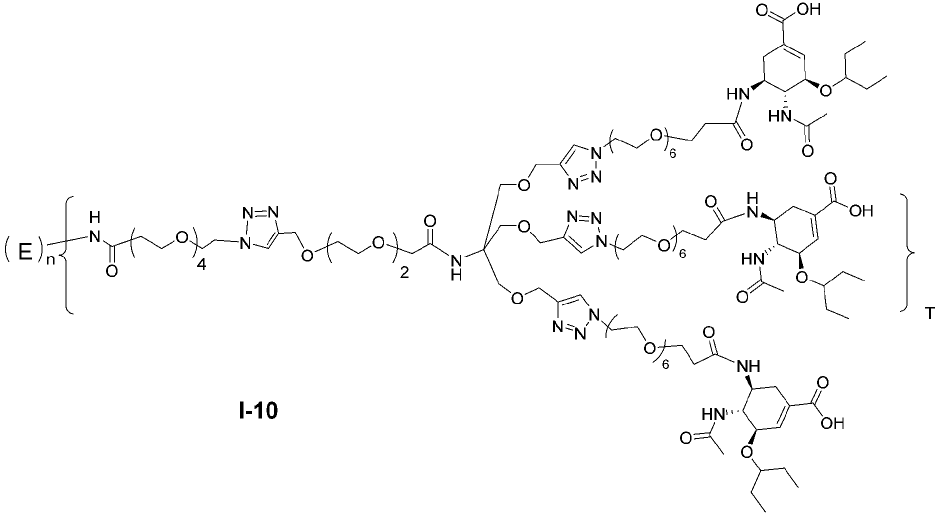 wherein E, n, and T are as defined in any one of claims 1 to 8.

10. A conjugate represented by Formula (II) or a pharmaceutically acceptable salt, a geometric or optical isomer, a hydrate, a solvate or a polymorph thereof, 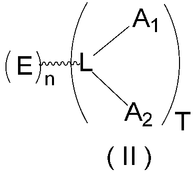
wherein A₁ and A₂ each independently are selected from the group consisting of Formula (A-I) to Formula (A-XIV), 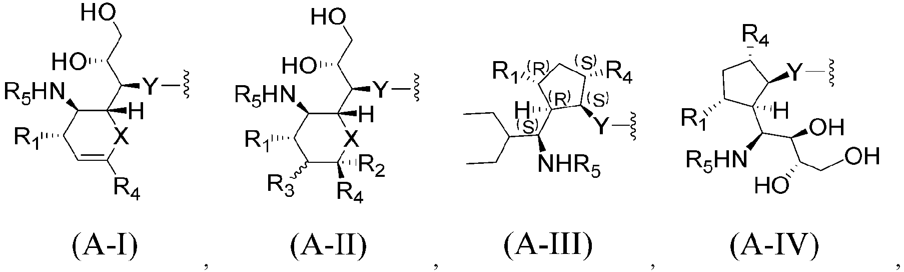 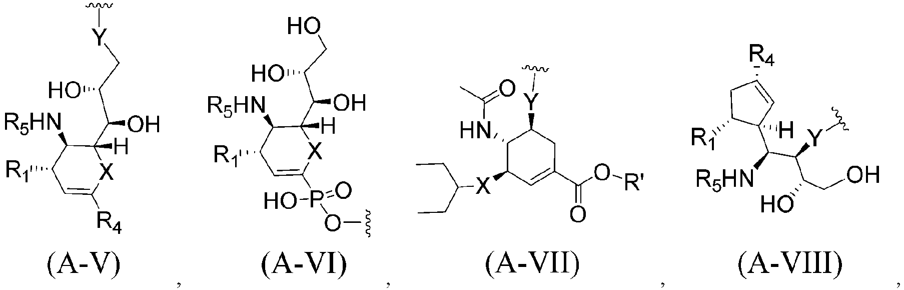 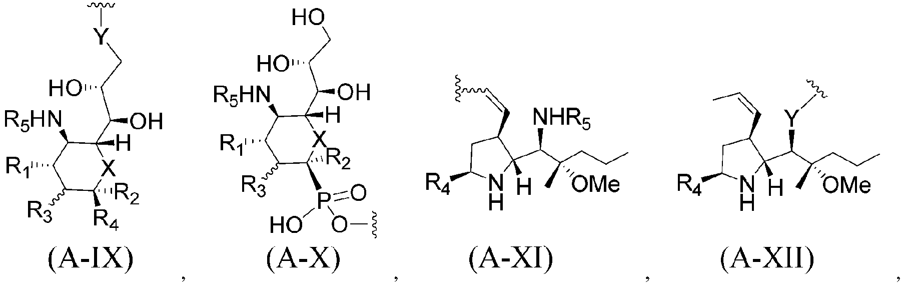 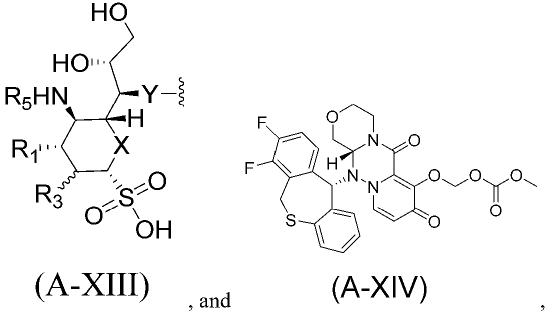
wherein, L may be linked to any possible sites of the compound of Formula (A-XIV);
wherein R₁ is selected from the group consisting of -OH, -NH₂, -NHC(=NH)NH₂ and -NHC(=NH)NHR₆;
R₂ and R₃ each independently are selected from the group consisting of -H, -OH, -F, -Cl and -Br;
R₄ is selected from the group consisting of -C(=O)OH, -P(=O)(OH)₂, -SO₃H, -C(=O)OCH₃ and -C(=O)OCF₃;
R₅ is selected from the group consisting of -C(=O)CH₃, -C(=O)CF₃, and -SO₂CH₃;
X is selected from the group consisting of -O- and -S-;
Y is selected from the group consisting of -O-, -S-, -NR₇-, -OC(=O)NR₇-, -OC(=S)NR₇-, -OC(=O)O-, -OC(=O)-, -NHC(=O)O-, -NHC(=O)-, -NHC(=NH)-, -NHC(=O)NR₇-, -NHC(=NH)NR₇-, -NHC(=S)NR₇-, -NHC(=S)-, -OCH₂C(=O)NR₇-, -NH(SO₂)- and -NH(SO₂)NR₇-;
R' is H or C₁-C₆ alkyl;
R₆ is selected from the group consisting of 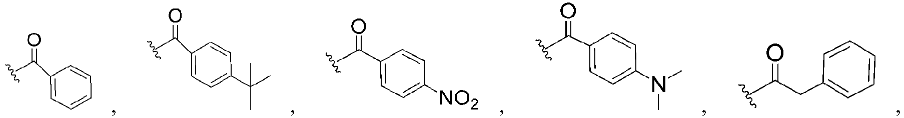 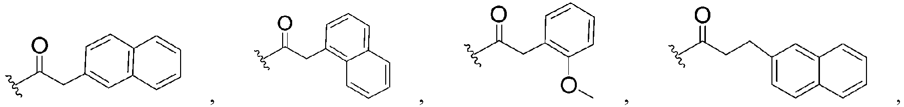 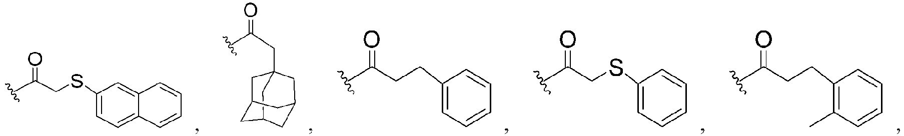 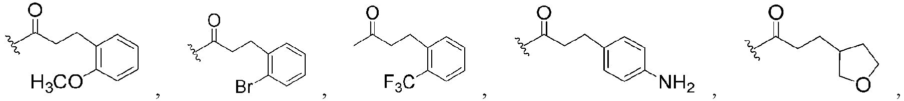 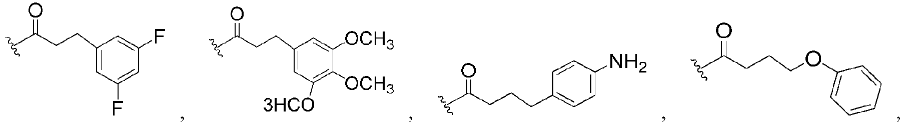 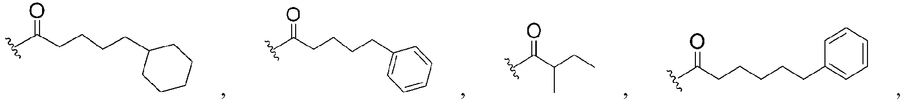 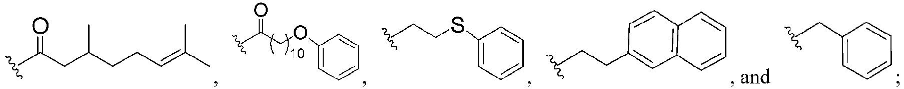
R₇ is selected from the group consisting of H, C₁-C₂₀ alkyl, C₃-C₂₀ cycloalkyl, C₃-C₂₀ heterocycloalkyl, C₅-C₁₅ aryl and C₅-C₁₅ heteroaryl; preferably, R₇ is H; preferably, R₇ is C₁-C₆ alkyl;
each E is a biomacromolecule; n is 1 or 2; T is a number between 1 and 20;
the wavy line linked to each E represents a covalent linking (e.g., via a covalent bond or a linker) of each A₁-L-A₂ to an N atom in the each E (e.g., an N atom on the side chain amino group of a lysine, arginine, asparagine, or glutamine residue), and L is a linker covalently linked to each of groups E, A₁, A₂.

11. The conjugate or a pharmaceutically acceptable salt, a geometric or optical isomer, a hydrate, a solvate or a polymorph thereof according to claim 10, wherein A₁ and A₂ each independently are selected from the group consisting of Formula (A-I) to Formula (A-XIII), 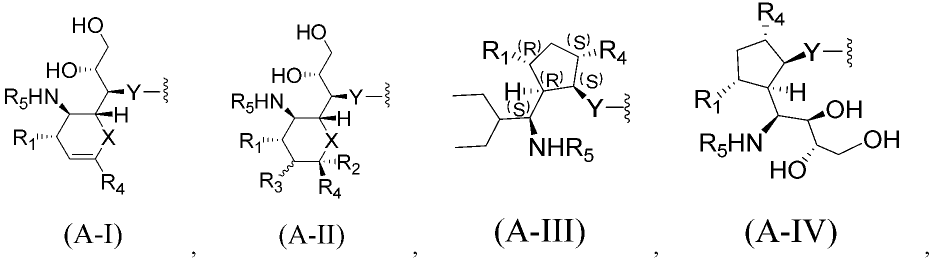 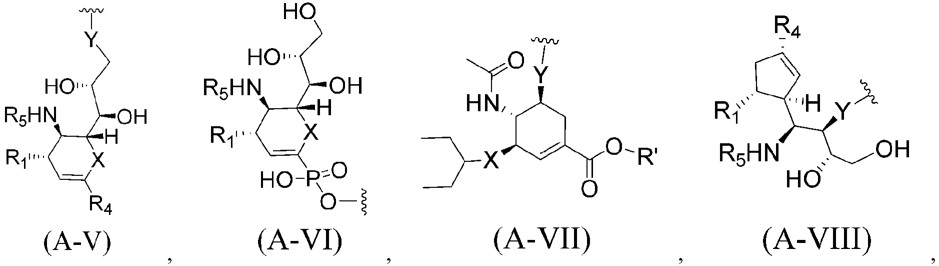 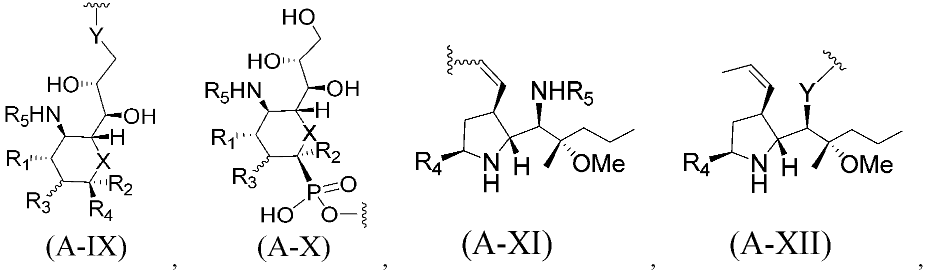 and 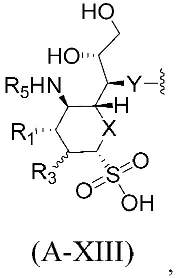
preferably, A₁ and A₂ each independently are selected from the group consisting of Formula (A-I) to Formula (A-IV) and Formula (A-VII), 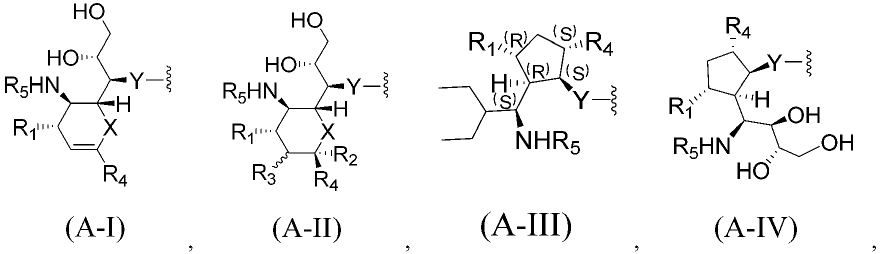 and 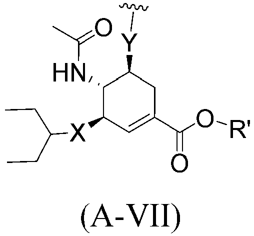
wherein R₁, R₂, R₃, R₄, R₅, R', X, and Y are as defined in claim 10;
each E comprises an Fc domain monomer (e.g., an Fc domain monomer having a sequence as set forth in any one of SEQ ID NO:1-68), albumin (e.g., albumin having a sequence as set forth in any one of SEQ ID NO:69-71), and an albumin-binding peptide, or an Fc-binding peptide; n is 1 or 2, wherein when n is 2 and E is the Fc domain monomer, the two E dimerize to form an Fc domain;
preferably, A₁ and A₂ each independently are selected from the group consisting of Formula (A-I) to Formula (A-III) and Formula (A-VII), 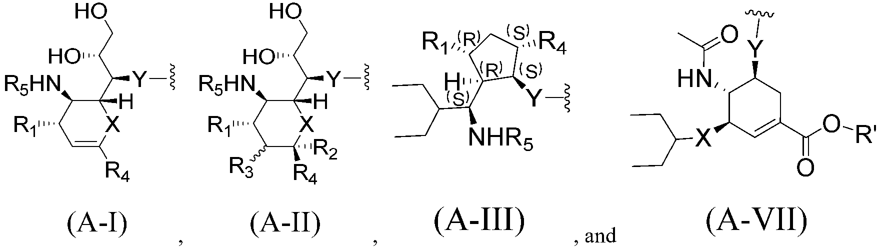
wherein R₁, R₂, R₃, R₄, R₅, R', X, and Y are as above defined;
preferably, A₁ and A₂ each independently are selected from the group consisting of Formula (A-I), Formula (A-III) and Formula (A-VII), 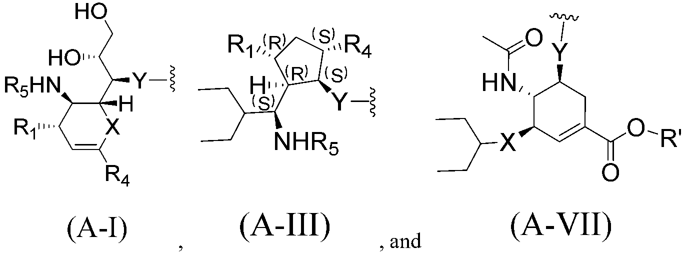
wherein R₁, R₄, R₅, R', X, and Y are as above defined.

12. The conjugate or a pharmaceutically acceptable salt, a geometric or optical isomer, a hydrate, a solvate or a polymorph thereof according to claim 10 or 11, wherein E comprises an Fc domain monomer (e.g., an Fc domain monomer having a sequence as set forth in any one of SEQ ID NO:1-68); n is 1 or 2, wherein when n is 2, the two E dimerize to form an Fc domain;
preferably, the Fc domain monomer has an amino acid sequence selected from the group consisting of:
i) any one of the sequences as set forth in SEQ ID NO: 1-68;
ii) a sequence with substitution, deletion or addition of one or several amino acids (e.g., substitution, deletion or addition of one, two, three, four or five amino acids) as compared with any one of the sequences as set forth in SEQ ID NO: 1-68; and
iii) a sequence with a sequence identity of at least 70% (such as 70%, 75%, 80%, 85%, 90%, 95%, 96%, 97%, 98%, 99% or 100%) as compared with any one of the sequences as set forth in SEQ ID NO: 1-68;
preferably, the substitution as described in (ii) is a conservative substitution;
preferably, E is an Fc domain monomer having a sequence with a sequence identity of at least 70% (such as 70%, 75%, 80%, 85%, 90%, 95%, 96%, 97%, 98%, 99% or 100%) as compared with the sequences as set forth in SEQ ID NO:64;
preferably, E is an Fc domain monomer having a sequence as set forth in SEQ ID NO:64;
preferably, R₁ is selected from the group consisting of -OH, -NH₂, and -NHC(=NH)NH₂; further preferably, R₁ is -NHC(=NH)NH₂;
preferably, R₄ is selected from the group consisting of -C(=O)OH, -C(=O)OCH₃ and -C(=O)OCF₃; further preferably, R₄ is selected from the group consisting of-C(=O)OH and -C(=O)OCH₃;
preferably, R₅ is selected from the group consisting of -C(=O)CH₃ and -C(=O)CF₃; further preferably, R₅ is -C(=O)CH₃;
preferably, R' is H or C₁-C₄ alkyl; further preferably, R' is H, methyl or ethyl; further preferably, R' is H or ethyl;
preferably, X is -O-;
preferably, Y is selected from the group consisting of -O-, -S-, and -NH-; further preferably, Y is selected from the group consisting of -O- and -NH-.

13. The conjugate or a pharmaceutically acceptable salt, a geometric or optical isomer, a hydrate, a solvate or a polymorph thereof according to any one of claims 10 to 12, wherein the conjugate has a structure represented by Formula (II-II-A), Formula (II-II-B) or Formula (II-II-C), 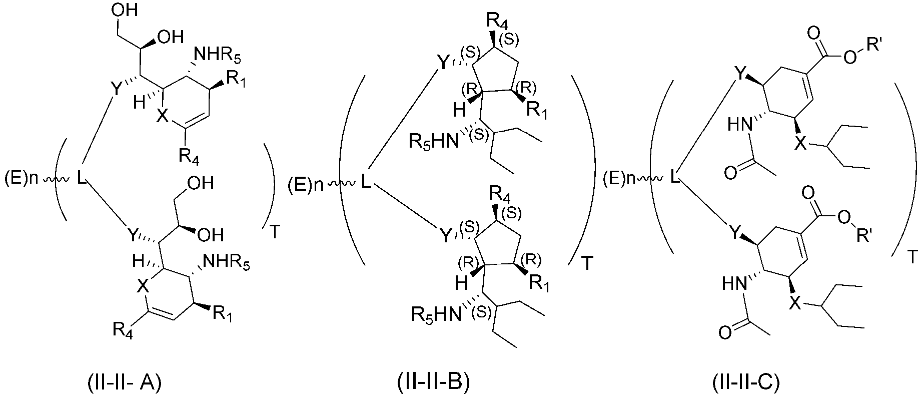 wherein R₁, R₄, R₅, R', X, Y, E, n, L, and T are as defined in any one of claims 10 to 12.

14. The conjugate or a pharmaceutically acceptable salt, a geometric or optical isomer, a hydrate, a solvate or a polymorph thereof according to any one of claims 10 to 13, wherein the conjugate has a structure represented by Formula (II-II-A'), Formula (II-II-B') or Formula (II-II-C'), 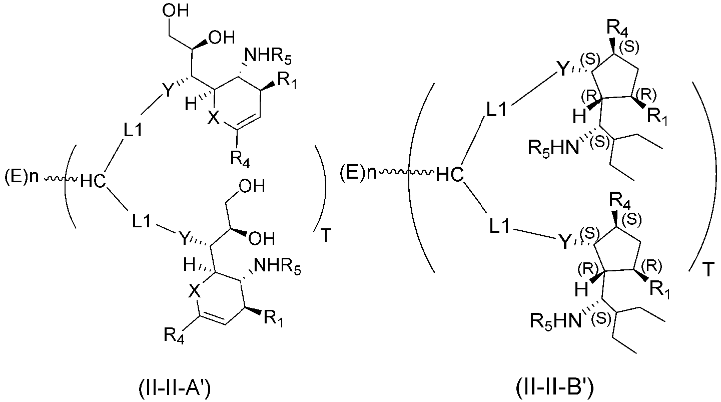 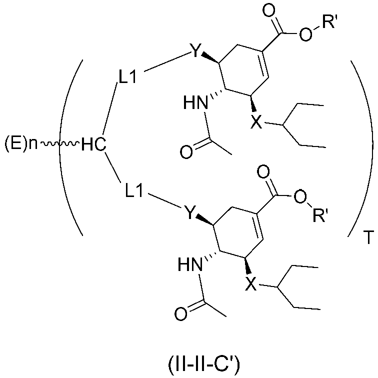
wherein R₁, R₄, R₅, R', X, Y, E, n, and T are as defined in any one of claims 10 to 12;
L₁ is selected from the group consisting of: 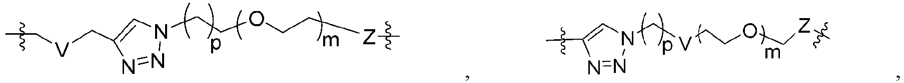 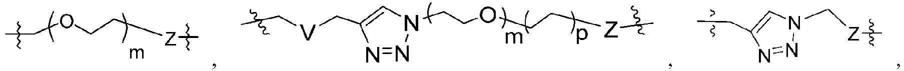 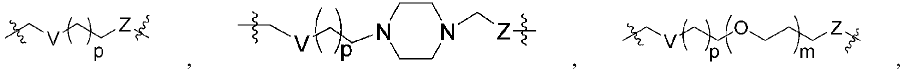 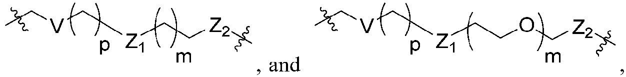 wherein V is -CH₂-, -O- or -S-; Z is - C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-; Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, - C(=O)- or -CH₂-; m and p each independently are an integer between 1 and 20;
preferably, L₁ is selected from the group consisting of: 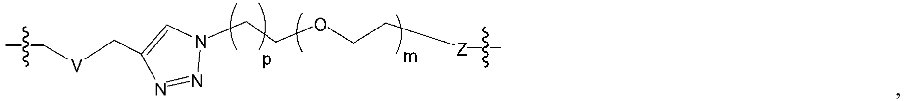 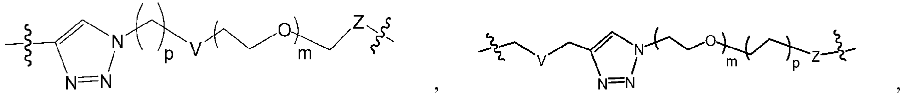 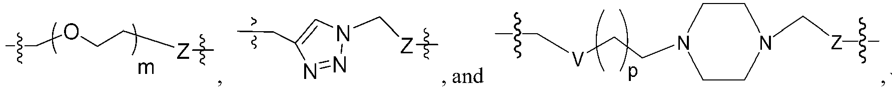 wherein V, p, m, Z are as above defined;
preferably, L₁ is 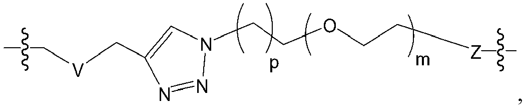 wherein V, p, m, and Z are as above defined;
preferably, V is -CH₂- or -O-; further preferably, V is -O-;
preferably, Z is -C(=O)-NH-, -CH₂-C(=O)-NH-, -NH-C(=O)-, -CH₂CH₂-C(=O)-, -N(CH₃)-C(=O)-, - N(CH₂CH₃)-C(=O)- or -C(=O)-; further preferably, Z is -NH-C(=O)- or -C(=O)-;
preferably, p is an integer between 1 and 10; further preferably, p is an integer between 1 and 6 (such as 1, 2, 3, 4, 5, 6);
preferably, m is an integer between 1 and 12; further preferably, m is an integer between 1 and 10 (such as 1, 2, 3, 4, 5, 6, 7, 8, 9).

15. The conjugate or a pharmaceutically acceptable salt, a geometric or optical isomer, a hydrate, a solvate or a polymorph thereof according to any one of claims 10 to 14, wherein the conjugate has a structure represented by Formula (II-II-A-1), Formula (II-II-B-1), Formula (II-II-B-2) or Formula (II-II-C-1), 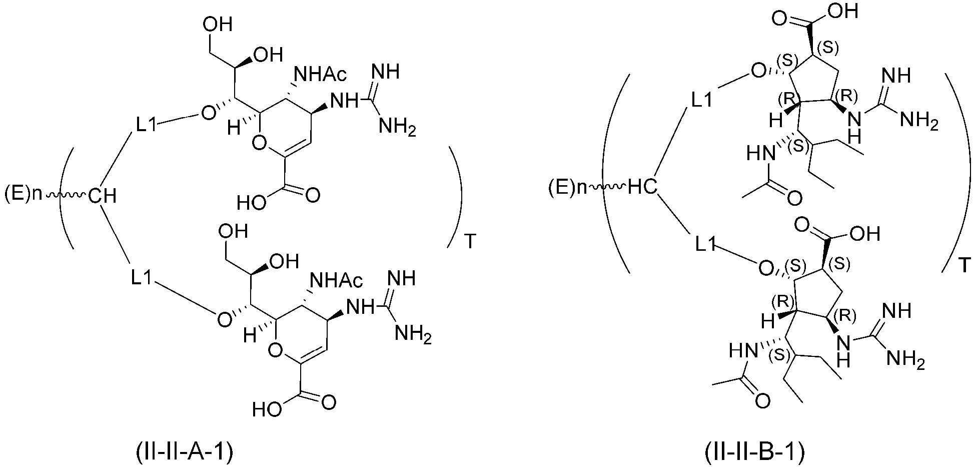 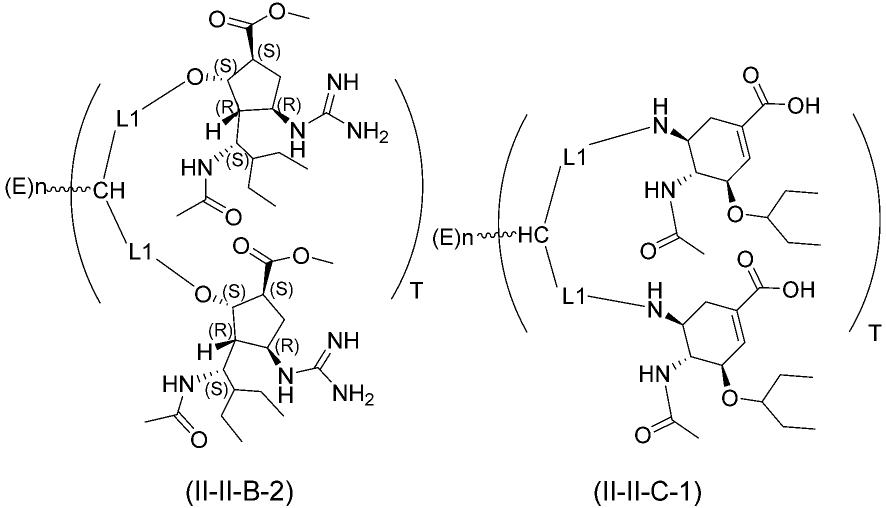 wherein E, n, and T are as defined in any one of claims 10 to 13, and L₁ is as defined in claim 14.

16. The conjugate or a pharmaceutically acceptable salt, a geometric or optical isomer, a hydrate, a solvate or a polymorph thereof according to any one of claims 10 to 15, wherein the conjugate has a structure represented by Formula (II-II-A-2), Formula (II-II-B-3), Formula (II-II-B-4) or Formula (II-II-C-2), 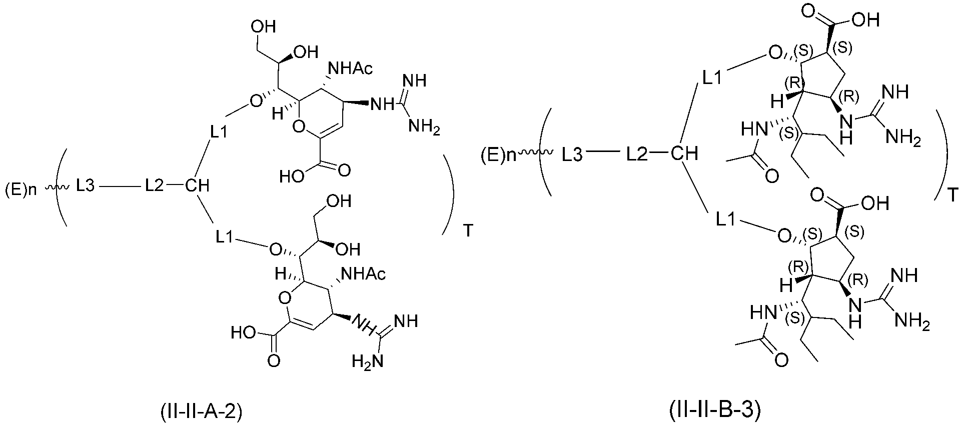 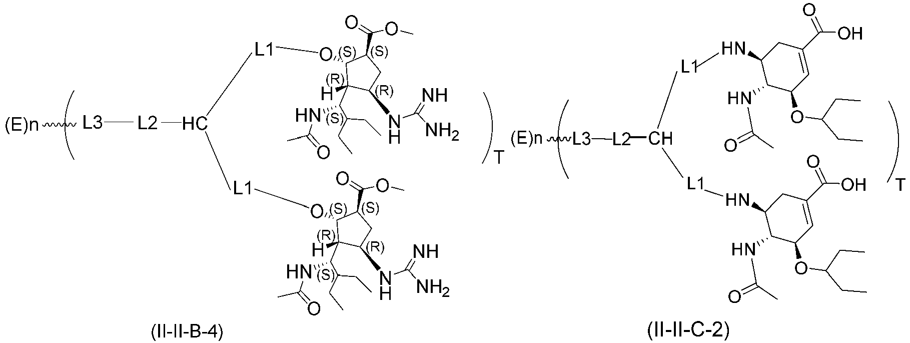
wherein E, n, and T are as defined in any one of claims 10 to 13, and L₁ is as defined in claim 14;
L₂ is selected from the group consisting of: 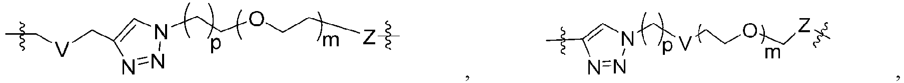 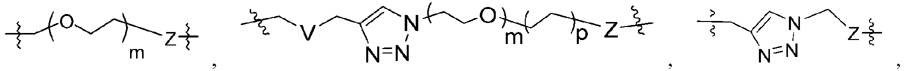 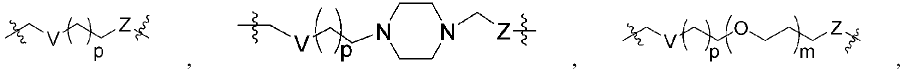 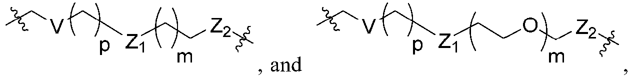 wherein V is -CH₂-, -O- or -S-; Z is - C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-; Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, - C(=O)- or -CH₂-; m and p each independently are an integer between 1 and 20;
preferably, L₂ is selected from the group consisting of: 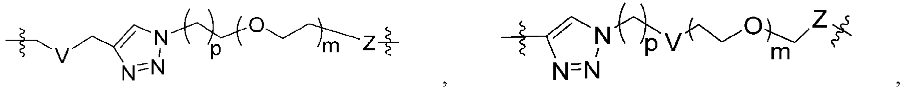 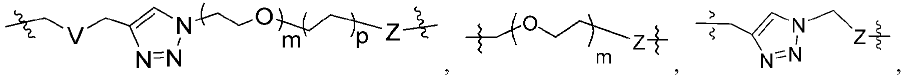 and 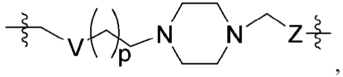 wherein V is -CH₂-, -O- or -S-; Z is -C(=O)-NH-, -CH₂-C(=O)-NH-, - NH-C(=O)-, -CH₂CH₂-C(=O)-, -N(CH₃)-C(=O)-, -N(CH₂CH₃)-C(=O)-, -O-C(=O)-, -C(=O)- or -CH₂-; m and p each independently are an integer between 1 and 20;
L₃ is 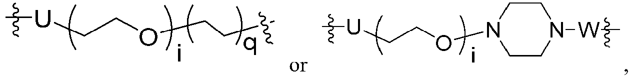 wherein U is -C(=O)-NH-, - NH-C(=O)-, -N(CH₃)-C(=O)-, -N(CH₂CH₃)-C(=O)-, -O-C(=O)-, -C(=O)-, or -CH₂-; W is -CH₂-, -O- or -S-; i and q each independently are an integer between 1 and 20, one end of L₃ is covalently linked to E, and the other end is covalently linked to L₂;
preferably, L₁ is 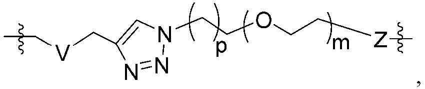 wherein V, p, m, and Z are as above defined; preferably, V is -CH₂- or -O-; further preferably, V is -O-; preferably, Z is -C(=O)-NH-, -CH₂-C(=O)-NH-, -NH-C(=O)-, -CH₂CH₂-C(=O)-, -N(CH₃)-C(=O)-, -N(CH₂CH₃)-C(=O)- or -C(=O)-; further preferably, Z is -NH-C(=O)- or -C(=O)-; preferably, p is an integer between 1 and 10; further preferably, p is an integer between 1 and 6 (such as 1, 2, 3, 4, 5, 6); preferably, m is an integer between 1 and 12; further preferably, m is an integer between 1 and 10 (such as 1, 2, 3, 4, 5, 6, 7, 8, 9);
preferably, L₂ is 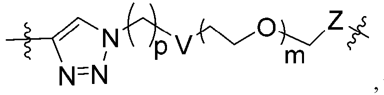 wherein V, p, m, and Z are as above defined; preferably, V is -CH₂- or -O-; further preferably, V is -O-; preferably, Z is -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-; further preferably, Z is -C(=O)-NH-; preferably, p is an integer between 1 and 10; further preferably, p is an integer between 1 and 6 (such as 1, 2, 3, 4, 5, 6); preferably, m is an integer between 1 and 12; further preferably, m is an integer between 1 and 10 (such as 1, 2, 3, 4, 5, 6, 7, 8, 9);
preferably, L₃ is 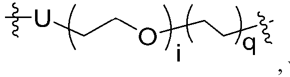 wherein U, i, and q are as above defined; preferably, U is -NH-C(=O)-, -N(CH₃)-C(=O)- or -N(CH₂CH₃)-C(=O)-; further preferably, U is -NH-C(=O)-; preferably, i is an integer between 1 and 12; further preferably, i is an integer between 1 and 10 (such as 1, 2, 3, 4, 5, 6, 7, 8, 9); preferably, q is an integer between 1 and 10; further preferably, q is an integer between 1 and 6 (such as 1, 2, 3, 4, 5, 6);
preferably, T is a number between 1 and 10; further preferably, T is a number between 1 and 9; further preferably, T is a number between 1 and 7; further preferably, T is a number between 1 and 5, further preferably, T is a number between 3 and 7, and further preferably, T is a number between 3 and 5.5.

17. The conjugate or a pharmaceutically acceptable salt, a geometric or optical isomer, a hydrate, a solvate or a polymorph thereof according to any one of claims 10 to 16, wherein the conjugate has a structure represented by Formula (II-II-A-3), Formula (II-II-B-5), Formula (II-II-B-6) or Formula (II-II-C-3), 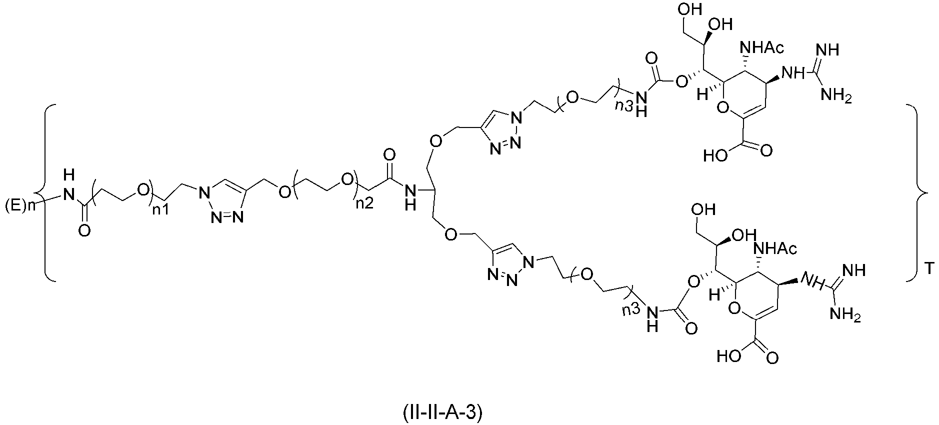 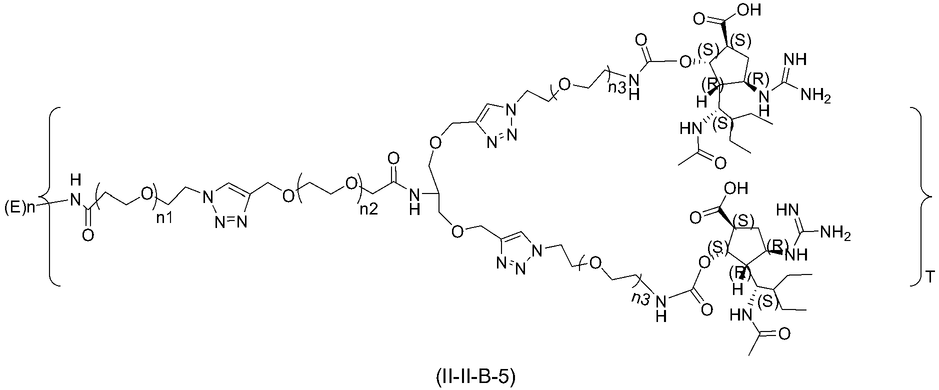 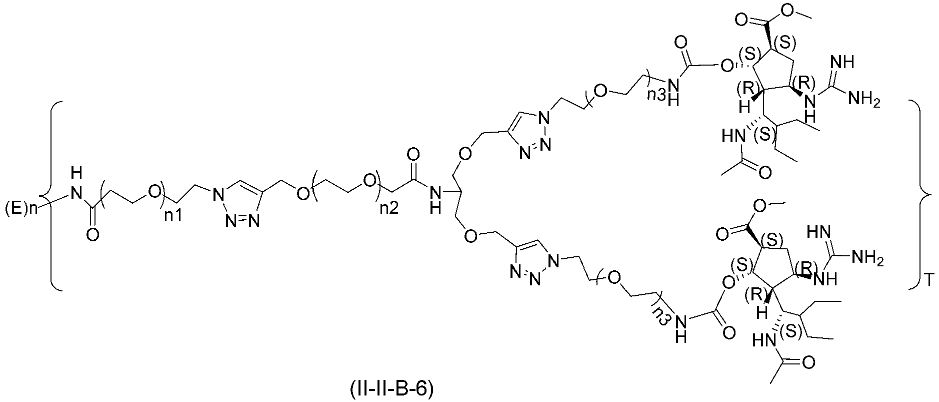 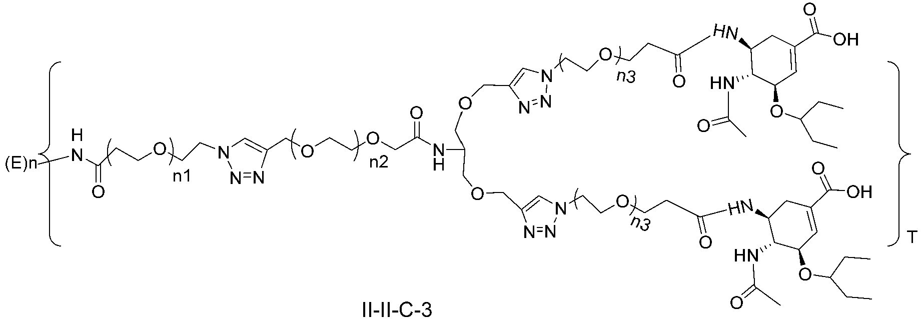
wherein E, n, and T are as defined in any one of claims 10 to 16; n1, n2, and n3 each independently are an integer between 1 and 20;
preferably, n1, n2, and n3 each independently are an integer between 1 and 12; further preferably, n1, n2, and n3 each independently are an integer between 1 and 10 (such as 1, 2, 3, 4, 5, 6, 7, 8, 9).

18. The conjugate or a pharmaceutically acceptable salt, a geometric or optical isomer, a hydrate, a solvate or a polymorph thereof according to any one of claims 10 to 17, wherein the conjugate is selected from the group consisting of: 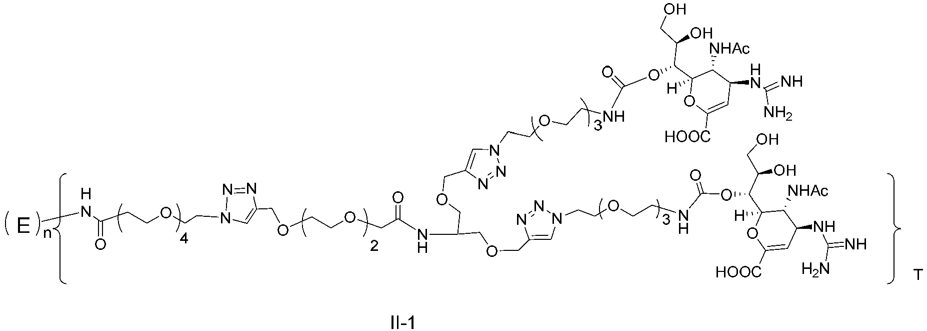 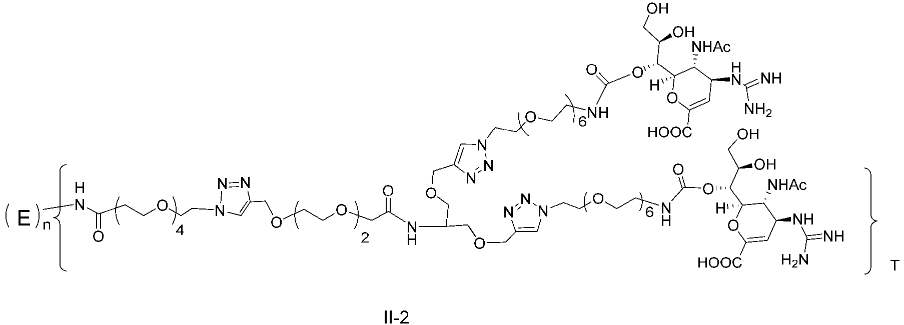 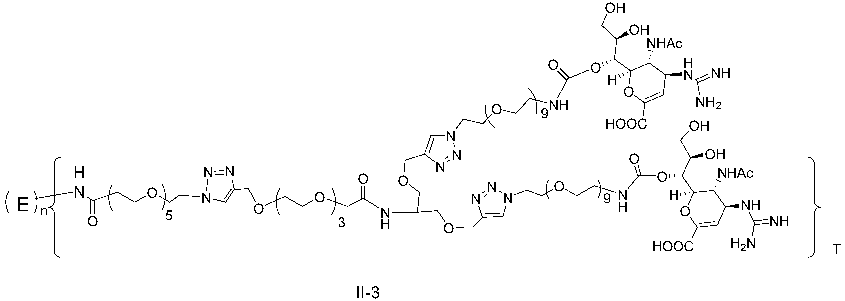 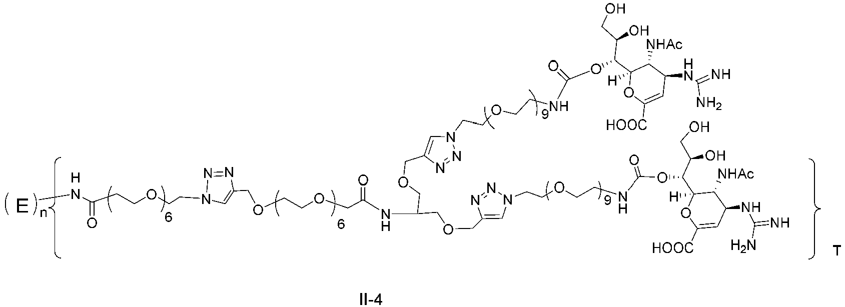 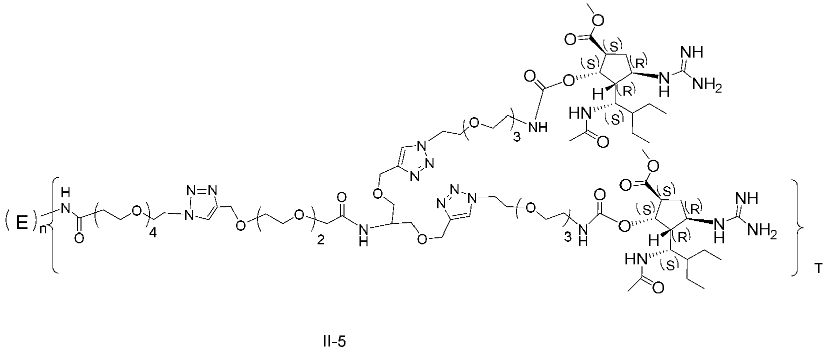 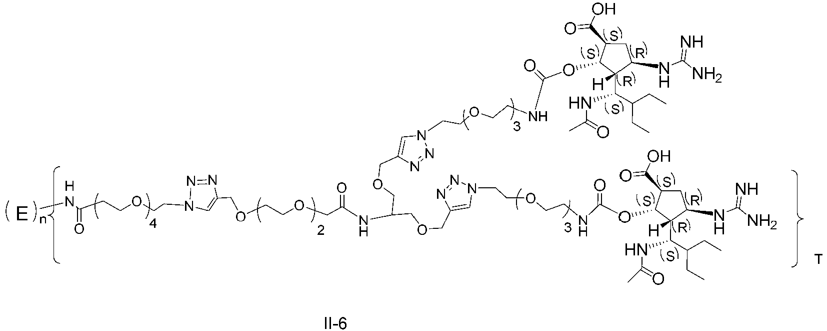 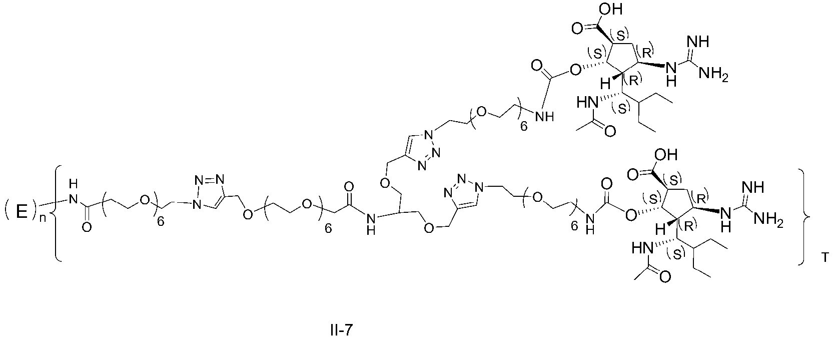 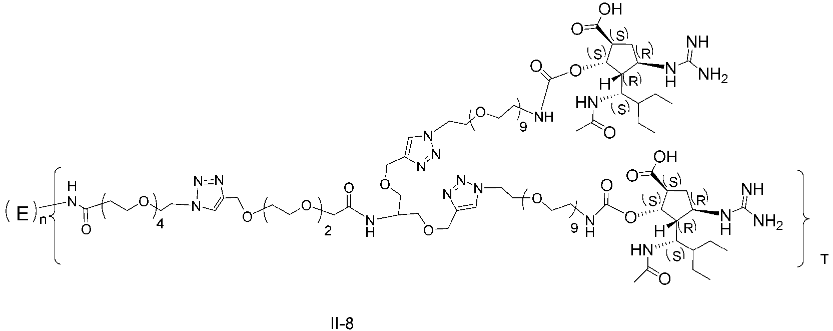 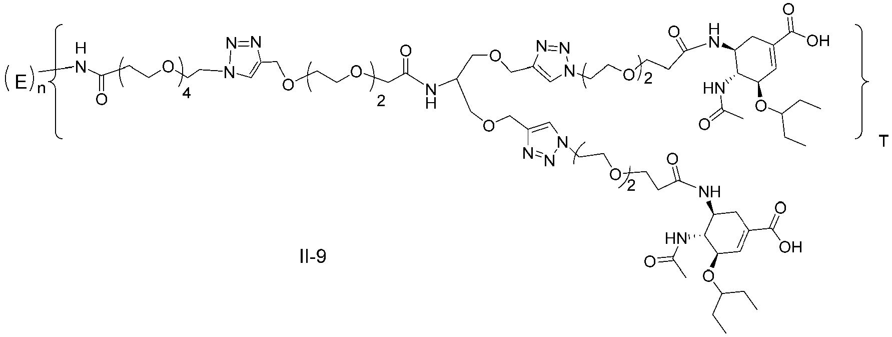 and 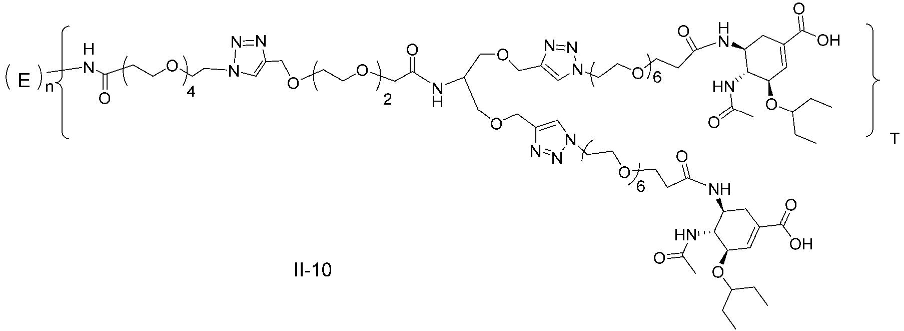 wherein E, n, and T are as defined in any one of claims 10 to 17.

19. A compound represented by Formula (IV) or a salt, a geometric or optical isomer, a hydrate, a solvate or a polymorph thereof, 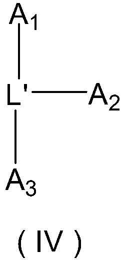
wherein A₁, A₂ and A₃ each independently are selected from the group consisting of Formula (A-I) to Formula (A-XIV), 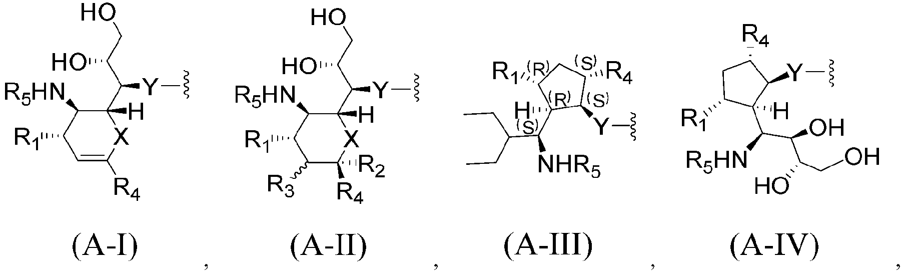 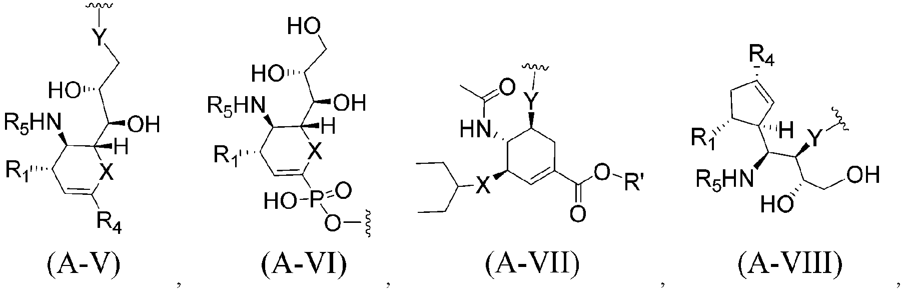 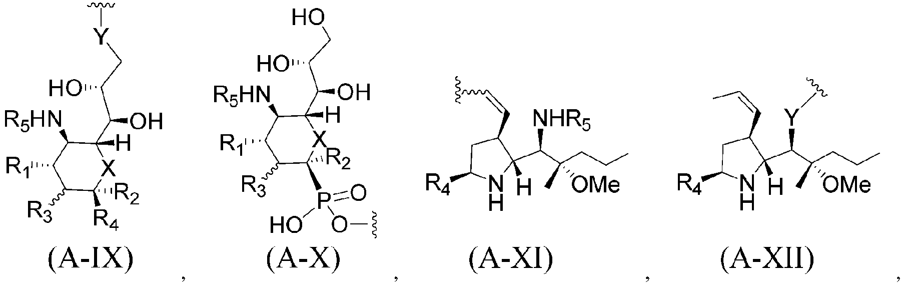 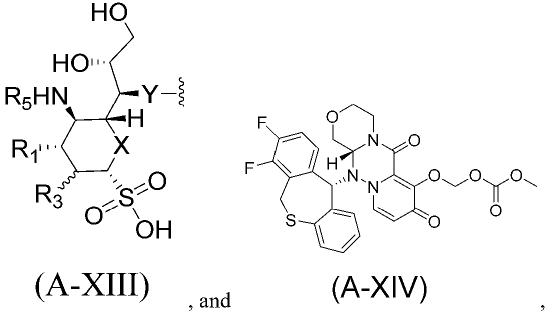
wherein, L may be linked to any possible sites of the compound of Formula (A-XIV);
wherein R₁ is selected from the group consisting of -OH, -NH₂, -NHC(=NH)NH₂ and -NHC(=NH)NHR₆;
R₂ and R₃ each independently are selected from the group consisting of -H, -OH, -F, -Cl and -Br;
R₄ is selected from the group consisting of -C(=O)OH, -P(=O)(OH)₂, -SO₃H, -C(=O)OCH₃ and -C(=O)OCF₃;
R₅ is selected from the group consisting of -C(=O)CH₃, -C(=O)CF₃, and -SO₂CH₃;
X is selected from the group consisting of -O- and -S-;
Y is selected from the group consisting of -O-, -S-, -NR₇-, -OC(=O)NR₇-, -OC(=S)NR₇-, -OC(=O)O-, -OC(=O)-, -NHC(=O)O-, -NHC(=O)-, -NHC(=NH)-, -NHC(=O)NR₇-, -NHC(=NH)NR₇-, -NHC(=S)NR₇-, -NHC(=S)-, -OCH₂C(=O)NR₇-, -NH(SO₂)- and -NH(SO₂)NR₇-;
R' is H or C₁-C₆ alkyl;
R₆ is selected from the group consisting of: 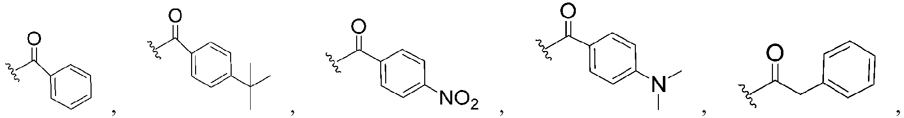 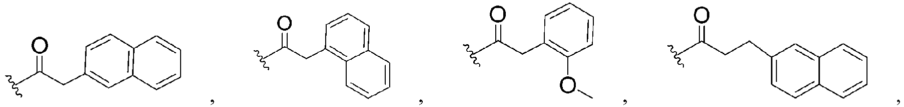 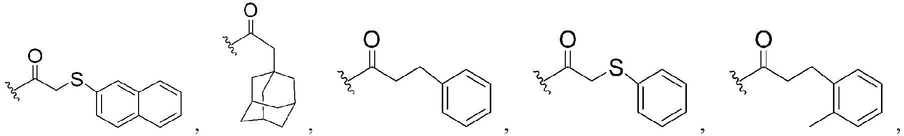 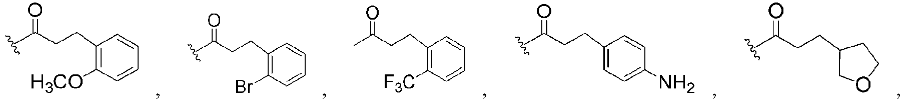 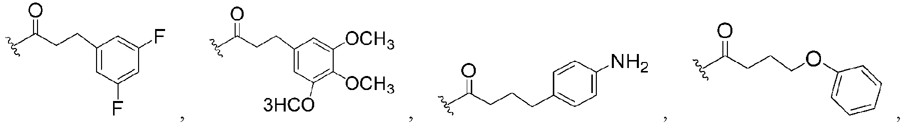 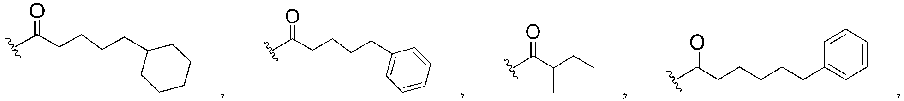 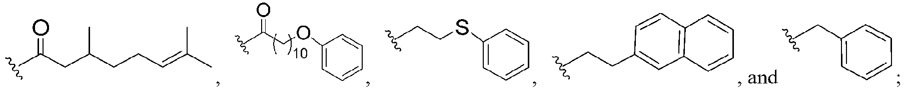
R₇ is selected from the group consisting of H, C₁-C₂₀ alkyl, C₃-C₂₀ cycloalkyl, C₃-C₂₀ heterocycloalkyl, C₅-C₁₅ aryl and C₅-C₁₅ heteroaryl; preferably, R₇ is H; preferably, R₇ is C₁-C₆ alkyl;
L' is a linker covalently linked to each of groups A₁, A₂ and A₃.

20. The compound represented or a salt, a geometric or optical isomer, a hydrate, a solvate or a polymorph thereof according to claim 19, wherein A₁, A₂ and A₃ each independently are selected from the group consisting of Formula (A-I) to Formula (A-XIII), 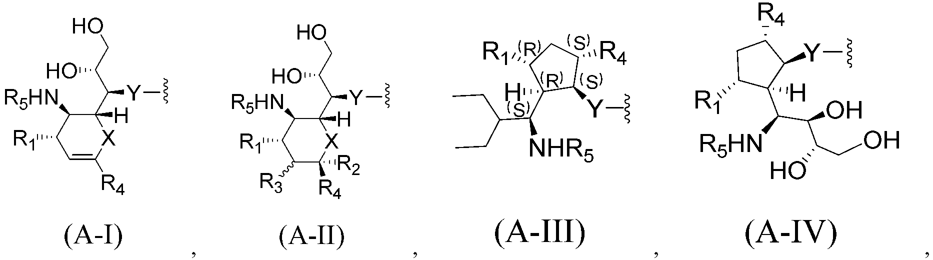 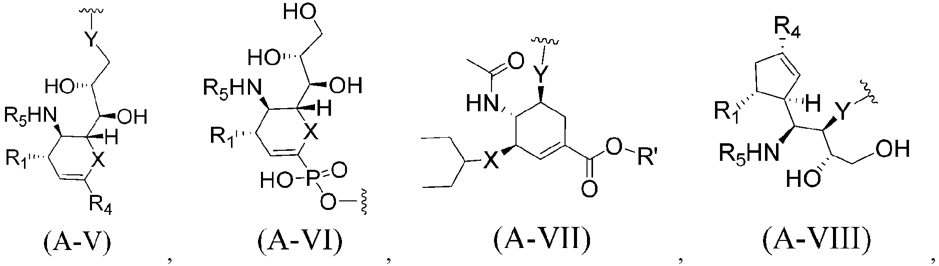 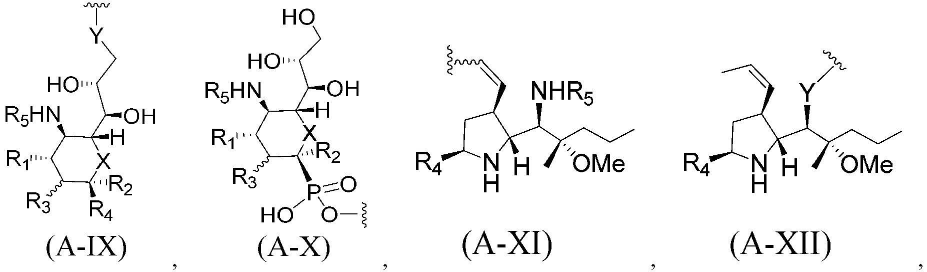 and 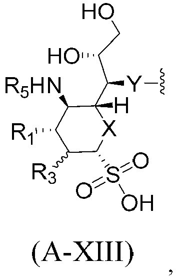
preferably, A₁, A₂, and A₃ each independently are selected from the group consisting of Formula (A-I) to Formula (A-IV) and Formula (A-VII), 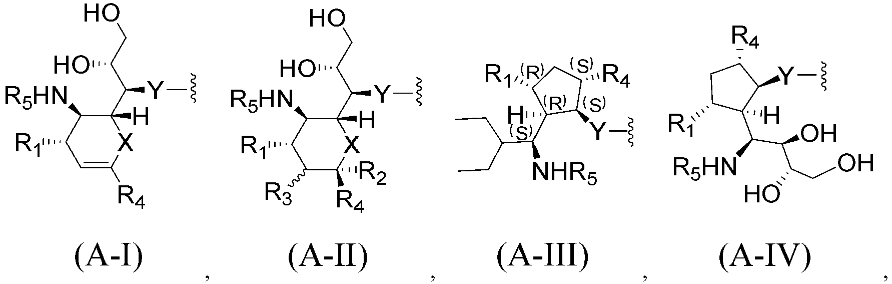 and 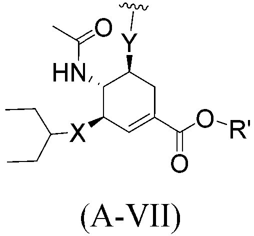
wherein R₁, R₂, R₃, R₄, R₅, R', X, and Y are as defined in claim 19;
preferably, A₁, A₂ and A₃ each independently are selected from the group consisting of Formula (A-I) to Formula (A-III) and Formula (A-VII), 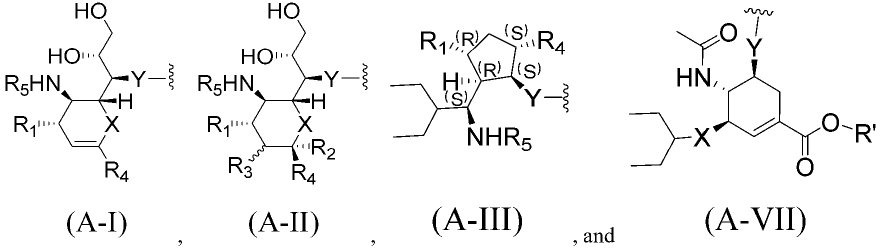
wherein R₁, R₂, R₃, R₄, R₅, R', X, and Y are as above defined;
preferably, A₁, A₂ and A₃ each independently are selected from the group consisting of Formula (A-I), Formula (A-III) and Formula (A-VII), 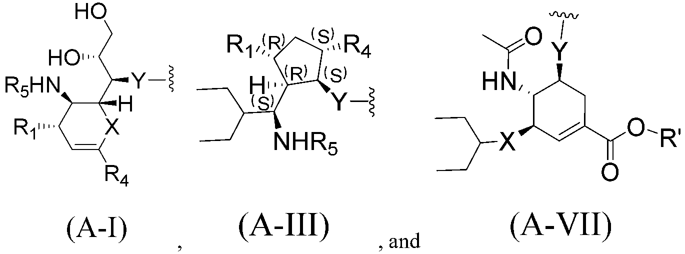
wherein R₁, R₄, R₅, R', X, and Y are as above defined.

21. The compound or a salt, a geometric or optical isomer, a hydrate, a solvate or a polymorph thereof according to claim 19 or 20, wherein
R₁ is selected from the group consisting of -OH, -NH₂, and -NHC(=NH)NH₂; further preferably, R₁ is -NHC(=NH)NH₂;
preferably, R₄ is selected from the group consisting of -C(=O)OH, -C(=O)OCH₃ and -C(=O)OCF₃; further preferably, R₄ is selected from the group consisting of -C(=O)OH and -C(=O)OCH₃;
preferably, R₅ is selected from the group consisting of-C(=O)CH₃ and -C(=O)CF₃; further preferably, R₅ is -C(=O)CH₃;
preferably, R' is H or C₁-C₄ alkyl; further preferably, R' is H, methyl or ethyl; further preferably, R' is H or ethyl;
preferably, X is -O-;
preferably, Y is selected from the group consisting of -O-, -S-, and -NH-; further preferably, Y is selected from the group consisting of -O- and -NH-.

22. The compound or a salt, a geometric or optical isomer, a hydrate, a solvate or a polymorph thereof according to any one of claims 19 to 21, wherein the compound has a structure represented by Formula (IV-I), 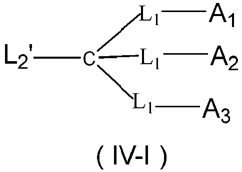
wherein A₁, A₂ and A₃ are as defined in any one of claims 19 to 21,
L₁ is selected from the group consisting of: 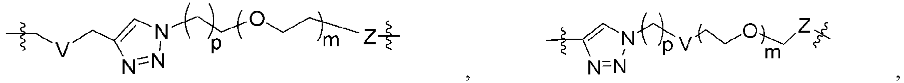 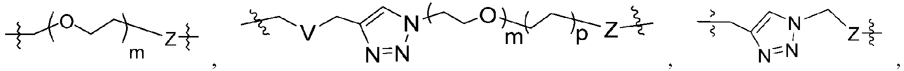 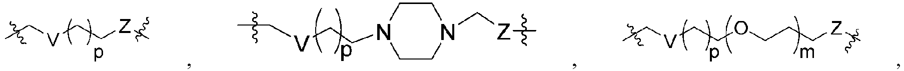 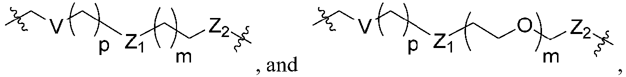
L₂' is selected from the group consisting of: 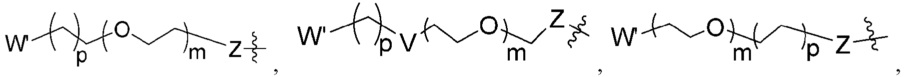 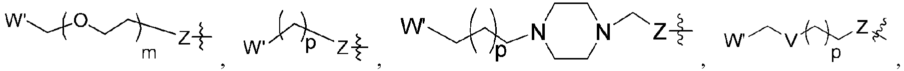 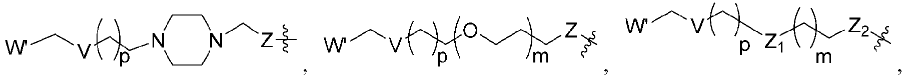 and 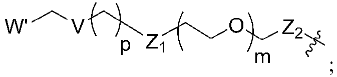
wherein V is -CH₂-, -O- or -S-; Z is -C(=O)-NH-, -CH₂-C(=O)-NH-, -NH-C(=O)-, -CH₂CH₂-C(=O)-, - N(CH₃)-C(=O)-, -N(CH₂CH₃)-C(=O)-, -O-C(=O)-, -C(=O)- or -CH₂-; or Z is 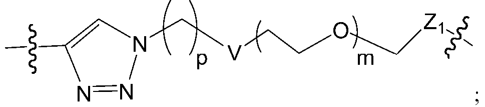 Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-; W' is 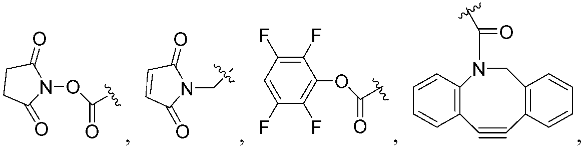 HC≡C-**,** -C(=O)OH, -NH₂ or N₃-; m and p each independently are an integer between 1 and 20;
preferably, L₁ is selected from the group consisting of: 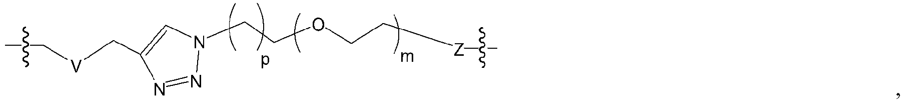 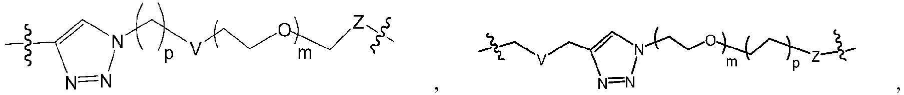 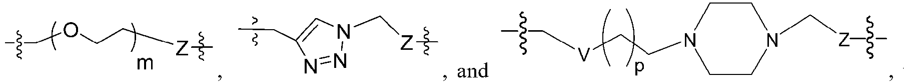 wherein V, p, m, and Z are as above defined;
preferably, L₁ is 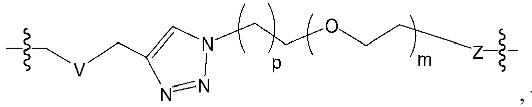 wherein V, p, m, and Z are as above defined;
preferably, L₂' is 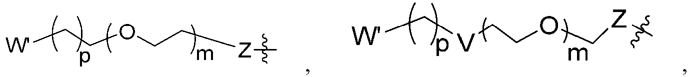 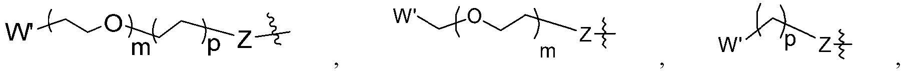 or 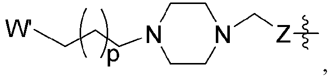 wherein W', V, p, m, and Z are as above defined;
preferably, L₂' is 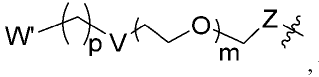 wherein W', V, p, m, and Z are as above defined;
preferably, L₂' is 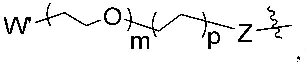 wherein W', p, m, and Z are as above defined;
preferably, V is -CH₂- or -O-; further preferably, V is -O-;
preferably, Z is -C(=O)-NH-, -CH₂-C(=O)-NH-, -NH-C(=O)-, -CH₂CH₂-C(=O)-, -N(CH₃)-C(=O)-, - N(CH₂CH₃)-C(=O)- or -C(=O)-; further preferably, Z is -NH-C(=O)- or -C(=O)-;
preferably, W' is HC≡C- , -C(=O)OH, -NH₂ or N₃-; further preferably, W' is HC≡C- ;
preferably, W' is 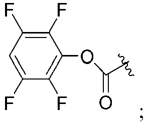
preferably, Z is 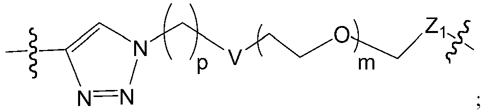
preferably, Z₁ is -C(=O)-NH-, -CH₂-C(=O)-NH-, -NH-C(=O)- or -CH₂CH₂-C(=O)-; further preferably, Z₁ is -C(=O)-NH- or -CH₂-C(=O)-NH-; more further preferably, Z₁ is -C(=O)-NH-;
preferably, p is an integer between 1 and 10; further preferably, p is an integer between 1 and 6 (such as 1, 2, 3, 4, 5, 6);
preferably, m is an integer between 1 and 12; further preferably, m is an integer between 1 and 10 (such as 1, 2, 3, 4, 5, 6, 7, 8, 9).

23. The compound or a salt, a geometric or optical isomer, a hydrate, a solvate or a polymorph thereof according to claims 19 to 22, wherein the compound has a structure represented by Formula (IV-I-1), Formula (IV-I-2), or Formula (IV-I-3): 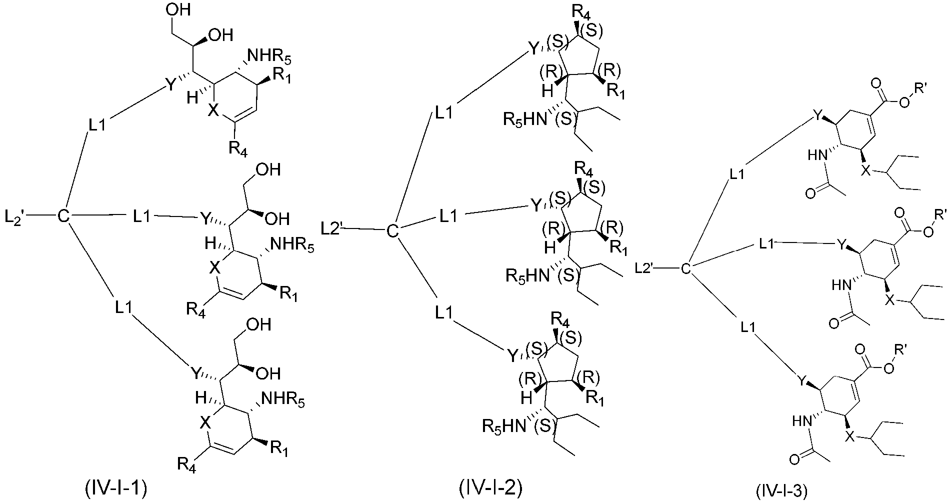 wherein R₁, R₄, R₅, R', X, and Y are as defined in any one of claims 19 to 21, and L₁ and L₂' are as defined in claim 22.

24. The compound or a salt, a geometric or optical isomer, a hydrate, a solvate or a polymorph thereof according to any one of claims 19 to 23, wherein the compound has a structure represented by Formula (IV-I-1-1), Formula (IV-I-2-1), Formula (IV-I-2-2) or Formula (IV-I-3-1): 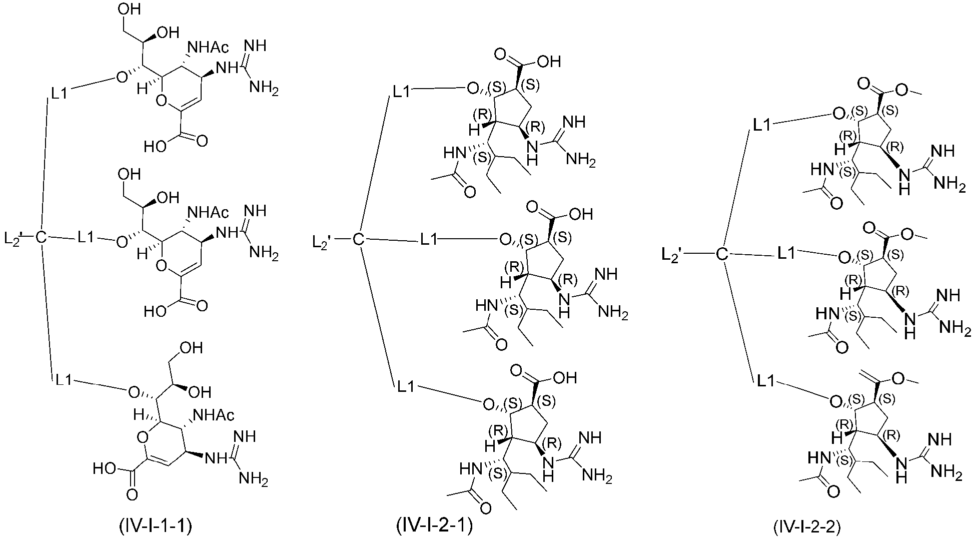 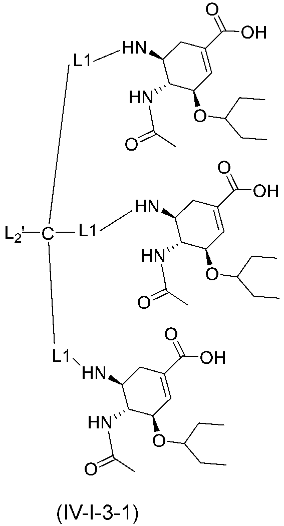 wherein L₁ and L₂' are as defined in claim 22.

25. The compound or a salt, a geometric or optical isomer, a hydrate, a solvate or a polymorph thereof according to claims 19 to 24, wherein the compound is selected from the group consisting of:
| Intermediates | Structures |
|---|---|
| Int-11 | 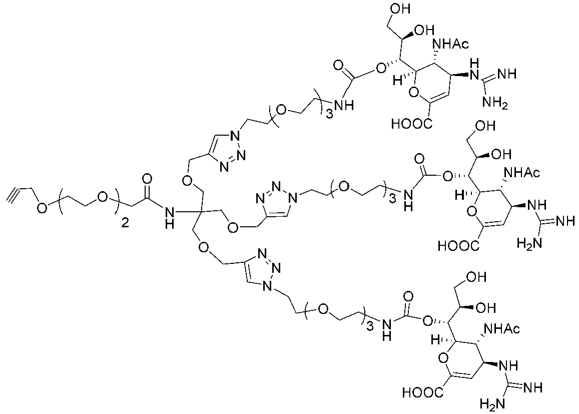 |
| Int-12 | 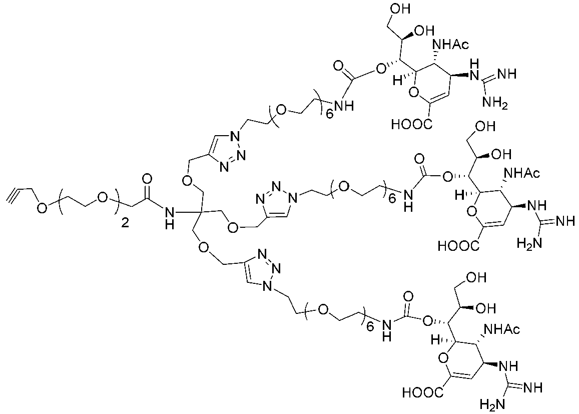 |
| Int-13 | 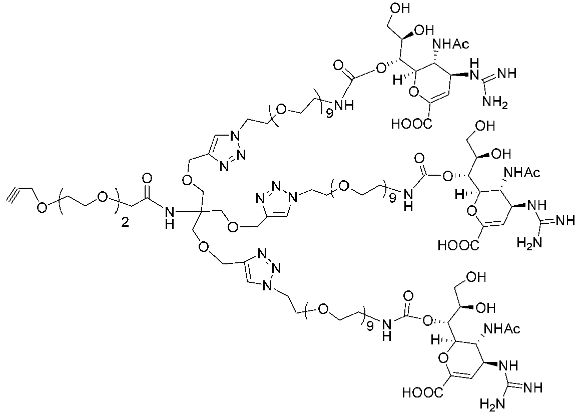 |
| Int-14 | 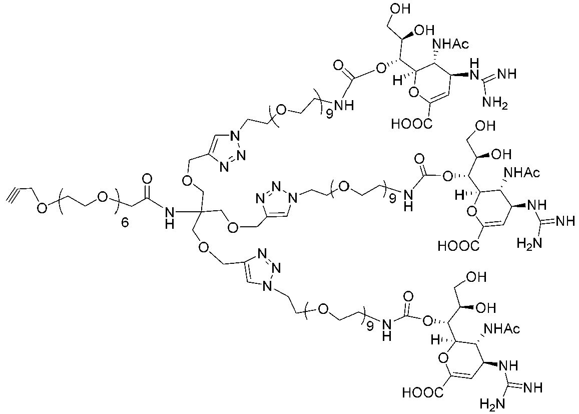 |
| Int-15 | 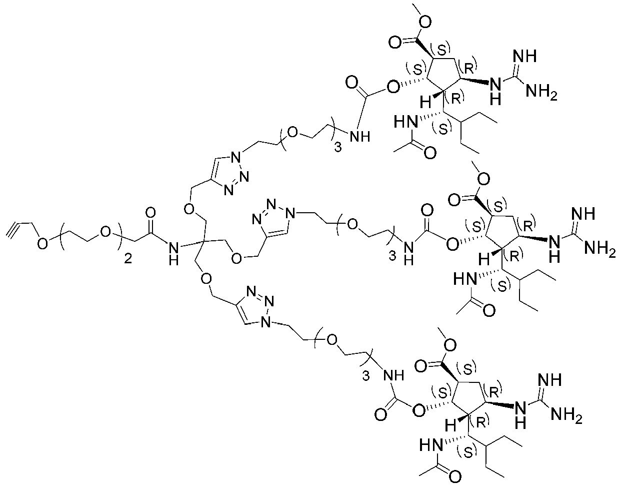 |
| Int-16 | 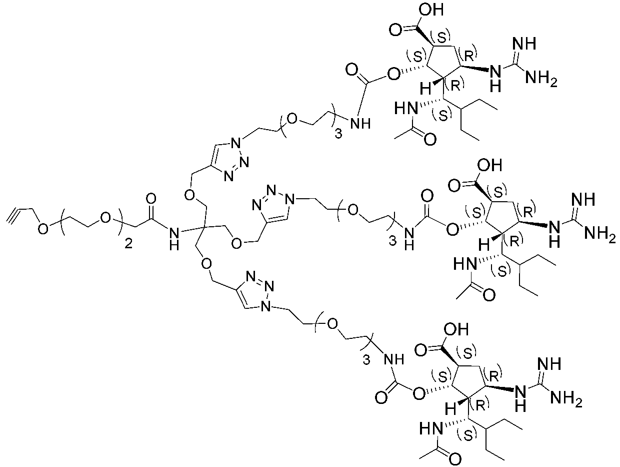 |
| Int-17 | 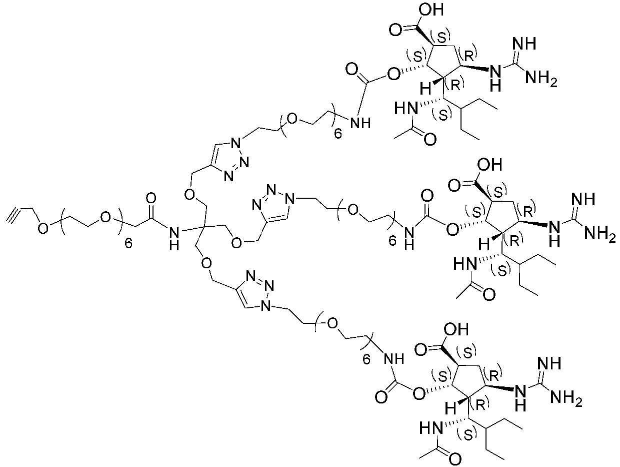 |
| Int-18 | 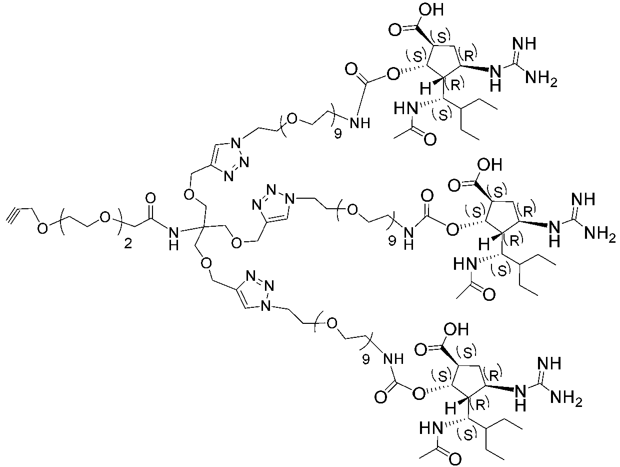 |
| Int-19 | 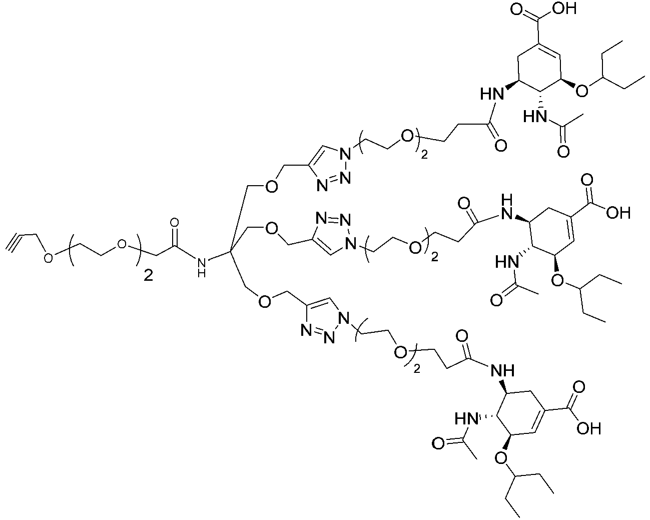 |
| Int-20 | 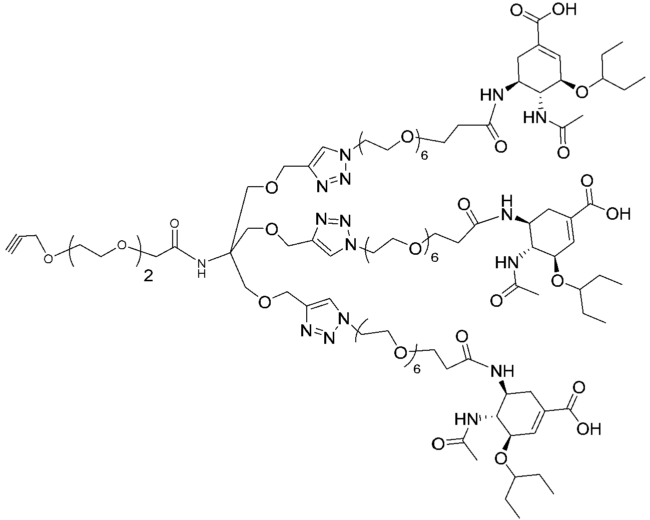 |
| Int-16-A | 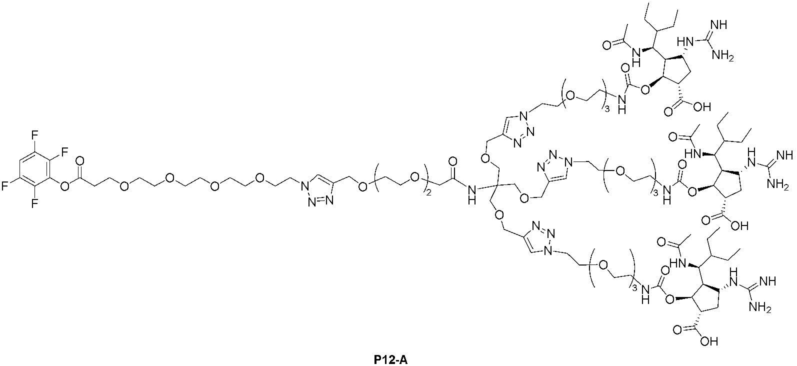 |

26. A compound represented by Formula (V) or a salt, a geometric or optical isomer, a hydrate, a solvate or a polymorph thereof, 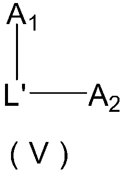
wherein A₁ and A₂ each independently are selected from the group consisting of (A-I) to Formula (A-XIV), 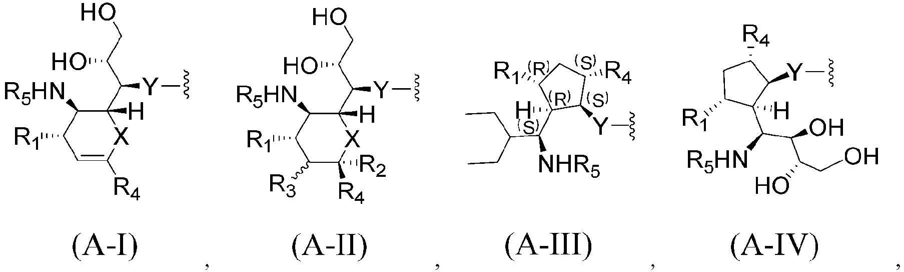 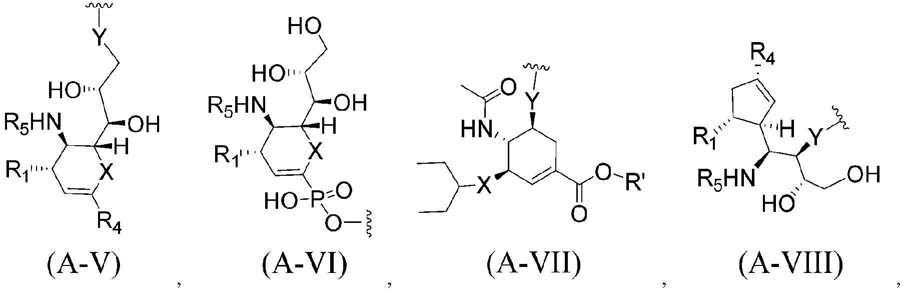 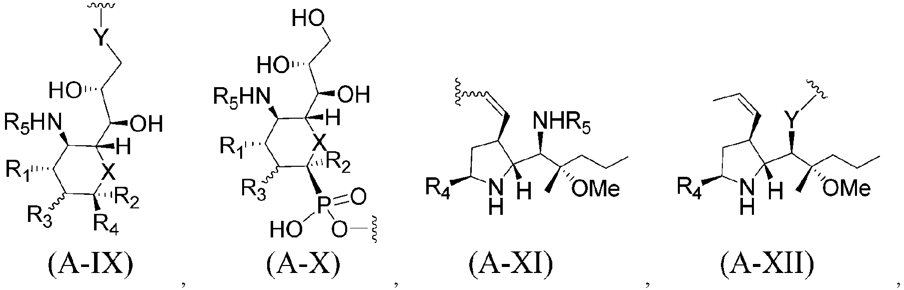 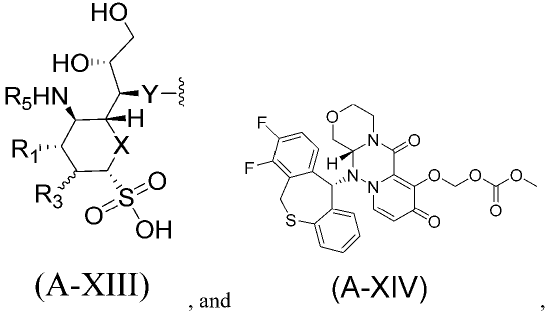
wherein, L may be linked to any possible sites of the compound of Formula (A-XIV);
wherein R₁ is selected from the group consisting of -OH, -NH₂, -NHC(=NH)NH₂ and -NHC(=NH)NHR₆;
R₂ and R₃ each independent are selected from the group consisting of -H, -OH, -F, -Cl and -Br;
R₄ is selected from the group consisting of -C(=O)OH, -P(=O)(OH)₂, -SO₃H, -C(=O)OCH₃ and -C(=O)OCF₃;
R₅ is selected from the group consisting of -C(=O)CH₃, -C(=O)CF₃, and -SO₂CH₃;
X is selected from the group consisting of -O- and -S-;
Y is selected from the group consisting of -O-, -S-, -NR₇-, -OC(=O)NR₇-, -OC(=S)NR₇-, -OC(=O)O-, -OC(=O)-, -NHC(=O)O-, -NHC(=O)-, -NHC(=NH)-, -NHC(=O)NR₇-, -NHC(=NH)NR₇-, -NHC(=S)NR₇-, -NHC(=S)-, -OCH₂C(=O)NR₇-, -NH(SO₂)- and -NH(SO₂)NR₇-;
R' is H or C₁-C₆ alkyl;
R₆ is selected from the group consisting of 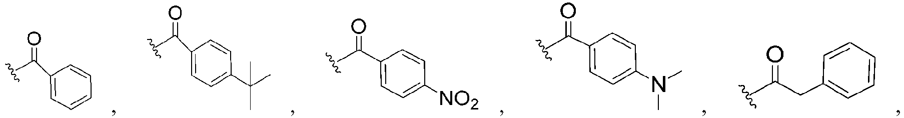 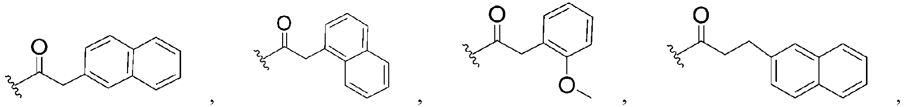 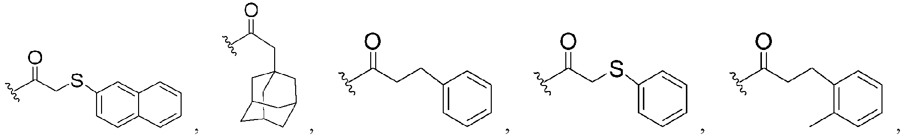 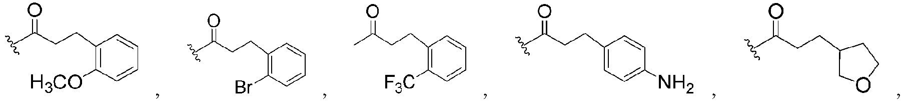 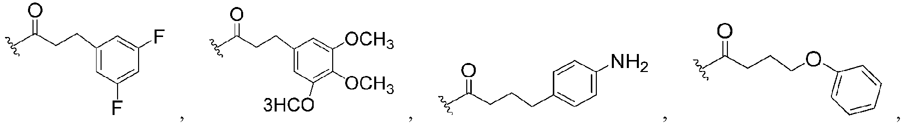 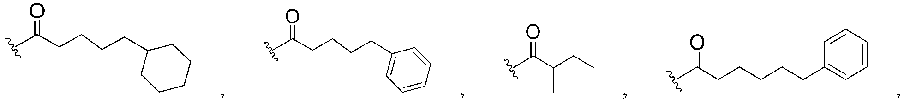 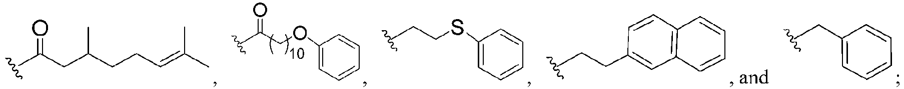
R₇ is selected from the group consisting of H, C₁-C₂₀ alkyl, C₃-C₂₀ cycloalkyl, C₃-C₂₀ heterocycloalkyl, C₅-C₁₅ aryl and C₅-C₁₅ heteroaryl; preferably, R₇ is H; preferably, R₇ is C₁-C₆ alkyl;
L' is a linker covalently linked to each of groups A₁ and A₂.

27. The compound or a salt, a geometric or optical isomer, a hydrate, a solvate or a polymorph thereof according to claim 26, wherein A₁ and A₂ each independently are selected from the group consisting of Formula (A-I) to Formula (A-XIII), 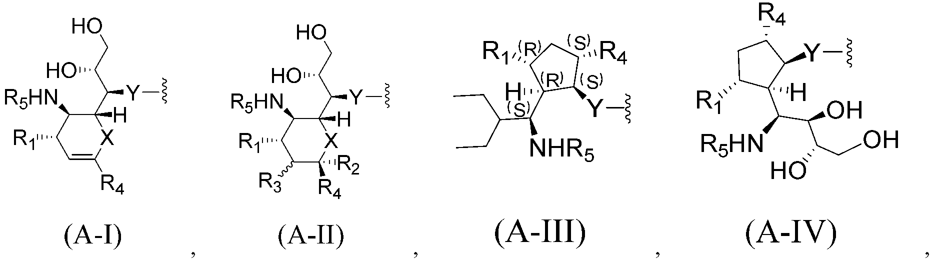 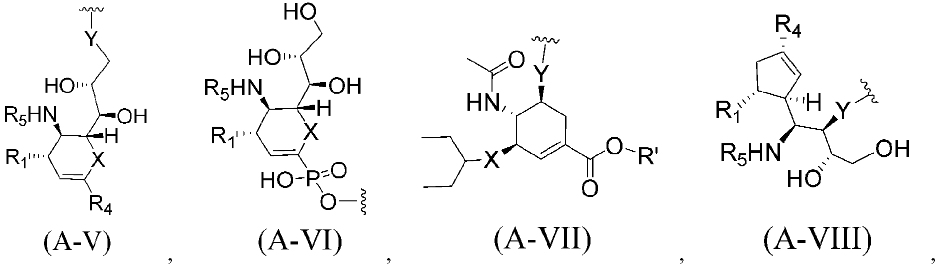 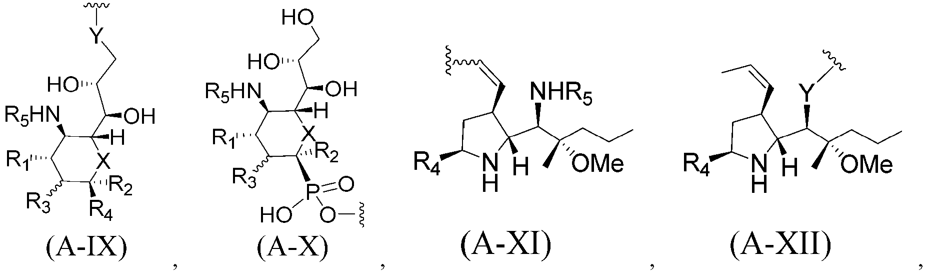 and 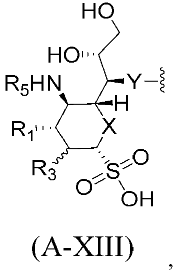
preferably, A₁ and A₂ each independently are selected from the group consisting of (A-I) to Formula (A-IV) and Formula (A-VII), 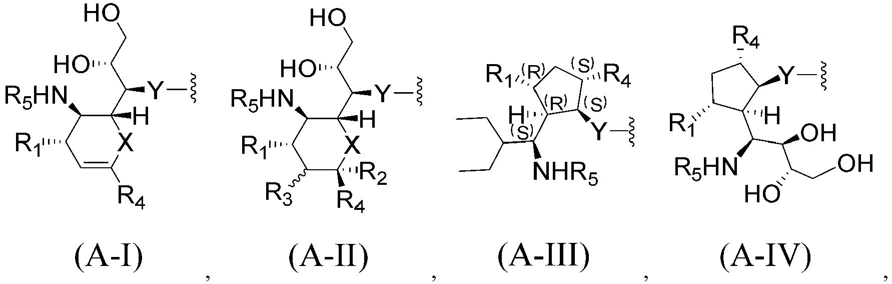 and 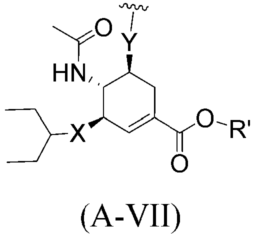
wherein R₁, R₂, R₃, R₄, R₅, R', X, and Y are as defined in claim 21;
preferably, A₁ and A₂ each independently are selected from the group consisting of Formula (A-I) to Formula (A-III) and Formula (A-VII), 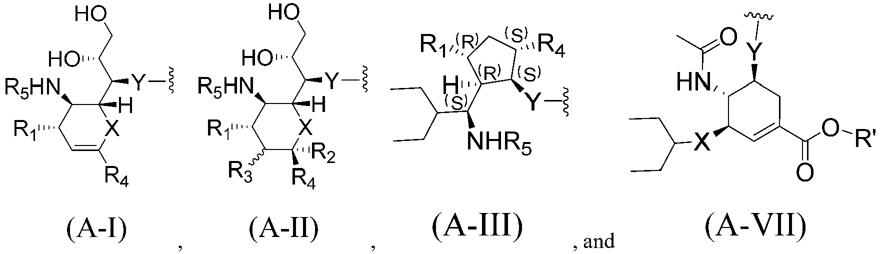
wherein R₁, R₂, R₃, R₄, R₅, R', X, and Y are as above defined;
preferably, A₁ and A₂ each independently are selected from the group consisting of Formula (A-I), Formula (A-III) and Formula (A-VII), 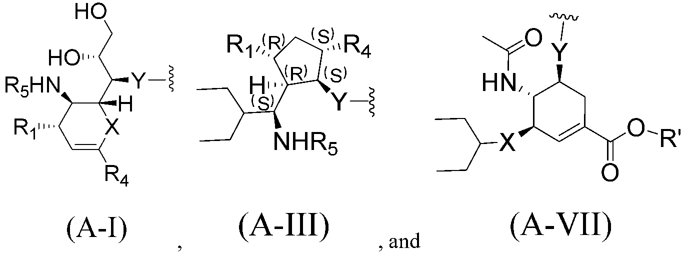
wherein R₁, R₄, R₅, R', X, and Y are as above defined.

28. The compound or a salt, a geometric or optical isomer, a hydrate, a solvate or a polymorph thereof according to claim 26 or 27, wherein
preferably, R₁ is selected from the group consisting of -OH, -NH₂, and -NHC(=NH)NH₂; further preferably, R₁ is -NHC(=NH)NH₂;
preferably, R₄ is selected from the group consisting of -C(=O)OH, -C(=O)OCH₃ and -C(=O)OCF₃; further preferably, R₄ is selected from the group consisting of -C(=O)OH and -C(=O)OCH₃;
preferably, R₅ is selected from the group consisting of -C(=O)CH₃ and -C(=O)CF₃; further preferably, R₅ is -C(=O)CH₃;
preferably, R' is H or C₁-C₄ alkyl; further preferably, R' is H, methyl or ethyl; further preferably, R' is H or ethyl;
preferably, X is -O-;
preferably, Y is selected from the group consisting of -O-, -S-, and -NH-; further preferably, Y is selected from the group consisting of -O- and -NH-.

29. The compound represented or a salt, a geometric or optical isomer, a hydrate, a solvate or a polymorph thereof according to any of claims 26 to 28, wherein the compound has a structure represented by Formula (V-I), 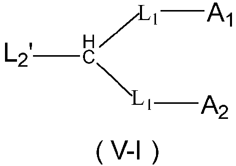
wherein A₁ and A₂ are as defined in any one of claims 26 to 28,
L₁ is selected from the group consisting of: 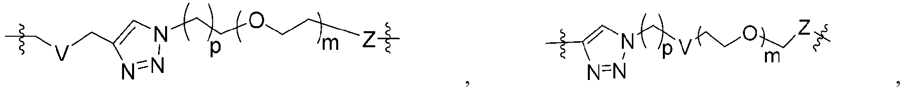 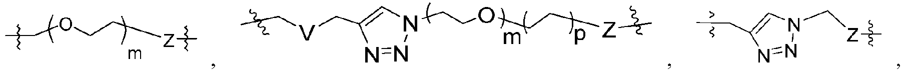 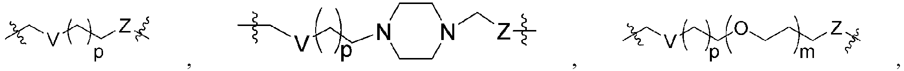 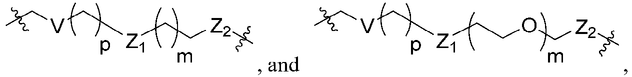
L₂' is selected from the group consisting of: 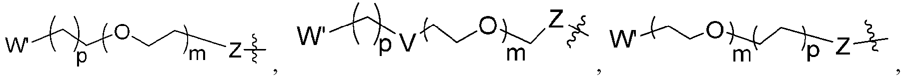 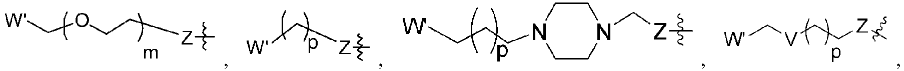 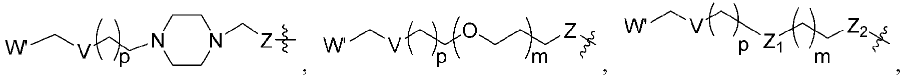 and 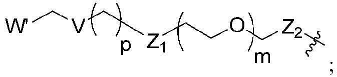
wherein V is -CH₂-, -O- or -S-; Z is -C(=O)-NH-, -CH₂-C(=O)-NH-, -NH-C(=O)-, -CH₂CH₂-C(=O)-, - N(CH₃)-C(=O)-, -N(CH₂CH₃)-C(=O)-, -O-C(=O)-, -C(=O)- or -CH₂-; Z₁ and Z₂ each independently are selected from the group consisting of -C(=O)-NH-, -CH₂-C(=O)-NH-, -NH-C(=O)-, -CH₂CH₂-C(=O)-, -N(CH₃)-C(=O)-, -N(CH₂CH₃)-C(=O)-, -O-C(=O)-, -C(=O)- or -CH₂-; W' is 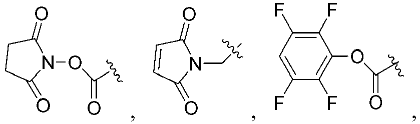 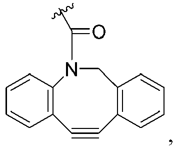 HC≡C-, -C(=O)OH, -NH₂ or N₃-; m and p each independently are an integer between 1 and 20;
preferably, L₁ is selected from the group consisting of: 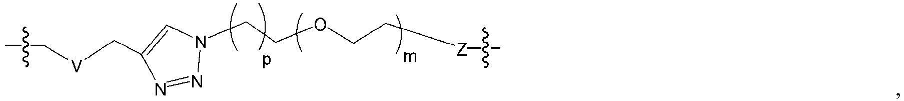 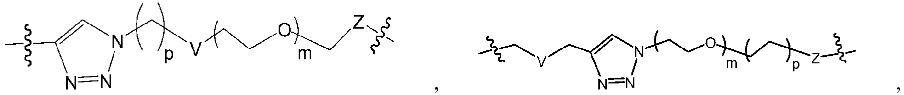 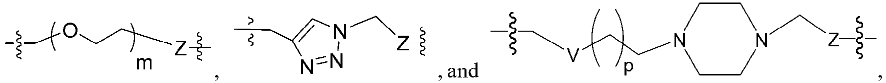 wherein V, p, m, and Z are as above defined;
preferably, L₁ is 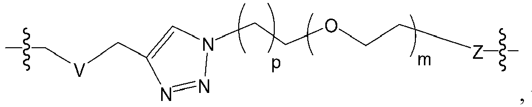 wherein V, p, m, and Z are as above defined;
preferably, L₂' is 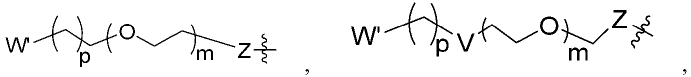 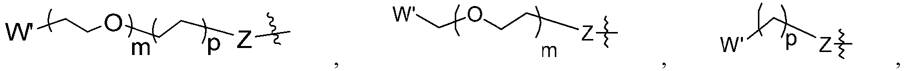 or 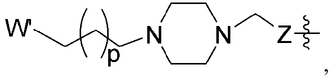 wherein W', V, p, m, and Z are as above defined;
preferably, L₂' is 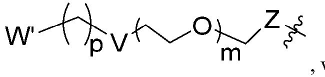 wherein W', V, p, m, and Z are as above defined;
preferably, V is -CH₂- or -O-; further preferably, V is -O-;
preferably, Z is -C(=O)-NH-, -CH₂-C(=O)-NH-, -NH-C(=O)-, -CH₂CH₂-C(=O)-, -N(CH₃)-C(=O)-, - N(CH₂CH₃)-C(=O)- or -C(=O)-; further preferably, Z is -NH-C(=O)- or -C(=O)-;
preferably, W' is HC≡C-, -C(=O)OH, -NH₂ or N₃-; further preferably, W' is HC≡C-;
preferably, p is an integer between 1 and 10; further preferably, p is an integer between 1 and 6 (such as 1, 2, 3, 4, 5, 6);
preferably, m is an integer between 1 and 12; further preferably, m is an integer between 1 and 10 (such as 1, 2, 3, 4, 5, 6, 7, 8, 9).

30. The compound or a salt, a geometric or optical isomer, a hydrate, a solvate or a polymorph thereof according to any of claims 26 to 29, wherein the compound has a structure represented by Formula (V-I-1), Formula (V-I-2), or Formula (V-I-3): 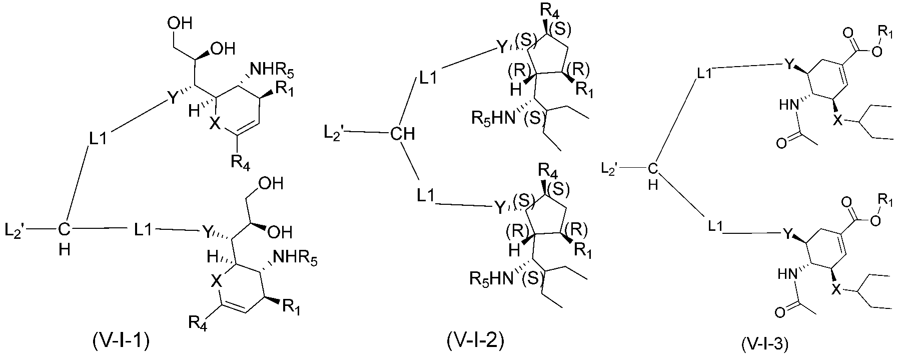 wherein R₁, R₄, R₅, R', X, and Y are as defined in any one of claims 26 to 28, and L₁ and L₂' are as defined in claim 29.

31. The compound or a salt, a geometric or optical isomer, a hydrate, a solvate or a polymorph thereof according to any one of claims 26 to 30, wherein the compound has a structure represented by Formula (V-I-1-1), Formula (V-I-2-1), Formula (V-I-2-2) or Formula (V-I-3-1): 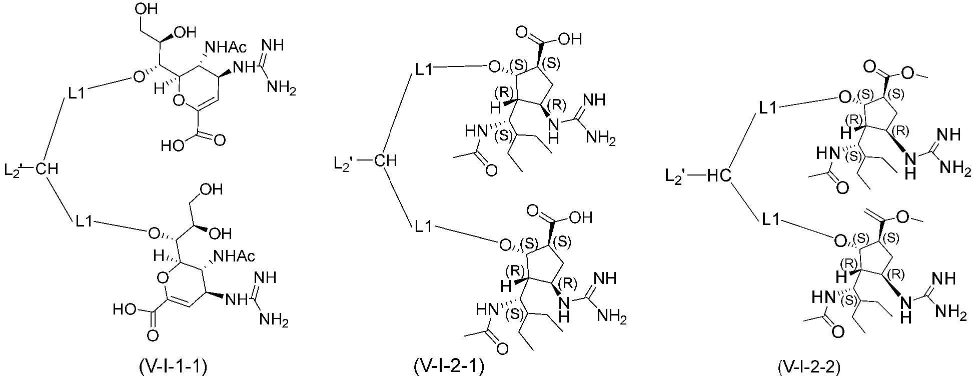 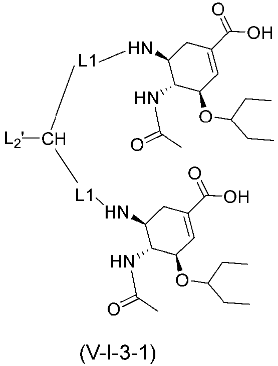 wherein L₁ and L₂' are as defined in claim 29.

32. The compound or a salt, a geometric or optical isomer, a hydrate, a solvate or a polymorph thereof according to any one of claims 26 to 31, wherein the compound is selected from the group consisting of:
| Intermediates | Structure |
|---|---|
| Int-1 | 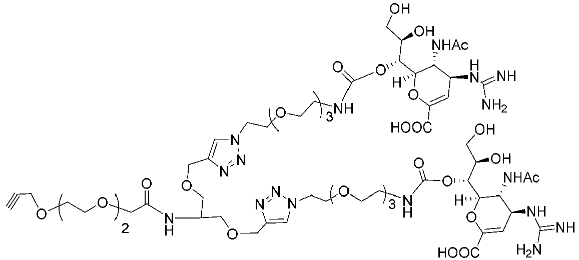 |
| Int-2 | 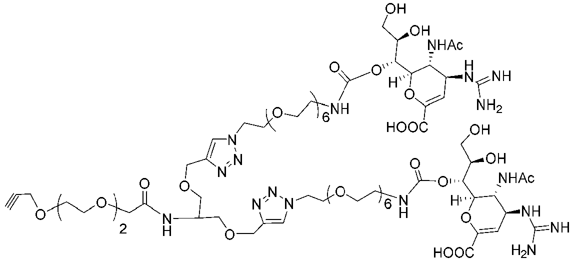 |
| Int-3 | 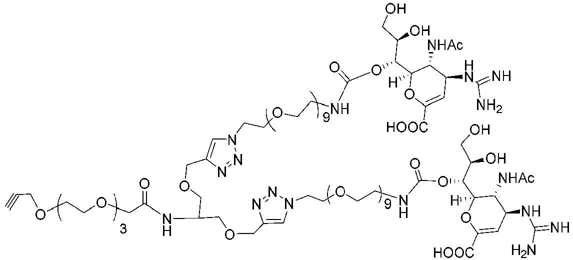 |
| Int-4 | 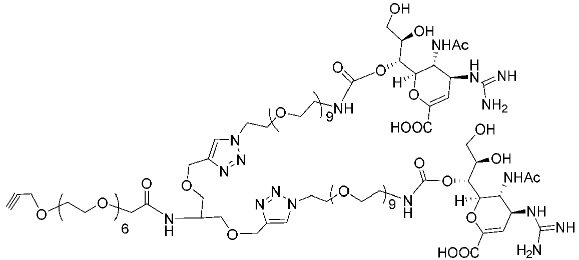 |
| Int-5 | 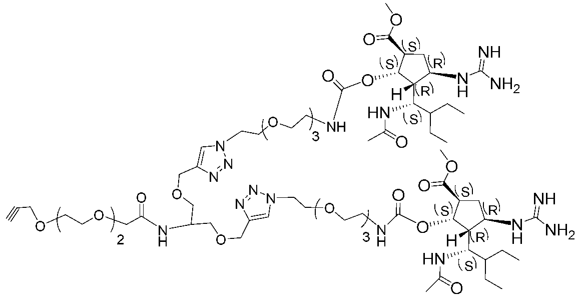 |
| Int-6 | 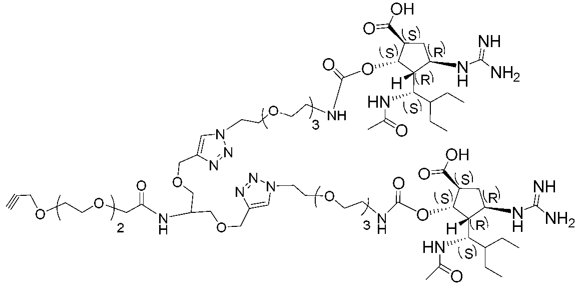 |
| Int-7 | 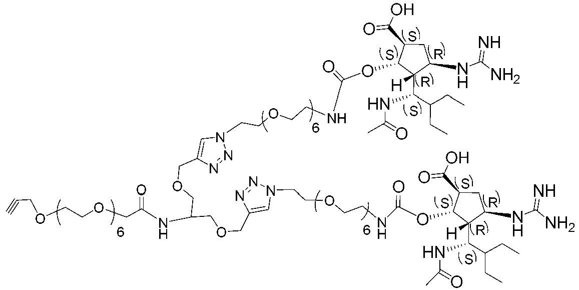 |
| Int-8 | 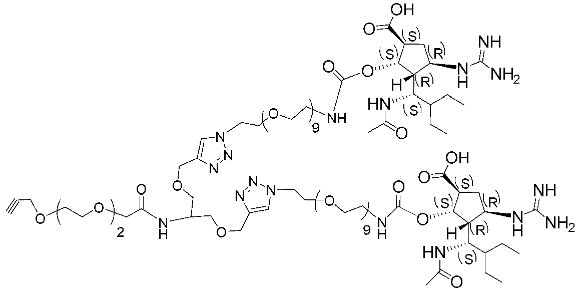 |
| Int-9 | 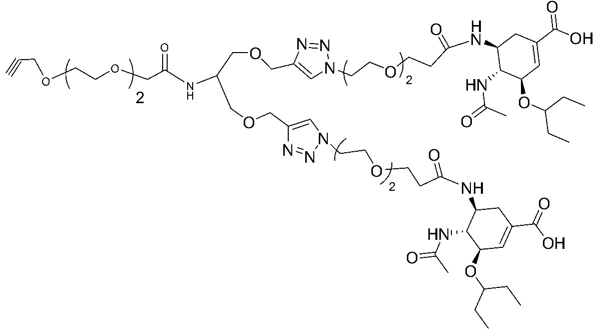 |
| Int-10 | 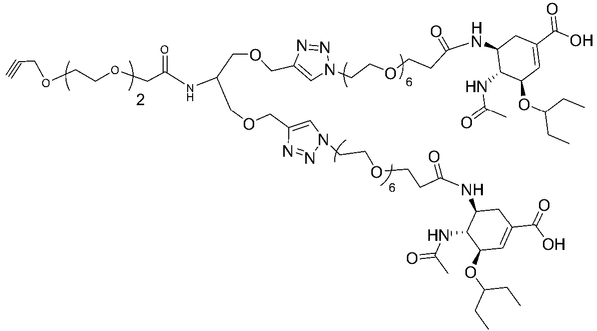 |

33. A pharmaceutical composition, comprising the conjugate or a pharmaceutically acceptable salt, a geometric or optical isomer, a hydrate, a solvate or a polymorph thereof according to any one of claims 1 to 18, or the compound represented or a salt, a geometric or optical isomer, a hydrate, a solvate or a polymorph thereof according to any one of claims 19 to 32, and a pharmaceutically acceptable carrier and/or excipient.

34. Use of the conjugate or a pharmaceutically acceptable salt, a geometric or optical isomer, a hydrate, a solvate or a polymorph thereof according to any one of claims 1 to 18, or the compound represented or a salt, a geometric or optical isomer, a hydrate, a solvate or a polymorph thereof according to any one of claims 19 to 32, or the pharmaceutical composition according to claim 33 in the manufacture of a medicament for treating or preventing a viral infection,
preferably, the viral infection is an infection caused by influenza virus or parainfluenza virus;
preferably, the viral infection is an infection caused by influenza A, B or C virus or parainfluenza virus;
preferably, the viral infection is an infection caused by avian influenza.

35. The use according to claim 34, wherein the subject may be further treated with an antiviral agent, wherein the antiviral agent is selected from the group consisting of oseltamivir, zanamivir, peramivir, lanimivir, amantadine, rimantadine, and baloxavir marboxil.

36. Use of the compound or a salt, a geometric or optical isomer, a hydrate, a solvate or a polymorph thereof according to any one of claims 19 to 32 as an intermediate in the preparation of a conjugate.
